(19) Europäisches Patentamt
European Patent Office
Office européen des brevets

(11) EP 4 589 016 A2

(12) # EUROPEAN PATENT APPLICATION

(43) Date of publication:
**23.07.2025 Bulletin 2025/30**

(21) Application number: **25163214.7**

(22) Date of filing: **13.11.2020**

(51) International Patent Classification (IPC):
***C12Q 1/6837*** *(2018.01)*

(52) Cooperative Patent Classification (CPC):
(C-Sets available)
**C12Q 1/6837** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.11.2019 US 201962935043 P**
**27.11.2019 US 201962941581 P**
**20.05.2020 US 202063027558 P**

(62) Document number(s) of the earlier application(s) in accordance with Art. 76 EPC:
**20833994.5 / 4 058 598**

(71) Applicant: **10x Genomics, Inc.**
**Pleasanton, CA 94588-3260 (US)**

(72) Inventors:
- **STOECKIUS, Marlon**
  **114 33 Stockholm (SE)**
- **GIACOMELLO, Stefania**
  **114 33 Stockholm (SE)**
- **BENT, Zachary**
  **Pleasanton, 94588-3260 (US)**
- **FREY, Meghan L. F.**
  **Pleasanton, 94588-3260 (US)**
- **GAGNON, Alexander**
  **Pleasanton, 94588-3260 (US)**
- **BORGSTROM, Erik Leonard Henrik**
  **114 33 Stockholm (SE)**

(74) Representative: **J A Kemp LLP**
**80 Turnmill Street**
**London EC1M 5QU (GB)**

Remarks:
•The complete document including Reference Table(s) and the Sequence Listing(s) can be downloaded from the EPO website
•This application was filed on 12-03-2025 as a divisional application to the application mentioned under INID code 62.
•Claims filed after the date of filing of the application / after the date of receipt of the divisional application (Rule 68(4) EPC).

(54) **GENERATING CAPTURE PROBES FOR SPATIAL ANALYSIS**

(57) The present disclosure relates to compositions, methods, and kits for generating capture probes on a substrate for identifying the location of analytes in a biological sample. In particular, disclosed is a method of generating a spatial array comprising: (a) providing a substrate comprising a plurality of acceptor oligonucleotides, wherein an acceptor oligonucleotide of the plurality of acceptor oligonucleotides comprises a spatial barcode and a first ligation handle, and wherein the 5' end of the acceptor oligonucleotide is attached to the substrate; (b) providing a plurality of universal splint oligonucleotides, wherein a universal splint oligonucleotide of the plurality of universal splint oligonucleotides comprises a sequence complementary to the first ligation handle and a sequence complementary to a second ligation handle present in a donor oligonucleotide of a plurality of donor oligonucleotides; and (c) ligating the donor oligonucleotide comprising a capture domain to the 3' end of the acceptor oligonucleotide to generate a capture probe, wherein the universal splint oligonucleotide is hybridized to the first ligation handle and the second ligation handle, thereby generating a spatial array.

EP 4 589 016 A2

(52) Cooperative Patent Classification (CPC): (Cont.)

C-Sets
**C12Q 1/6837, C12Q 2521/501, C12Q 2525/131, C12Q 2525/161, C12Q 2525/197, C12Q 2537/143, C12Q 2561/101, C12Q 2563/179, C12Q 2565/514**

## Description

### CROSS REFERENCE TO RELATED APPLICATIONS

This application claims priority to: U.S. Provisional Patent Application No. 62/935,043, filed November 13, 2019, U.S. Provisional Patent Application No. 62/941,581 filed November 27, 2019, and U.S. Provisional Patent Application No. 63/027,558 filed May 20, 2020. The contents of each of these application are incorporated by reference in their entireties.

### BACKGROUND

Cells within a tissue of a subject have differences in cell morphology and/or function due to varied analyte levels (e.g., gene and/or protein expression) within the different cells. The specific position of a cell within a tissue (e.g., the cell's position relative to neighboring cells or the cell's position relative to the tissue microenvironment) can affect, e.g., the cell's morphology, differentiation, fate, viability, proliferation, behavior, and signaling and crosstalk with other cells in the tissue.

Spatial heterogeneity has been previously studied using techniques that only provide data for a small handful of analytes in the context of an intact tissue or a portion of a tissue, or provide a lot of analyte data for single cells, but fail to provide information regarding the position of the single cell in a parent biological sample (e.g., tissue sample).

Arrays that capture spatial information of analytes within a biological sample can be prepared in various ways. Arrays can be designed with pluralities of capture probes. Capture probes can be designed to capture a substantial population of analytes (e.g., mRNA) or to capture specific (e.g., gene specific) analytes. The methods described herein provide for ways of preparing arrays with capture probes for capturing analytes.

### SUMMARY

Arrays that capture spatial information of analytes within a biological sample can be prepared in various ways. Arrays can be designed with pluralities of capture probes. Capture probes can be designed to capture a substantial population of analytes (e.g., mRNA) or to capture specific (e.g., gene specific) analytes. The methods described herein provide for ways of preparing arrays with capture probes for capturing analytes.

Generally, arrays can be manufactured in various ways including by various printing methods as described herein. However, printing methods can be time consuming and resource intense. Arrays including capture probes generally include the same capture domain (e.g., a poly(T) capture domain), however, capture probes including different capture domains allow for multiplexing (e.g., the simultaneous capture of different kinds of analytes or different species of the same type of analyte). Thus, for example, a first capture probe can be capable of capturing of a particular analyte (e.g., mRNA) and a second capture probe can be capable of capturing a particular species of mRNA, other RNA, genomic DNA, cDNA, or an analyte (e.g., protein) indirectly through an analyte capture sequence.

The present disclosure feature methods, compositions, and kits for generating a capture probe by ligating a plurality of donor oligonucleotides to a plurality of acceptor oligonucleotides including a spatial barcode. **In** some embodiments, the ligation reaction is facilitated by a splint oligonucleotide, where the splint oligonucleotide includes a sequence complementary to a portion of the acceptor oligonucleotide (e.g., a first ligation handle) and a portion of the donor oligonucleotide (e.g., a second ligation handle). **In** some embodiments, the donor oligonucleotide includes a capture domain to detect an analyte (e.g., directly or indirectly). **In** some embodiments, a first plurality of donor oligonucleotides including a first capture domain are ligated to acceptor oligonucleotides. **In** some embodiments, a second plurality of donor oligonucleotides including a second domain are ligated to acceptor oligonucleotides. **In** some embodiments, the first capture domain and the second capture domain are different (e.g., capture different analytes). Thus, an array can be generated with two or more species of capture probes (e.g., a first capture probe with a first capture domain and a second capture probe with a second capture domain) that allows for multiplex detection of analytes in a biological sample.

Provided herein are methods of generating a spatial array including (a) providing a substrate including a plurality of acceptor oligonucleotides, where a first acceptor oligonucleotide of the plurality of acceptor oligonucleotides includes a spatial barcode and a first ligation handle, and where the 5' end of the first acceptor oligonucleotide is attached to the substrate; (b) providing a plurality of universal splint oligonucleotides, where a universal splint oligonucleotide of the plurality of universal splint oligonucleotides includes a sequence complementary to the first ligation handle and a sequence complementary to a second ligation handle; (c) ligating a first plurality of donor oligonucleotides, where a first donor oligonucleotide of the first plurality of donor oligonucleotides includes the second ligation handle and a first capture domain, to the 3' end of a first acceptor oligonucleotide to generate a first capture probe, where the universal splint oligonucleotide is hybridized to the first ligation handle and the second ligation handle; and (d) ligating a second plurality of donor oligonucleotides, where a second donor oligonucleotide of the plurality of second donor oligonucleotides includes the second ligation handle and a second capture domain, to the 3' end of a second acceptor oligonucleotide to generate a second capture probe, where the universal splint oligonucleotide is hybridized to the first ligation handle and the second

ligation handle, thereby generating a spatial array.

In some embodiments, the plurality of acceptor oligonucleotides includes a cleavage domain, a unique molecular identifier, a functional domain, or any combination thereof.

In some embodiments, the first capture domain includes a poly(T) sequence.

In some embodiments, the second capture domain includes a gene-specific sequence. In some embodiments, the second capture domain includes a sequence at least 80% identical to SEQ ID NO: 20. In some embodiments, the second capture domain includes a sequence at least 85% identical to SEQ ID NO: 20. In some embodiments, the second capture domain includes a sequence at least 90% identical to SEQ ID NO: 20. In some embodiments, the second capture domain includes a sequence at least 95% identical to SEQ ID NO: 20. In some embodiments, the second capture domain includes SEQ ID NO: 20. In some embodiments, the first capture domain includes a random sequence. In some embodiments, the random sequence is a hexamer.

In some embodiments, the universal splint oligonucleotide includes a sequence at least 85% identical to SEQ ID NO: 13. In some embodiments, the universal splint oligonucleotide includes a sequence at least 90% identical to SEQ ID NO: 13. In some embodiments, the universal splint oligonucleotide includes SEQ ID NO: 13. In some embodiments, the first ligation handle includes a sequence at least 85% complementary to the first 7 nucleotides of SEQ ID NO: 13. In some embodiments, the first ligation handle includes a sequence complementary to the first 7 nucleotides of SEQ ID NO: 13. In some embodiments, the second ligation handle includes a sequence at least 85% complementary to nucleotides 8-14 of SEQ ID NO: 13. In some embodiments, the second ligation handle includes a sequence complementary to nucleotides 8-14 of SEQ ID NO: 13. In some embodiments, the universal splint oligonucleotide includes an inverted base at the 3' end. In some embodiments, the donor oligonucleotide includes one or more phosphorothioate bonds at the 3' end.

In some embodiments, the method includes contacting a biological sample to the spatial array after step (c). In some embodiments, the method includes permeabilizing the biological sample to allow an analyte in the biological sample to interact with the first capture probe or the second capture probe.

In some embodiments, the method includes migrating the analyte in the biological sample to the first capture probe and the second capture probe. In some embodiments, migrating includes passive migration. In some embodiments, migrating includes active migration.

Also provided herein are methods of generating a spatial array including (a) providing a substrate including a plurality of acceptor oligonucleotides, where an acceptor oligonucleotide of the plurality of acceptor oligonucleotides includes a spatial barcode and a first ligation handle, and where the 5' end of the acceptor oligonucleotide is attached to the substrate; (b) providing a plurality of universal splint oligonucleotides, where a universal splint oligonucleotide of the plurality of universal splint oligonucleotides includes a sequence complementary to the first ligation handle and a sequence complementary to a second ligation handle present in a donor oligonucleotide of a plurality of donor oligonucleotides; and (c) ligating the donor oligonucleotide including a capture domain to the 3' end of the acceptor oligonucleotide to generate a capture probe, where the universal splint oligonucleotide is hybridized to the first ligation handle and the second ligation handle, thereby generating a spatial array.

In some embodiments, the acceptor oligonucleotide includes a cleavage domain, a unique molecular identifier, a functional sequence, or any combination thereof.

In some embodiments, the universal splint oligonucleotide includes a sequence at least 85% identical to SEQ ID NO: 13. In some embodiments, the universal splint oligonucleotide includes a sequence at least 90% identical to SEQ ID NO: 13. In some embodiments, the universal splint oligonucleotide includes SEQ ID NO: 13. In some embodiments, the first ligation handle includes a sequence at least 85% complementary to the first 7 nucleotides of SEQ ID NO: 13. In some embodiments, the first ligation handle includes a sequence complementary to the first 7 nucleotides of SEQ ID NO: 13. In some embodiments, the second ligation handle includes a sequence at least 85% complementary to nucleotides 8-14 of SEQ ID NO: 13. In some embodiments, the second ligation handle includes a sequence complementary to nucleotides 8-14 of SEQ ID NO: 13.

In some embodiments, the method includes contacting a biological sample to the spatial array after step (c). In some embodiments, the method includes permeabilizing the biological sample to allow an analyte in the biological sample to interact with the capture probe.

In some embodiments, the method includes migrating the analyte in the biological sample to the capture probe. In some embodiments, migrating includes passive migration or active migration.

Also included herein are kits including (a) an array including a plurality of acceptor oligonucleotides, where an acceptor oligonucleotide of the plurality of acceptor oligonucleotides includes a spatial barcode and a first ligation handle; (b) a plurality of universal splint oligonucleotides; and (c) a ligase.

In some kits, the acceptor oligonucleotide includes SEQ ID NO: 12. In some kits, a universal splint oligonucleotide of the plurality of universal splint oligonucleotides includes a sequence at least 90% identical to SEQ ID NO: 13. In some kits, the splint oligonucleotide includes SEQ ID NO: 13.

In some kits, the ligase is T4 ligase. In some kits, the kit includes a reverse transcriptase. In some kits, the kit includes a DNA polymerase.

In some kits, the kit includes one or more permeabilization reagents. In some kits, the kit includes one or more RNase inhibitors. In some kits, the kit includes instructions for performing any of the methods described herein.

Also provided herein are compositions including (i) an acceptor oligonucleotide including a spatial barcode and a first ligation handle, where the 5' end of the acceptor oligonucleotide is attached to a substrate; (ii) a first donor oligonucleotide including a second ligation handle and a poly(T) capture domain; and (iii) a universal splint oligonucleotide including a sequence complementary to the first ligation handle and a sequence complementary to the second ligation handle, and where the universal splint oligonucleotide is hybridized to the first ligation handle of the acceptor oligonucleotide and the second ligation handle of the donor oligonucleotide.

Also provided herein are compositions including (i) a first acceptor oligonucleotide including a spatial barcode and a first ligation handle, where the 5' end of the acceptor oligonucleotide is attached to a substrate; (ii) a first donor oligonucleotide including a second ligation handle and a poly(T) capture domain; (iii) a second donor oligonucleotide including the second ligation handle and a sequence including SEQ ID NO: 20; (iv) a first universal splint oligonucleotide including a sequence complementary to the first ligation handle and a sequence complementary to the second ligation handle, and where the universal splint oligonucleotide is hybridized to the first ligation handle of the first acceptor oligonucleotide and the second ligation handle of the first donor oligonucleotide; and (v) a second universal splint oligonucleotide including a sequence complementary to the first ligation handle and a sequence complementary to the second ligation handle, and where the universal splint oligonucleotide is hybridized to the first ligation handle of the second acceptor oligonucleotide and the second ligation handle of the second donor oligonucleotide.

Also provided herein are compositions including at least one of a plurality of first acceptor oligonucleotides including a spatial barcode ligated to at least one of a plurality of first donor oligonucleotides including a first capture domain, where the plurality of first acceptor oligonucleotides is attached to a substrate, at least one of the plurality of first acceptor oligonucleotides including a spatial barcode and a first ligation handle, and at least one of a plurality of second donor oligonucleotides including a second ligation handle and a second capture domain, and a universal splint oligonucleotide, where the universal splint oligonucleotide is hybridized to a first ligation handle of at least one of a plurality of first acceptor oligonucleotides and the second ligation handle of at least one of the plurality of second donor oligonucleotides.

All publications, patents, patent applications, and information available on the internet and mentioned in this specification are herein incorporated by reference to the same extent as if each individual publication, patent, patent application, or item of information was specifically and individually indicated to be incorporated by reference. To the extent publications, patents, patent applications, and items of information incorporated by reference contradict the disclosure contained in the specification, the specification is intended to supersede and/or take precedence over any such contradictory material.

Where values are described in terms of ranges, it should be understood that the description includes the disclosure of all possible sub-ranges within such ranges, as well as specific numerical values that fall within such ranges irrespective of whether a specific numerical value or specific sub-range is expressly stated.

The term "each," when used in reference to a collection of items, is intended to identify an individual item in the collection but does not necessarily refer to every item in the collection, unless expressly stated otherwise, or unless the context of the usage clearly indicates otherwise.

Various embodiments of the features of this disclosure are described herein. However, it should be understood that such embodiments are provided merely by way of example, and numerous variations, changes, and substitutions can occur to those skilled in the art without departing from the scope of this disclosure. It should also be understood that various alternatives to the specific embodiments described herein are also within the scope of this disclosure.

## DESCRIPTION OF DRAWINGS

The following drawings illustrate certain embodiments of the features and advantages of this disclosure. These embodiments are not intended to limit the scope of the appended claims in any manner. Like reference symbols in the drawings indicate like elements.

**FIG. 1** is a schematic diagram showing an example of a barcoded capture probe, as described herein.

**FIG. 2** shows an exemplary method for generating a capture probe.

**FIG. 3** is an exemplary graph showing detection of cDNA with ligated and unligated polyT and Penk gene specific capture domains.

**FIGs. 4A-B** is a fluorescent image showing detection of Penk mRNA with a Penk specific capture probe generated by the method shown in **FIG. 2** (**FIG. 4A**) compared to a control tissue optimization (TO) slide (**FIG. 4B**).

**FIG. 5** shows an exemplary ligation-based method for generating a capture probe using two ligation handles and a universal splint oligonucleotide.

**FIGs. 6A-B** show H&E staining (**FIG. 6A**) of a biological sample and analyte capture with ligated poly(T) capture

domains (**FIG. 6B**).

**FIGs. 7A-B** are exemplary graphs showing detection of cDNA with ligated poly(T) capture domains (**FIG. 7A**) and controls (**FIG. 7B**).

**FIG. 8** shows exemplary Spearman and Pearson correlations between the ligated poly(T) subarray 1 raw counts (Y-axis) and the Printed poly(T) subarray raw counts (X-axis).

**FIGs. 9A-B** shows spatial gene expression for proenkephalin (Penk) (**FIG. 9A**) and Penk capture on a tissue optimization slide using a ligated Penk specific capture domain (**FIG. 9B**).

**FIG. 10** is an exemplary graph showing analyte capture for Penk target analytes with ligated poly(T) capture domains.

**FIGs. 11A-B** shows exemplary Spearman and Pearson correlation results from two separate subarrays (**FIGs. 11A-B**) between a printed poly(T) subarray raw counts and ligated Penk probe subarray raw counts.

**FIG. 12** shows the sequencing read distribution for an experiment using ligated poly(T) probes, printed poly(T) probes (positive control), ligated Penk 3' probe, ligated Penk middle 3' probe, and a plant ligated negative control probe.

**FIG. 13** shows capture of double C2-like domain containing protein gamma (Doc2g) gene using a ligated Doc2g specific capture domain (Doc2g-1 and Doc2g-2).

**FIG. 14** shows capture of potassium channel tetramerization domain containing 12 (Kctd12) gene using a ligated Kctd12 capture domain (Kctd12-1 and Kctd12-2).

**FIG. 15** shows Pearson correlation results between capture area 1 raw counts (y axis) and capture area 2 raw counts (x axis) for ligated capture domains in mouse brain tissue.

**FIG. 16** shows Pearson correlation results between capture area 1 raw counts (y axis) and capture area 2 raw counts (x axis) for ligated capture domains in human heart tissue.

**FIG. 17** shows spatial clustering results using ligated capture probes (top) and printed capture probes (bottom) in mouse brain tissue.

**FIGs. 18A-B** show inverted nucleotide base conformations including a 3' to 3' reversed linkage (**FIG. 18A**) and a 5' to 5' reversed linkage (**FIG. 18B**).

**FIGs. 19A-B** show successful ligation of a Cy5 labeled donor oligonucleotide to an acceptor oligonucleotide on a substrate via an image (**FIG. 19A**) and a graph (**FIG. 19B**).

**FIGs. 20A-B** show successful ligation of a Cy3 labeled donor oligonucleotide to an acceptor oligonucleotide on a substrate via an image (**FIG. 20A**) and a graph (**FIG. 20B**).

**FIGs. 21A-D** show an exemplary ligation scheme with a first donor oligonucleotide (**FIG. 21A**) ligated to an acceptor oligonucleotide (**FIG. 21B**), a second donor oligonucleotide ligated to another acceptor oligonucleotide (**FIG. 21C**), and followed by an exemplary qPCR scheme to detect successful ligation (**FIG. 21D**).

**FIG. 22** shows an exemplary ligation efficiency testing scheme.

**FIGs. 23A-B** show the DNA products detected after ligation (**FIG. 23A**) and a graph showing ligation efficiency at 22°C and 37°C (**FIG. 23B**).

**FIG. 24** is a graph showing the median UMIs at 50k raw reads/spot with arrays generated by ligation reactions at either 22°C, 30°C, or 37°C. A printed array serves as the control.

## DETAILED DESCRIPTION

### I. Introduction

This disclosure describes apparatus, systems, methods, and compositions for spatial analysis of biological samples. This section describes certain general terminology, analytes, sample types, and preparative steps that are referred to in later sections of the disclosure.

#### (a) Spatial Analysis

Tissues and cells can be obtained from any source. For example, tissues and cells can be obtained from single-cell or multicellular organisms (e.g., a mammal). Tissues and cells obtained from a mammal, e.g., a human, often have varied analyte levels (e.g., gene and/or protein expression) which can result in differences in cell morphology and/or function. The position of a cell or a subset of cells (e.g., neighboring cells and/or non-neighboring cells) within a tissue can affect, e.g., the cell's fate, behavior, morphology, and signaling and crosstalk with other cells in the tissue. Information regarding the differences in analyte levels (gene and/or protein expression) within different cells in a tissue of a mammal can also help physicians select or administer a treatment that will be effective and can allow researchers to identify and elucidate differences in cell morphology and/or cell function in the single-cell or multicellular organisms (e.g., a mammal) based on the detected differences in analyte levels within different cells in the tissue. Differences in analyte levels within different cells in a tissue of a mammal can also provide information on how tissues (e.g., healthy and diseased tissues) function and/or develop. Differences in analyte levels within different cells in a tissue of a mammal can also provide

information of different mechanisms of disease pathogenesis in a tissue and mechanism of action of a therapeutic treatment within a tissue. Differences in analyte levels within different cells in a tissue of a mammal can also provide information on drug resistance mechanisms and the development of the same in a tissue of a mammal. Differences in the presence or absence of analytes within different cells in a tissue of a multicellular organism (e.g., a mammal) can provide information on drug resistance mechanisms and the development of the same in a tissue of a multicellular organism.

The spatial analysis methodologies herein provide for the detection of differences in an analyte level (e.g., gene and/or protein expression) within different cells in a tissue of a mammal or within a single cell from a mammal. For example, spatial analysis methodologies can be used to detect the differences in analyte levels (e.g., gene and/or protein expression) within different cells in histological samples, the data from which can be reassembled to generate a three-dimensional map of analyte levels (e.g., gene and/or protein expression) of a tissue sample obtained from a mammal, e.g., with a degree of spatial resolution (e.g., single-cell resolution).

Spatial heterogeneity in developing systems has typically been studied via RNA hybridization, immunohistochemistry, fluorescent reporters, or purification or induction of pre-defined subpopulations and subsequent genomic profiling (e.g., RNA-seq). Such approaches, however, rely on a relatively small set of pre-defined markers, therefore introducing selection bias that limits discovery. These prior approaches also rely on *a priori* knowledge. RNA assays traditionally relied on staining for a limited number of RNA species. In contrast, single-cell RNA-sequencing allows for deep profiling of cellular gene expression (including non-coding RNA), but the established methods separate cells from their native spatial context.

Spatial analysis methodologies described herein provide a vast amount of analyte level and/or expression data for a variety of multiple analytes within a sample at high spatial resolution, e.g., while retaining the native spatial context. Spatial analysis methods include, e.g., the use of a capture probe including a spatial barcode (e.g., a nucleic acid sequence that provides information as to the position of the capture probe within a cell or a tissue sample (e.g., mammalian cell or a mammalian tissue sample) and a capture domain that is capable of binding to an analyte (e.g., a protein and/or nucleic acid) produced by and/or present in a cell. As described herein, the spatial barcode can be a nucleic acid that has a unique sequence, a unique fluorophore or a unique combination of fluorophores, a unique amino acid sequence, a unique heavy metal or a unique combination of heavy metals, or any other unique detectable agent. The capture domain can be any agent that is capable of binding to an analyte produced by and/or present in a cell (e.g., a nucleic acid that is capable of hybridizing to a nucleic acid from a cell (e.g., an mRNA, genomic DNA, mitochondrial DNA, or miRNA), a substrate including an analyte, a binding partner of an analyte, or an antibody that binds specifically to an analyte). A capture probe can also include a nucleic acid sequence that is complementary to a sequence of a universal forward and/or universal reverse primer. A capture probe can also include a cleavage site (e.g., a cleavage recognition site of a restriction endonuclease), a photolabile bond, a thermosensitive bond, or a chemical-sensitive bond.

The binding of an analyte to a capture probe can be detected using a number of different methods, e.g., nucleic acid sequencing, fluorophore detection, nucleic acid amplification, detection of nucleic acid ligation, and/or detection of nucleic acid cleavage products. In some examples, the detection is used to associate a specific spatial barcode with a specific analyte produced by and/or present in a cell (e.g., a mammalian cell).

Capture probes can be, e.g., attached to a surface, e.g., a solid array, a bead, or a coverslip. In some examples, capture probes are not attached to a surface. In some examples, capture probes can be encapsulated within, embedded within, or layered on a surface of a permeable composition (e.g., any of the substrates described herein). For example, capture probes can be encapsulated or disposed within a permeable bead (e.g., a gel bead). In some examples, capture probes can be encapsulated within, embedded within, or layered on a surface of a substrate (e.g., any of the exemplary substrates described herein, such as a hydrogel or a porous membrane).

In some examples, a cell or a tissue sample including a cell are contacted with capture probes attached to a substrate (e.g., a surface of a substrate), and the cell or tissue sample is permeabilized to allow analytes to be released from the cell and bind to the capture probes attached to the substrate. In some examples, analytes released from a cell can be actively directed to the capture probes attached to a substrate using a variety of methods, e.g., electrophoresis, chemical gradient, pressure gradient, fluid flow, or magnetic field.

In other examples, a capture probe can be directed to interact with a cell or a tissue sample using a variety of methods, e.g., inclusion of a lipid anchoring agent in the capture probe, inclusion of an agent that binds specifically to, or forms a covalent bond with a membrane protein in the capture probe, fluid flow, pressure gradient, chemical gradient, or magnetic field.

Non-limiting aspects of spatial analysis methodologies are described in WO 2011/127099, WO 2014/210233, WO 2014/210225, WO 2016/162309, WO 2018/091676, WO 2012/140224, WO 2014/060483, U.S. Patent No. 10,002,316, U.S. Patent No. 9,727,810, U.S. Patent Application Publication No. 2017/0016053, Rodriques et al., Science 363(6434):1463-1467, 2019; WO 2018/045186, Lee et al., Nat. Protoc. 10(3):442-458, 2015; WO 2016/007839, WO 2018/045181, WO 2014/163886, Trejo et al., PLoS ONE 14(2):e0212031, 2019, U.S. Patent Application Publication No. 2018/0245142, Chen et al., Science 348(6233):aaa6090, 2015, Gao et al., BMC Biol. 15:50, 2017, WO 2017/144338, WO 2018/107054, WO 2017/222453, WO 2019/068880, WO 2011/094669, U.S. Patent No. 7,709,198, U.S. Patent No.

8,604,182, U.S. Patent No. 8,951,726, U.S. Patent No. 9,783,841, U.S. Patent No. 10,041,949, WO 2016/057552, WO 2017/147483, WO 2018/022809, WO 2016/166128, WO 2017/027367, WO 2017/027368, WO 2018/136856, WO 2019/075091, U.S. Patent No. 10,059,990, WO 2018/057999, WO 2015/161173, and Gupta et al., Nature Biotechnol. 36:1197-1202, 2018, and can be used herein in any combination. Further non-limiting aspects of spatial analysis methodologies are described herein.

(b) General Terminology

Specific terminology is used throughout this disclosure to explain various aspects of the apparatus, systems, methods, and compositions that are described. This sub-section includes explanations of certain terms that appear in later sections of the disclosure. To the extent that the descriptions in this section are in apparent conflict with usage in other sections of this disclosure, the definitions in this section will control.

(i) Barcode

A "barcode" is a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes.

Barcodes can have a variety of different formats. For example, barcodes can include non-random, semi-random, and/or random nucleic acid and/or amino acid sequences, and synthetic nucleic acid and/or amino acid sequences. A barcode can be attached to an analyte or to another moiety or structure in a reversible or irreversible manner. A barcode can be added to, for example, a fragment of a deoxyribonucleic acid (DNA) or ribonucleic acid (RNA) sample before or during sequencing of the sample. Barcodes can allow for identification and/or quantification of individual sequencing-reads (e.g., a barcode can be or can include a unique molecular identifier or "UMI").

Barcodes can spatially-resolve molecular components found in biological samples, for example, at single-cell resolution (e.g., a barcode can be or can include a "spatial barcode"). In some embodiments, a barcode includes both a UMI and a spatial barcode. In some embodiments, a barcode includes two or more sub-barcodes that together function as a single barcode (e.g., a polynucleotide barcode). For example, a polynucleotide barcode can include two or more polynucleotide sequences (e.g., sub-barcodes) that may be separated by one or more non-barcode sequences.

(ii) Probe and Target

A "probe" or a "target," when used in reference to a nucleic acid or sequence of a nucleic acids, is intended as a semantic identifier for the nucleic acid or sequence in the context of a method or composition, and does not limit the structure or function of the nucleic acid or sequence beyond what is expressly indicated.

(iii) Adaptor, Adapter, and Tag

An "adaptor," an "adapter," and a "tag" are terms that are used interchangeably in this disclosure, and refer to species that can be coupled to a polynucleotide sequence (in a process referred to as "tagging") using any one of many different techniques including (but not limited to) ligation, hybridization, and tagmentation. Adaptors can also be nucleic acid sequences that add a function, e.g., spacer sequences, primer sequences/sites, barcode sequences, unique molecular identifier sequences.

(iv) Hybridizing, Hybridize, Annealing, and Anneal

The terms "hybridizing," "hybridize," "annealing," and "anneal" are used interchangeably in this disclosure, and refer to the pairing of substantially complementary or complementary nucleic acid sequences within two different molecules. Pairing can be achieved by any process in which a nucleic acid sequence joins with a substantially or fully complementary sequence through base pairing to form a hybridization complex. For purposes of hybridization, two nucleic acid sequences are "substantially complementary" if at least 60% (e.g., at least 70%, at least 80%, or at least 90%) of their individual bases are complementary to one another.

(v) Primer

A "primer" is a single-stranded nucleic acid sequence having a 3' end that can be used as a chemical substrate for a nucleic acid polymerase in a nucleic acid extension reaction. RNA primers are formed of RNA nucleotides, and are used in RNA synthesis, while DNA primers are formed of DNA nucleotides and used in DNA synthesis. Primers can also include

both RNA nucleotides and DNA nucleotides (e.g., in a random or designed pattern). Primers can also include other natural or synthetic nucleotides described herein that can have additional functionality. In some examples, DNA primers can be used to prime RNA synthesis and vice versa (e.g., RNA primers can be used to prime DNA synthesis). Primers can vary in length. For example, primers can be about 6 bases to about 120 bases. For example, primers can include up to about 25 bases.

(vi) Template Switching Oligonucleotide

A "template switching oligonucleotide" is an oligonucleotide that hybridizes to untemplated nucleotides added by a reverse transcriptase (e.g., enzyme with terminal transferase activity) during reverse transcription. In some embodiments, a template switching oligonucleotide hybridizes to untemplated poly(C) nucleotides added by a reverse transcriptase. In some embodiments, the template switching oligonucleotide adds a common 5' sequence to full-length cDNA that is used for cDNA amplification.

In some embodiments, the template switching oligonucleotide adds a common sequence onto the 5' end of the RNA being reverse transcribed. For example, a template switching oligonucleotide can hybridize to untemplated poly(C) nucleotides added onto the end of a cDNA molecule and provide a template for the reverse transcriptase to continue replication to the 5' end of the template switching oligonucleotide, thereby generating full-length cDNA ready for further amplification. In some embodiments, once a full-length cDNA molecule is generated, the template switching oligonucleotide can serve as a primer in a cDNA amplification reaction.

In some embodiments, a template switching oligonucleotide is added before, contemporaneously with, or after a reverse transcription, or other terminal transferase-based reaction. In some embodiments, a template switching oligonucleotide is included in the capture probe. In certain embodiments, methods of sample analysis using template switching oligonucleotides can involve the generation of nucleic acid products from analytes of the tissue sample, followed by further processing of the nucleic acid products with the template switching oligonucleotide.

Template switching oligonucleotides can include a hybridization region and a template region. The hybridization region can include any sequence capable of hybridizing to the target. In some embodiments, the hybridization region can, e.g., include a series of G bases to complement the overhanging C bases at the 3' end of a cDNA molecule. The series of G bases can include 1 G base, 2 G bases, 3 G bases, 4 G bases, 5 G bases, or more than 5 G bases. The template sequence can include any sequence to be incorporated into the cDNA. In other embodiments, the hybridization region can include at least one base in addition to at least one G base. In other embodiments, the hybridization can include bases that are not a G base. In some embodiments, the template region includes at least 1 (e.g., at least 2, 3, 4, 5 or more) tag sequences and/or functional sequences. In some embodiments, the template region and hybridization region are separated by a spacer.

In some embodiments, the template regions include a barcode sequence. The barcode sequence can act as a spatial barcode and/or as a unique molecular identifier (UMI). Template switching oligonucleotides can include deoxyribonucleic acids; ribonucleic acids; modified nucleic acids including 2-aminopurine, 2,6-diaminopurine (2-amino-dA), inverted dT, 5-methyl dC, 2'-deoxyInosine, Super T (5-hydroxybutynl-2'-deoxyuridine), Super G (8-aza-7-deazaguanosine), locked nucleic acids (LNAs), unlocked nucleic acids (UNAs, e.g., UNA-A, UNA-U, UNA-C, UNA-G), Iso-dG, Iso-dC, 2' fluoro bases (e.g., Fluoro C, Fluoro U, Fluoro A, and Fluoro G), or any combination of the foregoing.

In some embodiments, the length of a template switching oligonucleotide can be at least about 1, 2, 10, 20, 50, 75, 100, 150, 200, or 250 nucleotides or longer. In some embodiments, the length of a template switching oligonucleotide can be at most about 2, 10, 20, 50, 100, 150, 200, or 250 nucleotides or longer.

(vii) Splint Oligonucleotide

A "splint oligonucleotide" is an oligonucleotide that, when hybridized, acts as a "splint" to position two polynucleotides next to one another so that they can be ligated together. In some embodiments, the splint oligonucleotide is DNA or RNA. The splint oligonucleotide can include a nucleotide sequence that is partially complimentary to nucleotide sequences from two or more different oligonucleotides. In some embodiments, the splint oligonucleotide assists in ligating a "donor" oligonucleotide and an "acceptor" oligonucleotide. In general, an RNA ligase, a DNA ligase, or other variety or combination of ligases is used to ligate two splinted nucleotide sequences together.

In some embodiments, the splint oligonucleotide is between 10 and 50 oligonucleotides in length, e.g., between 10 and 45, 10 and 40, 10 and 35, 10 and 30, 10 and 25, or 10 and 20 oligonucleotides in length. In some embodiments, the splint oligonucleotide is between 15 and 50, 15 and 45, 15 and 40, 15 and 35, 15 and 30, 15 and 30, or 15 and 25 nucleotides in length.

(c) Analytes

The apparatus, systems, methods, and compositions described in this disclosure can be used to detect and

analyze a wide variety of different analytes. For the purpose of this disclosure, an "analyte" can include any biological substance, structure, moiety, or component to be analyzed. The term "target" can similarly refer to an analyte of interest.

Analytes can be broadly classified into one of two groups: nucleic acid analytes, and non-nucleic acid analytes. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral coat proteins, extracellular and intracellular proteins, antibodies, and antigen binding fragments. In some embodiments, the analyte can be an organelle (e.g., nuclei or mitochondria).

Cell surface features corresponding to analytes can include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, an extracellular matrix protein, a posttranslational modification (e.g., phosphorylation, glycosylation, ubiquitination, nitrosylation, methylation, acetylation or lipidation) state of a cell surface protein, a gap junction, and an adherens junction.

Analytes can be derived from a specific type of cell and/or a specific sub-cellular region. For example, analytes can be derived from cytosol, from cell nuclei, from mitochondria, from microsomes, and more generally, from any other compartment, organelle, or portion of a cell. Permeabilizing agents that specifically target certain cell compartments and organelles can be used to selectively release analytes from cells for analysis.

Examples of nucleic acid analytes include DNA analytes such as genomic DNA, methylated DNA, specific methylated DNA sequences, fragmented DNA, mitochondrial DNA, in situ synthesized PCR products, and RNA/DNA hybrids.

Examples of nucleic acid analytes also include RNA analytes such as various types of coding and non-coding RNA. Examples of the different types of RNA analytes include messenger RNA (mRNA), ribosomal RNA (rRNA), transfer RNA (tRNA), microRNA (miRNA), and viral RNA. The RNA can be a transcript (e.g., present in a tissue section). The RNA can be small (e.g., less than 200 nucleic acid bases in length) or large (e.g., RNA greater than 200 nucleic acid bases in length). Small RNAs mainly include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA), and small rDNA-derived RNA (srRNA). The RNA can be double-stranded RNA or single-stranded RNA. The RNA can be circular RNA. The RNA can be a bacterial rRNA (e.g., 16s rRNA or 23s rRNA).

Additional examples of analytes include mRNA and cell surface features (e.g., using the labelling agents described herein), mRNA and intracellular proteins (e.g., transcription factors), mRNA and cell methylation status, mRNA and accessible chromatin (e.g., ATAC-seq, DNase-seq, and/or MNase-seq), mRNA and metabolites (e.g., using the labelling agents described herein), a barcoded labelling agent (e.g., the oligonucleotide tagged antibodies described herein) and a V(D)J sequence of an immune cell receptor (e.g., T-cell receptor), mRNA and a perturbation agent (e.g., a CRISPR crRNA/sgRNA, TALEN, zinc finger nuclease, and/or antisense oligonucleotide as described herein). In some embodiments, a perturbation agent can be a small molecule, an antibody, a drug, an aptamer, a miRNA, a physical environmental (e.g., temperature change), or any other known perturbation agents.

Analytes can include a nucleic acid molecule with a nucleic acid sequence encoding at least a portion of a V(D)J sequence of an immune cell receptor (e.g., a TCR or BCR). In some embodiments, the nucleic acid molecule is cDNA first generated from reverse transcription of the corresponding mRNA, using a poly(T) containing primer. The generated cDNA can then be barcoded using a capture probe, featuring a barcode sequence (and optionally, a UMI sequence) that hybridizes with at least a portion of the generated cDNA. In some embodiments, a template switching oligonucleotide hybridizes to a poly(C) tail added to a 3'end of the cDNA by a reverse transcriptase enzyme. The original mRNA template and template switching oligonucleotide can then be denatured from the cDNA and the barcoded capture probe can then hybridize with the cDNA and a complement of the cDNA generated. Additional methods and compositions suitable for barcoding cDNA generated from mRNA transcripts including those encoding V(D)J regions of an immune cell receptor and/or barcoding methods and composition including a template switch oligonucleotide are described in PCT Patent Application PCT/US2017/057269, filed October 18, 2017, and U.S. Patent Application Serial No. 15/825,740, filed November 29, 2017, both of which are incorporated herein by reference in their entireties. V(D)J analysis can also be completed with the use of one or more labelling agents that bind to particular surface features of immune cells and associated with barcode sequences. The one or more labelling agents can include an MHC or MHC multimer.

As described above, the analyte can include a nucleic acid capable of functioning as a component of a gene editing reaction, such as, for example, clustered regularly interspaced short palindromic repeats (CRISPR)-based gene editing. Accordingly, the capture probe can include a nucleic acid sequence that is complementary to the analyte (e.g., a sequence that can hybridize to the CRISPR RNA (crRNA), single guide RNA (sgRNA), or an adapter sequence engineered into a crRNA or sgRNA).

In certain embodiments, an analyte can be extracted from a live cell. Processing conditions can be adjusted to

ensure that a biological sample remains live during analysis, and analytes are extracted from (or released from) live cells of the sample. Live cell-derived analytes can be obtained only once from the sample, or can be obtained at intervals from a sample that continues to remain in viable condition.

In general, the systems, apparatus, methods, and compositions can be used to analyze any number of analytes. For example, the number of analytes that are analyzed can be at least about 2, at least about 3, at least about 4, at least about 5, at least about 6, at least about 7, at least about 8, at least about 9, at least about 10, at least about 11, at least about 12, at least about 13, at least about 14, at least about 15, at least about 20, at least about 25, at least about 30, at least about 40, at least about 50, at least about 100, at least about 1,000, at least about 10,000, at least about 100,000 or more different analytes present in a region of the sample or within an individual feature of the substrate. Methods for performing multiplexed assays to analyze two or more different analytes will be discussed in a subsequent section of this disclosure.

(d) Biological Samples

(i) Types of Biological Samples

A "biological sample" is obtained from the subject for analysis using any of a variety of techniques including, but not limited to, biopsy, surgery, and laser capture microscopy (LCM), and generally includes cells and/or other biological material from the subject. In addition to the subjects described above, a biological sample can be obtained from non-mammalian organisms (e.g., a plants, an insect, an arachnid, a nematode (e.g., *Caenorhabditis elegans*), a fungi, an amphibian, or a fish (e.g., zebrafish)). A biological sample can be obtained from a prokaryote such as a bacterium, e.g., *Escherichia coli, Staphylococci* or *Mycoplasma pneumoniae;* an archaea; a virus such as Hepatitis C virus or human immunodeficiency virus; or a viroid. A biological sample can be obtained from a eukaryote, such as a patient derived organoid (PDO) or patient derived xenograft (PDX). The biological sample can include organoids, a miniaturized and simplified version of an organ produced in vitro in three dimensions that shows realistic micro-anatomy. Organoids can be generated from one or more cells from a tissue, embryonic stem cells, and/or induced pluripotent stem cells, which can self-organize in three-dimensional culture owing to their self-renewal and differentiation capacities. In some embodiments, an organoid is a cerebral organoid, an intestinal organoid, a stomach organoid, a lingual organoid, a thyroid organoid, a thymic organoid, a testicular organoid, a hepatic organoid, a pancreatic organoid, an epithelial organoid, a lung organoid, a kidney organoid, a gastruloid, a cardiac organoid, or a retinal organoid. Subjects from which biological samples can be obtained can be healthy or asymptomatic individuals, individuals that have or are suspected of having a disease (e.g., cancer) or a pre-disposition to a disease, and/or individuals that are in need of therapy or suspected of needing therapy.

Biological samples can be derived from a homogeneous culture or population of the subjects or organisms mentioned herein or alternatively from a collection of several different organisms, for example, in a community or ecosystem.

Biological samples can include one or more diseased cells. A diseased cell can have altered metabolic properties, gene expression, protein expression, and/or morphologic features. Examples of diseases include inflammatory disorders, metabolic disorders, nervous system disorders, and cancer. Cancer cells can be derived from solid tumors, hematological malignancies, cell lines, or obtained as circulating tumor cells.

Biological samples can also include fetal cells. For example, a procedure such as amniocentesis can be performed to obtain a fetal cell sample from maternal circulation. Sequencing of fetal cells can be used to identify any of a number of genetic disorders, including, e.g., aneuploidy such as Down's syndrome, Edwards syndrome, and Patau syndrome. Further, cell surface features of fetal cells can be used to identify any of a number of disorders or diseases.

Biological samples can also include immune cells. Sequence analysis of the immune repertoire of such cells, including genomic, proteomic, and cell surface features, can provide a wealth of information to facilitate an understanding the status and function of the immune system. By way of example, determining the status (e.g., negative or positive) of minimal residue disease (MRD) in a multiple myeloma (MM) patient following autologous stem cell transplantation is considered a predictor of MRD in the MM patient (see, e.g., U.S. Patent Application Publication No. 2018/0156784, the entire contents of which are incorporated herein by reference).

Examples of immune cells in a biological sample include, but are not limited to, B cells, T cells (e.g., cytotoxic T cells, natural killer T cells, regulatory T cells, and T helper cells), natural killer cells, cytokine induced killer (CIK) cells, myeloid cells, such as granulocytes (basophil granulocytes, eosinophil granulocytes, neutrophil granulocytes/hypersegmented neutrophils), monocytes/macrophages, mast cells, thrombocytes/megakaryocytes, and dendritic cells.

The biological sample can include any number of macromolecules, for example, cellular macromolecules and organelles (e.g., mitochondria and nuclei). The biological sample can be a nucleic acid sample and/or protein sample. The biological sample can be a carbohydrate sample or a lipid sample. The biological sample can be obtained as a tissue sample, such as a tissue section, biopsy, a core biopsy, needle aspirate, or fine needle aspirate. The sample can be a fluid sample, such as a blood sample, urine sample, or saliva sample. The sample can be a skin sample, a colon sample, a

cheek swab, a histology sample, a histopathology sample, a plasma or serum sample, a tumor sample, living cells, cultured cells, a clinical sample such as, for example, whole blood or blood-derived products, blood cells, or cultured tissues or cells, including cell suspensions.

Cell-free biological samples can include extracellular polynucleotides. Extracellular polynucleotides can be isolated from a bodily sample, e.g., blood, plasma, serum, urine, saliva, mucosal excretions, sputum, stool, and tears.

As discussed above, a biological sample can include a single analyte of interest, or more than one analyte of interest. Methods for performing multiplexed assays to analyze two or more different analytes in a single biological sample is discussed in a subsequent section of this disclosure.

(ii) Preparation of Biological Samples

A variety of steps can be performed to prepare a biological sample for analysis. Except where indicated otherwise, the preparative steps described below can generally be combined in any manner to appropriately prepare a particular sample for analysis.

(1) Freezing

In some embodiments, the biological sample (e.g., a tissue section as described above) can be prepared by deep freezing at a temperature suitable to maintain or preserve the integrity (e.g., the physical characteristics) of the tissue structure. Such a temperature can be, e.g., less than -20°C, or less than -25°C, -30°C, -40°C, -50°C, -60°C, -70°C, 80°C -90°C, - 100°C, -110°C, -120°C, -130°C, -140°C, -150°C, -160°C, -170°C, -180°C, -190°C, or - 200°C. The frozen tissue sample can be sectioned, e.g., thinly sliced, onto a substrate surface using any number of suitable methods. For example, a tissue sample can be prepared using a chilled microtome (e.g., a cryostat) set at a temperature suitable to maintain both the structural integrity of the tissue sample and the chemical properties of the nucleic acids in the sample. Such a temperature can be, e.g., less than -15°C, less than -20°C, or less than -25°C. A sample can be snap frozen in isopentane and liquid nitrogen. Frozen samples can be stored in a sealed container prior to embedding.

(2) Formalin Fixation and Paraffin Embedding

In some embodiments, the biological sample can be prepared using formalin-fixation and paraffin-embedding (FFPE), which are established methods. In some embodiments, cell suspensions and other non-tissue samples can be prepared using formalin-fixation and paraffin-embedding. Following fixation of the sample and embedding in a paraffin or resin block, the sample can be sectioned as described above. Prior to analysis, the paraffin-embedding material can be removed from the tissue section (e.g., deparaffinization) by incubating the tissue section in an appropriate solvent (e.g., xylene) followed by a rinse (e.g., 99.5% ethanol for 2 minutes, 96% ethanol for 2 minutes, and 70% ethanol for 2 minutes).

(3) Fixation

As an alternative to formalin fixation described above, a biological sample can be fixed in any of a variety of other fixatives to preserve the biological structure of the sample prior to analysis. For example, a sample can be fixed via immersion in ethanol, methanol, acetone, formaldehyde (e.g., 2% formaldehyde), paraformaldehyde-Triton, glutaraldehyde, or combinations thereof.

In some embodiments, acetone fixation is used with fresh frozen samples, which can include, but are not limited to, cortex tissue, mouse olfactory bulb, human brain tumor, human post-mortem brain, and breast cancer samples. In some embodiments, a compatible fixation method is chosen and/or optimized based on a desired workflow. For example, formaldehyde fixation may be chosen as compatible for workflows using IHC/IF protocols for protein visualization. As another example, methanol fixation may be chosen for workflows emphasizing RNA/DNA library quality. Acetone fixation may be chosen in some applications to permeabilize the tissue. When acetone fixation is performed, prepermeabilization steps (described below) may not be performed. Alternatively, acetone fixation can be performed in conjunction with permeabilization steps.

(4) Staining

To facilitate visualization, biological samples can be stained using a wide variety of stains and staining techniques. In some embodiments, a sample can be stained using any number of biological stains, including but not limited to, acridine orange, Bismarck brown, carmine, coomassie blue, cresyl violet, DAPI, eosin, ethidium bromide, acid fuchsine, hematoxylin, Hoechst stains, iodine, methyl green, methylene blue, neutral red, Nile blue, Nile red, osmium tetroxide, propidium iodide, rhodamine, or safranin.

The sample can be stained using known staining techniques, including Can-Grunwald, Giemsa, hematoxylin and eosin (H&E), Jenner's, Leishman, Masson's trichrome, Papanicolaou, Romanowsky, silver, Sudan, Wright's, and/or Periodic Acid Schiff (PAS) staining techniques. PAS staining is typically performed after formalin or acetone fixation.

In some embodiments, the biological sample can be stained using a detectable label (e.g., radioisotopes, fluorophores, chemiluminescent compounds, bioluminescent compounds, and dyes) as described elsewhere herein. In some embodiments, a biological sample is stained using only one type of stain or one technique. In some embodiments, staining includes biological staining techniques such as H&E staining. In some embodiments, staining includes identifying analytes using fluorescently-conjugated antibodies. In some embodiments, a biological sample is stained using two or more different types of stains, or two or more different staining techniques. For example, a biological sample can be prepared by staining and imaging using one technique (e.g., H&E staining and brightfield imaging), followed by staining and imaging using another technique (e.g., IHC/IF staining and fluorescence microscopy) for the same biological sample.

In some embodiments, biological samples can be destained. Methods of destaining or discoloring a biological sample are known in the art, and generally depend on the nature of the stain(s) applied to the sample. For example, H&E staining can be destained by washing the sample in HCl, or any other acid (e.g., selenic acid, sulfuric acid, hydroiodic acid, benzoic acid, carbonic acid, malic acid, phosphoric acid, oxalic acid, succinic acid, salicylic acid, tartaric acid, sulfurous acid, trichloroacetic acid, hydrobromic acid, hydrochloric acid, nitric acid, orthophosphoric acid, arsenic acid, selenous acid, chromic acid, citric acid, hydrofluoric acid, nitrous acid, isocyanic acid, formic acid, hydrogen selenide, molybdic acid, lactic acid, acetic acid, carbonic acid, hydrogen sulfide, or combinations thereof). In some embodiments, destaining can include 1, 2, 3, 4, 5, or more washes in an acid (e.g., HCl). In some embodiments, destaining can include adding HCl to a downstream solution (e.g., permeabilization solution). In some embodiments, destaining can include dissolving an enzyme used in the disclosed methods (e.g., pepsin) in an acid (e.g., HCl) solution. In some embodiments, after destaining hematoxylin with an acid, other reagents can be added to the destaining solution to raise the pH for use in other applications. For example, SDS can be added to an acid destaining solution in order to raise the pH as compared to the acid destaining solution alone. As another example, in some embodiments, one or more immunofluorescence stains are applied to the sample via antibody coupling. Such stains can be removed using techniques such as cleavage of disulfide linkages via treatment with a reducing agent and detergent washing, chaotropic salt treatment, treatment with antigen retrieval solution, and treatment with an acidic glycine buffer. Methods for multiplexed staining and destaining are described, for example, in Bolognesi et al., J. Histochem. Cytochem. 2017; 65(8): 431-444, Lin et al., Nat Commun. 2015; 6:8390, Pirici et al., J. Histochem. Cytochem. 2009; 57:567-75, and Glass et al., J. Histochem. Cytochem. 2009; 57:899-905, the entire contents of each of which are incorporated herein by reference.

(6) Tissue Permeabilization

In some embodiments, a biological sample can be permeabilized to facilitate transfer of analytes out of the sample, and/or to facilitate transfer of species (such as capture probes) into the sample. If a sample is not permeabilized sufficiently, the amount of analyte captured from the sample may be too low to enable adequate analysis. Conversely, if the tissue sample is too permeable, the relative spatial relationship of the analytes within the tissue sample can be lost. Hence, a balance between permeabilizing the tissue sample enough to obtain good signal intensity while still maintaining the spatial resolution of the analyte distribution in the sample is desirable.

In general, a biological sample can be permeabilized by exposing the sample to one or more permeabilizing agents. Suitable agents for this purpose include, but are not limited to, organic solvents (e.g., acetone, ethanol, and methanol), cross-linking agents (e.g., paraformaldehyde), detergents (e.g., saponin, Triton X-100™, Tween-20™, or sodium dodecyl sulfate (SDS)), and enzymes (e.g., trypsin, proteases (e.g., proteinase K). In some embodiments, the detergent is an anionic detergent (e.g., SDS or N-lauroylsarcosine sodium salt solution). In some embodiments, the biological sample can be permeabilized using any of the methods described herein (e.g., using any of the detergents described herein, e.g., SDS and/or N-lauroylsarcosine sodium salt solution) before or after enzymatic treatment (e.g., treatment with any of the enzymes described herein, e.g., trypin, proteases (e.g., pepsin and/or proteinase K)).

In some embodiments, a biological sample can be permeabilized by exposing the sample to greater than about 1.0 w/v % (e.g., greater than about 2.0 w/v %, greater than about 3.0 w/v %, greater than about 4.0 w/v%, greater than about 5.0 w/v %, greater than about 6.0 w/v %, greater than about 7.0 w/v %, greater than about 8.0 w/v %, greater than about 9.0 w/v %, greater than about 10.0 w/v %, greater than about 11.0 w/v %, greater than about 12.0 w/v %, or greater than about 13.0 w/v %) sodium dodecyl sulfate (SDS) and/or N-lauroylsarcosine or N-lauroylsarcosine sodium salt. In some embodiments, a biological sample can be permeabilized by exposing the sample (e.g., for about 5 minutes to about 1 hour, about 5 minutes to about 40 minutes, about 5 minutes to about 30 minutes, about 5 minutes to about 20 minutes, or about 5 minutes to about 10 minutes) to about 1.0 w/v % to about 14.0 w/v % (e.g., about 2.0 w/v % to about 14.0 w/v %, about 2.0 w/v % to about 12.0 w/v %, about 2.0 w/v % to about 10.0 w/v %, about 4.0 w/v % to about 14.0 w/v %, about 4.0 w/v % to about 12.0 w/v %, about 4.0 w/v % to about 10.0 w/v %, about 6.0 w/v % to about 14.0 w/v %, about 6.0 w/v % to about 12.0 w/v %, about 6.0 w/v % to about 10.0 w/v %, about 8.0 w/v % to about 14.0 w/v %, about 8.0 w/v % to about 12.0

w/v %, about 8.0 w/v % to about 10.0 w/v %, about 10.0 % w/v % to about 14.0 w/v %, about 10.0 w/v % to about 12.0 w/v %, or about 12.0 w/v % to about 14.0 w/v %) SDS and/or N-lauroylsarcosine salt solution and/or proteinase K (e.g., at a temperature of about 4% to about 35 °C, about 4 ° C to about 25 °C, about 4 ° C to about 20 °C, about 4 °C to about 10 °C, about 10 °C to about 25 °C, about 10 °C to about 20 °C, about 10 °C to about 15 °C, about 35 °C to about 50 °C, about 35 °C to about 45 °C, about 35 °C to about 40 °C, about 40 °C to about 50 °C, about 40 °C to about 45 °C, or about 45 °C to about 50 °C).

In some embodiments, the biological sample can be incubated with a permeabilizing agent to facilitate permeabilization of the sample. Additional methods for sample permeabilization are described, for example, in Jamur et al., Method Mol. Biol. 588:63-66, 2010, the entire contents of which are incorporated herein by reference.

Lysis Reagents

In some embodiments, the biological sample can be permeabilized by adding one or more lysis reagents to the sample. Examples of suitable lysis agents include, but are not limited to, bioactive reagents such as lysis enzymes that are used for lysis of different cell types, e.g., gram positive or negative bacteria, plants, yeast, mammalian, such as lysozymes, achromopeptidase, lysostaphin, labiase, kitalase, lyticase, and a variety of other commercially available lysis enzymes.

Other lysis agents can additionally or alternatively be added to the biological sample to facilitate permeabilization. For example, surfactant-based lysis solutions can be used to lyse sample cells. Lysis solutions can include ionic surfactants such as, for example, sarcosyl and sodium dodecyl sulfate (SDS). More generally, chemical lysis agents can include, without limitation, organic solvents, chelating agents, detergents, surfactants, and chaotropic agents.

In some embodiments, the biological sample can be permeabilized by non-chemical permeabilization methods. Non-chemical permeabilization methods are known in the art. For example, non-chemical permeabilization methods that can be used include, but are not limited to, physical lysis techniques such as electroporation, mechanical permeabilization methods (e.g., bead beating using a homogenizer and grinding balls to mechanically disrupt sample tissue structures), acoustic permeabilization (e.g., sonication), and thermal lysis techniques such as heating to induce thermal permeabilization of the sample.

Proteases

In some embodiments, a medium, solution, or permeabilization solution may contain one or more proteases. In some embodiments, a biological sample treated with a protease capable of degrading histone proteins can result in the generation of fragmented genomic DNA. The fragmented genomic DNA can be captured using the same capture domain (e.g., capture domain having a poly(T) sequence) used to capture mRNA. In some embodiments, a biological sample is treated with a protease capable of degrading histone proteins and an RNA protectant prior to spatial profiling in order to facilitate the capture of both genomic DNA and mRNA.

Additionally, the protease may be contained in a reaction mixture (solution), which also includes other components (e.g., buffer, salt, chelator (e.g., EDTA), and/or detergent (e.g., SDS, N-Lauroylsarcosine sodium salt solution)). The reaction mixture may be buffered, having a pH of about 6.5-8.5, e.g., about 7.0-8.0. Additionally, the reaction mixture may be used at any suitable temperature, such as about 10-50°C, e.g., about 10-44°C, 11-43°C, 12-42°C, 13-41°C, 14-40°C, 15-39°C, 16-38 °C, 17-37°C, e.g., about 10°C, 12°C, 15°C, 18°C, 20°C, 22°C, 25°C, 28°C, 30°C, 33°C, 35°C or 37 °C, preferably about 35-45°C, e.g., about 37°C.

Other Reagents

In some embodiments, a permeabilization solution can contain additional reagents or a biological sample may be treated with additional reagents in order to optimize biological sample permeabilization. In some embodiments, an additional reagent is an RNA protectant. As used herein, the term "RNA protectant" typically refers to a reagent that protects RNA from RNA nucleases (e.g., RNases). Any appropriate RNA protectant that protects RNA from degradation can be used. A non-limiting example of a RNA protectant includes organic solvents (e.g., at least 60%, 65%, 70%, 75%, 80%, 85%, 90%, or 95% v/v organic solvent), which include, without limitation, ethanol, methanol, propan-2-ol, acetone, trichloroacetic acid, propanol, polyethylene glycol, acetic acid, or a combination thereof. In some embodiments, a RNA protectant includes ethanol, methanol and/or propan-2-ol, or a combination thereof. In some embodiments, a RNA protectant includes RNAlater ICE (ThermoFisher Scientific). In some embodiments, the RNA protectant comprises at least about 60% ethanol. In some embodiments, the RNA protectant comprises about 60-95% ethanol, about 0-35% methanol and about 0-35% propan-2-ol, wherein the total amount of organic solvent in the medium is not more than about 95%. In some embodiments, the RNA protectant comprises about 60-95% ethanol, about 5-20% methanol and about 5-20% propan-2-ol, wherein the total amount of organic solvent in the medium is not more than about 95%.

In some embodiments, the RNA protectant includes a salt. The salt may include ammonium sulfate, ammonium

bisulfate, ammonium chloride, ammonium acetate, cesium sulfate, cadmium sulfate, cesium iron (II) sulfate, chromium (III) sulfate, cobalt (II) sulfate, copper (II) sulfate, lithium chloride, lithium acetate, lithium sulfate, magnesium sulfate, magnesium chloride, manganese sulfate, manganese chloride, potassium chloride, potassium sulfate, sodium chloride, sodium acetate, sodium sulfate, zinc chloride, zinc acetate and zinc sulfate. In some embodiments, the salt is a sulfate salt, for example, ammonium sulfate, ammonium bisulfate, cesium sulfate, cadmium sulfate, cesium iron (II) sulfate, chromium (III) sulfate, cobalt (II) sulfate, copper (II) sulfate, lithium sulfate, magnesium sulfate, manganese sulfate, potassium sulfate, sodium sulfate, or zinc sulfate. In some embodiments, the salt is ammonium sulfate. The salt may be present at a concentration of about 20 g/100 ml of medium or less, such as about 15g/100 ml, 10g/100 ml, 9g/100 ml, 8g/100 ml, 7g/100 ml, 6g/100 ml, 5g/100 ml or less, e.g., about 4g, 3g, 2g or 1g/100ml.

Additionally, the RNA protectant may be contained in a medium that further includes a chelator (e.g., EDTA), a buffer (e.g., sodium citrate, sodium acetate, potassium citrate, or potassium acetate, preferably sodium acetate), and/or buffered to a pH between about 4-8 (e.g., about 5).

In some embodiments, the biological sample is treated with one or more RNA protectants before, contemporaneously with, or after permeabilization. For example, a biological sample is treated with one or more RNA protectants prior to treatment with one or more permeabilization reagents (e.g., one or more proteases). In another example, a biological sample is treated with a solution including one or more RNA protectants and one or more permeabilization reagents (e.g., one or more proteases). In yet another example, a biological sample is treated with one or more RNA protectants after the biological sample has been treated with one or more permeabilization reagents (e.g., one or more proteases). In some embodiments, a biological sample is treated with one or more RNA protectants prior to fixation.

In some embodiments, identifying the location of the captured analyte in the biological sample includes a nucleic acid extension reaction. In some embodiments where a capture probe captures a fragmented genomic DNA molecule, a nucleic acid extension reaction includes DNA polymerase. For example, a nucleic acid extension reaction includes using a DNA polymerase to extend the capture probe that is hybridized to the captured analyte (e.g., fragmented genomic DNA) using the captured analyte (e.g., fragmented genomic DNA) as a template. The product of the extension reaction includes a spatially-barcoded analyte (e.g., spatially-barcoded fragmented genomic DNA). The spatially-barcoded analyte (e.g., spatially-barcoded fragmented genomic DNA) can be used to identify the spatial location of the analyte in the biological sample. Any DNA polymerase that is capable of extending the capture probe using the captured analyte as a template can be used for the methods described herein. Non-limiting examples of DNA polymerases include T7 DNA polymerase; Bsu DNA polymerase; and E.coli DNA Polymerase pol I.

## Selective Permeabilization/Selective Lysis

In some embodiments, biological samples can be processed to selectively release an analyte from a subcellular region of a cell according to established methods. In some embodiments, a method provided herein can include detecting at least one biological analyte present in a subcellular region of a cell in a biological sample. As used herein, a "subcellular region" can refer to any subcellular region. For example, a subcellular region can refer to cytosol, a mitochondria, a nucleus, a nucleolus, an endoplasmic reticulum, a lysosome, a vesicle, a Golgi apparatus, a plastid, a vacuole, a ribosome, cytoskeleton, or combinations thereof. In some embodiments, the subcellular region comprises at least one of cytosol, a nucleus, a mitochondria, and a microsome. In some embodiments, the subcellular region is cytosol. In some embodiments, the subcellular region is a nucleus. In some embodiments, the subcellular region is a mitochondria. In some embodiments, the subcellular region is a microsome.

For example, a biological analyte can be selectively released from a subcellular region of a cell by selective permeabilization or selective lysing. In some embodiments, "selective permeabilization" can refer to a permeabilization method that can permeabilize a membrane of a subcellular region while leaving a different subcellular region substantially intact (e.g., biological analytes are not released from subcellular region due to the applied permeabilization method). Non-limiting examples of selective permeabilization methods include using electrophoresis and/or applying a permeabilization reagent. In some embodiments, "selective lysing" can refer to a lysis method that can lyse a membrane of a subcellular region while leaving a different subcellular region substantially intact (e.g., biological analytes are not released from subcellular region due to the applied lysis method). Several methods for selective permeabilization or lysis are known to one of skill in the art including the methods described in Lu et al. Lab Chip. 2005 Jan;5(1):23-9; Niklas et al. Anal Biochem. 2011 Sep 15;416(2):218-27; Cox and Emili. Nat Protoc. 2006;1(4):1872-8; Chiang et al. J Biochem. Biophys. Methods. 2000 Nov 20;46(1-2):53-68; and Yamauchi and Herr et al. Microsyst. Nanoeng. 2017;3. pii: 16079; each of which is incorporated herein by reference in its entirety.

In some embodiments, "selective permeabilization" or "selective lysis" refer to the selective permeabilization or selective lysis of a specific cell type. For example, "selective permeabilization" or "selective lysis" can refer to lysing one cell type while leaving a different cell type substantially intact (e.g., biological analytes are not released from the cell due to the applied permeabilization or lysis method). A cell that is a "different cell type" than another cell can refer to a cell from a different taxonomic kingdom, a prokaryotic cell versus a eukaryotic cell, a cell from a different tissue type, etc. Many

methods are known to one of skill in the art for selectively permeabilizing or lysing different cell types. Non-limiting examples include applying a permeabilization reagent, electroporation, and/or sonication. See, e.g., International Application No. WO 2012/168003; Han et al. Microsyst Nanoeng. 2019 Jun 17;5:30; Gould et al. Oncotarget. 2018 Mar 20; 9(21): 15606-15615; Oren and Shai. Biochemistry. 1997 Feb 18;36(7):1826-35; Algayer et al. Molecules. 2019 May 31;24(11). pii: E2079; Hipp et al. Leukemia. 2017 Oct;31(10):2278; International Application No. WO 2012/168003; and U.S. Patent No. 7,785,869; all of which are incorporated by reference herein in their entireties.

In some embodiments, applying a selective permeabilization or lysis reagent comprises contacting the biological sample with a hydrogel comprising the permeabilization or lysis reagent.

In some embodiments, the biological sample is contacted with two or more arrays (e.g., flexible arrays, as described herein). For example, after a subcellular region is permeabilized and a biological analyte from the subcellular region is captured on a first array, the first array can be removed, and a biological analyte from a different subcellular region can be captured on a second array.

(7) Other Reagents

Additional reagents can be added to a biological sample to perform various functions prior to analysis of the biological sample. In some embodiments, nuclease inhibitors such as DNase and RNase inactivating agents or protease inhibitors, and/or chelating agents such as EDTA, can be added to the biological sample. In other embodiments nucleases, such as DNase or RNAse, or proteases, such as pepsin or proteinase K, are added to the sample. In some embodiments, additional reagents may be dissolved in a solution or applied as a medium to the sample. In some embodiments, additional reagents (e.g., pepsin) may be dissolved in HCl prior to applying to the sample. For example, hematoxylin, from an H&E stain, can be optionally removed from the biological sample by washing in dilute HCl (0.001M to 0.1M) prior to further processing. In some embodiments, pepsin can be dissolved in dilute HCl (0.001M to 0.1M) prior to further processing. In some embodiments, biological samples can be washed additional times (e.g., 2, 3, 4, 5, or more times) in dilute HCl prior to incubation with a protease (e.g., pepsin), but after proteinase K treatment.

In some embodiments, the biological sample can be treated with one or more enzymes. For example, one or more endonucleases to fragment DNA, DNA polymerase enzymes, and dNTPs used to amplify nucleic acids can be added. Other enzymes that can also be added to the biological sample include, but are not limited to, polymerase, transposase, ligase, and DNAse, and RNAse.

In some embodiments, reverse transcriptase enzymes can be added to the sample, including enzymes with terminal transferase activity, primers, and template switch oligonucleotides (TSOs). Template switching can be used to increase the length of a cDNA, e.g., by appending a predefined nucleic acid sequence to the cDNA. In some embodiments, the appended nucleic acid sequence comprises one or more ribonucleotides.

In some embodiments, additional reagents can be added to improve the recovery of one or more target molecules (e.g., cDNA molecules, mRNA transcripts). For example, addition of carrier RNA to a RNA sample workflow process can increase the yield of extracted RNA/DNA hybrids from the biological sample. In some embodiments, carrier molecules are useful when the concentration of input or target molecules is low as compared to remaining molecules. Generally, single target molecules cannot form a precipitate, and addition of the carrier molecules can help in forming a precipitate. Some target molecule recovery protocols use carrier RNA to prevent small amounts of target nucleic acids present in the sample from being irretrievably bound. In some embodiments, carrier RNA can be added immediately prior to a second strand synthesis step. In some embodiments, carrier RNA can be added immediately prior to a second strand cDNA synthesis on oligonucleotides released from an array. In some embodiments, carrier RNA can be added immediately prior to a post in vitro transcription clean-up step. In some embodiments, carrier RNA can be added prior to amplified RNA purification and quantification. In some embodiments, carrier RNA can be added before RNA quantification. In some embodiments, carrier RNA can be added immediately prior to both a second strand cDNA synthesis and a post in vitro transcription clean-up step.

II. General Spatial Array-Based Analytical Methodology

Provided herein are methods, apparatus, systems, and compositions for spatial array-based analysis of biological samples.

(a) Spatial Analysis Methods

Array-based spatial analysis methods involve the transfer of one or more analytes from a biological sample to an array of features on a substrate, where each feature is associated with a unique spatial location on the array. Subsequent analysis of the transferred analytes includes determining the identity of the analytes and the spatial location of each analyte within the biological sample. The spatial location of each analyte within the biological sample is determined based on the

feature to which each analyte is bound on the array, and the feature's relative spatial location within the array.

There are at least two general methods to associate a spatial barcode with one or more neighboring cells, such that the spatial barcode identifies the one or more cells, and/or contents of the one or more cells, as associated with a particular spatial location. One general method is to promote analytes out of a cell and towards the spatially-barcoded array.

Another general method is to cleave the spatially-barcoded capture probes from an array, and promote the spatially-barcoded capture probes towards and/or into or onto the biological sample.

Using the methods, compositions, systems, kits, and devices described herein, RNA transcripts present in biological samples (e.g., tissue samples) can be used for spatial transcriptome analysis. In particular, in some cases, the barcoded oligonucleotides may be configured to prime, replicate, and consequently yield barcoded extension products from an RNA template, or derivatives thereof. For example, in some cases, the barcoded oligonucleotides may include mRNA specific priming sequences, e.g., poly-T primer segments that allow priming and replication of mRNA in a reverse transcription reaction or other targeted priming sequences. Alternatively or additionally, random RNA priming may be carried out using random N-mer primer segments of the barcoded oligonucleotides. Reverse transcriptases (RTs) can use an RNA template and a primer complementary to the 3' end of the RNA template to direct the synthesis of the first strand complementary DNA (cDNA). Many RTs can be used in this reverse transcription reactions, including, for example, avian myeloblastosis virus (AMV) reverse transcriptase, moloney murine leukemia virus (M-MuLV or MMLV), and other variants thereof. Some recombinant M-MuLV reverse transcriptase, such as, for example, PROTOSCRIPT® II reverse transcriptase, can have reduced RNase H activity and increased thermostability when compared to its wild type counterpart, and provide higher specificity, higher yield of cDNA and more full-length cDNA products with up to 12 kilobase (kb) in length. In some embodiments, the reverse transcriptase enzyme is a mutant reverse transcriptase enzyme such as, but not limited to, mutant MMLV reverse transcriptase. In another embodiment, the reverse transcriptase is a mutant MMLV reverse transcriptase such as, but not limited to, one or more variants described in US Patent Publication No. 20180312822 and US Provisional Patent Application No. 62/946,885 filed on December 11, 2019, both of which are incorporated herein by reference in their entireties.

In a non-limiting example of the workflows described above, a biological sample (e.g., tissue section), can be fixed with methanol, stained with hematoxylin and eosin, and imaged. Optionally, the sample can be destained prior to permeabilization. The images can be used to map spatial gene expression patterns back to the biological sample. A permeabilization enzyme can be used to permeabilize the biological sample directly on the slide. Analytes (e.g., polyadenylated mRNA) released from the overlying cells of the biological sample can be captured by capture probes within a capture area on a substrate. Reverse transcription (RT) reagents can be added to permeabilized biological samples. Incubation with the RT reagents can produce spatially-barcoded full-length cDNA from the captured analytes (e.g., polyadenylated mRNA). Second strand reagents (e.g., second strand primers, enzymes) can be added to the biological sample on the slide to initiate second strand synthesis. The resulting cDNA can be denatured from the capture probe template and transferred (e.g., to a clean tube) for amplification, and/or library construction. The spatially-barcoded, full-length cDNA can be amplified via PCR prior to library construction. The amplicons can then be enzymatically fragmented and/or size-selected in order to provide for desired amplicon size. In some embodiments, when utilizing an Illumina® library preparation methodology, P5 and P7 sequences can be added to the amplifcons thereby allowing for capture of the library preparation on a sequencing flowcell (e.g., on Illumina sequencing instruments). Additionally, i7 and i5 can index sequences be added as sample indexes if multiple libraries are to be pooled and sequenced together. Further, Read 1 and Read 2 sequences can be added to the library preparation for sequencing purposes. The aforementioned sequences can be added to a library preparation sample, fore example, via End Repair, A-tailing, Adaptor Ligation, and/or PCR. The cDNA fragments can then be sequenced using, for example, paired-end sequencing using TruSeq Read 1 and TruSeq Read 2 as sequencing primer sites.

In some embodiments, performing correlative analysis of data produced by this workflow, and other workflows described herein, can yield over 95% correlation of genes expressed across two capture areas (e.g., 95% or greater, 96% or greater, 97% or greater, 98% or greater, or 99% or greater). When performing the described workflows using single cell RNA sequencing of nuclei, in some embodiments, correlative analysis of the data can yield over 90% (e.g., over 90%, 91%, 92%, 93%, 94%, 95%, 96%, 97%, 98%, or 99%) correlation of genes expressed across two capture areas.

In some embodiments, after cDNA is generated (e.g., by reverse transcription) the cDNA can be amplified directly on the substrate surface. Generating multiple copies of the cDNA (e.g., cDNA synthesized from captured analytes) via amplification directly on the substrate surface can improve final sequencing library complexity. Thus, in some embodiments, cDNA can be amplified directly on the substrate surface by isothermal nucleic acid amplification. In some embodiments, isothermal nucleic acid amplification can amplify RNA or DNA.

In some embodiments, isothermal amplification can be faster than a standard PCR reaction. In some embodiments, isothermal amplification can be linear amplification (e.g., asymmetrical with a single primer), or exponential amplification (e.g., with two primers). In some embodiments, isothermal nucleic acid amplification can be performed by a template-switching oligonucleotide primer. In some embodiments, the template switching oligonucleotide adds a common

sequence onto the 5' end of the RNA being reverse transcribed. For example, after a capture probe interacts with an analyte (e.g., mRNA) and reverse transcription is performed such that additional nucleotides are added to the end of the cDNA creating a 3' overhang as described herein. In some embodiments, a template switching oligonucleotide hybridizes to untemplated poly(C) nucleotides added by a reverse transcriptase to continue replication to the 5' end of the template switching oligonucleotide, thereby generating full-length cDNA ready for further amplification. In some embodiments, the template switching oligonucleotide adds a common 5' sequence to full-length cDNA that is used for cDNA amplification (e.g., a reverse complement of the template switching oligonucleotide).

In some embodiments, once a full-length cDNA molecule is generated, the template switching oligonucleotide can serve as a primer in a cDNA amplification reaction (e.g., with a DNA polymerase). In some embodiments, double stranded cDNA (e.g., first strand cDNA and second strand reverse complement cDNA) can be amplified via isothermal amplification with either a helicase or recombinase, followed by a strand displacing DNA polymerase. The strand displacing DNA polymerase can generate a displaced second strand resulting in an amplified product.

In any of isothermal amplification methods described herein, barcode exchange (e.g., spatial barcode) can occur after the first amplification cycle where there are unused capture probes on the substrate surface. In some embodiments, the free 3'OH end of the unused capture probes can be blocked by any suitable 3'OH blocking method. In some embodiments, the 3'OH can be blocked by hairpin ligation.

Isothermal nucleic acid amplification can be used in addition to, or as an alternative to standard PCR reactions (e.g., a PCR reaction that requires heating to about 95°C to denature double stranded DNA). Isothermal nucleic acid amplification generally does not require the use of a thermocycler, however in some embodiments, isothermal amplification can be performed in a thermocycler. In some embodiments, isothermal amplification can be performed from about 35°C to about 75°C. In some embodiments, isothermal amplification can be performed from about 40°C, about 45°C, about 50°C, about 55°C, about 60°C, about 65°C, or about 70°C or anywhere in between depending on the polymerase and auxiliary enzymes used.

Isothermal nucleic acid amplification techniques are known in the art, and can be used alone or in combination with any of the spatial methods described herein. For example, non-limiting examples of suitable isothermal nucleic acid amplification techniques include transcription mediated amplification, nucleic acid sequence-based amplification, signal mediated amplification of RNA technology, strand displacement amplification, rolling circle amplification, loop-mediated isothermal amplification of DNA (LAMP), isothermal multiple displacement amplification, recombinase polymerase amplification, helicase-dependent amplification, single primer isothermal amplification, and circular helicase-dependent amplification (See, e.g., Gill and Ghaemi, Nucleic acid isothermal amplification technologies: a review, Nucleosides, Nucleotides, & Nucleic Acids, 27(3), 224-43, doi: 10.1080/15257770701845204 (2008), which is incorporated herein by reference in its entirety).

In some embodiments, the isothermal nucleic acid amplification is helicase-dependent nucleic acid amplification. Helicase-dependent isothermal nucleic acid amplification is described in Vincent, et. al., Helicase-dependent isothermal DNA amplification, EMBO Rep., 795-800 (2004) and U.S. Patent No. 7,282,328, which are both incorporated herein by reference in their entireties. Further, helicase-dependent nucleic acid amplification on a substrate (e.g., on-chip) is described in Andresen, et. al., Helicase-dependent amplification: use in OnChip amplification and potential for point-of-care diagnostics, Expert Rev Mol Diagn., 9, 645-650, doi: 10.1586/erm.09.46 (2009), which is incorporated herein by reference in its entirety. In some embodiments, the isothermal nucleic acid amplification is recombinase polymerase nucleic acid amplification. Recombinase polymerase nucleic acid amplification is described in Piepenburg, et al., DNA Detection Using Recombinant Proteins, PLoS Biol., 4, 7 e204 (2006) and Li, et. al., Review: a comprehensive summary of a decade development of the recombinase polymerase amplification, Analyst, 144, 31-67, doi: 10.1039/C8AN01621F (2019), both of which are incorporated herein by reference in their entireties.

Generally, isothermal amplification techniques use standard PCR reagents (e.g., buffer, dNTPs etc.) known in the art. Some isothermal amplification techniques can require additional reagents. For example, helicase dependent nucleic acid amplification uses a single-strand binding protein and an accessory protein. In another example, recombinase polymerase nucleic acid amplification uses recombinase (e.g., T4 UvsX), recombinase loading factor (e.g., TF UvsY), single-strand binding protein (e.g., T4 gp32), crowding agent (e.g., PEG-35K), and ATP.

After isothermal nucleic acid amplification of the full-length cDNA described by any of the methods herein, the isothermally amplified cDNAs (e.g., single-stranded or double-stranded) can be recovered from the substrate, and optionally followed by amplification with typical cDNA PCR in microcentrifuge tubes. The sample can then be used with any of the spatial methods described herein.

(i) Immunohistochemistry and Immunofluorescence

In some embodiments, immunofluorescence or immunohistochemistry protocols (direct and indirect staining techniques) can be performed as a part of, or in addition to, the exemplary spatial workflows presented herein. For example, tissue sections can be fixed according to methods described herein. The biological sample can be transferred to

an array (e.g., capture probe array), wherein analytes (e.g., proteins) are probed using immunofluorescence protocols. For example, the sample can be rehydrated, blocked, and permeabilized (3XSSC, 2% BSA, 0.1% Triton X, 1 U/μl RNAse inhibitor for 10 min at 4°C) before being stained with fluorescent primary antibodies (1:100 in 3XSSC, 2% BSA, 0.1% Triton X, 1 U/μl RNAse inhibitor for 30 min at 4°C). The biological sample can be washed, coverslipped (in glycerol + 1 U/μl RNAse inhibitor), imaged (e.g., using a confocal microscope or other apparatus capable of fluorescent detection), washed, and processed according to analyte capture or spatial workflows described herein.

As used herein, an "antigen retrieval buffer" can improve antibody capture in IF/IHC protocols. An exemplary protocol for antigen retrieval can be preheating the antigen retrieval buffer (e.g., to 95°C), immersing the biological sample in the heated antigen retrieval buffer for a predetermined time, and then removing the biological sample from the antigen retrieval buffer and washing the biological sample.

In some embodiments, optimizing permeabilization can be useful for identifying intracellular analytes. Permeabilization optimization can include selection of permeabilization agents, concentration of permeabilization agents, and permeabilization duration. Tissue permeabilization is discussed elsewhere herein.

In some embodiments, blocking an array and/or a biological sample in preparation of labeling the biological sample decreases unspecific binding of the antibodies to the array and/or biological sample (decreases background). Some embodiments provide for blocking buffers/blocking solutions that can be applied before and/or during application of the label, wherein the blocking buffer can include a blocking agent, and optionally a surfactant and/or a salt solution. **In** some embodiments, a blocking agent can be bovine serum albumin (BSA), serum, gelatin (e.g., fish gelatin), milk (e.g., non-fat dry milk), casein, polyethylene glycol (PEG), polyvinyl alcohol (PVA), or polyvinylpyrrolidone (PVP), biotin blocking reagent, a peroxidase blocking reagent, levamisole, Camoy's solution, glycine, lysine, sodium borohydride, pontamine sky blue, Sudan Black, trypan blue, FITC blocking agent, and/or acetic acid. The blocking buffer/blocking solution can be applied to the array and/or biological sample prior to and/or during labeling (e.g., application of fluorophore-conjugated antibodies) to the biological sample.

**In** some embodiments, additional steps or optimizations can be included in performing IF/IHC protocols in conjunction with spatial arrays. Additional steps or optimizations can be included in performing spatially-tagged analyte capture agent workflows discussed herein.

In some embodiments, provided herein are methods for spatially detecting an analyte (e.g., detecting the location of an analyte, e.g., a biological analyte) from a biological sample (e.g., an analyte present in a biological sample, such as a tissue section) that include: (a) providing a biological sample on a substrate; (b) staining the biological sample on the substrate, imaging the stained biological sample, and selecting the biological sample or subsection of the biological sample (e.g., region of interest) to subject to analysis; (c) providing an array comprising one or more pluralities of capture probes on a substrate; (d) contacting the biological sample with the array, thereby allowing a capture probe of the one or more pluralities of capture probes to capture the analyte of interest; and (e) analyzing the captured analyte, thereby spatially detecting the analyte of interest. Any variety of staining and imaging techniques as described herein or known in the art can be used in accordance with methods described herein. In some embodiments, the staining includes optical labels as described herein, including, but not limited to, fluorescent, radioactive, chemiluminescent, calorimetric, or colorimetric detectable labels. In some embodiments, the staining includes a fluorescent antibody directed to a target analyte (e.g., cell surface or intracellular proteins) in the biological sample. In some embodiments, the staining includes an immunohistochemistry stain directed to a target analyte (e.g., cell surface or intracellular proteins) in the biological sample. In some embodiments, the staining includes a chemical stain such as hematoxylin and eosin (H&E) or periodic acid-schiff (PAS). In some embodiments, significant time (e.g., days, months, or years) can elapse between staining and/or imaging the biological sample and performing analysis. In some embodiments, reagents for performing analysis are added to the biological sample before, contemporaneously with, or after the array is contacted to the biological sample. In some embodiments, step (d) includes placing the array onto the biological sample. In some embodiments, the array is a flexible array where the plurality of spatially-barcoded features (e.g., a substrate with capture probes, a bead with capture probes) are attached to a flexible substrate. In some embodiments, measures are taken to slow down a reaction (e.g., cooling the temperature of the biological sample or using enzymes that preferentially perform their primary function at lower or higher temperature as compared to their optimal functional temperature) before the array is contacted with the biological sample. In some embodiments, step (e) is performed without bringing the biological sample out of contact with the array. In some embodiments, step (e) is performed after the biological sample is no longer in contact with the array. In some embodiments, the biological sample is tagged with an analyte capture agent before, contemporaneously with, or after staining and/or imaging of the biological sample. In such cases, significant time (e.g., days, months, or years) can elapse between staining and/or imaging and performing analysis. In some embodiments, the array is adapted to facilitate biological analyte migration from the stained and/or imaged biological sample onto the array (e.g., using any of the materials or methods described herein). In some embodiments, a biological sample is permeabilized before being contacted with an array. In some embodiments, the rate of permeabilization is slowed prior to contacting a biological sample with an array (e.g., to limit diffusion of analytes away from their original locations in the biological sample). In some embodiments, modulating the rate of permeabilization (e.g., modulating the activity of a permeabilization reagent) can occur by modulating a condition that

the biological sample is exposed to (e.g., modulating temperature, pH, and/or light). In some embodiments, modulating the rate of permeabilization includes use of external stimuli (e.g., small molecules, enzymes, and/or activating reagents) to modulate the rate of permeabilization. For example, a permeabilization reagent can be provided to a biological sample prior to contact with an array, which permeabilization reagent is inactive until a condition (e.g., temperature, pH, and/or light) is changed or an external stimulus (e.g., a small molecule, an enzyme, and/or an activating reagent) is provided.

In some embodiments, provided herein are methods for spatially detecting an analyte (e.g., detecting the location of an analyte, e.g., a biological analyte) from a biological sample (e.g., present in a biological sample such as a tissue section) that include: (a) providing a biological sample on a substrate; (b) staining the biological sample on the substrate, imaging the stained biological sample, and selecting the biological sample or subsection of the biological sample (e.g., a region of interest) to subject to spatial transcriptomic analysis; (c) providing an array comprising one or more pluralities of capture probes on a substrate; (d) contacting the biological sample with the array, thereby allowing a capture probe of the one or more pluralities of capture probes to capture the biological analyte of interest; and (e) analyzing the captured biological analyte, thereby spatially detecting the biological analyte of interest.

(b) Capture Probes

A "capture probe" refers to any molecule capable of capturing (directly or indirectly) and/or labelling an analyte (e.g., an analyte of interest) in a biological sample. In some embodiments, the capture probe is a nucleic acid or a polypeptide. In some embodiments, the capture probe is a conjugate (e.g., an oligonucleotide-antibody conjugate). In some embodiments, the capture probe includes a barcode (e.g., a spatial barcode and/or a unique molecular identifier (UMI)) and a capture domain.

(i) Capture Domain

As discussed above, each capture probe includes at least one capture domain. The "capture domain" can be an oligonucleotide, a polypeptide, a small molecule, or any combination thereof, that binds specifically to a desired analyte. In some embodiments, a capture domain can be used to capture or detect a desired analyte.

In some embodiments, the capture domain is a functional nucleic acid sequence configured to interact with one or more analytes, such as one or more different types of nucleic acids (e.g., RNA molecules and DNA molecules). In some embodiments, the functional nucleic acid sequence can include an N-mer sequence (e.g., a random N-mer sequence), which N-mer sequences are configured to interact with a plurality of DNA molecules. In some embodiments, the functional sequence can include a poly(T) sequence, which poly(T) sequences are configured to interact with messenger RNA (mRNA) molecules via the poly(A) tail of an mRNA transcript. In some embodiments, the functional nucleic acid sequence is the binding target of a protein (e.g., a transcription factor, a DNA binding protein, or a RNA binding protein), where the analyte of interest is a protein.

Capture probes can include ribonucleotides and/or deoxyribonucleotides as well as synthetic nucleotide residues that are capable of participating in Watson-Crick type or analogous base pair interactions. In some embodiments, the capture domain is capable of priming a reverse transcription reaction to generate cDNA that is complementary to the captured RNA molecules. In some embodiments, the capture domain of the capture probe can prime a DNA extension (polymerase) reaction to generate DNA that is complementary to the captured DNA molecules. In some embodiments, the capture domain can template a ligation reaction between the captured DNA molecules and a surface probe that is directly or indirectly immobilized on the substrate. In some embodiments, the capture domain can be ligated to one strand of the captured DNA molecules. For example, SplintR ligase along with RNA or DNA sequences (e.g., degenerate RNA) can be used to ligate a single-stranded DNA or RNA to the capture domain. In some embodiments, ligases with RNA-templated ligase activity, e.g., SplintR ligase, T4 RNA ligase 2 or KOD ligase, can be used to ligate a single-stranded DNA or RNA to the capture domain. In some embodiments, a capture domain includes a splint oligonucleotide. In some embodiments, a capture domain captures a splint oligonucleotide.

In some embodiments, the capture domain is located at the 3' end of the capture probe and includes a free 3' end that can be extended, e.g., by template dependent polymerization, to form an extended capture probe as described herein. In some embodiments, the capture domain includes a nucleotide sequence that is capable of hybridizing to nucleic acid, e.g., RNA or other analyte, present in the cells of the biological sample contacted with the array. In some embodiments, the capture domain can be selected or designed to bind selectively or specifically to a target nucleic acid. For example, the capture domain can be selected or designed to capture mRNA by way of hybridization to the mRNA poly(A) tail. Thus, in some embodiments, the capture domain includes a poly(T) DNA oligonucleotide, e.g., a series of consecutive deoxythymidine residues linked by phosphodiester bonds, which is capable of hybridizing to the poly(A) tail of mRNA. In some embodiments, the capture domain can include nucleotides that are functionally or structurally analogous to a poly(T) tail. For example, a poly(U) oligonucleotide or an oligonucleotide included of deoxythymidine analogues. In some embodiments, the capture domain includes at least 10, 11, 12, 13, 14, 15, 16, 17, 18, 19, or 20 nucleotides. In some embodiments,

the capture domain includes at least 25, 30, or 35 nucleotides.

In some embodiments, a capture probe includes a capture domain having a sequence that is capable of binding to mRNA and/or genomic DNA. For example, the capture probe can include a capture domain that includes a nucleic acid sequence (e.g., a poly(T) sequence) capable of binding to a poly(A) tail of an mRNA and/or to a poly(A) homopolymeric sequence present in genomic DNA. In some embodiments, a homopolymeric sequence is added to an mRNA molecule or a genomic DNA molecule using a terminal transferase enzyme in order to produce an analyte that has a poly(A) or poly(T) sequence. For example, a poly(A) sequence can be added to an analyte (e.g., a fragment of genomic DNA) thereby making the analyte capable of capture by a poly(T) capture domain.

In some embodiments, random sequences, e.g., random hexamers or similar sequences, can be used to form all or a part of the capture domain. For example, random sequences can be used in conjunction with poly(T) (or poly(T) analogue) sequences. Thus, where a capture domain includes a poly(T) (or a "poly(T)-like") oligonucleotide, it can also include a random oligonucleotide sequence (e.g., "poly(T)-random sequence" probe). This can, for example, be located 5' or 3' of the poly(T) sequence, e.g., at the 3' end of the capture domain. The poly(T)-random sequence probe can facilitate the capture of the mRNA poly(A) tail. In some embodiments, the capture domain can be an entirely random sequence. In some embodiments, degenerate capture domains can be used.

In some embodiments, a pool of two or more capture probes form a mixture, where the capture domain of one or more capture probes includes a poly(T) sequence and the capture domain of one or more capture probes includes random sequences. In some embodiments, a pool of two or more capture probes form a mixture where the capture domain of one or more capture probes includes poly(T)-like sequence and the capture domain of one or more capture probes includes random sequences. In some embodiments, a pool of two or more capture probes form a mixture where the capture domain of one or more capture probes includes a poly(T)-random sequences and the capture domain of one or more capture probes includes random sequences. In some embodiments, probes with degenerate capture domains can be added to any of the preceding combinations listed herein. In some embodiments, probes with degenerate capture domains can be substituted for one of the probes in each of the pairs described herein.

The capture domain can be based on a particular gene sequence or particular motif sequence or common/-conserved sequence, that it is designed to capture (i.e., a sequence-specific capture domain). Thus, in some embodiments, the capture domain is capable of binding selectively to a desired sub-type or subset of nucleic acid, for example a particular type of RNA, such as mRNA, rRNA, tRNA, SRP RNA, tmRNA, snRNA, snoRNA, SmY RNA, scaRNA, gRNA, RNase P, RNase MRP, TERC, SL RNA, aRNA, cis-NAT, crRNA, lncRNA, miRNA, piRNA, siRNA, shRNA, tasiRNA, rasiRNA, 7SK, eRNA, ncRNA or other types of RNA. In a non-limiting example, the capture domain can be capable of binding selectively to a desired subset of ribonucleic acids, for example, microbiome RNA, such as 16S rRNA. In other non-limiting examples, the capture domain can be capable of selectively binding to a sequence in a DNA molecule, for example a cDNA or gDNA molecule. For example, a capture domain could selectively bind a DNA of interest in cancer or disease states.

In some embodiments, a capture domain includes an "anchor" or "anchoring sequence", which is a sequence of nucleotides that is designed to ensure that the capture domain hybridizes to the intended analyte. In some embodiments, an anchor sequence includes a sequence of nucleotides, including a 1-mer, 2-mer, 3-mer or longer sequence. In some embodiments, the short sequence is random. For example, a capture domain including a poly(T) sequence can be designed to capture a mRNA. In such embodiments, an anchoring sequence can include a random 3-mer (e.g., GGG) that helps ensure that the poly(T) capture domain hybridizes to an mRNA. In some embodiments, an anchoring sequence can be VN, N, or NN. Alternatively, the sequence can be designed using a specific sequence of nucleotides. In some embodiments, the anchor sequence is at the 3' end of the capture domain. In some embodiments, the anchor sequence is at the 5' end of the capture domain.

In some embodiments, capture domains of capture probes are blocked prior to contacting the biological sample with the array, and blocking probes can be used when the nucleic acid in the biological sample is modified prior to its capture on the array. In some embodiments, the blocking probe is used to block or modify the free 3' end of the capture domain. In some embodiments, blocking probes can be hybridized to the capture probes to mask the free 3' end of the capture domain, e.g., hairpin probes, partially double stranded probes, or complementary sequences. In some embodiments, the free 3' end of the capture domain can be blocked by chemical modification, e.g., addition of an azidomethyl group as a chemically reversible capping moiety such that the capture probes do not include a free 3' end. Blocking or modifying the capture probes, particularly at the free 3' end of the capture domain, prior to contacting the biological sample with the array, prevents modification of the capture probes, e.g., prevents the addition of a poly(A) tail to the free 3' end of the capture probes.

Non-limiting examples of 3' modifications include dideoxy C-3' (3'-ddC), 3' inverted dT, 3' C3 spacer, 3'Amino, and 3' phosphorylation. In some embodiments, the nucleic acid in the biological sample can be modified such that it can be captured by the capture domain. For example, an adaptor sequence (including a binding domain capable of binding to the capture domain of the capture probe) can be added to the end of the nucleic acid, e.g., fragmented genomic DNA. In some embodiments, this is achieved by ligation of the adaptor sequence or extension of the nucleic acid. In some embodiments,

an enzyme is used to incorporate additional nucleotides at the end of the nucleic acid sequence, e.g., a poly(A) tail. In some embodiments, the capture probes can be reversibly masked or modified such that the capture domain of the capture probe does not include a free 3' end. In some embodiments, the 3' end is removed, modified, or made inaccessible so that the capture domain is not susceptible to the process used to modify the nucleic acid of the biological sample, e.g., ligation or extension.

In some embodiments, the capture domain of the capture probe is modified to allow the removal of any modifications of the capture probe that occur during modification of the nucleic acid molecules of the biological sample. In some embodiments, the capture probes can include an additional sequence downstream of the capture domain, e.g., 3' to the capture domain, namely a blocking domain.

In some embodiments, the capture domain of the capture probe can be a non-nucleic acid domain. Examples of suitable capture domains that are not exclusively nucleic-acid based include, but are not limited to, proteins, peptides, aptamers, antigens, antibodies, and molecular analogs that mimic the functionality of any of the capture domains described herein.

(ii) Cleavage Domain

Each capture probe can optionally include at least one cleavage domain. The cleavage domain represents the portion of the probe that is used to reversibly attach the probe to an array feature, as will be described further herein. Further, one or more segments or regions of the capture probe can optionally be released from the array feature by cleavage of the cleavage domain. As an example, spatial barcodes and/or universal molecular identifiers (UMIs) can be released by cleavage of the cleavage domain.

In some embodiments, the cleavage domain linking the capture probe to a feature is a bond capable of cleavage by an enzyme. An enzyme can be added to cleave the cleavage domain, resulting in release of the capture probe from the feature. As another example, heating can also result in degradation of the cleavage domain and release of the attached capture probe from the array feature. In some embodiments, laser radiation is used to heat and degrade cleavage domains of capture probes at specific locations. In some embodiments, the cleavage domain is a photo-sensitive chemical bond (e.g., a chemical bond that dissociates when exposed to light such as ultraviolet light). In some embodiments, the cleavage domain can be an ultrasonic cleavage domain. For example, ultrasonic cleavage can depend on nucleotide sequence, length, pH, ionic strength, temperature, and the ultrasonic frequency (e.g., 22 kHz, 44 kHz) (Grokhovsky, S.L., Specificity of DNA cleavage by ultrasound, Molecular Biology, 40(2), 276-283 (2006)).

Oligonucleotides with photo-sensitive chemical bonds (e.g., photo-cleavable linkers) have various advantages. They can be cleaved efficiently and rapidly (e.g., in nanoseconds and milliseconds). In some cases, photo-masks can be used such that only specific regions of the array are exposed to cleavable stimuli (e.g., exposure to UV light, exposure to light, exposure to heat induced by laser). When a photo-cleavable linker is used, the cleavable reaction is triggered by light, and can be highly selective to the linker and consequently biorthogonal. Typically, wavelength absorption for the photocleavable linker is located in the near-UV range of the spectrum. In some embodiments, $\lambda_{max}$ of the photocleavable linker is from about 300 nm to about 400 nm, or from about 310 nm to about 365 nm. In some embodiments, $\lambda$max of the photocleavable linker is about 300 nm, about 312 nm, about 325 nm, about 330 nm, about 340 nm, about 345 nm, about 355 nm, about 365 nm, or about 400 nm.

Non-limiting examples of a photo-sensitive chemical bond that can be used in a cleavage domain include those described in Leriche et al. Bioorg Med Chem. 2012 Jan 15;20(2):571-82 and U.S. Publication No. 2017/0275669, both of which are incorporated by reference herein in their entireties. For example, linkers that comprise photo-sensitive chemical bonds include 3-amino-3-(2-nitrophenyl)propionic acid (ANP), phenacyl ester derivatives, 8-quinolinyl benzenesulfonate, dicoumarin, 6-bromo-7-alkixycoumarin-4-ylmethoxycarbonyl, a bimane-based linker, and a bis-arylhydrazone based linker. In some embodiments, the photo-sensitive bond is part of a cleavable linker such as an ortho-nitrobenzyl (ONB) linker below:

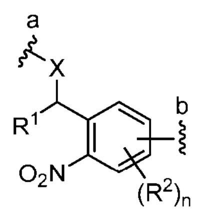

wherein:

X is selected from O and NH;

$R^1$ is selected from H and $C_{1-3}$ alkyl;

$R^2$ is selected from H and $C_{1-3}$ alkoxy;

n is 1, 2, or 3; and

a and b each represent either the point of attachment of the linker to the substrate, or the point of attachment of the linker to the capture probe.

In some embodiments, at least one spacer is included between the substrate and the ortho-nitrobenzyl (ONB) linker, and at least one spacer is included between the ortho-nitrobenzyl (ONB) linker and the capture probe. In some aspects of these embodiments, the spacer comprises at least one group selected from C1-6 alkylene, C2-6 alkenylene, C2-6 alkynylene, C=O, O, S, NH, -(C=O)O-, -(C=O)NH-, -S-S-, ethylene glycol, polyethyleneglycol, propylene glycol, and polypropyleneglycol, or any combination thereof. In some embodiments, X is O. In some embodiments, X is NH. In some embodiments, $R^1$ is H. In some embodiments, $R^1$ is $C_{1-3}$ alkyl. In some embodiments, $R^1$ is methyl. In some embodiments, $R^2$ is H. In some embodiments, $R^2$ is $C_{1-3}$ alkoxy. In some embodiments, $R^2$ is methoxy. In some embodiments, $R^1$ is H and $R^2$ is H. In some embodiments, $R^1$ is H and $R^2$ is methoxy. In some embodiments, $R^1$ is methyl and $R^2$ is H. In some embodiments, $R^1$ is methyl and $R^2$ is methoxy.

In some embodiments, the photocleavable linker has formula:

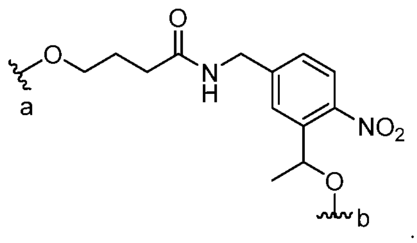

In some embodiments, the photocleavable linker has formula:

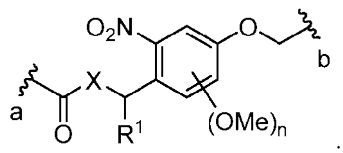

In some embodiments, the photocleavable linker has formula:

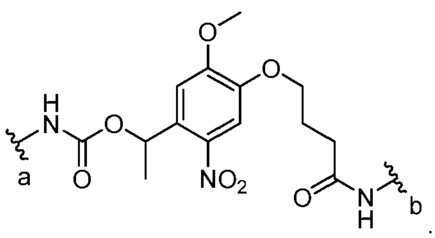

In some embodiments, the photocleavable linker has formula:

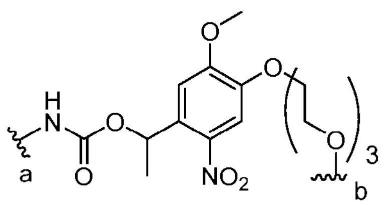

In some embodiments, the photocleavable linker has formula:

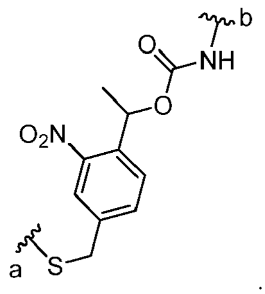

Without being bound to any particular theory, it is believed that excitation of the ortho-nitrobenzyl (ONB) linker leads to Norrish-type hydrogen abstraction in the γ-position, followed by formation of azinic acid, which is highly reactive and rearranges into nitroso compound, resulting in the complete cleavage of the linker, as shown on the following scheme:

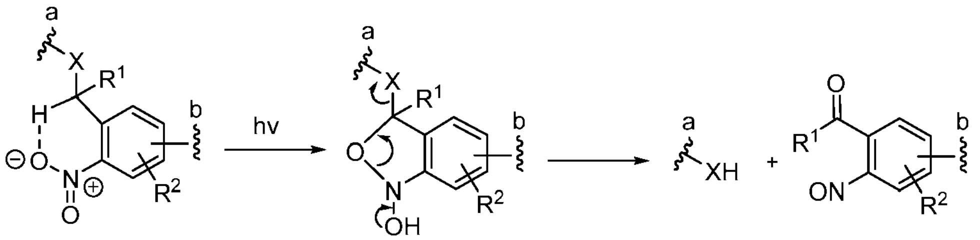


In some embodiments, the photocleavable linker is 3-amino-3-(2-nitrophenyl)propionic acid (ANP) linker:

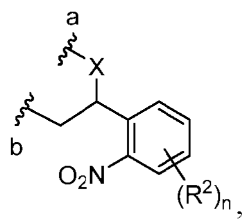

wherein X, $R^2$, n, a, and b are as described herein for the ortho-nitrobenzyl (ONB) linker.

In some embodiments, the photocleavable linker has formula:

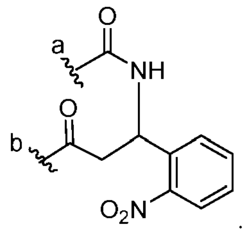

In some embodiments, the photocleavable linker is phenacyl ester linker:

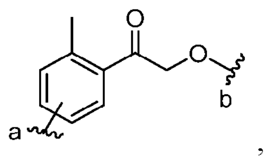

wherein a and b are as described herein for the ortho-nitrobenzyl (ONB) linker.

Other examples of photo-sensitive chemical bonds that can be used in a cleavage domain include halogenated nucleosides such as bromodeoxyuridine (BrdU). BrdU is an analog of thymidine that can be readily incorporated into oligonucleotides (e.g., in the cleavage domain of a capture probe), and is sensitive to UVB light (280-320 nm range). Upon exposure to UVB light, a photo-cleavage reaction occurs (e.g., at a nucleoside immediately 5' to the site of BrdU

incorporation (Doddridge et al. Chem. Comm., 1998, 18:1997-1998 and Cook et al. Chemistry and Biology. 1999, 6:451-459)) that results in release of the capture probe from the feature.

Other examples of cleavage domains include labile chemical bonds such as, but not limited to, ester linkages (e.g., cleavable with an acid, a base, or hydroxylamine), a vicinal diol linkage (e.g., cleavable via sodium periodate), a Diels-Alder linkage (e.g., cleavable via heat), a sulfone linkage (e.g., cleavable via a base), a silyl ether linkage (e.g., cleavable via an acid), a glycosidic linkage (e.g., cleavable via an amylase), a peptide linkage (e.g., cleavable via a protease), an abasic or apurinic/apyrimidinic (AP) site (e.g., cleavable with an alkali or an AP endonuclease), or a phosphodiester linkage (e.g., cleavable via a nuclease (e.g., DNAase)).

In some embodiments, the cleavage domain includes a sequence that is recognized by one or more enzymes capable of cleaving a nucleic acid molecule, e.g., capable of breaking the phosphodiester linkage between two or more nucleotides. A bond can be cleavable via other nucleic acid molecule targeting enzymes, such as restriction enzymes (e.g., restriction endonucleases). For example, the cleavage domain can include a restriction endonuclease (restriction enzyme) recognition sequence. Restriction enzymes cut double-stranded or single stranded DNA at specific recognition nucleotide sequences known as restriction sites. In some embodiments, a rare-cutting restriction enzyme, e.g., enzymes with a long recognition site (at least 8 base pairs in length), is used to reduce the possibility of cleaving elsewhere in the capture probe.

In some embodiments, the cleavage domain includes a poly(U) sequence which can be cleaved by a mixture of Uracil DNA glycosylase (UDG) and the DNA glycosylase-lyase Endonuclease VIII, commercially known as the USER™ enzyme. Releasable capture probes can be available for reaction once released. Thus, for example, an activatable capture probe can be activated by releasing the capture probes from a feature.

In some embodiments, where the capture probe is attached indirectly to a substrate, e.g., via a surface probe, the cleavage domain includes one or more mismatch nucleotides, so that the complementary parts of the surface probe and the capture probe are not 100% complementary (for example, the number of mismatched base pairs can be one, two, or three base pairs). Such a mismatch is recognized, e.g., by the MutY and T7 endonuclease I enzymes, which results in cleavage of the nucleic acid molecule at the position of the mismatch. As described herein a "surface probe" can be any moiety present on the surface of the substrate capable of attaching to an agent (e.g., a capture probe). In some embodiments, the surface probe is an oligonucleotide. In some embodiments, the surface probe is part of the capture probe.

In some embodiments, where the capture probe is attached (e.g., immobilized) to a feature indirectly, e.g., via a surface probe, the cleavage domain includes a nickase recognition site or sequence. Nickases are endonucleases which cleave only a single strand of a DNA duplex. Thus, the cleavage domain can include a nickase recognition site close to the 5' end of the surface probe (and/or the 5' end of the capture probe) such that cleavage of the surface probe or capture probe destabilizes the duplex between the surface probe and capture probe thereby releasing the capture probe) from the feature.

Nickase enzymes can also be used in some embodiments where the capture probe is attached (e.g., immobilized) to the feature directly. For example, the substrate can be contacted with a nucleic acid molecule that hybridizes to the cleavage domain of the capture probe to provide or reconstitute a nickase recognition site, e.g., a cleavage helper probe. Thus, contact with a nickase enzyme will result in cleavage of the cleavage domain thereby releasing the capture probe from the feature. Such cleavage helper probes can also be used to provide or reconstitute cleavage recognition sites for other cleavage enzymes, e.g., restriction enzymes.

Some nickases introduce single-stranded nicks only at particular sites on a DNA molecule, by binding to and recognizing a particular nucleotide recognition sequence. A number of naturally-occurring nickases have been discovered, of which at present the sequence recognition properties have been determined for at least four. Nickases are described in U.S. Patent No. 6,867,028, which is incorporated herein by reference in its entirety. **In** general, any suitable nickase can be used to bind to a complementary nickase recognition site of a cleavage domain. Following use, the nickase enzyme can be removed from the assay or inactivated following release of the capture probes to prevent unwanted cleavage of the capture probes.

Examples of suitable capture domains that are not exclusively nucleic-acid based include, but are not limited to, proteins, peptides, aptamers, antigens, antibodies, and molecular analogs that mimic the functionality of any of the capture domains described herein.

In some embodiments, a cleavage domain is absent from the capture probe. Examples of substrates with attached capture probes lacking a cleavage domain are described for example in Macosko et al., (2015) Cell 161, 1202-1214, the entire contents of which are incorporated herein by reference.

In some embodiments, the region of the capture probe corresponding to the cleavage domain can be used for some other function. For example, an additional region for nucleic acid extension or amplification can be included where the cleavage domain would normally be positioned. In such embodiments, the region can supplement the functional domain or even exist as an additional functional domain. In some embodiments, the cleavage domain is present but its use is optional.

(iii) Functional Domain

Each capture probe can optionally include at least one functional domain. Each functional domain typically includes a functional nucleotide sequence for a downstream analytical step in the overall analysis procedure.

In some embodiments, the capture probe can include a functional domain for attachment to a sequencing flow cell, such as, for example, a P5 sequence for Illumina® sequencing. In some embodiments, the capture probe or derivative thereof can include another functional domain, such as, for example, a P7 sequence for attachment to a sequencing flow cell for Illumina® sequencing. The functional domains can be selected for compatibility with a variety of different sequencing systems, e.g., 454 Sequencing, Ion Torrent Proton or PGM, Illumina X10, etc., and the requirements thereof.

In some embodiments, the functional domain includes a primer. The primer can include an R1 primer sequence for Illumina® sequencing, and in some embodiments, an R2 primer sequence for Illumina® sequencing. Examples of such capture probes and uses thereof are described in U.S. Patent Publication Nos. 2014/0378345 and 2015/0376609, the entire contents of each of which are incorporated herein by reference.

(iv) Spatial Barcode

As discussed above, the capture probe can include one or more spatial barcodes (e.g., two or more, three or more, four or more, five or more) spatial barcodes. A "spatial barcode" is a contiguous nucleic acid segment or two or more non-contiguous nucleic acid segments that function as a label or identifier that conveys or is capable of conveying spatial information. In some embodiments, a capture probe includes a spatial barcode that possesses a spatial aspect, where the barcode is associated with a particular location within an array or a particular location on a substrate.

A spatial barcode can be part of an analyte, or independent from an analyte (e.g., part of the capture probe). A spatial barcode can be a tag attached to an analyte (e.g., a nucleic acid molecule) or a combination of a tag in addition to an endogenous characteristic of the analyte (e.g., size of the analyte or end sequence(s)). A spatial barcode can be unique. In some embodiments where the spatial barcode is unique, the spatial barcode functions both as a spatial barcode and as a unique molecular identifier (UMI), associated with one particular capture probe.

Spatial barcodes can have a variety of different formats. For example, spatial barcodes can include polynucleotide spatial barcodes; random nucleic acid and/or amino acid sequences; and synthetic nucleic acid and/or amino acid sequences. In some embodiments, a spatial barcode is attached to an analyte in a reversible or irreversible manner. In some embodiments, a spatial barcode is added to, for example, a fragment of a DNA or RNA sample before, during, and/or after sequencing of the sample. In some embodiments, a spatial barcode allows for identification and/or quantification of individual sequencing-reads. In some embodiments, a spatial barcode is a used as a fluorescent barcode for which fluorescently labeled oligonucleotide probes hybridize to the spatial barcode.

In some embodiments, the spatial barcode is a nucleic acid sequence that does not substantially hybridize to analyte nucleic acid molecules in a biological sample. In some embodiments, the spatial barcode has less than 80% sequence identity (e.g., less than 70%, 60%, 50%, or less than 40% sequence identity) to the nucleic acid sequences across a substantial part (e.g., 80% or more) of the nucleic acid molecules in the biological sample.

The spatial barcode sequences can include from about 6 to about 20 or more nucleotides within the sequence of the capture probes. In some embodiments, the length of a spatial barcode sequence can be about 6, 7, 8, 9, 10, 11, 12, 13, 14, 15, 16, 17, 18, 19, 20 nucleotides or longer. In some embodiments, the length of a spatial barcode sequence can be at least about 6, 7, 8, 9, 10, 11, 12, 13, 14, 15, 16, 17, 18, 19, 20 nucleotides or longer. In some embodiments, the length of a spatial barcode sequence is at most about 6, 7, 8, 9, 10, 11, 12, 13, 14, 15, 16, 17, 18, 19, 20 nucleotides or shorter.

These nucleotides can be completely contiguous, e.g., in a single stretch of adjacent nucleotides, or they can be separated into two or more separate subsequences that are separated by 1 or more nucleotides. Separated spatial barcode subsequences can be from about 4 to about 16 nucleotides in length. In some embodiments, the spatial barcode subsequence can be about 4, 5, 6, 7, 8, 9, 10, 11, 12, 13, 14, 15, 16 nucleotides or longer. In some embodiments, the spatial barcode subsequence can be at least about 4, 5, 6, 7, 8, 9, 10, 11, 12, 13, 14, 15, 16 nucleotides or longer. In some embodiments, the spatial barcode subsequence can be at most about 4, 5, 6, 7, 8, 9, 10, 11, 12, 13, 14, 15, 16 nucleotides or shorter.

For multiple capture probes that are attached to a common array feature, the one or more spatial barcode sequences of the multiple capture probes can include sequences that are the same for all capture probes coupled to the feature, and/or sequences that are different across all capture probes coupled to the feature.

Capture probes attached to a single array feature can include identical (or common) spatial barcode sequences, different spatial barcode sequences, or a combination of both. Capture probes attached to a feature can include multiple sets of capture probes. Capture probes of a given set can include identical spatial barcode sequences. The identical spatial barcode sequences can be different from spatial barcode sequences of capture probes of another set.

The plurality of capture probes can include spatial barcode sequences (e.g., nucleic acid barcode sequences) that are associated with specific locations on a spatial array. For example, a first plurality of capture probes can be

associated with a first region, based on a spatial barcode sequence common to the capture probes within the first region, and a second plurality of capture probes can be associated with a second region, based on a spatial barcode sequence common to the capture probes within the second region. The second region may or may not be associated with the first region. Additional pluralities of capture probes can be associated with spatial barcode sequences common to the capture probes within other regions. In some embodiments, the spatial barcode sequences can be the same across a plurality of capture probe molecules.

In some embodiments, multiple different spatial barcodes are incorporated into a single arrayed capture probe. For example, a mixed but known set of spatial barcode sequences can provide a stronger address or attribution of the spatial barcodes to a given spot or location, by providing duplicate or independent confirmation of the identity of the location. In some embodiments, the multiple spatial barcodes represent increasing specificity of the location of the particular array point.

(v) Unique Molecular Identifier

The capture probe can include one or more (e.g., two or more, three or more, four or more, five or more) Unique Molecular Identifiers (UMIs). A unique molecular identifier is a contiguous nucleic acid segment or two or more non-contiguous nucleic acid segments that function as a label or identifier for a particular analyte, or for a capture probe that binds a particular analyte (e.g., via the capture domain).

A UMI can be unique. A UMI can include one or more specific polynucleotides sequences, one or more random nucleic acid and/or amino acid sequences, and/or one or more synthetic nucleic acid and/or amino acid sequences, or combinations thereof.

In some embodiments, the UMI is a nucleic acid sequence that does not substantially hybridize to analyte nucleic acid molecules in a biological sample. In some embodiments, the UMI has less than 80% sequence identity (e.g., less than 70%, 60%, 50%, or less than 40% sequence identity) to the nucleic acid sequences across a substantial part (e.g., 80% or more) of the nucleic acid molecules in the biological sample.

The UMI can include from about 6 to about 20 or more nucleotides within the sequence of the capture probes. In some embodiments, the length of a UMI sequence can be about 6, 7, 8, 9, 10, 11, 12, 13, 14, 15, 16, 17, 18, 19, 20 nucleotides or longer. In some embodiments, the length of a UMI sequence can be at least about 6, 7, 8, 9, 10, 11, 12, 13, 14, 15, 16, 17, 18, 19, 20 nucleotides or longer. In some embodiments, the length of a UMI sequence is at most about 6, 7, 8, 9, 10, 11, 12, 13, 14, 15, 16, 17, 18, 19, 20 nucleotides or shorter.

These nucleotides can be completely contiguous, i.e., in a single stretch of adjacent nucleotides, or they can be separated into two or more separate subsequences that are separated by 1 or more nucleotides. Separated UMI subsequences can be from about 4 to about 16 nucleotides in length. In some embodiments, the UMI subsequence can be about 4, 5, 6, 7, 8, 9, 10, 11, 12, 13, 14, 15, 16 nucleotides or longer. In some embodiments, the UMI subsequence can be at least about 4, 5, 6, 7, 8, 9, 10, 11, 12, 13, 14, 15, 16 nucleotides or longer. In some embodiments, the UMI subsequence can be at most about 4, 5, 6, 7, 8, 9, 10, 11, 12, 13, 14, 15, 16 nucleotides or shorter.

In some embodiments, a UMI is attached to an analyte in a reversible or irreversible manner. In some embodiments, a UMI is added to, for example, a fragment of a DNA or RNA sample before, during, and/or after sequencing of the analyte. In some embodiments, a UMI allows for identification and/or quantification of individual sequencing-reads. In some embodiments, a UMI is a used as a fluorescent barcode for which fluorescently labeled oligonucleotide probes hybridize to the UMI.

(vi) Other Aspects of Capture Probes

For capture probes that are attached to an array feature, an individual array feature can include one or more capture probes. In some embodiments, an individual array feature includes hundreds or thousands of capture probes. In some embodiments, the capture probes are associated with a particular individual feature, where the individual feature contains a capture probe including a spatial barcode unique to a defined region or location on the array.

In some embodiments, a particular feature can contain capture probes including more than one spatial barcode (e.g., one capture probe at a particular feature can include a spatial barcode that is different than the spatial barcode included in another capture probe at the same particular feature, while both capture probes include a second, common spatial barcode), where each spatial barcode corresponds to a particular defined region or location on the array. For example, multiple spatial barcode sequences associated with one particular feature on an array can provide a stronger address or attribution to a given location by providing duplicate or independent confirmation of the location. In some embodiments, the multiple spatial barcodes represent increasing specificity of the location of the particular array point. In a non-limiting example, a particular array point can be coded with two different spatial barcodes, where each spatial barcode identifies a particular defined region within the array, and an array point possessing both spatial barcodes identifies the sub-region where two defined regions overlap, e.g., such as the overlapping portion of a Venn diagram.

In another non-limiting example, a particular array point can be coded with three different spatial barcodes, where the first spatial barcode identifies a first region within the array, the second spatial barcode identifies a second region, where the second region is a subregion entirely within the first region, and the third spatial barcode identifies a third region, where the third region is a subregion entirely within the first and second subregions.

In some embodiments, capture probes attached to array features are released from the array features for sequencing. Alternatively, in some embodiments, capture probes remain attached to the array features, and the probes are sequenced while remaining attached to the array features (e.g., via in situ sequencing). Further aspects of the sequencing of capture probes are described in subsequent sections of this disclosure.

In some embodiments, an array feature can include different types of capture probes attached to the feature. For example, the array feature can include a first type of capture probe with a capture domain designed to bind to one type of analyte, and a second type of capture probe with a capture domain designed to bind to a second type of analyte. In general, array features can include one or more (e.g., two or more, three or more, four or more, five or more, six or more, eight or more, ten or more, 12 or more, 15 or more, 20 or more, 30 or more, 50 or more) different types of capture probes attached to a single array feature.

In some embodiments, the capture probe is a nucleic acid. In some embodiments, the capture probe is attached to the array feature via its 5' end. In some embodiments, the capture probe includes from the 5' to 3' end: one or more barcodes (e.g., a spatial barcode and/or a UMI) and one or more capture domains. In some embodiments, the capture probe includes from the 5' to 3' end: one barcode (e.g., a spatial barcode or a UMI) and one capture domain. In some embodiments, the capture probe includes from the 5' to 3' end: a cleavage domain, a functional domain, one or more barcodes (e.g., a spatial barcode and/or a UMI), and a capture domain. In some embodiments, the capture probe includes from the 5' to 3' end: a cleavage domain, a functional domain, one or more barcodes (e.g., a spatial barcode and/or a UMI), a second functional domain, and a capture domain. In some embodiments, the capture probe includes from the 5' to 3' end: a cleavage domain, a functional domain, a spatial barcode, a UMI, and a capture domain. In some embodiments, the capture probe does not include a spatial barcode. In some embodiments, the capture probe does not include a UMI. In some embodiments, the capture probe includes a sequence for initiating a sequencing reaction.

In some embodiments, the capture probe is immobilized on a feature via its 3' end. In some embodiments, the capture probe includes from the 3' to 5' end: one or more barcodes (e.g., a spatial barcode and/or a UMI) and one or more capture domains. In some embodiments, the capture probe includes from the 3' to 5' end: one barcode (e.g., a spatial barcode or a UMI) and one capture domain. In some embodiments, the capture probe includes from the 3' to 5' end: a cleavage domain, a functional domain, one or more barcodes (e.g., a spatial barcode and/or a UMI), and a capture domain. In some embodiments, the capture probe includes from the 3' to 5' end: a cleavage domain, a functional domain, a spatial barcode, a UMI, and a capture domain.

In some embodiments, a capture probe includes an in situ synthesized oligonucleotide. The in situ synthesized oligonucleotide can be attached to a substrate, or to a feature on a substrate. In some embodiments, the in situ synthesized oligonucleotide includes one or more constant sequences, one or more of which serves as a priming sequence (e.g., a primer for amplifying target nucleic acids). The in situ synthesized oligonucleotide can, for example, include a constant sequence at the 3'end that is attached to a substrate, or attached to a feature on a substrate. Additionally or alternatively, the in situ synthesized oligonucleotide can include a constant sequence at the free 5' end. In some embodiments, the one or more constant sequences can be a cleavable sequence. In some embodiments, the in situ synthesized oligonucleotide includes a barcode sequence, e.g., a variable barcode sequence. The barcode can be any of the barcodes described herein. The length of the barcode can be approximately 8 to 16 nucleotides (e.g., 8, 9, 10, 11, 12, 13, 14, 15, or 16 nucleotides). The length of the in situ synthesized oligonucleotide can be less than 100 nucleotides (e.g., less than 90, 80, 75, 70, 60, 50, 45, 40, 35, 30, 25 or 20 nucleotides). In some instances, the length of the in situ synthesized oligonucleotide is about 20 to about 40 nucleotides. Exemplary in situ synthesized oligonucleotides are produced by Affymetrix. In some embodiments, the in situ synthesized oligonucleotide is attached to a feature of an array.

Additional oligonucleotides can be ligated to an in situ synthesized oligonucleotide to generate a capture probe. For example, a primer complementary to a portion of the in situ synthesized oligonucleotide (e.g., a constant sequence in the oligonucleotide) can be used to hybridize an additional oligonucleotide and extend (using the in situ synthesized oligonucleotide as a template e.g., a primer extension reaction) to form a double stranded oligonucleotide and to further create a 3' overhang. In some embodiments, the 3' overhang can be created by template-independent ligases (e.g., terminal deoxynucleotidyl transferase (TdT) or poly(A) polymerase). An additional oligonucleotide comprising one or more capture domains can be ligated to the 3' overhang using a suitable enzyme (e.g., a ligase) and a splint oligonucleotide, to generate a capture probe. Thus, in some embodiments, a capture probe is a product of two or more oligonucleotide sequences, (e.g., the in situ synthesized oligonucleotide and the additional oligonucleotide) that are ligated together. In some embodiments, one of the oligonucleotide sequences is an in situ synthesized oligonucleotide.

In some embodiments, the capture probe can be prepared using a splint oligonucleotide (e.g., any of the splint oligonucleotides described herein). Two or more oligonucleotides can be ligated together using a splint oligonucleotide and any variety of ligases known in the art or described herein (e.g., SplintR ligase).

One of the oligonucleotides can include, for example, a constant sequence (e.g., a sequence complementary to a portion of a splint oligonucleotide), a degenerate sequence, and/or a capture domain (e.g., as described herein). One of the oligonucleotides can also include a sequence compatible for ligating or hybridizing to an analyte of interest in the biological sample. An analyte of interest (e.g., an mRNA) can also be used as a splint oligonucleotide to ligate further oligonucleotides onto the capture probe. In some embodiments, the capture probe is generated by having an enzyme add polynucleotides at the end of an oligonucleotide sequence. The capture probe can include a degenerate sequence, which can function as a unique molecular identifier.

A degenerate sequence, which is a sequence in which some positions of a nucleotide sequence contain a number of possible bases. A degenerate sequence can be a degenerate nucleotide sequence including about or at least 5, 6, 7, 8, 9, 10, 11, 12, 13, 14, 15, 16, 17, 18, 19, 20, 25, 30, 35, 40, 45, or 50 nucleotides. In some embodiments, a nucleotide sequence contains 1, 2, 3, 4, 5, 6, 7, 8, 9, 10, 15, 20, 25, or more degenerate positions within the nucleotide sequence. In some embodiments, the degenerate sequence is used as a UMI.

In some embodiments, a capture probe includes a restriction endonuclease recognition sequence or a sequence of nucleotides cleavable by specific enzyme activities. For example, uracil sequences can be enzymatically cleaved from a nucleotide sequence using uracil DNA glycosylase (UDG) or Uracil Specific Excision Reagent (USER). As another example, other modified bases (e.g., modified by methylation) can be recognized and cleaved by specific endonucleases. The capture probes can be subjected to an enzymatic cleavage, which removes the blocking domain and any of the additional nucleotides that are added to the 3' end of the capture probe during the modification process. Removal of the blocking domain reveals and/or restores the free 3' end of the capture domain of the capture probe. In some embodiments, additional nucleotides can be removed to reveal and/or restore the 3' end of the capture domain of the capture probe.

In some embodiments, a blocking domain can be incorporated into the capture probe when it is synthesized, or after its synthesis. The terminal nucleotide of the capture domain is a reversible terminator nucleotide (e.g., 3'-O-blocked reversible terminator and 3'-unblocked reversible terminator), and can be included in the capture probe during or after probe synthesis.

(vii) Extended Capture Probes

An "extended capture probe" is a capture probe with an enlarged nucleic acid sequence. For example, where the capture probe includes nucleic acid, an "extended 3' end" indicates that further nucleotides were added to the most 3' nucleotide of the capture probe to extend the length of the capture probe, for example, by standard polymerization reactions utilized to extend nucleic acid molecules including templated polymerization catalyzed by a polymerase (e.g., a DNA polymerase or reverse transcriptase).

In some embodiments, extending the capture probe includes generating cDNA from the captured (hybridized) RNA. This process involves synthesis of a complementary strand of the hybridized nucleic acid, e.g., generating cDNA based on the captured RNA template (the RNA hybridized to the capture domain of the capture probe). Thus, in an initial step of extending the capture probe, e.g., the cDNA generation, the captured (hybridized) nucleic acid, e.g., RNA, acts as a template for the extension, e.g., reverse transcription, step.

In some embodiments, the capture probe is extended using reverse transcription. For example, reverse transcription includes synthesizing cDNA (complementary or copy DNA) from RNA, e.g., (messenger RNA), using a reverse transcriptase. In some embodiments, reverse transcription is performed while the tissue is still in place, generating an analyte library, where the analyte library includes the spatial barcodes from the adjacent capture probes. In some embodiments, the capture probe is extended using one or more DNA polymerases.

In some embodiments, the capture domain of the capture probe includes a primer for producing the complementary strand of the nucleic acid hybridized to the capture probe, e.g., a primer for DNA polymerase and/or reverse transcription. The nucleic acid, e.g., DNA and/or cDNA, molecules generated by the extension reaction incorporate the sequence of the capture probe. The extension of the capture probe, e.g., a DNA polymerase and/or reverse transcription reaction, can be performed using a variety of suitable enzymes and protocols.

In some embodiments, a full-length DNA, e.g., cDNA, molecule is generated. In some embodiments, a "full-length" DNA molecule refers to the whole of the captured nucleic acid molecule. However, if the nucleic acid, e.g., RNA, was partially degraded in the tissue sample, then the captured nucleic acid molecules will not be the same length as the initial RNA in the tissue sample. In some embodiments, the 3' end of the extended probes, e.g., first strand cDNA molecules, is modified. For example, a linker or adaptor can be ligated to the 3' end of the extended probes. This can be achieved using single stranded ligation enzymes such as T4 RNA ligase or Circligase™ (available from Lucigen, Middleton, WI). In some embodiments, template switching oligonucleotides are used to extend cDNA in order to generate a full-length cDNA (or as close to a full-length cDNA as possible). In some embodiments, a second strand synthesis helper probe (a partially double stranded DNA molecule capable of hybridizing to the 3' end of the extended capture probe), can be ligated to the 3' end of the extended probe, e.g., first strand cDNA, molecule using a double stranded ligation enzyme such as T4 DNA ligase. Other enzymes appropriate for the ligation step are known in the art and include, e.g., Tth DNA

ligase, Taq DNA ligase, *Thermococcus* sp. (strain 9°N) DNA ligase (9°N™ DNA ligase, New England Biolabs), Ampligase™ (available from Lucigen, Middleton, WI), and SplintR (available from New England Biolabs, Ipswich, MA). In some embodiments, a polynucleotide tail, e.g., a poly(A) tail, is incorporated at the 3' end of the extended probe molecules. In some embodiments, the polynucleotide tail is incorporated using a terminal transferase active enzyme.

In some embodiments, double-stranded extended capture probes are treated to remove any unextended capture probes prior to amplification and/or analysis, e.g., sequence analysis. This can be achieved by a variety of methods, e.g., using an enzyme to degrade the unextended probes, such as an exonuclease enzyme, or purification columns.

In some embodiments, extended capture probes are amplified to yield quantities that are sufficient for analysis, e.g., via DNA sequencing. In some embodiments, the first strand of the extended capture probes (e.g., DNA and/or cDNA molecules) acts as a template for the amplification reaction (e.g., a polymerase chain reaction).

In some embodiments, the amplification reaction incorporates an affinity group onto the extended capture probe (e.g., RNA-cDNA hybrid) using a primer including the affinity group. In some embodiments, the primer includes an affinity group and the extended capture probes includes the affinity group. The affinity group can correspond to any of the affinity groups described previously.

In some embodiments, the extended capture probes including the affinity group can be coupled to an array feature specific for the affinity group. In some embodiments, the substrate can include an antibody or antibody fragment. In some embodiments, the array feature includes avidin or streptavidin and the affinity group includes biotin. In some embodiments, the array feature includes maltose and the affinity group includes maltose-binding protein. In some embodiments, the array feature includes maltose-binding protein and the affinity group includes maltose. In some embodiments, amplifying the extended capture probes can function to release the extended probes from the array feature, insofar as copies of the extended probes are not attached to the array feature.

In some embodiments, the extended capture probe or complement or amplicon thereof is released from an array feature. The step of releasing the extended capture probe or complement or amplicon thereof from an array feature can be achieved in a number of ways. In some embodiments, an extended capture probe or a complement thereof is released from the feature by nucleic acid cleavage and/or by denaturation (e.g., by heating to denature a double-stranded molecule).

In some embodiments, the extended capture probe or complement or amplicon thereof is released from the array feature by physical means. For example, methods for inducing physical release include denaturing double stranded nucleic acid molecules. Another method for releasing the extended capture probes is to use a solution that interferes with the hydrogen bonds of the double stranded molecules. In some embodiments, the extended capture probe is released by applying heated water such as water or buffer of at least 85°C, e.g., at least 90, 91, 92, 93, 94, 95, 96, 97, 98, or 99°C. In some embodiments, a solution including salts, surfactants, etc. that can further destabilize the interaction between the nucleic acid molecules is added to release the extended capture probe from the array feature. In some embodiments, a formamide solution can be used to destabilize the interaction between nucleic acid molecules to release the extended capture probe from the array feature.

(viii) Amplification of Capture Probes

In some embodiments, methods are provided herein for amplifying a capture probe affixed to a spatial array, where amplification of the capture probe increases the number of capture domains and spatial barcodes on the spatial array. In some embodiments where a capture probe is amplified, the amplification is performed by rolling circle amplification. In some embodiments, the capture probe to be amplified includes sequences (e.g., docking sequences, functional sequences, and/or primer sequences) that enable rolling circle amplification. In one example, the capture probe can include a functional sequence that is capable of binding to a primer used for amplification. In another example, the capture probe can include one or more docking sequences (e.g., a first docking sequence and a second docking sequence) that can hybridize to one or more oligonucleotides (e.g., a padlock probe(s)) used for rolling circle amplification. In some embodiments, additional probes are affixed to the substrate, where the additional probes include sequences (e.g., a docking sequence(s), a functional sequence(s), and/or a primer sequence(s)) that enable rolling circle amplification. In some embodiments, the spatial array is contacted with an oligonucleotide (e.g., a padlock probe). As used herein, a "padlock probe" refers to an oligonucleotide that has, at its 5' and 3' ends, sequences that are complementary to adjacent or nearby target sequences (e.g., docking sequences) on a capture probe. Upon hybridization to the target sequences (e.g., docking sequences), the two ends of the padlock probe are either brought into contact or an end is extended until the two ends are brought into contact, allowing circularization of the padlock probe by ligation (e.g., ligation using any of the methods described herein). In some embodiments, after circularization of the oligonucleotide, rolling circle amplification can be used to amplify the ligation product, which includes at least a capture domain and a spatial barcode from the capture probe. In some embodiments, amplification of the capture probe using a padlock oligonucleotide and rolling circle amplification increases the number of capture domains and the number of spatial barcodes on the spatial array.

In some embodiments, a method of increasing capture efficiency of a spatial array includes amplifying all or part of a capture probe affixed to a substrate. For example, amplification of all or part of the capture probes affixed to the substrate can increase the capture efficiency of the spatial array by increasing the number of capture domains and spatial barcodes. In some embodiments, a method of determining a location of an analyte in a biological sample includes using a spatial array having increased capture efficiency (e.g., a spatial array where a capture probe has been amplified as described herein). For example, the capture efficiency of a spatial array can be increased by amplification of all or part of the capture probe prior to contact with a biological sample. The amplification results in an increased number of capture domains that enable capture of more analytes as compared to a spatial array where the capture probe was not amplified prior to contacting the biological sample. In some embodiments, a method of producing a spatial array that has increased capture efficiency includes amplifying all or part of a capture probe. In some embodiments where a spatial array having increased capture efficiency is produced by amplifying all or part of a capture probe, the amplification increases the number of capture domains and the number of spatial barcodes on the spatial array. In some embodiments, a method of determining the location of a capture probe (i.e., a capture probe on a feature) on a spatial array includes amplifying all or part of a capture probe. For example, amplification of the capture probe affixed to the substrate can increase the number of spatial barcodes used for direct decoding (e.g., direct decoding using any of the methods described herein including, without limitation, in situ sequencing) of the location of the capture probe.

(ix) Analyte Capture Agents

This disclosure also provides methods and materials for using analyte capture agents for spatial profiling of biological analytes (e.g., mRNA, genomic DNA, accessible chromatin, and cell surface or intracellular proteins and/or metabolites). As used herein, an "analyte capture agent" (also referred to previously at times as a "cell labelling" agent") refers to an agent that interacts with an analyte (e.g., an analyte in a sample) and with a capture probe (e.g., a capture probe attached to a substrate) to identify the analyte. In some embodiments, the analyte capture agent includes an analyte binding moiety and a capture agent barcode domain.

As used herein, the term "analyte binding moiety" refers to a molecule or moiety capable of binding to a macromolecular constituent (e.g., an analyte, e.g., a biological analyte). In some embodiments of any of the spatial profiling methods described herein, the analyte binding moiety of the analyte capture agent that binds to a biological analyte can include, but is not limited to, an antibody, or an epitope binding fragment thereof, a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The analyte binding moiety can bind to the macromolecular constituent (e.g., analyte) with high affinity and/or with high specificity. The analyte binding moiety can include a nucleotide sequence (e.g., an oligonucleotide), which can correspond to at least a portion or an entirety of the analyte binding moiety. The analyte binding moiety can include a polypeptide and/or an aptamer (e.g., a polypeptide and/or an aptamer that binds to a specific target molecule, e.g., an analyte). The analyte binding moiety can include an antibody or antibody fragment (e.g., an antigen-binding fragment) that binds to a specific analyte (e.g., a polypeptide).

In some embodiments, an analyte binding moiety of an analyte capture agent includes one or more antibodies or antigen binding fragments thereof. The antibodies or antigen binding fragments including the analyte binding moiety can specifically bind to a target analyte. In some embodiments, the analyte is a protein (e.g., a protein on a surface of the biological sample (e.g., a cell) or an intracellular protein). In some embodiments, a plurality of analyte capture agents comprising a plurality of analyte binding moieties bind a plurality of analytes present in a biological sample. In some embodiments, the plurality of analytes includes a single species of analyte (e.g., a single species of polypeptide). In some embodiments in which the plurality of analytes includes a single species of analyte, the analyte binding moieties of the plurality of analyte capture agents are the same. In some embodiments in which the plurality of analytes includes a single species of analyte, the analyte binding moieties of the plurality of analyte capture agents are the different (e.g., members of the plurality of analyte capture agents can have two or more species of analyte binding moieties, wherein each of the two or more species of analyte binding moieties binds a single species of analyte, e.g., at different binding sites). In some embodiments, the plurality of analytes includes multiple different species of analyte (e.g., multiple different species of polypeptides).

An analyte capture agent can include an analyte binding moiety. The analyte binding moiety can be an antibody. Exemplary, non-limiting antibodies that can be used as analyte binding moieties in an analyte capture agent or that can be used in the IHC/IF applications disclosed herein include any of the following including variations thereof: A-ACT,, A-AT, ACTH, Actin-Muscle-specific, Actin-Smooth Muscle (SMA), AE1, AE1/AE3, AE3, AFP, AKT Phosphate, ALK-1, Amyloid A, Androgen Receptor, Annexin A1, B72.3, BCA-225, BCL-1 (Cyclin D1), BCL-1/CD20, BCL-2, BCL-2/BCL-6, BCL-6, Ber-EP4, Beta-amyloid, Beta-catenin, BG8 (Lewis Y), BOB-1, CA 19.9, CA 125, CAIX, Calcitonin, Caldesmon, Calponin, Calretinin, CAM 5.2, CAM 5.2/AE1, CD1a, CD2, CD3 (M), CD3 (P), CD3/CD20, CD4, CD5, CD7, CD8, CD10, CD14, CD15, CD20, CD21, CD22, CD 23, CD25, CD30, CD31, CD33, CD34, CD35, CD43, CD45 (LCA), CD45RA, CD56, CD57,

CD61, CD68, CD71, CD74, CD79a, CD99, CD117 (c-KIT), CD123, CD138, CD163, CDX-2, CDX-2/CK-7, CEA (M), CEA (P), Chromogranin A, Chymotrypsin, CK-5, CK-5/6, CK-7, CK-7/TTF-1, CK-14, CK-17, CK-18, CK-19, CK-20, CK-HMW, CK-LMW, CMV-IH, COLL-IV, COX-2, D2-40, DBA44, Desmin, DOG1, EBER-ISH, EBV (LMP1), E-Cadherin, EGFR, EMA, ER, ERCC1, Factor VIII (vWF), Factor XIIIa, Fascin, FLI-1, FHS, Galectin-3, Gastrin, GCDFP-15, GFAP, Glucagon, Glycophorin A, Glypican-3, Granzyme B, Growth Hormone (GH), GST, HAM 56, HMBE-1, HBP, HCAg, HCG, Hemoglobin A, HEP B CORE (HBcAg), HEP B SURF, (HBsAg), HepPar1, HER2, Herpes I, Herpes II, HHV-8, HLA-DR, HMB 45, HPL, HPV-IHC, HPV (6/11)-ISH, HPV (16/18)-ISH, HPV (31/33)-ISH, HPV WSS-ISH, HPV High-ISH, HPV Low-ISH, HPV High & Low-ISH, IgA, IgD, IgG, IgG4, IgM, Inhibin, Insulin, JC Virus-ISH, Kappa-ISH, KER PAN, Ki-67, Lambda-IHC, Lambda-ISH, LH, Lipase, Lysozyme (MURA), Mammaglobin, MART-1, MBP, M-Cell Tryptase, MEL-5, Melan-A,, Melan-A/Ki-67, Mesothelin, MiTF, MLH-1, MOC-31, MPO, MSH-2, MSH-6, MUC1, MUC2, MUC4, MUC5AC, MUM-1, MYO D1, Myogenin, Myoglobin, Myoin Heavy Chain, Napsin A, NB84a, NEW-N, NF, NK1-C3, NPM, NSE, OCT-2, OCT-3/4, OSCAR, p16, p21, p27/Kip1, p53, p57, p63, p120, P504S, Pan Melanoma, PANC.POLY, Parvovirus B19, PAX-2, PAX-5, PAX-5/CD43, PAX=5/CD5, PAX-8, PC, PD1, Perforin, PGP 9.5, PLAP, PMS-2, PR, Prolactin, PSA, PSAP, PSMA, PTEN, PTH, PTS, RB, RCC, S6, S100, Serotonin, Somatostatin, Surfactant (SP-A), Synaptophysin, Synuclein, TAU, TCL-1, TCR beta, TdT, Thrombomodulin, Thyroglobulin, TIA-1, TOXO, TRAP, TriView™ breast, TriView™ prostate, Trypsin, TS, TSH, TTF-1, Tyrosinase, Ubiqutin, Uroplakin, VEGF, Villin, Vimentin (VIM), VIP, VZV, WT1 (M) N-Terminus, WT1 (P) C-Terminus, ZAP-70.

Further, exemplary, non-limiting antibodies that can be used as analyte binding moieties in an analyte capture agent or that can be used in the IHC/IF applications disclosed herein include any of the following antibodies (and variations thereof) to: cell surface proteins, intracellular proteins, kinases (e.g., AGC kinase family (e.g., AKT1, AKT2, PDK1, Protein Kinase C, ROCK1, ROCK2, SGK3), CAMK kinase family (e.g., AMPK1, AMPK2, CAMK, Chk1, Chk2, Zip), CK1 kinase family, TK kinase family (e.g., Abl2, AXL, CD167, CD246/ALK, c-Met, CSK, c-Src, EGFR, ErbB2 (HER2/neu), ErbB3, ErbB4, FAK, Fyn, LCK, Lyn, PKT7, Syk, Zap70), STE kinase family (e.g., ASK1, MAPK, MEK1, MEK2, MEK3 MEK4, MEK5, PAK1, PAK2, PAK4, PAK6), CMGC kinase family (e.g., Cdk2, Cdk4, Cdk5, Cdk6, Cdk7, Cdk9, Erk1, GSK3, Jnk/MAPK8, Jnk2/MAPK9, JNK3/MAPK10, p38/MAPK), and TKL kinase family (e.g., ALK1, ILK1, IRAK1, IRAK2, IRAK3, IRAK4, LIMK1, LIMK2, M3K11, RAF1, RIP1, RIP3, VEGFR1, VEGFR2, VEGFR3), Aurora A kinase, Aurora B kinase, IKK, Nemo-like kinase, PINK, PLK3, ULK2, WEE1, transcription factors (e.g., FOXP3, ATF3, BACH1, EGR, ELF3, FOXA1, FOXA2, FOX01, GATA), growth factor receptors, tumor suppressors (e.g., anti-p53, anti-BLM, anti-Cdk2, anti-Chk2, anti-BRCA-1, anti-NBS1, anti-BRCA-2, anti-WRN, anti-PTEN, anti-WT1, anti-p38).

In some embodiments, analyte capture agents are capable of binding to analytes present inside a cell. In some embodiments, analyte capture agents are capable of binding to cell surface analytes that can include, without limitation, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, an extracellular matrix protein, a posttranslational modification (e.g., phosphorylation, glycosylation, ubiquitination, nitrosylation, methylation, acetylation or lipidation) state of a cell surface protein, a gap junction, and an adherens junction. In some embodiments, the analyte capture agents are capable of binding to cell surface analytes that are post-translationally modified. In such embodiments, analyte capture agents can be specific for cell surface analytes based on a given state of posttranslational modification (e.g., phosphorylation, glycosylation, ubiquitination, nitrosylation, methylation, acetylation or lipidation), such that a cell surface analyte profile can include posttranslational modification information of one or more analytes.

In some embodiments, the analyte capture agent includes a capture agent barcode domain that is conjugated or otherwise attached to the analyte binding moiety. In some embodiments, the capture agent barcode domain is covalently-linked to the analyte binding moiety. In some embodiments, a capture agent barcode domain is a nucleic acid sequence. In some embodiments, a capture agent barcode domain includes an analyte binding moiety barcode and an analyte capture sequence.

As used herein, the term "analyte binding moiety barcode" refers to a barcode that is associated with or otherwise identifies the analyte binding moiety. In some embodiments, by identifying an analyte binding moiety and its associated analyte binding moiety barcode, the analyte to which the analyte binding moiety binds can also be identified. An analyte binding moiety barcode can be a nucleic acid sequence of a given length and/or sequence that is associated with the analyte binding moiety. An analyte binding moiety barcode can generally include any of the variety of aspects of barcodes described herein. For example, an analyte capture agent that is specific to one type of analyte can have coupled thereto a first capture agent barcode domain (e.g., that includes a first analyte binding moiety barcode), while an analyte capture agent that is specific to a different analyte can have a different capture agent barcode domain (e.g., that includes a second barcode analyte binding moiety barcode) coupled thereto. In some aspects, such a capture agent barcode domain can include an analyte binding moiety barcode that permits identification of the analyte binding moiety to which the capture agent barcode domain is coupled. The selection of the capture agent barcode domain can allow significant diversity in terms of sequence, while also being readily attachable to most analyte binding moieties (e.g., antibodies or aptamers) as

well as being readily detected, (e.g., using sequencing or array technologies).

In some embodiments, the capture agent barcode domain of an analyte capture agent includes an analyte capture sequence. As used herein, the term "analyte capture sequence" refers to a region or moiety configured to hybridize to, bind to, couple to, or otherwise interact with a capture domain of a capture probe. In some embodiments, an analyte capture sequence includes a nucleic acid sequence that is complementary to or substantially complementary to the capture domain of a capture probe such that the analyte capture sequence hybridizes to the capture domain of the capture probe. In some embodiments, an analyte capture sequence comprises a poly(A) nucleic acid sequence that hybridizes to a capture domain that comprises a poly(T) nucleic acid sequence. In some embodiments, an analyte capture sequence comprises a poly(T) nucleic acid sequence that hybridizes to a capture domain that comprises a poly(A) nucleic acid sequence. In some embodiments, an analyte capture sequence comprises a non-homopolymeric nucleic acid sequence that hybridizes to a capture domain that comprises a non-homopolymeric nucleic acid sequence that is complementary (or substantially complementary) to the non-homopolymeric nucleic acid sequence of the analyte capture region.

In some embodiments of any of the spatial analysis methods described herein that employ an analyte capture agent, the capture agent barcode domain can be directly coupled to the analyte binding moiety, or they can be attached to a bead, molecular lattice, e.g., a linear, globular, cross-slinked, or other polymer, or other framework that is attached or otherwise associated with the analyte binding moiety, which allows attachment of multiple capture agent barcode domains to a single analyte binding moiety. Attachment (coupling) of the capture agent barcode domains to the analyte binding moieties can be achieved through any of a variety of direct or indirect, covalent or non-covalent associations or attachments. For example, in the case of a capture agent barcode domain coupled to an analyte binding moiety that includes an antibody or antigen-binding fragment, such capture agent barcode domains can be covalently attached to a portion of the antibody or antigen-binding fragment using chemical conjugation techniques (e.g., Lightning-Link® antibody labelling kits available from Innova Biosciences). In some embodiments, a capture agent barcode domain can be coupled to an antibody or antigen-binding fragment using non-covalent attachment mechanisms (e.g., using biotinylated antibodies and oligonucleotides or beads that include one or more biotinylated linker(s), coupled to oligonucleotides with an avidin or streptavidin linker.) Antibody and oligonucleotide biotinylation techniques can be used, and are described for example in Fang et al., Nucleic Acids Res. (2003), 31(2): 708-715, the entire contents of which are incorporated by reference herein. Likewise, protein and peptide biotinylation techniques have been developed and can be used, and are described for example in U.S. Patent No. 6,265,552, the entire contents of which are incorporated by reference herein. Furthermore, click reaction chemistry such as a methyltetrazine-PEG5-NHS ester reaction, a TCO-PEG4-NHS ester reaction, or the like, can be used to couple capture agent barcode domains to analyte binding moieties. The reactive moiety on the analyte binding moiety can also include amine for targeting aldehydes, amine for targeting maleimide (e.g., free thiols), azide for targeting click chemistry compounds (e.g., alkynes), biotin for targeting streptavidin, phosphates for targeting EDC, which in turn targets active ester (e.g., NH2). The reactive moiety on the analyte binding moiety can be a chemical compound or group that binds to the reactive moiety on the analyte binding moiety. Exemplary strategies to conjugate the analyte binding moiety to the capture agent barcode domain include the use of commercial kits (e.g., Solulink, Thunder link), conjugation of mild reduction of hinge region and maleimide labelling, stain-promoted click chemistry reaction to labeled amides (e.g., copper-free), and conjugation of periodate oxidation of sugar chain and amine conjugation. In the cases where the analyte binding moiety is an antibody, the antibody can be modified prior to or contemporaneously with conjugation of the oligonucleotide. For example, the antibody can be glycosylated with a chemical substrate-permissive mutant of β-1,4-galactosyltransferase, GalT (Y289L) and azide-bearing uridine diphosphate-N-acetylgalactosamine analog uridine diphosphate -GalNAz. The modified antibody can be conjugated to an oligonucleotide with a dibenzocyclooctyne-PEG4-NHS group. In some embodiments, certain steps (e.g., COOH activation (e.g., EDC) and homobifunctional cross linkers) can be avoided to prevent the analyte binding moieties from conjugating to themselves. In some embodiments of any of the spatial profiling methods described herein, the analyte capture agent (e.g., analyte binding moiety coupled to an oligonucleotide) can be delivered into the cell, e.g., by transfection (e.g., using transfectamine, cationic polymers, calcium phosphate or electroporation), by transduction (e.g., using a bacteriophage or recombinant viral vector), by mechanical delivery (e.g., magnetic beads), by lipid (e.g., 1,2-Dioleoyl-sn-glycero-3-phosphocholine (DOPC)), or by transporter proteins. An analyte capture agent can be delivered into a cell using exosomes. For example, a first cell can be generated that releases exosomes comprising an analyte capture agent. An analyte capture agent can be attached to an exosome membrane. An analyte capture agent can be contained within the cytosol of an exosome. Released exosomes can be harvested and provided to a second cell, thereby delivering the analyte capture agent into the second cell. An analyte capture agent can be releasable from an exosome membrane before, during, or after delivery into a cell. In some embodiments, the cell is permeabilized to allow the analyte capture agent to couple with intracellular constituents (such as, without limitation, intracellular proteins, metabolites, and nuclear membrane proteins). Following intracellular delivery, analyte capture agents can be used to analyze intracellular constituents as described herein.

In some embodiments of any of the spatial profiling methods described herein, the capture agent barcode domain coupled to an analyte capture agent can include modifications that render it non-extendable by a polymerase. In some

embodiments, when binding to a capture domain of a capture probe or nucleic acid in a sample for a primer extension reaction, the capture agent barcode domain can serve as a template, not a primer. When the capture agent barcode domain also includes a barcode (e.g., an analyte binding moiety barcode), such a design can increase the efficiency of molecular barcoding by increasing the affinity between the capture agent barcode domain and unbarcoded sample nucleic acids, and eliminate the potential formation of adaptor artifacts. In some embodiments, the capture agent barcode domain can include a random N-mer sequence that is capped with modifications that render it non-extendable by a polymerase. In some cases, the composition of the random N-mer sequence can be designed to maximize the binding efficiency to free, unbarcoded ssDNA molecules. The design can include a random sequence composition with a higher GC content, a partial random sequence with fixed G or C at specific positions, the use of guanosines, the use of locked nucleic acids, or any combination thereof.

A modification for blocking primer extension by a polymerase can be a carbon spacer group of different lengths or a dideoxynucleotide. In some embodiments, the modification can be an abasic site that has an apurine or apyrimidine structure, a base analog, or an analogue of a phosphate backbone, such as a backbone of N-(2-aminoethyl)-glycine linked by amide bonds, tetrahydrofuran, or 1', 2'-Dideoxyribose. The modification can also be a uracil base, 2'OMe modified RNA, C3-18 spacers (e.g., structures with 3-18 consecutive carbon atoms, such as C3 spacer), ethylene glycol multimer spacers (e.g., spacer 18 (hexaethyleneglycol spacer), biotin, di-deoxynucleotide triphosphate, ethylene glycol, amine, or phosphate.

In some embodiments of any of the spatial profiling methods described herein, the capture agent barcode domain coupled to the analyte binding moiety includes a cleavable domain. For example, after the analyte capture agent binds to an analyte (e.g., a cell surface analyte), the capture agent barcode domain can be cleaved and collected for downstream analysis according to the methods as described herein. In some embodiments, the cleavable domain of the capture agent barcode domain includes a U-excising element that allows the species to release from the bead. In some embodiments, the U-excising element can include a single-stranded DNA (ssDNA) sequence that contains at least one uracil. The species can be attached to a bead via the ssDNA sequence. The species can be released by a combination of uracil-DNA glycosylase (e.g., to remove the uracil) and an endonuclease (e.g., to induce an ssDNA break). If the endonuclease generates a 5' phosphate group from the cleavage, then additional enzyme treatment can be included in downstream processing to eliminate the phosphate group, e.g., prior to ligation of additional sequencing handle elements, e.g., Illumina full P5 sequence, partial P5 sequence, full R1 sequence, and/or partial R1 sequence.

In some embodiments, multiple different species of analytes (e.g., polypeptides) from the biological sample can be subsequently associated with the one or more physical properties of the biological sample. For example, the multiple different species of analytes can be associated with locations of the analytes in the biological sample. Such information (e.g., proteomic information when the analyte binding moiety(ies) recognizes a polypeptide(s)) can be used in association with other spatial information (e.g., genetic information from the biological sample, such as DNA sequence information, transcriptome information (i.e., sequences of transcripts), or both). For example, a cell surface protein of a cell can be associated with one or more physical properties of the cell (e.g., a shape, size, activity, or a type of the cell). The one or more physical properties can be characterized by imaging the cell. The cell can be bound by an analyte capture agent comprising an analyte binding moiety that binds to the cell surface protein and an analyte binding moiety barcode that identifies that analyte binding moiety, and the cell can be subjected to spatial analysis (e.g., any of the variety of spatial analysis methods described herein). For example, the analyte capture agent bound to the cell surface protein can be bound to a capture probe (e.g., a capture probe on an array), which capture probe includes a capture domain that interacts with an analyte capture sequence present on the capture agent barcode domain of the analyte capture agent. All or part of the capture agent barcode domain (including the analyte binding moiety barcode) can be copied with a polymerase using a 3' end of the capture domain as a priming site, generating an extended capture probe that includes the all or part of complementary sequence that corresponds to the capture probe (including a spatial barcode present on the capture probe) and a copy of the analyte binding moiety barcode. In some embodiments, an analyte capture agent with an extended capture agent barcode domain that includes a sequence complementary to a spatial barcode of a capture probe is called a "spatially-tagged analyte capture agent."

In some embodiments, the spatial array with spatially-tagged analyte capture agents can be contacted with a sample, where the analyte capture agent(s) associated with the spatial array capture the target analyte(s). The analyte capture agent(s) containing the extended capture probe(s), which includes a sequence complementary to the spatial barcode(s) of the capture probe(s) and the analyte binding moiety barcode(s), can then be denatured from the capture probe(s) of the spatial array. This allows the spatial array to be reused. The sample can be dissociated into non-aggregated cells (e.g., single cells) and analyzed by the single cell / droplet methods described herein. The spatially-tagged analyte capture agent can be sequenced to obtain the nucleic acid sequence of the spatial barcode of the capture probe and the analyte binding moiety barcode of the analyte capture agent. The nucleic acid sequence of the extended capture probe can thus be associated with an analyte (e.g., cell surface protein), and in turn, with the one or more physical properties of the cell (e.g., a shape or cell type). In some embodiments, the nucleic acid sequence of the extended capture probe can be associated with an intracellular analyte of a nearby cell, where the intracellular analyte was released using any of the cell

permeabilization or analyte migration techniques described herein.

In some embodiments of any of the spatial profiling methods described herein, the capture agent barcode domains released from the analyte capture agents can then be subjected to sequence analysis to identify which analyte capture agents were bound to analytes. Based upon the capture agent barcode domains that are associated with a feature (e.g., a feature at a particular location) on a spatial array and the presence of the analyte binding moiety barcode sequence, an analyte profile can be created for a biological sample. Profiles of individual cells or populations of cells can be compared to profiles from other cells, e.g., 'normal' cells, to identify variations in analytes, which can provide diagnostically relevant information. In some embodiments, these profiles can be useful in the diagnosis of a variety of disorders that are characterized by variations in cell surface receptors, such as cancer and other disorders.

In some embodiments of any of the spatial profiling methods described herein, the methods are used to identify immune cell profiles. Immune cells express various adaptive immunological receptors relating to immune function, such as T cell receptors (TCRs) and B cell receptors (BCRs). T cell receptors and B cell receptors play a part in the immune response by specifically recognizing and binding to antigens and aiding in their destruction.

The T cell receptor, or TCR, is a molecule found on the surface of T cells that is generally responsible for recognizing fragments of antigen as peptides bound to major histocompatibility complex (MHC) molecules. The TCR is generally a heterodimer of two chains, each of which is a member of the immunoglobulin superfamily, possessing an N-terminal variable (V) domain, and a C terminal constant domain. In humans, in 95% of T cells, the TCR consists of an alpha (a) and beta (β) chain, whereas in 5% of T cells, the TCR consists of gamma and delta (γ/δ) chains. This ratio can change during ontogeny and in diseased states as well as in different species. When the TCR engages with antigenic peptide and MHC (peptide/MHC or pMHC), the T lymphocyte is activated through signal transduction.

Each of the two chains of a TCR contains multiple copies of gene segments - a variable 'V' gene segment, a diversity 'D' gene segment, and a joining 'J' gene segment. The TCR alpha chain (TCRa) is generated by recombination of V and J segments, while the beta chain (TCRb) is generated by recombination of V, D, and J segments. Similarly, generation of the TCR gamma chain involves recombination of V and J gene segments, while generation of the TCR delta chain occurs by recombination of V, D, and J gene segments. The intersection of these specific regions (V and J for the alpha or gamma chain, or V, D and J for the beta or delta chain) corresponds to the CDR3 region that is important for antigen-MHC recognition. Complementarity determining regions (e.g., CDR1, CDR2, and CDR3), or hypervariable regions, are sequences in the variable domains of antigen receptors (e.g., T cell receptor and immunoglobulin) that can complement an antigen. Most of the diversity of CDRs is found in CDR3, with the diversity being generated by somatic recombination events during the development of T lymphocytes. A unique nucleotide sequence that arises during the gene arrangement process can be referred to as a clonotype.

The B cell receptor, or BCR, is a molecule found on the surface of B cells. The antigen binding portion of a BCR is composed of a membrane-bound antibody that, like most antibodies (e.g., immunoglobulins), has a unique and randomly determined antigen-binding site. The antigen binding portion of a BCR includes membrane-bound immunoglobulin molecule of one isotype (e.g., IgD, IgM, IgA, IgG, or IgE). When a B cell is activated by its first encounter with a cognate antigen, the cell proliferates and differentiates to generate a population of antibody-secreting plasma B cells and memory B cells. The various immunoglobulin isotypes differ in their biological features, structure, target specificity, and distribution. A variety of molecular mechanisms exist to generate initial diversity, including genetic recombination at multiple sites.

The BCR is composed of two genes IgH and IgK (or IgL) coding for antibody heavy and light chains. Immunoglobulins are formed by recombination among gene segments, sequence diversification at the junctions of these segments, and point mutations throughout the gene. Each heavy chain gene contains multiple copies of three different gene segments - a variable 'V' gene segment, a diversity 'D' gene segment, and a joining 'J' gene segment. Each light chain gene contains multiple copies of two different gene segments for the variable region of the protein - a variable 'V' gene segment and a joining 'J' gene segment.

The recombination can generate a molecule with one of each of the V, D, and J segments. Furthermore, several bases can be deleted and others added (called N and P nucleotides) at each of the two junctions, thereby generating further diversity. After B cell activation, a process of affinity maturation through somatic hypermutation occurs. In this process, progeny cells of the activated B cells accumulate distinct somatic mutations throughout the gene with higher mutation concentration in the CDR regions leading to the generation of antibodies with higher affinity to the antigens.

In addition to somatic hypermutation, activated B cells undergo the process of isotype switching. Antibodies with the same variable segments can have different forms (isotypes) depending on the constant segment. Whereas all naive B cells express IgM (or IgD), activated B cells mostly express IgG but also IgM, IgA, and IgE. This expression switching from IgM (and/or IgD) to IgG, IgA, or IgE occurs through a recombination event causing one cell to specialize in producing a specific isotype. A unique nucleotide sequence that arises during the gene arrangement process can similarly be referred to as a clonotype.

Certain methods described herein are utilized to analyze the various sequences of TCRs and BCRs from immune cells, for example, various clonotypes. In some embodiments, the methods are used to analyze the sequence of a TCR alpha chain, a TCR beta chain, a TCR delta chain, a TCR gamma chain, or any fragment thereof (e.g., variable regions

including V(D)J or VJ regions, constant regions, transmembrane regions, fragments thereof, combinations thereof, and combinations of fragments thereof). In some embodiments, the methods described herein can be used to analyze the sequence of a B cell receptor heavy chain, B cell receptor light chain, or any fragment thereof (e.g., variable regions including V(D)J or VJ regions, constant regions, transmembrane regions, fragments thereof, combinations thereof, and combinations of fragments thereof).

Where immune cells are to be analyzed, primer sequences useful in any of the various operations for attaching barcode sequences and/or amplification reactions can include gene specific sequences which target genes or regions of genes of immune cell proteins, for example immune receptors. Such gene sequences include, but are not limited to, sequences of various T cell receptor alpha variable genes (TRAV genes), T cell receptor alpha joining genes (TRAJ genes), T cell receptor alpha constant genes (TRAC genes), T cell receptor beta variable genes (TRBV genes), T cell receptor beta diversity genes (TRBD genes), T cell receptor beta joining genes (TRBJ genes), T cell receptor beta constant genes (TRBC genes), T cell receptor gamma variable genes (TRGV genes), T cell receptor gamma joining genes (TRGJ genes), T cell receptor gamma constant genes (TRGC genes), T cell receptor delta variable genes (TRDV genes), T cell receptor delta diversity genes (TRDD genes), T cell receptor delta joining genes (TRDJ genes), and T cell receptor delta constant genes (TRDC genes).

In some embodiments, the analyte binding moiety is based on the Major Histocompatibility Complex (MHC) class I or class II. In some embodiments, the analyte binding moiety is an MHC multimer including, without limitation, MHC dextramers, MHC tetramers, and MHC pentamers (see, for example, U.S. Patent Application Publication Nos. US 2018/0180601 and US 2017/0343545, the entire contents of each of which are incorporated herein by reference. MHCs (e.g., a soluble MHC monomer molecule), including full or partial MHC-peptides, can be used as analyte binding moieties of analyte capture agents that are coupled to capture agent barcode domains that include an analyte binding moiety barcode that identifies its associated MHC (and, thus, for example, the MHC's TCR binding partner). In some embodiments, MHCs are used to analyze one or more cell-surface features of a T-cell, such as a TCR. In some cases, multiple MHCs are associated together in a larger complex (MHC multi-mer) to improve binding affinity of MHCs to TCRs via multiple ligand binding synergies.

(c) Substrates

For the spatial array-based analytical methods described herein, a substrate functions as a support for direct or indirect attachment of capture probes to features of the array. In addition, in some embodiments, a substrate (e.g., the same substrate or a different substrate) can be used to provide support to a biological sample, particularly, for example, a thin tissue section. Accordingly, a "substrate" is a support that is insoluble in aqueous liquid and which allows for positioning of biological samples, analytes, features, and/or capture probes on the substrate.

Further, a "substrate" as used herein, and when not preceded by the modifier "chemical", refers to a member with at least one surface that generally functions to provide physical support for biological samples, analytes, and/or any of the other chemical and/or physical moieties, agents, and structures described herein. Substrates can be formed from a variety of solid materials, gel-based materials, colloidal materials, semi-solid materials (e.g., materials that are at least partially cross-linked), materials that are fully or partially cured, and materials that undergo a phase change or transition to provide physical support. Examples of substrates that can be used in the methods and systems described herein include, but are not limited to, slides (e.g., slides formed from various glasses, slides formed from various polymers), hydrogels, layers and/or films, membranes (e.g., porous membranes), flow cells, cuvettes, wafers, plates, or combinations thereof. **In** some embodiments, substrates can optionally include functional elements such as recesses, protruding structures, microfluidic elements (e.g., channels, reservoirs, electrodes, valves, seals), and various markings, as will be discussed in further detail below.

(i) Substrate Attributes

A substrate can generally have any suitable form or format. For example, a substrate can be flat, curved, e.g., convexly or concavely curved towards the area where the interaction between a biological sample, e.g., tissue sample, and a substrate takes place. **In** some embodiments, a substrate is flat, e.g., planar, chip, or slide. A substrate can contain one or more patterned surfaces within the substrate (e.g., channels, wells, projections, ridges, divots, etc.).

A substrate can be of any desired shape. For example, a substrate can be typically a thin, flat shape (e.g., a square or a rectangle). In some embodiments, a substrate structure has rounded corners (e.g., for increased safety or robustness). In some embodiments, a substrate structure has one or more cut-off corners (e.g., for use with a slide clamp or cross-table). In some embodiments, where a substrate structure is flat, the substrate structure can be any appropriate type of support having a flat surface (e.g., a chip or a slide such as a microscope slide).

Substrates can optionally include various structures such as, but not limited to, projections, ridges, and channels. A substrate can be micropatterned to limit lateral diffusion (e.g., to prevent overlap of spatial barcodes). A substrate

modified with such structures can be modified to allow association of analytes, features (e.g., beads), or probes at individual sites. For example, the sites where a substrate is modified with various structures can be contiguous or non-contiguous with other sites.

In some embodiments, the surface of a substrate can be modified so that discrete sites are formed that can only have or accommodate a single feature. In some embodiments, the surface of a substrate can be modified so that features adhere to random sites.

In some embodiments, the surface of a substrate is modified to contain one or more wells, using techniques such as (but not limited to) stamping, microetching, or molding techniques. In some embodiments in which a substrate includes one or more wells, the substrate can be a concavity slide or cavity slide. For example, wells can be formed by one or more shallow depressions on the surface of the substrate. In some embodiments, where a substrate includes one or more wells, the wells can be formed by attaching a cassette (e.g., a cassette containing one or more chambers) to a surface of the substrate structure.

In some embodiments, the structures of a substrate (e.g., wells or features) can each bear a different capture probe. Different capture probes attached to each structure can be identified according to the locations of the structures in or on the surface of the substrate. Exemplary substrates include arrays in which separate structures are located on the substrate including, for example, those having wells that accommodate features.

In some embodiments where the substrate is modified to contain one or more structures, including but not limited to, wells, projections, ridges, features, or markings, the structures can include physically altered sites. For example, a substrate modified with various structures can include physical properties, including, but not limited to, physical configurations, magnetic or compressive forces, chemically functionalized sites, chemically altered sites, and/or electrostatically altered sites. In some embodiments where the substrate is modified to contain various structures, including but not limited to wells, projections, ridges, features, or markings, the structures are applied in a pattern. Alternatively, the structures can be randomly distributed.

The substrate (e.g., or a bead or a feature on an array) can include tens to hundreds of thousands or millions of individual oligonucleotide molecules (e.g., at least about 10,000, 50,000, 100,000, 500,000, 1,000,000, 10,000,000, 100,000,000, 1,000,000,000, or 10,000,000,000 oligonucleotide molecules).

In some embodiments, a substrate includes one or more markings on a surface of a substrate, e.g., to provide guidance for correlating spatial information with the characterization of the analyte of interest. For example, a substrate can be marked with a grid of lines (e.g., to allow the size of objects seen under magnification to be easily estimated and/or to provide reference areas for counting objects). In some embodiments, fiducial markers can be included on a substrate. Such markings can be made using techniques including, but not limited to, printing, sand-blasting, and depositing on the surface.

In some embodiments, imaging can be performed using one or more fiducial markers, i.e., objects placed in the field of view of an imaging system which appear in the image produced. Fiducial markers are typically used as a point of reference or measurement scale. Fiducial markers can include, but are not limited to, detectable labels such as fluorescent, radioactive, chemiluminescent, and colorimetric labels. The use of fiducial markers to stabilize and orient biological samples is described, for example, in Carter et al., Applied Optics 46:421-427, 2007), the entire contents of which are incorporated herein by reference. In some embodiments, a fiducial marker can be a physical particle (e.g., a nanoparticle, a microsphere, a nanosphere, a bead, a post, or any of the other exemplary physical particles described herein or known in the art).

In some embodiments, a fiducial marker can be present on a substrate to provide orientation of the biological sample. In some embodiments, a microsphere can be coupled to a substrate to aid in orientation of the biological sample. In some examples, a microsphere coupled to a substrate can produce an optical signal (e.g., fluorescence). In another example, a microsphere can be attached to a portion (e.g., corner) of an array in a specific pattern or design (e.g., hexagonal design) to aid in orientation of a biological sample on an array of features on the substrate. In some embodiments, a quantum dot can be coupled to the substrate to aid in the orientation of the biological sample. In some examples, a quantum dot coupled to a substrate can produce an optical signal.

In some embodiments, a fiducial marker can be an immobilized molecule with which a detectable signal molecule can interact to generate a signal. For example, a marker nucleic acid can be linked or coupled to a chemical moiety capable of fluorescing when subjected to light of a specific wavelength (or range of wavelengths). Such a marker nucleic acid molecule can be contacted with an array before, contemporaneously with, or after the tissue sample is stained to visualize or image the tissue section. Although not required, it can be advantageous to use a marker that can be detected using the same conditions (e.g., imaging conditions) used to detect a labelled cDNA.

In some embodiments, fiducial markers are included to facilitate the orientation of a tissue sample or an image thereof in relation to an immobilized capture probes on a substrate. Any number of methods for marking an array can be used such that a marker is detectable only when a tissue section is imaged. For instance, a molecule, e.g., a fluorescent molecule that generates a signal, can be immobilized directly or indirectly on the surface of a substrate. Markers can be provided on a substrate in a pattern (e.g., an edge, one or more rows, one or more lines, etc.).

In some embodiments, a fiducial marker can be randomly placed in the field of view. For example, an oligonucleotide containing a fluorophore can be randomly printed, stamped, synthesized, or attached to a substrate (e.g., a glass slide) at a random position on the substrate. A tissue section can be contacted with the substrate such that the oligonucleotide containing the fluorophore contacts, or is in proximity to, a cell from the tissue section or a component of the cell (e.g., an mRNA or DNA molecule). An image of the substrate and the tissue section can be obtained, and the position of the fluorophore within the tissue section image can be determined (e.g., by reviewing an optical image of the tissue section overlaid with the fluorophore detection). In some embodiments, fiducial markers can be precisely placed in the field of view (e.g., at known locations on a substrate). In this instance, a fiducial marker can be stamped, attached, or synthesized on the substrate and contacted with a biological sample. Typically, an image of the sample and the fiducial marker is taken, and the position of the fiducial marker on the substrate can be confirmed by viewing the image.

In some embodiments, a fiducial marker can be an immobilized molecule (e.g., a physical particle) attached to the substrate. For example, a fiducial marker can be a nanoparticle, e.g., a nanorod, a nanowire, a nanocube, a nanopyramid, or a spherical nanoparticle. In some examples, the nanoparticle can be made of a heavy metal (e.g., gold). In some embodiments, the nanoparticle can be made from diamond. In some embodiments, the fiducial marker can be visible by eye.

As noted herein, any of the fiducial markers described herein (e.g., microspheres, beads, or any of the other physical particles described herein) can be located at a portion (e.g., corner) of an array in a specific pattern or design (e.g., hexagonal design) to aid in orientation of a biological sample on an array of features on the substrate. In some embodiments, the fiducial markers located at a portion (e.g., corner) of an array (e.g., an array on a substrate) can be patterned or designed in at least 1, at least 2, at least 3, or at least 4 unique patterns. In some examples, the fiducial markers located at the corners of the array (e.g., an array on a substrate) can have four unique patterns of fiducial markers.

In some examples, fiducial markers can surround the array. In some embodiments the fiducial markers allow for detection of, e.g., mirroring. In some embodiments, the fiducial markers may completely surround the array. In some embodiments, the fiducial markers may not completely surround the array. In some embodiments, the fiducial markers identify the corners of the array. In some embodiments, one or more fiducial markers identify the center of the array. In some embodiments, the fiducial markers comprise patterned spots, wherein the diameter of one or more patterned spot fiducial markers is approximately 100 micrometers. The diameter of the fiducial markers can be any useful diameter including, but not limited to, 50 micrometers to 500 micrometers in diameter. The fiducial markers may be arranged in such a way that the center of one fiducial marker is between 100 micrometers and 200 micrometers from the center of one or more other fiducial markers surrounding the array. In some embodiments, the array with the surrounding fiducial markers is approximately 8 mm by 8 mm. In some embodiments, the array without the surrounding fiducial markers is smaller than 8 mm by 50 mm.

In some embodiments, an array can be enclosed within a frame. Put another way, the perimeter of an array can have fiducial markers such that the array is enclosed, or substantially enclosed. In some embodiments, the perimeter of an array can be fiducial markers (e.g., any fiducial marker described herein). In some embodiments, the perimeter of an array can be uniform. For example, the fiducial markings can connect, or substantially connect, consecutive corners of an array in such a fashion that the non-comer portion of the array perimeter is the same on all sides (e.g., four sides) of the array. In some embodiments, the fiducial markers attached to the non-comer portions of the perimeter can be pattered or designed to aid in the orientation of the biological sample on the array. In some embodiments, the particles attached to the non-comer portions of the perimeter can be patterned or designed in at least 1, at least 2, at least 3, or at least 4 patterns. In some embodiments, the patterns can have at least 2, at least 3, or at least 4 unique patterns of fiducial markings on the non-comer portion of the array perimeter.

In some embodiments, an array can include at least two fiducial markers (e.g., at least 3, at least 4, at least 5, at least 6, at least 7, at least 8, at least 9, at least 10, at least 12, at least 15, at least 20, at least 30, at least 40, at least 50, at least 60, at least 70, at least 80, at least 90, at least 100 fiducial markers or more (e.g., several hundred, several thousand, or tens of thousands of fiducial markers)) in distinct positions on the surface of a substrate. Fiducial markers can be provided on a substrate in a pattern (e.g., an edge, one or more rows, one or more lines, etc.).

A wide variety of different substrates can be used for the foregoing purposes. In general, a substrate can be any suitable support material. Exemplary substrates include, but are not limited to, glass, modified and/or functionalized glass, hydrogels, films, membranes, plastics (including e.g., acrylics, polystyrene, copolymers of styrene and other materials, polypropylene, polyethylene, polybutylene, polyurethanes, Teflon™, cyclic olefins, polyimides etc.), nylon, ceramics, resins, Zeonor, silica or silica-based materials including silicon and modified silicon, carbon, metals, inorganic glasses, optical fiber bundles, and polymers, such as polystyrene, cyclic olefin copolymers (COCs), cyclic olefin polymers (COPs), polypropylene, polyethylene polycarbonate, or combinations thereof.

Among the examples of substrate materials discussed above, polystyrene is a hydrophobic material suitable for binding negatively charged macromolecules because it normally contains few hydrophilic groups. For nucleic acids immobilized on glass slides, by increasing the hydrophobicity of the glass surface the nucleic acid immobilization can be increased. Such an enhancement can permit a relatively more densely packed formation (e.g., provide improved

specificity and resolution).

In another example, a substrate can be a flow cell. Flow cells can be formed of any of the foregoing materials, and can include channels that permit reagents, solvents, features, and analytes to pass through the flow cell. In some embodiments, a hydrogel embedded biological sample is assembled in a flow cell (e.g., the flow cell is utilized to introduce the hydrogel to the biological sample). In some embodiments, a hydrogel embedded biological sample is not assembled in a flow cell. In some embodiments, the hydrogel embedded biological sample can then be prepared and/or isometrically expanded as described herein.

(ii) Conductive Substrates

Conductive substrates (e.g., electrophoretic compatible arrays) generated as described herein can be used in the spatial detection of analytes. For example, an electrophoretic field can be applied to facilitate migration of analytes towards the barcoded oligonucleotides (e.g., capture probes) on the array (e.g., capture probes immobilized on paper, capture probes immobilized in a hydrogel film, or capture probes immobilized on a glass slide having a conductive coating). In some embodiments, an electrophoresis assembly can be arranged. For example, an anode and a cathode can be arranged such that an array of capture probes (e.g., capture probes immobilized on paper, capture probes immobilized in a hydrogel film, or capture probes immobilized on a glass slide having a conductive coating) and a biological sample are positioned between the anode and the cathode. In such embodiments, analytes in the biological sample are actively migrated toward the capture probes on the conductive substrate and captured. The biological sample can be prepared (e.g., permeabilized) according to any method described herein. In some embodiments, after electrophoretic-assisted capture of the analytes, the barcoded oligonucleotides (e.g., capture probes) and captured analytes can be collected, processed, and/or analyzed (e.g., sequenced) using any of the methods described herein.

In some embodiments, a conductive substrate can include glass (e.g., a glass slide) that has been coated with a substance or otherwise modified to confer conductive properties to the glass. In some embodiments, a glass slide can be coated with a conductive coating. In some embodiments, a conductive coating includes tin oxide (TO) or indium tin oxide (ITO). In some embodiments, a conductive coating includes a transparent conductive oxide (TCO). In some embodiments, a conductive coating includes aluminum doped zinc oxide (AZO). In some embodiments, a conductive coating includes fluorine doped tin oxide (FTO).

In some embodiments, arrays that are spotted or printed with oligonucleotides (e.g., capture probes, e.g., any of the variety of capture probes described herein) can be generated on a conductive substrate (e.g., any of the conductive substrates described herein). For example, the arrays described herein can be compatible with active analyte capture methods (e.g., any of the analyte capture methods described herein, including without limitation, electrophoretic capture methods). In some embodiments, a conductive substrate is a porous medium. Non-limiting examples of porous media that can be used in methods described herein that employ active analyte capture include a nitrocellulose or nylon membrane. In some embodiments, a porous medium that can be used in methods described herein that employ active analyte capture includes paper. In some embodiments, the oligonucleotides can be printed on a paper substrate. In some embodiments, the printed oligonucleotides can interact with the substrate (e.g., interact with fibers of the paper). In some embodiments, printed oligonucleotides can covalently bind the substrate (e.g., to fibers of the paper). In some embodiments, oligonucleotides in a molecular precursor solution can be printed on a conductive substrate (e.g., paper). In some embodiments, a molecular precursor solution can polymerize, thereby generating gel pads on the conductive substrate (e.g., paper). In some embodiments, a molecular precursor solution can be polymerized by light (e.g., photocured). In some embodiments, gel beads (e.g., any of the variety of gel beads described herein) containing oligonucleotides (e.g., barcoded oligonucleotides such as capture probes) can be printed on a conductive substrate (e.g., paper). In some embodiments, the printed oligonucleotides can be covalently attached into the gel matrix.

(iii) Coatings

In some embodiments, a substrate is coated with a surface treatment such as poly(L)-lysine. Additionally or alternatively, the substrate can be treated by silanation, e.g., with epoxy-silane, amino-silane, and/or by a treatment with polyacrylamide.

In some embodiments, a substrate is treated in order to minimize or reduce non-specific analyte hybridization within or between features. For example, treatment can include coating the substrate with a hydrogel, film, and/or membrane that creates a physical barrier to non-specific hybridization. Any suitable hydrogel can be used. For example, hydrogel matrices prepared according to the methods set forth in U.S. Patent Nos. 6,391,937, 9,512,422, and 9,889,422, and U.S. Patent Application Publication Nos. U.S. 2017/0253918 and U.S. 2018/0052081, can be used. The entire contents of each of the foregoing documents is incorporated herein by reference.

Treatment can include adding a functional group that is reactive or capable of being activated such that it becomes reactive after application of a stimulus (e.g., photoreactive functional groups). Treatment can include treating

with polymers having one or more physical properties (e.g., mechanical, electrical, magnetic, and/or thermal) that minimize non-specific binding (e.g., that activate a substrate at certain locations to allow analyte hybridization at those locations).

A "removable coating" is a coating that can be removed from the surface of a substrate upon application of a releasing agent. In some embodiments, a removable coating includes a hydrogel as described herein, e.g., a hydrogel including a polypeptide-based material. Non-limiting examples of a hydrogel featuring a polypeptide-based material include a synthetic peptide-based material featuring a combination of spider silk and a transmembrane segment of human muscle L-type calcium channel (e.g., PEPGEL®), an amphiphilic 16 residue peptide containing a repeating arginine-alanine-aspartate-alanine sequence (RADARADARADARADA) (e.g., PURAMATRIX®), EAK16 (AEAEAKAKAEAEAKAK), KLD12 (KLDLKLDLKLDL), and PGMATRIX™.

In some embodiments, the hydrogel in the removable coating is a stimulus-responsive hydrogel. A stimulus-responsive hydrogel can undergo a gel-to-solution and/or gel-to-solid transition upon application of one or more external triggers (e.g., a releasing agent). See, e.g., Willner, Acc. Chem. Res. 50:657-658, 2017, which is incorporated herein by reference in its entirety. Non-limiting examples of a stimulus-responsive hydrogel include a thermoresponsive hydrogel, a pH-responsive hydrogel, a light-responsive hydrogel, a redox-responsive hydrogel, an analyte-responsive hydrogel, or a combination thereof. In some embodiments, a stimulus-responsive hydrogel can be a multi-stimuli-responsive hydrogel.

A "releasing agent" or "extemal trigger" is an agent that allows for the removal of a removable coating from a substrate when the releasing agent is applied to the removable coating. An external trigger or releasing agent can include physical triggers such as thermal, magnetic, ultrasonic, electrochemical, and/or light stimuli as well as chemical triggers such as pH, redox reactions, supramolecular complexes, and/or biocatalytically driven reactions. See e.g., Echeverria, et al., Gels (2018), 4, 54; doi:10.3390/gels4020054, which is incorporated herein by reference in its entirety. The type of "releasing agent" or "extemal trigger" can depend on the type of removable coating. For example, a removable coating featuring a redox-responsive hydrogel can be removed upon application of a releasing agent that includes a reducing agent such as dithiothreitol (DTT). As another example, a pH-responsive hydrogel can be removed upon the application of a releasing agent that changes the pH.

(iv) Gel Substrates

In some embodiments, gel beads containing oligonucleotides (e.g., barcoded oligonucleotides such as capture probes) can be deposited on a substrate (e.g., a glass slide). In some embodiments, gel pads can be deposited on a substrate (e.g., a glass slide). In some embodiments, gel pads or gel beads are deposited on a substrate in an arrayed format. In some embodiments in which gel pads or gel beads are deposited on a substrate in an arrayed format, a hydrogel molecular precursor solution can be applied on top of the array (e.g., the array of gel pads or gel beads on a glass slide). In some embodiments, a hydrogel molecular precursor solution can be polymerized such that the deposited gel pads or gel beads are immobilized within the polymerized hydrogel. Any suitable method of polymerization can be used or (e.g., any of the variety of methods described herein). In some embodiments, a polymerized hydrogel that includes the gel pads or gel beads can be removed (e.g., peeled) from the substrate (e.g., glass slide) such that the gel beads or gel pads are secured in the hydrogel. In some embodiments, a polymerized hydrogel that includes the gel pads or gel beads is a conductive substrate (as described herein) that can be used in accordance with any of the variety of analyte capture methods described herein (e.g., electrophoretic migration of analytes for capture).

Arrays can be prepared by depositing features (e.g., droplets, beads) on a substrate surface to produce a spatially-barcoded array. Methods of depositing (e.g., droplet manipulation) features are known in the art (see, U.S. Patent Application Publication No. 2008/0132429, Rubina, A.Y., et al., Biotechniques. 2003 May; 34(5):1008-14, 1016-20, 1022 and Vasiliskov et al. Biotechniques. 1999 September; 27(3):592-4, 596-8, 600 passim. each herein incorporated by reference in its entirety). A feature can be printed or deposited at a specific location on the substrate (e.g., inkjet printing). In some embodiments, each feature can have a unique oligonucleotide that functions as a spatial barcode. In some embodiments, each feature can have capture probes for multiplexing (e.g., capturing multiple analytes or multiple types of analytes, e.g., proteins and nucleic acids). In some embodiments, a feature can be printed or deposited at the specific location using an electric field. A feature can contain a photo-crosslinkable polymer precursor and an oligonucleotide. In some embodiments, the photo-crosslinkable polymer precursor can be deposited into a patterned feature on the substrate (e.g., well).

A "photo-crosslinkable polymer precursor" refers to a compound that cross-links and/or polymerizes upon exposure to light. In some embodiments, one or more photoinitiators may also be included to induce and/or promote polymerization and/or crosslinking. See, e.g., Choi et al. Biotechniques. 2019 Jan;66(1):40-53, which is incorporated herein by reference in its entirety.

Non-limiting examples of photo-crosslinkable polymer precursors include polyethylene (glycol) diacrylate (PEGDA), gelatin-methacryloyl (GelMA), and methacrylated hyaluronic acid (MeHA). In some embodiments, a photo-crosslinkable polymer precursor comprises polyethylene (glycol) diacrylate (PEGDA), gelatin-methacryloyl (GelMA),

methacrylated hyaluronic acid (MeHA), or a combination thereof. In some embodiments, a photo-crosslinkable polymer precursor (e.g., PAZAM) can be covalently linked (e.g., cross-linked) to a substrate. In some embodiments, a photo-crosslinkable polymer precursor is not covalently linked to a substrate surface. For example, a silane-free acrylamide can be used (See U.S. Patent Application Publication No. 2011/0059865, herein incorporated by reference in its entirety). The photo-crosslinkable polymer precursor in a feature (e.g., droplet or bead) can be polymerized by any known method. The oligonucleotides can be polymerized in a cross-linked gel matrix (e.g., copolymerized or simultaneously polymerized). In some embodiments, the features containing the photo-crosslinkable polymer precursor deposited on the substrate surface can be exposed to UV light. The UV light can induce polymerization of the photo-crosslinkable polymer precursor and result in the features becoming a gel matrix (e.g., gel pads) on the substrate surface (e.g., array).

Polymerization methods for hydrogel subunits can be selected to form hydrogels with different properties (e.g., pore volume, swelling properties, biodegradability, conduction, transparency, and/or permeability of the hydrogel). For example, a hydrogel can include pores of sufficient volume to allow the passage of macromolecules, (e.g., nucleic acids, proteins, chromatin, metabolites, gRNA, antibodies, carbohydrates, peptides, metabolites, and/or small molecules) to/from the sample (e.g., tissue section). It is known that pore volume generally decreases with increasing concentration of hydrogel subunits and generally increases with an increasing ratio of hydrogel subunits to cross-linker. Therefore, a hydrogel composition can be prepared that includes a concentration of hydrogel subunits that allows the passage of such biological macromolecules.

In some embodiments, hydrogel formation on a substrate occurs before, contemporaneously with, or after features (e.g., beads) are attached to the substrate. For example, when a capture probe is attached (e.g., directly or indirectly) to a substrate, hydrogel formation can be performed on the substrate already containing the capture probes.

(d) Arrays

In many of the methods described herein, features (as described further below) are collectively positioned on a substrate. An "array" is a specific arrangement of a plurality of features that is either irregular or forms a regular pattern. Individual features in the array differ from one another based on their relative spatial locations. In general, at least two of the plurality of features in the array include a distinct capture probe (e.g., any of the examples of capture probes described herein).

Arrays can be used to measure large numbers of analytes simultaneously. In some embodiments, oligonucleotides are used, at least in part, to create an array. For example, one or more copies of a single species of oligonucleotide (e.g., capture probe) can correspond to or be directly or indirectly attached to a given feature in the array. In some embodiments, a given feature in the array includes two or more species of oligonucleotides (e.g., capture probes). In some embodiments, the two or more species of oligonucleotides (e.g., capture probes) attached directly or indirectly to a given feature on the array include a common (e.g., identical) spatial barcode.

(i) Arrays for Analyte Capture

In some embodiments, an array can include a capture probe attached directly or indirectly to the substrate. The capture probe can include a capture domain (e.g., a nucleotide sequence) that can specifically bind (e.g., hybridize) to a target analyte (e.g., mRNA, DNA, or protein) within a sample. In some embodiments, the binding of the capture probe to the target (e.g., hybridization) can be detected and quantified by detection of a visual signal, e.g., a fluorophore, a heavy metal (e.g., silver ion), or chemiluminescent label, which has been incorporated into the target. In some embodiments, the intensity of the visual signal correlates with the relative abundance of each analyte in the biological sample. Since an array can contain thousands or millions of capture probes (or more), an array can interrogate many analytes in parallel.

In some embodiments, a substrate includes one or more capture probes that are designed to capture analytes from one or more organisms. In a non-limiting example, a substrate can contain one or more capture probes designed to capture mRNA from one organism (e.g., a human) and one or more capture probes designed to capture DNA from a second organism (e.g., a bacterium).

The capture probes can be attached to a substrate or feature using a variety of techniques. In some embodiments, the capture probe is directly attached to a feature that is fixed on an array. In some embodiments, the capture probes are immobilized to a substrate by chemical immobilization. For example, a chemical immobilization can take place between functional groups on the substrate and corresponding functional elements on the capture probes. Exemplary corresponding functional elements in the capture probes can either be an inherent chemical group of the capture probe, e.g., a hydroxyl group, or a functional element can be introduced on to the capture probe. An example of a functional group on the substrate is an amine group. In some embodiments, the capture probe to be immobilized includes a functional amine group or is chemically modified in order to include a functional amine group. Means and methods for such a chemical modification are well known in the art.

In some embodiments, the capture probe is a nucleic acid. In some embodiments, the capture probe is

immobilized on a substrate or feature via its 5' end. In some embodiments, the capture probe is immobilized on a substrate or feature via its 5' end and includes from the 5' to 3' end: one or more barcodes (e.g., a spatial barcode and/or a UMI) and one or more capture domains. In some embodiments, the capture probe is immobilized on a substrate or feature via its 5' end and includes from the 5' to 3' end: one barcode (e.g., a spatial barcode or a UMI) and one capture domain. In some embodiments, the capture probe is immobilized on a substrate or feature via its 5' end and includes from the 5' to 3' end: a cleavage domain, a functional domain, one or more barcodes (e.g., a spatial barcode and/or a UMI), and a capture domain.

In some embodiments, the capture probe is immobilized on a substrate or feature via its 5' end and includes from the 5' to 3' end: a cleavage domain, a functional domain, one or more barcodes (e.g., a spatial barcode and/or a UMI), a second functional domain, and a capture domain. In some embodiments, the capture probe is immobilized on a substrate or feature via its 5' end and includes from the 5' to 3' end: a cleavage domain, a functional domain, a spatial barcode, a UMI, and a capture domain. In some embodiments, the capture probe is immobilized on a substrate or feature via its 5' end and does not include a spatial barcode. In some embodiments, the capture probe is immobilized on a substrate or feature via its 5' end and does not include a UMI. In some embodiments, the capture probe includes a sequence for initiating a sequencing reaction.

In some embodiments, the capture probe is immobilized on a substrate or feature via its 3' end. In some embodiments, the capture probe is immobilized on a substrate or feature via its 3' end and includes from the 3' to 5' end: one or more barcodes (e.g., a spatial barcode and/or a UMI) and one or more capture domains. In some embodiments, the capture probe is immobilized on a substrate or feature via its 3' end and includes from the 3' to 5' end: one barcode (e.g., a spatial barcode or a UMI) and one capture domain. In some embodiments, the capture probe is immobilized on a substrate or feature via its 3' end and includes from the 3' to 5' end: a cleavage domain, a functional domain, one or more barcodes (e.g., a spatial barcode and/or a UMI), and a capture domain. In some embodiments, the capture probe is immobilized on a substrate or feature via its 3' end and includes from the 3' to 5' end: a cleavage domain, a functional domain, a spatial barcode, a UMI, and a capture domain.

The localization of the functional group within the capture probe to be immobilized can be used to control and shape the binding behavior and/or orientation of the capture probe, e.g., the functional group can be placed at the 5' or 3' end of the capture probe or within the sequence of the capture probe. In some embodiments, a capture probe can further include a substrate. A typical substrate for a capture probe to be immobilized includes moieties which are capable of binding to such capture probes, e.g., to amine-functionalized nucleic acids. Examples of such substrates are carboxy, aldehyde, or epoxy substrates.

In some embodiments, the substrates on which capture probes can be immobilized can be chemically activated, e.g., by the activation of functional groups available on the substrate. The term "activated substrate" relates to a material in which interacting or reactive chemical functional groups are established or enabled by chemical modification procedures. For example, a substrate including carboxyl groups can be activated before use. Furthermore, certain substrates contain functional groups that can react with specific moieties already present in the capture probes.

In some embodiments, a covalent linkage is used to directly couple a capture probe to a substrate. In some embodiments a capture probe is indirectly coupled to a substrate through a linker separating the "first" nucleotide of the capture probe from the substrate, e.g., a chemical linker. In some embodiments, a capture probe does not bind directly to the substrate, but interacts indirectly, for example by binding to a molecule which itself binds directly or indirectly to the substrate. In some embodiments, the capture probe is indirectly attached to a substrate (e.g., attached to a substrate via a solution including a polymer).

In some embodiments where the capture probe is immobilized on a feature of the array indirectly, e.g., via hybridization to a surface probe capable of binding the capture probe, the capture probe can further include an upstream sequence (5' to the sequence that hybridizes to the nucleic acid, e.g., RNA of the tissue sample) that is capable of hybridizing to 5' end of a surface probe. Alone, the capture domain of the capture probe can be seen as a capture domain oligonucleotide, which can be used in the synthesis of the capture probe in embodiments where the capture probe is immobilized on the array indirectly.

In some embodiments, a substrate is comprised of an inert material or matrix (e.g., glass slides) that has been functionalized by, for example, treating the substrate with a material comprising reactive groups which enable immobilization of capture probes. See, for example, WO 2017/019456, the entire contents of which is herein incorporated by reference. Non-limiting examples include polyacrylamide hydrogels supported on an inert substrate (e.g., glass slide; see WO 2005/065814 and U.S. Patent Application No. 2008/0280773, the entire contents of which is incorporated herein by reference).

In some embodiments, an oligonucleotide (e.g., a capture probe) can be attached to a substrate or feature according to the methods set forth in U.S. Patent Nos. 6,737,236, 7,259,258, 7,375,234, 7,427,678, 5,610,287, 5,807,522, 5,837,860, and 5,472,881; U.S. Patent Application Publication Nos. 2008/0280773 and 2011/0059865; Shalon et al. (1996) Genome Research, 639-645; Rogers et al. (1999) Analytical Biochemistry 266, 23-30; Stimpson et al. (1995) Proc. Natl. Acad. Sci. USA 92, 6379-6383; Beattie et al. (1995) Clin. Chem. 45, 700-706; Lamture et al. (1994) Nucleic Acids Research 22, 2121-2125; Beier et al. (1999) Nucleic Acids Research 27, 1970-1977; Joos et al. (1997) Analytical

Biochemistry 247, 96-101; Nikiforov et al. (1995) Analytical Biochemistry 227, 201-209; Timofeev et al. (1996) Nucleic Acids Research 24, 3142-3148; Chrisey et al. (1996) Nucleic Acids Research 24, 3031-3039; Guo et al. (1994) Nucleic Acids Research 22, 5456-5465; Running and Urdea (1990) BioTechniques 8, 276-279; Fahy et al. (1993) Nucleic Acids Research 21, 1819-1826; Zhang *et al*. (1991) 19, 3929-3933; and Rogers et al. (1997) Gene Therapy 4, 1387-1392. The entire contents of each of the foregoing documents is incorporated herein by reference.

(ii) Generation of Capture Probes in an Array Format

Arrays can be prepared by a variety of methods. In some embodiments, arrays are prepared through the synthesis (e.g., in situ synthesis) of oligonucleotides on the array, or by jet printing or lithography. For example, light-directed synthesis of high-density DNA oligonucleotides can be achieved by photolithography or solid-phase DNA synthesis. To implement photolithographic synthesis, synthetic linkers modified with photochemical protecting groups can be attached to a substrate and the photochemical protecting groups can be modified using a photolithographic mask (applied to specific areas of the substrate) and light, thereby producing an array having localized photo-deprotection. Many of these methods are known in the art, and are described e.g., in Miller et al., "Basic concepts of microarrays and potential applications in clinical microbiology." Clinical Microbiology Reviews 22.4 (2009): 611-633; US201314111482A; US9593365B2; US2019203275; and WO2018091676, which are each incorporated herein by reference in its entirety.

(1) Spotting or Printing

In some embodiments, oligonucleotides (e.g., capture probes) can be "spotted" or "printed" onto a substrate to form an array. The oligonucleotides can be applied by either noncontact or contact printing. A noncontact printer can use the same method as computer printers (e.g., bubble jet or inkjet) to expel small droplets of probe solution onto the substrate. The specialized inkjet-like printer can expel nanoliter to picoliter volume droplets of oligonucleotide solution, instead of ink, onto the substrate. In contact printing, each print pin directly applies the oligonucleotide solution onto a specific location on the surface. The oligonucleotides can be attached to the substrate surface by the electrostatic interaction of the negative charge of the phosphate backbone of the DNA with a positively charged coating of the substrate surface or by UV-cross-linked covalent bonds between the thymidine bases in the DNA and amine groups on the treated substrate surface. In some embodiments, the substrate is a glass slide. In some embodiments, the oligonucleotides (e.g., capture probes) are attached to a substrate by a covalent bond to a chemical matrix, e.g., epoxy-silane, amino-silane, lysine, polyacrylamide, etc.

(2) In situ Synthesis

Capture probes arrays can be prepared by in situ synthesis. In some embodiments, capture probe arrays can be prepared using photolithography. Photolithography typically relies on UV masking and light-directed combinatorial chemical synthesis on a substrate to selectively synthesize probes directly on the surface of an array, one nucleotide at a time per spot, for many spots simultaneously. In some embodiments, a substrate contains covalent linker molecules that have a protecting group on the free end that can be removed by light. UV light is directed through a photolithographic mask to deprotect and activate selected sites with hydroxyl groups that initiate coupling with incoming protected nucleotides that attach to the activated sites. The mask is designed in such a way that the exposure sites can be selected, and thus specify the coordinates on the array where each nucleotide can be attached. The process can be repeated, a new mask is applied activating different sets of sites and coupling different bases, allowing different oligonucleotides to be constructed at each site. This process can be used to synthesize hundreds of thousands of different oligonucleotides. In some embodiments, maskless array synthesizer technology can be used. Programmable micromirrors can create digital masks that reflect the desired pattern of UV light to deprotect the features.

In some embodiments, the inkjet spotting process can also be used for in situ oligonucleotide synthesis. The different nucleotide precursors plus catalyst can be printed on the substrate, and are then combined with coupling and deprotection steps. This method relies on printing picoliter volumes of nucleotides on the array surface in repeated rounds of base-by-base printing that extends the length of the oligonucleotide probes on the array.

(3) Ligation

In some embodiments, an array comprising barcoded probes can be generated through ligation of a plurality of oligonucleotides. In some instances, an oligonucleotide of the plurality contains a portion of a barcode, and the complete barcode is generated upon ligation of the plurality of oligonucleotides. For example, a first oligonucleotide containing a first portion of a barcode can be attached to a substrate (e.g., using any of the methods of attaching an oligonucleotide to a substrate described herein), and a second oligonucleotide containing a second portion of the barcode can then be ligated

onto the first oligonucleotide to generate a complete barcode. Different combinations of the first, second and any additional portions of a barcode can be used to increase the diversity of the barcodes. In instances where the second oligonucleotide is also attached to the substrate prior to ligation, the first and/or the second oligonucleotide can be attached to the substrate via a surface linker which contains a cleavage site. Upon ligation, the ligated oligonucleotide can be linearized by cleaving at the cleavage site.

To increase the diversity of the barcodes, a plurality of second oligonucleotides comprising two or more different barcode sequences can be ligated onto a plurality of first oligonucleotides that comprise the same barcode sequence, thereby generating two or more different species of barcodes. To achieve selective ligation, a first oligonucleotide attached to a substrate containing a first portion of a barcode can initially be protected with a protective group (e.g., a photocleavable protective group), and the protective group can be removed prior to ligation between the first and second oligonucleotide. In instances where the barcoded probes on an array are generated through ligation of two or more oligonucleotides, a concentration gradient of the oligonucleotides can be applied to a substrate such that different combinations of the oligonucleotides are incorporated into a barcoded probe depending on its location on the substrate.

Probes can be generated by directly ligating additional oligonucleotides onto existing oligonucleotides via a splint oligonucleotide. In some embodiments, oligonucleotides on an existing array can include a recognition sequence that can hybridize with a splint oligonucleotide. The recognition sequence can be at the free 5’ end or the free 3’ end of an oligonucleotide on the existing array. Recognition sequences useful for the methods of the present disclosure may not contain restriction enzyme recognition sites or secondary structures (e.g., hairpins), and may include high contents of Guanine and Cytosine nucleotides.

(4) Polymerases

Barcoded probes on an array can also be generated by adding single nucleotides to existing oligonucleotides on an array, for example, using polymerases that function in a template-independent manner. Single nucleotides can be added to existing oligonucleotides in a concentration gradient, thereby generating probes with varying length, depending on the location of the probes on the array.

(iii) Features

A "feature" is an entity that acts as a support or repository for various molecular entities used in sample analysis. In some embodiments, some or all of the features in an array are functionalized for analyte capture. In some embodiments, functionalized features include one or more capture probe(s). Examples of features include, but are not limited to, a bead, a spot of any two- or three-dimensional geometry (e.g., an ink jet spot, a masked spot, a square on a grid), a well, and a hydrogel pad. In some embodiments, features are directly or indirectly attached or fixed to a substrate. In some embodiments, the features are not directly or indirectly attached or fixed to a substrate, but instead, for example, are disposed within an enclosed or partially enclosed three dimensional space (e.g., wells or divots).

In addition to those above, a wide variety of other features can be used to form the arrays described herein. For example, in some embodiments, features that are formed from polymers and/or biopolymers that are jet printed, screen printed, or electrostatically deposited on a substrate can be used to form arrays. Jet printing of biopolymers is described, for example, in PCT Patent Application Publication No. WO 2014/085725. Jet printing of polymers is described, for example, in de Gans et al., Adv Mater. 16(3): 203-213 (2004). Methods for electrostatic deposition of polymers and biopolymers are described, for example, in Hoyer et al., Anal. Chem. 68(21): 3840-3844 (1996). The entire contents of each of the foregoing references are incorporated herein by reference.

As another example, in some embodiments, features are formed by metallic micro- or nanoparticles. Suitable methods for depositing such particles to form arrays are described, for example, in Lee et al., Beilstein J. Nanotechnol. 8: 1049-1055 (2017), the entire contents of which are incorporated herein by reference.

As a further example, in some embodiments, features are formed by magnetic particles that are assembled on a substrate. Examples of such particles and methods for assembling arrays are described in Ye et al., Scientific Reports 6: 23145 (2016), the entire contents of which are incorporated herein by reference.

As another example, in some embodiments, features correspond to regions of a substrate in which one or more optical labels have been incorporated, and/or which have been altered by a process such as permanent photobleaching. Suitable substrates to implement features in this manner include a wide variety of polymers, for example. Methods for forming such features are described, for example, in Moshrefzadeh et al., Appl. Phys. Lett. 62: 16 (1993), the entire contents of which are incorporated herein by reference.

As yet another example, in some embodiments, features can correspond to colloidal particles assembled (e.g., via self-assembly) to form an array. Suitable colloidal particles are described for example in Sharma, Resonance 23(3): 263-275 (2018), the entire contents of which are incorporated herein by reference.

As a further example, in some embodiments, features can be formed via spot-array photopolymerization of a

monomer solution on a substrate. In particular, two-photon and three-photon polymerization can be used to fabricate features of relatively small (e.g., submicron) dimensions. Suitable methods for preparing features on a substrate in this manner are described for example in Nguyen et al., Materials Today 20(6): 314-322 (2017), the entire contents of which are incorporated herein by reference.

In some embodiments, features are directly or indirectly attached or fixed to a substrate that is liquid permeable. In some embodiments, features are directly or indirectly attached or fixed to a substrate that is biocompatible. In some embodiments, features are directly or indirectly attached or fixed to a substrate that is a hydrogel.

(e) Analyte Capture

In this section, general aspects of methods and systems for capturing analytes are described. Individual method steps and system features can be present in combination in many different embodiments; the specific combinations described herein do not in any way limit other combinations of steps or features.

(i) Conditions for capture

Generally, analytes can be captured when contacting a biological sample with a substrate including capture probes (e.g., substrate with capture probes embedded, spotted, printed on the substrate or a substrate with features (e.g., beads, wells) comprising capture probes).

As used herein, "contact," "contacted," and/ or "contacting," a biological sample with a substrate refers to any contact (e.g., direct or indirect) such that capture probes can interact (e.g., capture) with analytes from the biological sample. For example, a substrate may be near or adjacent to the biological sample without direct physical contact, yet capable of capturing analytes from the biological sample. In some embodiments a biological sample is in direct physical contact with a substrate. In some embodiments, a biological sample is in indirect physical contact with a substrate. For example, a liquid layer may be between the biological sample and the substrate. In some embodiments, analytes diffuse through a liquid layer. In some embodiments capture probes diffuse through a liquid layer. In some embodiments reagents may be delivered via a liquid layer between a biological sample and a substrate. In some embodiments, indirect physical contact may include a second substrate (e.g., a hydrogel, a film, a porous membrane) between the biological sample and the first substrate comprising capture probes. In some embodiments, reagents may be delivered by a second substrate to a biological sample.

In some embodiments, a cell immobilization agent can be used to contact a biological sample with a substrate (e.g., by immobilizing non-aggregated or disaggregated sample on a spatially-barcoded array prior to analyte capture). A "cell immobilization agent" as used herein can refer to an agent (e.g., an antibody), attached to a substrate, which can bind to a cell surface marker. Non-limiting examples of a cell surface marker include CD45, CD3, CD4, CD8, CD56, CD19, CD20, CD11c, CD14, CD33, CD66b, CD34, CD41, CD61, CD235a, CD146, and epithelial cellular adhesion molecule (EpCAM). A cell immobilization agent can include any probe or component that can bind to (e.g., immobilize) a cell or tissue when on a substrate. A cell immobilization agent attached to the surface of a substrate can be used to bind a cell that has a cell surface maker. The cell surface marker can be a ubiquitous cell surface marker, wherein the purpose of the cell immobilization agent is to capture a high percentage of cells within the sample. The cell surface marker can be a specific, or more rarely expressed, cell surface marker, wherein the purpose of the cell immobilization agent is to capture a specific cell population expressing the target cell surface marker. Accordingly, a cell immobilization agent can be used to selectively capture a cell expressing the target cell surface marker from a population of cells that do not have the same cell surface marker.

Capture probes on a substrate (or on a feature on the substrate) may interact with released analytes through a capture domain, described elsewhere, to capture analytes. In some embodiments, certain steps are performed to enhance the transfer or capture of analytes to the capture probes of the array. Examples of such modifications include, but are not limited to, adjusting conditions for contacting the substrate with a biological sample (e.g., time, temperature, orientation, pH levels, pre-treating of biological samples, etc.), using force to transport analytes (e.g., electrophoretic, centrifugal, mechanical, etc.), performing amplification reactions to increase the amount of biological analytes (e.g., PCR amplification, in situ amplification, clonal amplification), and/or using labeled probes for detecting of amplicons and barcodes.

In some embodiments, an array is adapted in order to facilitate biological analyte migration. Non-limiting examples of adapting an array to facilitate biological analyte migration include arrays with substrates containing nanopores, nanowells, and/or microfluidic channels; arrays with porous membranes; and arrays with substrates that are made of hydrogel. In some cases, the array substrate is liquid permeable. In some cases, the array is a coverslip or slide that includes nanowells or patterning, (e.g., via fabrication). In some cases where the substrate includes nanopores, nanowells, and/or microfluidic channels, these structures can facilitate exposure of the biological sample to reagents (e.g., reagents for permeabilization, biological analyte capture, and/or a nucleic acid extension reaction), thereby increasing analyte capture efficiency as compared to a substrate lacking such characteristics.

In some embodiments, analyte capture is facilitated by treating a biological sample with permeabilization reagents. If a biological sample is not permeabilized sufficiently, the amount of analyte captured on a substrate can be too low to enable adequate analysis. Conversely, if a biological sample is too permeable, an analyte can diffuse away from its origin in the biological sample, such that the relative spatial relationship of the analytes within the biological sample is lost. Hence, a balance between permeabilizing the biological sample enough to obtain good signal intensity while still maintaining the spatial resolution of the analyte distribution in the biological sample is desired. Methods of preparing biological samples to facilitate analyte capture are known in the art and can be modified depending on the biological sample and how the biological sample is prepared (e.g., fresh frozen, FFPE, etc.).

(ii) Passive capture methods

In some embodiments, analytes can be migrated from a sample to a substrate. Methods for facilitating migration can be passive (e.g., diffusion) and/or active (e.g., electrophoretic migration of nucleic acids). Non-limiting examples of passive migration can include simple diffusion and osmotic pressure created by the rehydration of dehydrated objects.

Passive migration by diffusion uses concentration gradients. Diffusion is movement of untethered objects toward equilibrium. Therefore, when there is a region of high object concentration and a region of low object concentration, the object (e.g., a capture probe, an analyte, etc.) moves to an area of lower concentration. **In** some embodiments, untethered analytes move down a concentration gradient.

**In** some embodiments, different reagents may be added to the biological sample, such that the biological sample is rehydrated while improving capture of analytes. **In** some embodiments, the biological sample can be contacted with a shrunken array as described herein. **In** some embodiments, the biological sample and/or the shrunken array can be rehydrated with permeabilization reagents. **In** some embodiments, the biological sample and/or the shrunken array can be rehydrated with a staining solution (e.g., hematoxylin and eosin stain).

(iii) Diffusion-Resistant Media / Lids

To increase efficiency by encouraging analyte diffusion toward the spatially-barcoded capture probes, a diffusion-resistant medium can be used. In general, molecular diffusion of biological analytes occurs in all directions, including toward the capture probes (i.e., toward the spatially-barcoded array), and away from the capture probes (i.e., into the bulk solution). Increasing diffusion toward the spatially-barcoded array reduces analyte diffusion away from the spatially-barcoded array and increases the capturing efficiency of the capture probes.

In some embodiments, a biological sample is placed on the top of a spatially-barcoded substrate and a diffusion-resistant medium is placed on top of the biological sample. For example, the diffusion-resistant medium can be placed onto an array that has been placed in contact with a biological sample. In some embodiments, the diffusion-resistant medium and spatially-barcoded array are the same component. For example, the diffusion-resistant medium can contain spatially-barcoded capture probes within or on the diffusion-resistant medium (e.g., coverslip, slide, hydrogel, or membrane). In some embodiments, a sample is placed on a substrate and a diffusion-resistant medium is placed on top of the biological sample. Additionally, a spatially-barcoded capture probe array can be placed in close proximity over a diffusion-resistant medium. For example, a diffusion-resistant medium may be sandwiched between a spatially-barcoded array and a sample on a substrate. In some embodiments, a diffusion-resistant medium is disposed or spotted onto a sample. In other embodiments, a diffusion-resistant medium is placed in close proximity to a sample.

In general, a diffusion-resistant medium can be any material known to limit diffusivity of biological analytes. For example, a diffusion-resistant medium can be a solid lid (e.g., coverslip or glass slide). In some embodiments, a diffusion-resistant medium may be made of glass, silicon, paper, hydrogel polymer monoliths, or other material. In some embodiments, the glass side can be an acrylated glass slide. In some embodiments, the diffusion-resistant medium is a porous membrane. In some embodiments, the material may be naturally porous. In some embodiments, the material may have pores or wells etched into solid material. In some embodiments, the pore volume can be manipulated to minimize loss of target analytes. In some embodiments, the membrane chemistry can be manipulated to minimize loss of target analytes. In some embodiments, the diffusion-resistant medium (e.g., hydrogel) is covalently attached to a substrate (e.g., glass slide). In some embodiments, a diffusion-resistant medium can be any material known to limit diffusivity of poly(A) transcripts. In some embodiments, a diffusion-resistant medium can be any material known to limit the diffusivity of proteins. In some embodiments, a diffusion-resistant medium can be any material know to limit the diffusivity of macromolecular constituents.

In some embodiments, a diffusion-resistant medium includes one or more diffusion-resistant media. For example, one or more diffusion-resistant media can be combined in a variety of ways prior to placing the media in contact with a biological sample including, without limitation, coating, layering, or spotting. As another example, a hydrogel can be placed onto a biological sample followed by placement of a lid (e.g., glass slide) on top of the hydrogel.

In some embodiments, a force (e.g., hydrodynamic pressure, ultrasonic vibration, solute contrasts, microwave

radiation, vascular circulation, or other electrical, mechanical, magnetic, centrifugal, and/or thermal forces) is applied to control diffusion and enhance analyte capture. In some embodiments, one or more forces and one or more diffusion-resistant media are used to control diffusion and enhance capture. For example, a centrifugal force and a glass slide can used contemporaneously. Any of a variety of combinations of a force and a diffusion-resistant medium can be used to control or mitigate diffusion and enhance analyte capture.

In some embodiments, a diffusion-resistant medium, along with the spatially-barcoded array and sample, is submerged in a bulk solution. In some embodiments, a bulk solution includes permeabilization reagents. In some embodiments, a diffusion-resistant medium includes at least one permeabilization reagent. In some embodiments, a diffusion-resistant medium (i.e. hydrogel) is soaked in permeabilization reagents before contacting the diffusion-resistant medium to the sample. In some embodiments, a diffusion-resistant medium can include wells (e.g., micro-, nano-, or picowells) containing a permeabilization buffer or reagents. In some embodiments, a diffusion-resistant medium can include permeabilization reagents. In some embodiments, a diffusion-resistant medium can contain dried reagents or monomers to deliver permeabilization reagents when the diffusion-resistant medium is applied to a biological sample. In some embodiments, a diffusion-resistant medium is added to the spatially-barcoded array and sample assembly before the assembly is submerged in a bulk solution. In some embodiments, a diffusion-resistant medium is added to the spatially-barcoded array and sample assembly after the sample has been exposed to permeabilization reagents. In some embodiments, permeabilization reagents are flowed through a microfluidic chamber or channel over the diffusion-resistant medium. In some embodiments, the flow controls the sample's access to the permeabilization reagents. In some embodiments, target analytes diffuse out of the sample and toward a bulk solution and get embedded in a spatially-barcoded capture probe-embedded diffusion-resistant medium. In some embodiments, a free solution is sandwiched between the biological sample and a diffusion-resistant medium.

(iv) Active capture methods

In some of the methods described herein, an analyte in a biological sample (e.g., in a cell or tissue section) can be transported (e.g., passively or actively) to a capture probe (e.g., a capture probe affixed to a substrate (e.g., a substrate or bead)).

For example, analytes can be transported to a capture probe (e.g., an immobilized capture probe) using an electric field (e.g., using electrophoresis), pressure, fluid flow, gravity, temperature, and/or a magnetic field. For example, analytes can be transported through, e.g., a gel (e.g., hydrogel), a fluid, or a permeabilized cell, to a capture probe (e.g., an immobilized capture probe) using a pressure gradient, a chemical concentration gradient, a temperature gradient, and/or a pH gradient. For example, analytes can be transported through a gel (e.g., hydrogel), a fluid, or a permeabilized cell, to a capture probe (e.g., an immobilized capture probe).

In some examples, an electrophoretic field can be applied to analytes to facilitate migration of analytes towards a capture probe. In some examples, a sample containing analytes contacts a substrate having capture probes fixed on the substrate (e.g., a slide, cover slip, or bead), and an electric current is applied to promote the directional migration of charged analytes towards capture probes on a substrate. An electrophoresis assembly (e.g., an electrophoretic chamber), where a biological sample is in contact with a cathode and capture probes (e.g., capture probes fixed on a substrate), and where the capture probes are in contact with the biological sample and an anode, can be used to apply the current.

In some embodiments, methods utilizing an active capture method can employ a conductive substrate (e.g., any of the conductive substrates described herein). In some embodiments, a conductive substrate includes paper, a hydrogel film, or a glass slide having a conductive coating. In some embodiments, a conductive substrate (e.g., any of the conductive substrates described herein) includes one or more capture probes.

Non-limiting examples of permeabilization solutions include, enzymes (e.g., proteinase K, pepsin, and collagenase), detergents (e.g., sodium dodecyl sulfate (SDS), polyethylene glycol tert-octylphenyl ether, polysorbate 80, and polysorbate 20), ribonuclease inhibitors, buffers optimized for electrophoresis, buffers optimized for permeabilization, buffers optimized for hybridization, or combinations thereof. Permeabilization reagents can also include but are not limited to a dried permeabilization reagent, a permeabilization buffer, a buffer without a permeabilization reagent, a permeabilization gel, and a permeabilization solution. In some examples, biological samples (e.g., tissue samples) can be permeabilized first and then be subjected to electrophoresis.

In some embodiments, 3, 4, 5, 6, 7, 8, 9, 10, or more biological samples can be placed on a same substrate and be subjected to electrophoresis simultaneously. In some embodiments, 2, 3, 4, 5, 6, 7, 8, 9, 10, or more top substrates can be placed above a same bottom substrate containing one or more samples in order to simultaneously subject the one or more samples to electrophoresis. In some embodiments, 2, 3, 4, 5, 6, 7, 8, 9, 10, or more top substrates can be perpendicularly placed (e.g., at about 90 degrees) above a same bottom substrate containing one or more samples in order to simultaneously subject the one or more samples to electrophoresis. In some embodiments, 2, 3, 4, 5, 6, 7, 8, 9, 10, or more top substrates can be placed in a parallel orientation above a same bottom substrate containing one or more biological samples in order to simultaneously subject the one or more samples to electrophoresis. In some embodiments, a

configuration of top substrates can be arranged above a same bottom substrate containing one or more biological samples in order to simultaneously subject the one or more samples to electrophoresis. In some embodiments, a first configuration of top substrates can be arranged above a second array of bottom substrates containing one or more biological samples in order to simultaneously subject the one or more biological samples to electrophoresis. In some embodiments, simultaneously subjecting two or more biological samples on a same substrate to electrophoresis can provide the advantage of a more effective workflow. In some embodiments, one or more of the top substrates can contain the biological sample.

In some embodiments, methods utilizing an active capture method can include one or more solutions between the biological sample and the substrate (e.g., a substrate including capture probes). In some embodiments, the one or more solutions between the biological sample and the substrate including capture probes can include a permeabilization buffer (e.g., any of the permeabilization buffers described herein). In some embodiments, the one or more solutions between the biological sample and the substrate including capture probes can include an electrophoresis buffer.

In some embodiments, actively capturing analytes can include one or more porous materials between the biological sample and the substrate including capture probes. In some embodiments, the one or more porous materials between the biological sample and substrate including capture probes can include a paper or a blotting membrane. In some embodiments, the one or more porous materials between the biological sample and the substrate including capture probes can include a gel containing one or more solutions. For example, in a non-limiting way, the gel can be a SDS-PAGE gel. In some embodiments, the one or more porous materials between the biological sample and the substrate including capture probes can contain a permeabilization buffer. In some embodiments, the one or more porous materials between the biological sample and the substrate including capture probes can contain an electrophoresis buffer. In some embodiments, actively capturing analytes can include one or more solutions and one or more porous materials between the biological sample and the substrate including capture probes.

In some embodiments, the one or more porous materials between the biological sample and the substrate including capture probes (e.g., an array) can act as a filter to separate analytes (e.g., analytes of interest) from other molecules or analytes present in the biological sample. In some embodiments, the analytes (e.g., analytes of interest) are RNA transcripts. In some embodiments, the one or more porous materials between the biological sample and the substrate including capture probes can act as a filter to separate RNA transcripts from other molecules (e.g., analytes) such as proteins, lipids and/or other nucleic acids. In some embodiments, the one or more porous materials between the biological sample and the substrate including capture probes can act as a filter to separate the analytes and other molecules based on physicochemical properties. For example, in a non-limiting way, analytes can be separated on properties such as charge, size (e.g., length, radius of gyration, effective diameters, etc.), hydrophobicity, hydrophilicity, molecular binding (e.g., immunoaffinity), and combinations thereof. In some embodiments, the one or more porous materials between the biological sample and the substrate including capture probes can separate the analytes from other molecules to reduce non-specific binding near the capture probes and therefore improve binding between the analytes and the capture probes, thus improving subsequent assay performance.

In some embodiments, the one or more porous materials between the biological sample and the substrate including capture probes can act as molecular sieving matrices for electrophoretic analyte separation. For example, in a non-limiting way, separation of analytes can occur based on physicochemical properties such as charge, size (e.g., length, radius of gyration, and effective diameters, etc.), electrophoretic mobility, zeta potential, isoelectric point, hydrophobicity, hydrophilicity, molecular binding (e.g., immunoaffinity), and combinations thereof. In some embodiments, the one or more porous materials between the biological sample and the substrate including capture probes can be of a uniform pore size. In some embodiments, the one or more porous materials between the biological sample and the substrate including capture probes can have discontinuities in pore sizes, as generally used in different gel electrophoresis schemes. In some embodiments, the one or more porous materials between the biological sample and the substrate including capture probes can have gradients in pore sizes. For example, the one or more porous materials (e.g., a hydrogel) can have a gradient of pore sizes such that the gradient separates the analytes as the analytes migrate to the substrate including capture probes (e.g., an array).

In some embodiments, the one or more porous materials between the biological sample and the substrate including capture probes can separate the analytes based on length. For example, shorter analytes will have a higher electrophoretic mobility, and therefore migrate faster towards the capture probes relative to longer analytes in an electrophoretic setup. In some embodiments, the one or more porous materials between the biological sample and the substrate including capture probes separate the analytes based on length, such that only shorter analytes can migrate through the one or more porous materials to reach the capture probes, while longer analytes cannot reach the capture probes.

In some embodiments, specific subsets of analytes (e.g., a subset of transcripts) can be captured by applying an electrophoretic field for a certain amount of time. In some embodiments, specific subsets of analytes (e.g., a subset of transcripts) can be captured by selecting different porous materials (e.g., porous materials with different compositions) between the biological sample and the substrate including capture probes. In some embodiments, specific subsets of analytes (e.g., a subset of transcripts) can be captured by applying an electrophoretic field for a certain amount of time and

selecting different porous materials between the biological sample and the substrate including capture probes (e.g., an array).

In some embodiments, the one or more porous materials between the biological sample and the substrate including capture probes can have discontinuities in pore sizes that can cause an increase in the concentration of the migrating analytes (e.g., "stacking"). For example, the one or more porous materials (e.g., a hydrogel) between the biological sample and the substrate including capture probes can have discontinuities in pore sizes that can cause an increase in the concentration of the analytes near the capture probes resulting in favorable binding kinetics and increased sensitivity. In some embodiments, the one or more porous materials between the biological sample and the substrate including capture probes can have discontinuities in pore sizes that enhance the separation between migrating analytes of different sizes and/or lengths. In some embodiments, the one or more porous materials between the biological sample and the substrate including capture probes can include a first porous material and a second porous material, with the first porous material having a larger pore size than the second porous material. In some embodiments, the first porous material is located on the surface, or near the surface, of the biological sample. In some embodiments, the second porous material (e.g., second porous material with a smaller pore size than the first porous material) can be placed on the surface, or near the surface, of the first porous material. In some embodiments, as analytes migrate (e.g., migrate via electrophoresis) from the biological sample through the first porous material and the second porous material sequentially, the migrating analytes can collect (e.g., "stack") at the interface between the first porous material and the second porous material.

In some embodiments, the one or more porous materials between the biological sample and the substrate including capture probes can include a gradient in pore sizes for continuous stacking as analytes migrate through decreasing pore sizes (e.g., decreasing pore diameter). In some embodiments, the one or more porous materials between the biological sample and the substrate including capture probes can include a gradient in pore sizes such that the pores decrease in diameter as the analytes migrate from the biological sample to the substrate including capture probes. In some embodiments, the pore size gradient can increase the resolution among analytes of different sizes. In some embodiments, the pore size gradient can increase the concentration of the analytes near the capture probes. In some embodiments, the pore size gradient can continuously reduce the speed at which the analytes migrate and collect (e.g., "stack") as the analytes migrate through the gradient of decreasing pore sizes (e.g., decreasing pore diameter).

In some embodiments, the one or more porous materials between the biological sample and the substrate including capture probes can include a gradient gel for continuous stacking as analytes migrate through decreasing pore sizes (e.g., decreasing pore diameter) of the gradient gel. In some embodiments, the gradient gel can have pores with a decreasing diameter as the analytes migrate toward the capture probes. In some embodiments, the gradient gel can increase the separation resolution among analytes of different sizes. In some embodiments, the gradient gel can increase the concentration of analytes near the capture probes. In some embodiments, the gradient gel can continuously reduce the speed at which analytes migrate and collect (e.g., "stack") as the analytes migrate through the gradient gel of decreasing pore sizes (e.g., decreasing in diameter).

In some embodiments, a biological sample can be placed in a first substrate holder (e.g., a substrate holder described herein). In some embodiments, a spatially-barcoded capture probe array (e.g., capture probes, barcoded array) can be placed on a second substrate holder (e.g., a substrate holder described herein). In some embodiments, a biological sample can be placed in a first substrate holder that also contains capture probes. In some embodiments, the first substrate holder, the second substrate holder, or both can be conductive (e.g. any of the conductive substrates described herein). In some embodiments, the first substrate holder including the biological sample, the second substrate holder including capture probes, or both, can be contacted with permeabilization reagents (e.g., a permeabilization buffer) and analytes can be migrated from the biological sample toward the barcoded array using an electric field.

In some embodiments, electrophoresis can be applied to a biological sample on a barcoded array while in contact with a permeabilization buffer. In some embodiments, electrophoresis can be applied to a biological sample on a barcoded array while in contact with an electrophoresis buffer (e.g. a buffer that lacks permeabilization reagents). In some embodiments, the permeabilization buffer can be replaced with an electrophoresis buffer after a desired amount of time. In some embodiments, electrophoresis can be applied simultaneously with the permeabilization buffer or electrophoresis buffer. In some embodiments, electrophoresis can be applied after a desired amount of time of contact between the biological sample and the permeabilization buffer or electrophoresis buffer.

In some embodiments, the biological sample can be placed on a substrate (e.g., a porous membrane, a hydrogel, paper, etc.). In some embodiments, the biological sample placed on the substrate can have a gap (e.g., a space) between the substrate and the substrate holder (e.g., conductive substrate holder). In some embodiments, the barcoded array can be placed on a substrate (e.g., a porous membrane, a hydrogel, paper, etc.). In some embodiments, the barcoded array can have a gap between the substrate and substrate holder (e.g., conductive substrate holder). In some embodiments, the barcoded array can be placed in direct proximity to the biological sample or at a desired distance from the biological sample. In some embodiments, a buffer reservoir can be used between the substrate holder (e.g., conductive substrate holder) and the barcoded array, the biological sample, or both. This setup allows the analytes to be migrated to a barcoded array while not in proximity with the electrodes (e.g. conductive substrate holder), thus resulting in more stable

electrophoresis.

In some embodiments, a combination of at least two buffers with different ionic compositions can be used to differentially migrate analytes based on their ionic mobility (e.g., isotachophoresis (ITP)). For example, using two or more buffers with different ionic compositions can increase the concentration of analytes prior to contact with a barcoded array. Isotachophoresis includes at least two buffers that contain a common counter-ion (e.g., ions that have different charge sign than the analytes) and different co-ions (e.g., ions that have the same charge sign as the analytes) (Smejkal P., et al., Microfluidic isotachophoresis: A review, Electrophoresis, 34.11 1493-1509, (2013) which is incorporated herein by reference in its entirety). In some embodiments, one buffer can contain a co-ion with a higher ionic mobility (e.g. speed at which they travel through solution in an electric field) than the analytes (e.g., the "leading" buffer). In some embodiments, a second buffer can contain a co-ion with a lower ionic mobility than the analytes (e.g., the "trailing" buffer). In some embodiments, a third buffer can contain a co-ion with an ionic mobility that is between the electric mobility of the analytes. In some embodiments, a biological sample can be placed on a first substrate holder (e.g., a conductive substrate holder) and the barcoded array can be placed on a second substrate holder (e.g., a second conductive substrate holder) and contacted with a permeabilization buffer and the analytes can be migrated away from the biological sample and toward the barcoded array using an electric field. As the electric field is applied to the biological sample the analytes can be concentrated in the buffer as they are migrated toward the capture probes. In some embodiments, isotachophoresis can be used with gel-based separations (e.g., any of the gel-based separations described herein).

In some embodiments, a permeabilization buffer can be applied to a region of interest (e.g., region of interest as described herein) in a biological sample. In some embodiments, permeabilization reagents (e.g. a hydrogel containing permeabilization reagents) can be applied to a region of interest in a biological sample. For example, a region of interest can be a region that is smaller in area relative to the overall area of the biological sample. In some embodiments, the permeabilization buffer or permeabilization reagents can be contacted with the biological sample and a substrate including capture probes (e.g., an array). In some embodiments, the biological sample can have more than one region of interest (e.g. two, three). In some embodiments, the biological sample, the substrate including capture probes, or both, can be placed in a conductive substrate holder. In some embodiments, analytes can be released from the region(s) of interest and migrated from the biological sample toward the capture probes with an electric field.

In some embodiments, electrophoretic transfer of analytes can be performed while retaining the relative spatial locations of analytes in a biological sample while minimizing passive diffusion of an analyte away from its location in a biological sample. In some embodiments, an analyte captured by a capture probe (e.g., capture probes on a substrate) retains the spatial location of the analyte present in the biological sample from which it was obtained (e.g., the spatial location of the analyte that is captured by a capture probe on a substrate when the analyte is actively migrated to the capture probe by electrophoretic transfer can be more precise or representative of the spatial location of the analyte in the biological sample than when the analyte is not actively migrated to the capture probe). In some embodiments, electrophoretic transport and binding process is described by the Damköhler number (Da), which is a ratio of reaction and mass transport rates. The fraction of analytes bound and the shape of the biological sample will depend on the parameters in the Da. There parameters include electromigration velocity $U_e$(depending on analyte electrophoretic mobility $\mu_e$ and electric field strength E), density of capture probes (e.g., barcoded oligonucleotides) $p_0$, the binding rate between probes (e.g., barcoded oligonucleotides) and analytes $k_{on}$, and capture area thickness $L$.

$$\mathrm{Da} \sim \frac{k_{on} p_0 L}{\mu_e E}$$

Fast migration (e.g., electromigration) can reduce assay time and can minimize molecular diffusion of analytes.

In some embodiments, electrophoretic transfer of analytes can be performed while retaining the relative spatial alignment of the analytes in the sample. As such, an analyte captured by the capture probes (e.g., capture probes on a substrate) retains the spatial information of the cell or the biological sample from which it was obtained. Applying an electrophoretic field to analytes can also result in an increase in temperature (e.g., heat). In some embodiments, the increased temperature (e.g., heat) can facilitate the migration of the analytes towards a capture probe.

In some examples, a spatially-addressable microelectrode array is used for spatially-constrained capture of at least one charged analyte of interest by a capture probe. For example, a spatially-addressable microelectrode array can allow for discrete (e.g., localized) application of an electric field rather than a uniform electric field. The spatially-addressable microelectrode array can be independently addressable. In some embodiments, the electric field can be applied to one or more regions of interest in a biological sample. The electrodes may be adjacent to each other or distant from each other. The microelectrode array can be configured to include a high density of discrete sites having a small area for applying an electric field to promote the migration of charged analyte(s) of interest. For example, electrophoretic capture can be performed on a region of interest using a spatially-addressable microelectrode array.

A high density of discrete sites on a microelectrode array can be used. The surface can include any suitable density of discrete sites (e.g., a density suitable for processing the sample on the conductive substrate in a given amount of

time). In one embodiment, the surface has a density of discrete sites greater than or equal to about 500 sites per 1 $mm^2$. In some embodiments, the surface has a density of discrete sites of about 100, about 200, about 300, about 400, about 500, about 600, about 700, about 800, about 900, about 1,000, about 2,000, about 3,000, about 4,000, about 5,000, about 6,000, about 7,000, about 8,000, about 9,000, about 10,000, about 20,000, about 40,000, about 60,000, about 80,000, about 100,000, or about 500,000 sites per 1 $mm^2$. In some embodiments, the surface has a density of discrete sites of at least about 200, at least about 300, at least about 400, at least about 500, at least about 600, at least about 700, at least about 800, at least about 900, at least about 1,000, at least about 2,000, at least about 3,000, at least about 4,000, at least about 5,000, at least about 6,000, at least about 7,000, at least about 8,000, at least about 9,000, at least about 10,000, at least about 20,000, at least about 40,000, at least about 60,000, at least about 80,000, at least about 100,000, or at least about 500,000 sites per 1 $mm^2$.

(v) Targeted Analysis

In some aspects, arrays include a plurality of capture probes that bind to one or more specific biological targets in a sample. The capture probes can be directly or indirectly attached to a substrate. The capture probe can be or include, for example, DNA or RNA. In some aspects, the capture probes on an array can be immobilized, e.g., attached or bound, to the array via their 5' or 3' ends, depending on the chemical matrix of the array. In some aspects, the probes are attached via a 3' linkage, thereby leaving a free 5' end. In some aspects, the probes are attached via a 5' linkage, thereby leaving a free 3' end. In some aspects, the probes are immobilized indirectly. For example, a probe can be attached to a bead, which bead can be deposited on a substrate. A capture probe as disclosed in this section can include any of the various components of a capture probe as provided throughout this disclosure (e.g., spatial barcodes, UMIs, functional domains, cleavage domains, etc.).

In some aspects, a capture probe or plurality of capture probes interact with an analyte specific for a particular species or organism (e.g., host or pathogen). In some aspects, the probe or plurality of probes can be used to detect a viral, bacterial, or plant protein or nucleic acid. In some aspects, the capture probe or plurality of capture probes can be used to detect the presence of a pathogen (e.g., bacteria or virus) in the biological sample. In some aspects, the capture probe or plurality of capture probes can be used to detect the expression of a particular nucleic acid associated with a pathogen (e.g., presence of 16S ribosomal RNA or Human Immunodeficiency Virus (HIV) RNA in a human sample).

In some aspects, the capture domain in the capture probe can interact with one or more specific analytes (e.g., an analyte or a subset of analytes out of the pool of total analytes). The specific analyte(s) to be detected can be any of a variety of biological molecules including but not limited to proteins, nucleic acids, lipids, carbohydrates, ions, small molecules, subcellular targets, or multicomponent complexes containing any of the above. In some embodiments, the analyte(s) can be localized to subcellular location(s), including, for example, organelles, e.g., mitochondria, Golgi apparatus, endoplasmic reticulum, chloroplasts, endocytic vesicles, exocytic vesicles, vacuoles, lysosomes, etc. In some embodiments, analyte(s) can be peptides or proteins, including without limitation antibodies and enzymes.

In some aspects, analytes from a biological sample interact with one or more capture probes (e.g., one or more capture probes immobilized directly or indirectly on a substrate), and the capture probes interact with specific analytes in the biological sample. In some aspects, the capture probes are allowed to interact with (e.g., hybridize to) specific analytes, e.g., under appropriate conditions where oligonucleotide capture probes can hybridize to the target nucleic acids. In some aspects, analytes that did not hybridize to capture probes are removed (e.g., analytes that do not interact with capture domains of the capture probes). In some embodiments, removal of analytes that did not interact with a capture probe can be accomplished by, e.g., washing the sample to remove such analytes.

In some aspects, a capture probe or plurality of capture probes includes a capture domain that interacts with an analyte or analytes present in a biological sample. In some aspects, the capture probe or plurality of capture probes includes a capture domain that detects the presence or level amount (e.g., expression level) of a particular analyte or analytes of interest. The capture domain of a capture probe (immobilized directly or indirectly on a substrate) can be capable of binding selectively to a desired subtype or subset of nucleic acid. In some aspects, for example, the capture domain binds to a subset of nucleic acids in a genome or a subset of nucleic acids in a transcriptome. In some aspects, the analyte(s) can include one or more nucleic acids. In some aspects, the capture probe or plurality of capture probes can be used to detect the expression of a particular transcript (e.g., a particular mRNA). In some aspects, a capture probe or plurality of capture probes can be specific for (e.g., binds to) an individual change in a nucleic acid or protein (e.g., a mutation or single nucleotide polymorphism (SNP)).

In some aspects, the biological sample includes an analyte that is or includes a nucleic acid. The nucleic acid can be RNA or DNA. In some aspects, the capture probe or plurality of capture probes detects DNA copy number of a particular set of nucleic acid analyte or analytes. For example, capture probe or plurality of capture probes provided herein can be used to detect DNA copy number of nucleic acids that share homology to each other.

In some aspects, the capture probe or plurality of capture probes includes a capture domain that detects the presence or level amount (e.g., expression level) of one or more RNA transcripts (e.g., specific RNA transcripts). In some

aspects, the capture probe or plurality of capture probes includes a capture domain that detects the presence or amount (e.g., expression level) of one or more non-coding RNAs (e.g., microRNA, transfer RNA (tRNA), ribosomal RNA (rRNA), small interfering RNA (siRNA) and small nucleolar RNA (snoRNA). In some aspects, the probe or plurality of probes includes a capture domain that detects the presence or level amount (e.g., expression level) of one or more proteins (e.g., proteins expressed of a nucleic acid of interest).

In some aspects, the capture probe or plurality of capture probes can be specific for a particular protein. In some aspects, the capture probe or plurality of capture probes can be used to detect the presence of a particular protein of interest. In some aspects, the capture probe or plurality of capture probes can be used to detect translation of a particular protein. In some aspects, the capture probe or plurality of capture probes can specifically interact with an active region of an enzyme, a binding domain of an immunoglobulin, defined domains of proteins, whole proteins, synthetic peptides, peptides with introduced mutations, aptamer, or any combination thereof. In some aspects, the analyte(s) can include one or more proteins. In some aspects, the analyte(s) can include one or more nucleic acids and one or more proteins.

In some aspects, the capture probe or plurality of capture probes can be used to detect particular post-translational modifications of a particular protein. In such embodiments, analyte capture agents can be specific for cell surface analytes having a given state of posttranslational modification (e.g., phosphorylation, glycosylation, ubiquitination, nitrosylation, methylation, acetylation or lipidation), such that a cell surface analyte profile can include posttranslational modification information of one or more analytes.

In some aspects, the capture probe or plurality of capture probes can be specific for a particular set of nucleic acids (e.g., nucleic acids that are associated with a specific cellular pathway or pathways). In some aspects, the set of nucleic acids is DNA. In some aspects, the set of nucleic acids is RNA. In some aspects, the set of nucleic acids has similar and/or homologous sequences. In some aspects, the set of nucleic acids encodes for analytes that function in a similar cellular pathway. In some aspects, the set of nucleic acids encodes for analytes that are expressed in a certain pathological state (e.g., cancer, Alzheimer's, or Parkinson's disease). In some aspects, the set of nucleic acids encodes for analytes that are over-expressed in a certain pathological state. In some aspects, the set of nucleic acids encodes for analytes that are under-expressed in a certain pathological state.

In some aspects, the capture probe or plurality of capture probes can be specific for a particular nucleic acid, or detection or expression of a particular set of proteins (e.g., in a similar cellular pathway). In some aspects, the set of proteins has similar functional domains. In some aspects, the set of proteins functions in a similar cellular pathway. In some aspects, the set of proteins is expressed in a certain pathological state (e.g., cancer, Alzheimer's or Parkinson's disease). In some aspects, the set of proteins is over-expressed in a certain pathological state. In some aspects, the set of proteins is under-expressed in a certain pathological state.

In some embodiments, a capture probe includes a capture domain that is capable of binding to more than one analyte. In some embodiments, a capture domain can bind to one or more analytes that are about 80% identical, about 85% identical, about 90% identical, about 95% identical, about 96% identical, about 97% identical, about 98% identical, about 99% identical, 100% identical to the target analyte. In some aspects, the capture probe can bind to an analyte that is about 80% identical, about 85% identical, about 90% identical, about 95% identical, about 96% identical, about 97% identical, about 98% identical, or about 99% identical to each other. In some embodiments, a capture domain can bind to a conserved region of one or more analytes, in which the conserved regions are about 80% identical, about 85% identical, about 90% identical, about 95% identical, about 96% identical, about 97% identical, about 98% identical, about 99% identical, 100% identical to the target analyte.

In some aspects, a capture probe or plurality of capture probes interacts with two or more analytes (e.g., nucleic acids or proteins) that are not similar in sequence and/or do not share a conserved domain. In some embodiments, a capture probe includes two or more capture domains, each of which interacts with a different analyte. In such embodiments, members of the two or more capture domains can be adjacent to each other in the capture probe and/or members of the two or more capture domains can be separated from each other in the capture probe by one or more domains (e.g., nucleic acid domains). For example, in some aspects, the sets of analytes that are detected include mutational changes in the targeted nucleic acids or proteins. In some aspects, the capture probe or plurality of capture probes detects sets of nucleic acids or proteins (e.g., non-homologous nucleic acids or proteins) that are individually mutated during a pathogenic state. In some aspects, the pathogenic state is cancer.

In some aspects, a capture probe or plurality of capture probes include capture domains that can be used to detect analytes that are typically detected using diagnostic panels. In some aspects, the capture probe or plurality of capture probes are used to detect changes in one or more analytes. In some aspects, the analyte changes include one or more of increased analyte expression, decreased analyte expression, mutated nucleic acid sequences, or any combination thereof. In some aspects, the changes in the analytes are associated with and/or lead to manifestation of a pathogenic state in a subject. In some aspects, the detected changes are compared to a reference analyte or analytes.

(vi) Polypeptide Capture

Provided herein are methods and materials for identifying the location of a polypeptide in a biological sample. In some embodiments, an analyte (e.g., a polypeptide analyte) can be directly captured on a substrate. For example, polypeptide analytes can be captured by amine groups on a functionalized substrate. In other examples, an analyte (e.g., a polypeptide analyte) can be captured via an analyte binding moiety directly attached to a substrate. In some embodiments, the substrate may be populated with analyte minding moieties directly attached to the substrate as well as spatially-barcoded capture probes directly attached to the substrate. In other embodiments, an analyte (e.g., a polypeptide analyte) can be captured via an analyte binding moiety indirectly attached to a substrate. In an example, the substrate may be populated with capture probes that are bound to an analyte capture agent, wherein the analyte capture domain of the analyte capture agent binds to the capture domain of the capture probe and the analyte binding moiety binds the polypeptide analyte.

In some embodiments, an analyte (e.g., a polypeptide analyte) can be directly captured or immobilized on a substrate. Direct immobilization may be achieved by covalently coupling the polypeptide analyte to the substrate via amide bonds between the carboxylic acid of the C-terminal amino acid residue and a functionalized substrate surface. For example, a substrate (e.g., a glass coverslip or slide) can be functionalized through amino-silanization with aminopropyltriethoxysilane. The substrate surfaces are further passivated by overnight incubation with polyethylene glycol (PEG)-NHS solution, and functionalized slides can be stored in a vacuum desiccator until use. The t-butyloxycarbonyl protecting groups can be removed by incubating the substrate with 90% TFA (v/v in water) for 5 hours before use, thus exposing free amine groups for peptide immobilization. The resulting functionalized substrate is stable to multiple cycles of Edman degradation and washing steps.

In some embodiments, methods for capturing polypeptides in a biological sample include providing a substrate where an analyte binding moiety is directly immobilized on the substrate. In some embodiments, direct immobilization is achieved through chemical modification of the substrate and/or chemical modification of the analyte binding moiety. For example, a substrate can be prepared with free amines on the surface. When exposed to an analyte binding moiety with a free carboxylic acid on the C-terminal residue, the free amines can form amide bonds with the carboxylic acid thereby covalently coupling the analyte binding moiety to the substrate. Substrates and/or analyte binding moieties can be modified in any manner that facilitates covalent bonding of the analyte binding moiety to the substrate. Non-limiting examples of chemical modification that can be used to covalently bind the analyte binding moiety to the substrate include are described herein.

In some embodiments, methods for capturing analyte polypeptides include providing a substrate (e.g., an array) where the analyte binding moiety is indirectly attached to the substrate. For example, an analyte binding moiety can be indirectly attached to a substrate via an oligonucleotide (e.g., a capture agent barcode domain or capture agent barcode domain hybridized to a capture probe) or other domain capable of binding to both the substrate and the analyte binding domain. The capture agent barcode domain is described elsewhere herein. The capture agent barcode domain can be modified to include a cleavage domain, which can attach to a substrate using any of the chemistries described herein. In some embodiments, the capture agent barcode domain can include an analyte capture sequence as described herein, wherein the analyte capture sequence can hybridize to the capture domain of a capture probe. In some embodiments, a substrate (e.g., an array) containing capture probes can be modified to capture polypeptide analytes by hybridizing the analyte capture sequence of the analyte capture agent to the capture domain of a capture probe.

In some embodiments, methods for capturing analyte polypeptides include providing a substrate (e.g., an array) and providing an analyte capture agent to the biological sample. For example, after drying and fixing sectioned tissue samples, the tissue samples can be positioned on a substrate (e.g., a spatial array), rehydrated, blocked, and permeabilized (e.g., 3XSSC, 2% BSA, 0.1% Triton X, 1 U/μl RNAse inhibitor for 10 min at 4°C) before being stained with fluorescent primary antibodies (1:100) and a pool of analyte capture agents (in 3XSSC, 2% BSA, 0.1% Triton X, 1 U/μl RNAse inhibitor for 30 min at 4°C). The biological sample can be washed, coverslipped (in glycerol + 1 U/μl RNAse inhibitor), imaged for detected analytes (e.g., using a confocal microscope or other apparatus capable of fluorescent detection), and washed again. The analyte-bound analyte capture agents can be released from the biological sample (e.g., the biological sample can be treated with proteinase, e.g., proteinase K) and migrated to the spatial array. An analyte capture sequence of the analyte-bound analyte capture agent can be captured by a capture probe capture domain, and the capture agent barcode domain can be extended to produce a spatially-tagged analyte capture agent. The spatially-tagged analyte capture agents can be processed according to spatial workflows described herein.

In some embodiments, methods for capturing analyte polypeptides include providing blocking probes to analyte capture agents before introducing the analyte capture agents to a biological sample. In some embodiments, the blocking probes can be alternatively or additionally provided in any of the rehydrating or blocking buffers provided herein. In some embodiments, the analyte capture agent analyte capture sequence can be blocked prior to binding to the capture probe capture domain using a blocking probe sequence complementary to the analyte capture sequence. Blocking the capture agent barcode domain, particularly the free 3' end of the capture agent barcode domain (e.g., analyte capture sequence),

prior to contacting the analyte capture agents with the biological sample and/or substrate, can prevent binding of the analyte capture sequence of the capture agent barcode domains, e.g., prevents the binding of a poly(A) tail to the capture probe capture domain. In some embodiments, blocking the analyte capture agent analyte capture domain reduces non-specific background staining. In some embodiments, the blocking probes are reversible, such that the blocking probes can be removed from the analyte capture sequence during or after the time that analyte capture agents are in contact with the biological sample. In some embodiments, the blocking probe can be removing with RNAse treatment (e.g., RNAse H treatment).

In some embodiments, methods for capturing polypeptides in a biological sample include active transfer (e.g., electrophoresis). For example, the biological sample is placed on a conductive substrate and contacted with a spatial array including one or more analyte binding moieties. An electric filed can be applied to the conductive substrate to promote migration of the polypeptides towards the analyte binding moieties, as described herein.

In some embodiments, methods for identifying the spatial location of a polypeptide in a biological sample include determining the sequence of a captured polypeptide. In some embodiments, the sequence of the captured polypeptide is determined through detection of amino acid residues labeled with a detectable label (e.g., radiolabel of a fluorophore). Non-limiting examples of detectable labels that can be used for labelling the captured polypeptide include fluorophores and radiolabels. In some embodiments, the polypeptides are labeled at specific amino acid residues only (e.g., not all amino acid residues are labeled). In some embodiments, the polypeptide is labeled prior to contacting the biological sample with the substrate. In some embodiments, a captured polypeptide is labeled with fluorophores using standard coupling schemes (see Hernandez et al., New J. Chem. 41:462-469 (2017)). For example, polypeptides may be labeled by reaction with Atto647N-NHS, Atto647Niodoacetamide, TMR-NHS, or JF549-NHS, as appropriate, to label lysines (via NHS) or cysteines (via iodoacetamide). In addition, serine or threonine phosphorylation sites may be selectively labeled via beta elimination followed by conjugate addition via thiols to substitute thiol-linked fluorophores in place of phosphates (see Stevens et al., Rapid Commun. Mass Specrtom., 15: 2157-2162 (2005)). The number of fluorophores incorporated into a polypeptide is any number that may be spectrally resolved. In some instances, four or more fluorophores are utilized.

In some embodiments, a captured polypeptide is radiolabeled. In some embodiments, specific amino acids can be labeled with an isotope. Non-limiting examples of isotopes used to label amino acids include $^{3}H$, $^{14}C$, $^{15}N$, $^{32}P$, and $^{125}I$. In some embodiments, the isotope is incorporated into the selected amino acid prior to incorporation into a polypeptide. In some embodiments, the radiolabeled amino acid can be incorporated into the polypeptide after polypeptide formation.

In some embodiments, the sequence of the captured polypeptide is determined using Edman degradation (and in some embodiments successive rounds of Edman degradation). In such cases, a polypeptide is captured, and the polypeptide sequence can be resolved by imaging the substrate following repeated rounds of Edman degradation. For example, the substrate is imaged following each Edman reaction in order to capture the detectable labels that are produced due to the removal of amino acids that are a byproduct of the reaction. The information obtained by the Edman degradation can be complied to identify a polypeptide. In some embodiments, the biological sample is visualized or imaged using light or fluorescence microscopy.

(vii) Region of Interest

A biological sample can have regions that show morphological feature(s) that may indicate the presence of disease or the development of a disease phenotype. For example, morphological features at a specific site within a tumor biopsy sample can indicate the aggressiveness, therapeutic resistance, metastatic potential, migration, stage, diagnosis, and/or prognosis of cancer in a subject. A change in the morphological features at a specific site within a tumor biopsy sample often correlate with a change in the level or expression of an analyte in a cell within the specific site, which can, in turn, be used to provide information regarding the aggressiveness, therapeutic resistance, metastatic potential, migration, stage, diagnosis, and/or prognosis of cancer in a subject. A region or area within a biological sample that is selected for specific analysis (e.g., a region in a biological sample that has morphological features of interest) is often described as "a region of interest."

A region of interest in a biological sample can be used to analyze a specific area of interest within a biological sample, and thereby, focus experimentation and data gathering to a specific region of a biological sample (rather than an entire biological sample). This results in increased time efficiency of the analysis of a biological sample.

A region of interest can be identified in a biological sample using a variety of different techniques, e.g., expansion microscopy, bright field microscopy, dark field microscopy, phase contrast microscopy, electron microscopy, fluorescence microscopy, reflection microscopy, interference microscopy, confocal microscopy, and visual identification (e.g., by eye), and combinations thereof. For example, the staining and imaging of a biological sample can be performed to identify a region of interest. In some examples, the region of interest can correspond to a specific structure of cytoarchitecture. In some embodiments, a biological sample can be stained prior to visualization to provide contrast between the different regions of the biological sample. The type of stain can be chosen depending on the type of biological sample and the region of the cells to be stained. In some embodiments, more than one stain can be used to visualize different aspects of the

biological sample, e.g., different regions of the sample, specific cell structures (e.g., organelles), or different cell types. In other embodiments, the biological sample can be visualized or imaged without staining the biological sample.

In some embodiments, staining and imaging a biological sample prior to contacting the biological sample with a spatial array is performed to select samples for spatial analysis. In some embodiments, the staining includes applying a fiducial marker as described herein, including fluorescent, radioactive, chemiluminescent, or colorimetric detectable markers. In some embodiments, the staining and imaging of biological samples allows the user to identify the specific sample (or region of interest) the user wishes to assess.

In some examples, an array (e.g., any of the exemplary arrays described herein) can be contacted with only a portion of a biological sample (e.g., a cell, a tissue section, or a region of interest). In some examples, a biological sample is contacted with only a portion of an array (e.g., any of the exemplary arrays described herein). In some embodiments, capture probes on an array corresponding to regions of interest of a biological sample (e.g., proximal to the region of interest) can be selectively cleaved and analyzed. For example, capture probes on an array may be deactivated or eliminated outside of areas corresponding to regions of interest of a biological sample. In some embodiments, capture probes including a photocleavable bond and on the array in areas corresponding to regions of interest of a biological sample can be selectively cleaved by using light. A mirror, mirror array, a lens, a moving stage, and/or a photomask can be used to direct the light to regions of the array that correspond to areas outside one or more regions of interest in the biological sample. Some embodiments include deactivating or eliminating capture probes, e.g., capture probes comprising a photocleavable bond as described herein, using light. In some embodiments, a laser, e.g., a scanning laser, can be used to deactivate or eliminate capture probes. In some embodiments, the eliminated member of the plurality of capture probes can be washed away. In some embodiments, regions of interest can be labeled with different heavy metals, and a laser can sequentially ablate these regions of interest before mass spectrometry identification. A laser can, for example, deactivate or eliminate capture probes through UV light destruction of DNA, heat, inducing a chemical reaction that prevents the capture probes from moving to the next step, inducing photocleavage of a photocleavable bond, or a combination thereof. In some examples, a portion of the array can be deactivated such that it does not interact with the analytes in the biological sample (e.g., optical deactivation, chemical deactivation, heat deactivation, or blocking of the capture probes in the array (e.g., using blocking probes)). In some embodiments, the capture probes can be blocked (e.g., masked or modified) prior to contacting the biological sample with the array. For example, the free 3' end of the capture probe can be blocked or modified prior to contacting the biological sample with the array to avoid modification of the capture probes (e.g., to avoid the removal or modification or the free 3' OH group on the end of the capture probes). In some embodiments, the capture probes can be blocked prior to contacting the biological sample to the array. In some embodiments, the blocking probe is used to block or modify the free 3' end of the capture domain of the capture probe. In some embodiments, the blocking probes can be hybridized to the capture probe. In some embodiments, the free 3' end of the capture domain can be blocked by chemical modification.

In some examples, a region of interest can be removed from a biological sample and then the region of interest can be contacted to the array (e.g., any of the arrays described herein). A region of interest can be removed from a biological sample using microsurgery, laser capture microdissection, chunking, a microtome, dicing, trypsinization, labelling, and/or fluorescence-assisted cell sorting, and the like. In some embodiments, the biological sample is dissected using laser capture microdissection, retaining one or more portions of biological sample for analysis and/or discarding one or more portions of biological sample. In some embodiments, the biological sample is dissected on the array. In some embodiments, one or more regions of interest are selected using spatially addressable microelectrode arrays.

In some examples, a region of interest can be permeabilized or lysed while areas outside the region of interest are not permeabilized or lysed (e.g., Kashyap et al. Sci Rep. 2016; 6: 29579, herein incorporated by reference in its entirety). For example, in some embodiments, a region of interest can be contacted with a hydrogel comprising a permeabilization or lysing reagent. In some embodiments, the area(s) outside the region of interest are not contacted with the hydrogel comprising the permeabilization or lysing reagent. In some embodiments, the eliminated members of the plurality of capture probes are washed away after the permeabilization of the biological sample.

(g) Analysis of Captured Analytes

(i) Sample Removal from an Array

In some embodiments, after contacting a biological sample with a substrate that includes capture probes, a removal step can optionally be performed to remove all or a portion of the biological sample from the substrate. In some embodiments, the removal step includes enzymatic and/or chemical degradation of cells of the biological sample. For example, the removal step can include treating the biological sample with an enzyme (e.g., a proteinase, e.g., proteinase K) to remove at least a portion of the biological sample from the substrate. In some embodiments, the removal step can include ablation of the tissue (e.g., laser ablation).

In some embodiments, provided herein are methods for spatially detecting an analyte (e.g., detecting the location

of an analyte, e.g., a biological analyte) from a biological sample (e.g., present in a biological sample), the method comprising: (a) optionally staining and/or imaging a biological sample on a substrate; (b) permeabilizing (e.g., providing a solution comprising a permeabilization reagent to) the biological sample on the substrate; (c) contacting the biological sample with an array comprising a plurality of capture probes, wherein a capture probe of the plurality captures the biological analyte; and (d) analyzing the captured biological analyte, thereby spatially detecting the biological analyte; wherein the biological sample is fully or partially removed from the substrate.

In some embodiments, a biological sample is not removed from the substrate. For example, the biological sample is not removed from the substrate prior to releasing a capture probe (e.g., a capture probe bound to an analyte) from the substrate. In some embodiments, such releasing comprises cleavage of the capture probe from the substrate (e.g., via a cleavage domain). In some embodiments, such releasing does not comprise releasing the capture probe from the substrate (e.g., a copy of the capture probe bound to an analyte can be made and the copy can be released from the substrate, e.g., via denaturation). In some embodiments, the biological sample is not removed from the substrate prior to analysis of an analyte bound to a capture probe after it is released from the substrate. In some embodiments, the biological sample remains on the substrate during removal of a capture probe from the substrate and/or analysis of an analyte bound to the capture probe after it is released from the substrate. In some embodiments, the biological sample remains on the substrate during removal (e.g., via denaturation) of a copy of the capture probe (e.g., complement). In some embodiments, analysis of an analyte bound to capture probe from the substrate can be performed without subjecting the biological sample to enzymatic and/or chemical degradation of the cells (e.g., permeabilized cells) or ablation of the tissue (e.g., laser ablation).

In some embodiments, at least a portion of the biological sample is not removed from the substrate. For example, a portion of the biological sample can remain on the substrate prior to releasing a capture probe (e.g., a capture prove bound to an analyte) from the substrate and/or analyzing an analyte bound to a capture probe released from the substrate. In some embodiments, at least a portion of the biological sample is not subjected to enzymatic and/or chemical degradation of the cells (e.g., permeabilized cells) or ablation of the tissue (e.g., laser ablation) prior to analysis of an analyte bound to a capture probe from the substrate.

In some embodiments, provided herein are methods for spatially detecting an analyte (e.g., detecting the location of an analyte, e.g., a biological analyte) from a biological sample (e.g., present in a biological sample) that include: (a) optionally staining and/or imaging a biological sample on a substrate; (b) permeabilizing (e.g., providing a solution comprising a permeabilization reagent to) the biological sample on the substrate; (c) contacting the biological sample with an array comprising a plurality of capture probes, wherein a capture probe of the plurality captures the biological analyte; and (d) analyzing the captured biological analyte, thereby spatially detecting the biological analyte; where the biological sample is not removed from the substrate.

In some embodiments, provided herein are methods for spatially detecting a biological analyte of interest from a biological sample that include: (a) staining and imaging a biological sample on a substrate; (b) providing a solution comprising a permeabilization reagent to the biological sample on the substrate; (c) contacting the biological sample with an array on a substrate, wherein the array comprises one or more capture probe pluralities thereby allowing the one or more pluralities of capture probes to capture the biological analyte of interest; and (d) analyzing the captured biological analyte, thereby spatially detecting the biological analyte of interest; where the biological sample is not removed from the substrate.

In some embodiments, the method further includes selecting a region of interest in the biological sample to subject to spatial transcriptomic analysis. In some embodiments, one or more of the one or more capture probes include a capture domain. In some embodiments, one or more of the one or more capture probe pluralities comprise a unique molecular identifier (UMI). In some embodiments, one or more of the one or more capture probe pluralities comprise a cleavage domain. In some embodiments, the cleavage domain comprises a sequence recognized and cleaved by a uracil-DNA glycosylase, apurinic/apyrimidinic (AP) endonuclease (APE1), U uracil-specific excision reagent (USER), and/or an endonuclease VIII. In some embodiments, one or more capture probes do not comprise a cleavage domain and is not cleaved from the array.

(ii) Extended Capture Probes

In some embodiments, a capture probe can be extended (an "extended capture probe," e.g., as described herein (e.g., Section II(b)(vii))). For example, extending a capture probe can include generating cDNA from a captured (hybridized) RNA. This process involves synthesis of a complementary strand of the hybridized nucleic acid, e.g., generating cDNA based on the captured RNA template (the RNA hybridized to the capture domain of the capture probe). Thus, in an initial step of extending a capture probe, e.g., the cDNA generation, the captured (hybridized) nucleic acid, e.g., RNA, acts as a template for the extension, e.g., reverse transcription, step.

In some embodiments, the capture probe is extended using reverse transcription. For example, reverse transcription includes synthesizing cDNA (complementary or copy DNA) from RNA, e.g., (messenger RNA), using a

reverse transcriptase. In some embodiments, reverse transcription is performed while the tissue is still in place, generating an analyte library, where the analyte library includes the spatial barcodes from the adjacent capture probes. In some embodiments, the capture probe is extended using one or more DNA polymerases.

In some embodiments, a capture domain of a capture probe includes a primer for producing the complementary strand of a nucleic acid hybridized to the capture probe, e.g., a primer for DNA polymerase and/or reverse transcription. The nucleic acid, e.g., DNA and/or cDNA, molecules generated by the extension reaction incorporate the sequence of the capture probe. The extension of the capture probe, e.g., a DNA polymerase and/or reverse transcription reaction, can be performed using a variety of suitable enzymes and protocols.

In some embodiments, a full-length DNA (e.g., cDNA) molecule is generated. In some embodiments, a "full-length" DNA molecule refers to the whole of the captured nucleic acid molecule. However, if a nucleic acid (e.g., RNA) was partially degraded in the tissue sample, then the captured nucleic acid molecules will not be the same length as the initial RNA in the tissue sample. In some embodiments, the 3' end of the extended probes, e.g., first strand cDNA molecules, is modified. For example, a linker or adaptor can be ligated to the 3' end of the extended probes. This can be achieved using single stranded ligation enzymes such as T4 RNA ligase or Circligase™ (available from Lucigen, Middleton, WI). In some embodiments, template switching oligonucleotides are used to extend cDNA in order to generate a full-length cDNA (or as close to a full-length cDNA as possible). In some embodiments, a second strand synthesis helper probe (a partially double stranded DNA molecule capable of hybridizing to the 3' end of the extended capture probe), can be ligated to the 3' end of the extended probe, e.g., first strand cDNA, molecule using a double stranded ligation enzyme such as T4 DNA ligase. Other enzymes appropriate for the ligation step are known in the art and include, e.g., Tth DNA ligase, Taq DNA ligase, *Thermococcus* sp. (strain 9°N) DNA ligase (9°N™ DNA ligase, New England Biolabs), Ampligase™ (available from Lucigen, Middleton, WI), and SplintR (available from New England Biolabs, Ipswich, MA). In some embodiments, a polynucleotide tail, e.g., a poly(A) tail, is incorporated at the 3' end of the extended probe molecules. In some embodiments, the polynucleotide tail is incorporated using a terminal transferase active enzyme.

In some embodiments, double-stranded extended capture probes are treated to remove any unextended capture probes prior to amplification and/or analysis, e.g., sequence analysis. This can be achieved by a variety of methods, e.g., using an enzyme to degrade the unextended probes, such as an exonuclease enzyme, or purification columns.

In some embodiments, extended capture probes are amplified to yield quantities that are sufficient for analysis, e.g., via DNA sequencing. In some embodiments, the first strand of the extended capture probes (e.g., DNA and/or cDNA molecules) acts as a template for the amplification reaction (e.g., a polymerase chain reaction).

In some embodiments, the amplification reaction incorporates an affinity group onto the extended capture probe (e.g., RNA-cDNA hybrid) using a primer including the affinity group. In some embodiments, the primer includes an affinity group and the extended capture probes includes the affinity group. The affinity group can correspond to any of the affinity groups described previously.

In some embodiments, the extended capture probes including the affinity group can be coupled to a substrate specific for the affinity group. In some embodiments, the substrate can include an antibody or antibody fragment. In some embodiments, the substrate includes avidin or streptavidin and the affinity group includes biotin. In some embodiments, the substrate includes maltose and the affinity group includes maltose-binding protein. In some embodiments, the substrate includes maltose-binding protein and the affinity group includes maltose. In some embodiments, amplifying the extended capture probes can function to release the extended probes from the surface of the substrate, insofar as copies of the extended probes are not immobilized on the substrate.

In some embodiments, the extended capture probe or complement or amplicon thereof is released. The step of releasing the extended capture probe or complement or amplicon thereof from the surface of the substrate can be achieved in a number of ways. In some embodiments, an extended capture probe or a complement thereof is released from the array by nucleic acid cleavage and/or by denaturation (e.g., by heating to denature a double-stranded molecule).

In some embodiments, the extended capture probe or complement or amplicon thereof is released from the surface of the substrate (e.g., array) by physical means. For example, where the extended capture probe is indirectly immobilized on the array substrate, e.g., via hybridization to a surface probe, it can be sufficient to disrupt the interaction between the extended capture probe and the surface probe. Methods for disrupting the interaction between nucleic acid molecules include denaturing double stranded nucleic acid molecules are known in the art. A straightforward method for releasing the DNA molecules (i.e., of stripping the array of extended probes) is to use a solution that interferes with the hydrogen bonds of the double stranded molecules. In some embodiments, the extended capture probe is released by a applying heated solution, such as water or buffer, of at least 85°C, e.g., at least 90, 91, 92, 93, 94, 95, 96, 97, 98, or 99°C. In some embodiments, a solution including salts, surfactants, etc. that can further destabilize the interaction between the nucleic acid molecules is added to release the extended capture probe from the substrate.

In some embodiments, where the extended capture probe includes a cleavage domain, the extended capture probe is released from the surface of the substrate by cleavage. For example, the cleavage domain of the extended capture probe can be cleaved by any of the methods described herein. In some embodiments, the extended capture probe

is released from the surface of the substrate, e.g., via cleavage of a cleavage domain in the extended capture probe, prior to the step of amplifying the extended capture probe.

In some embodiments, probes complementary to the extended capture probe can be contacted with the substrate. In some embodiments, the biological sample can be in contact with the substrate when the probes are contacted with the substrate. In some embodiments, the biological sample can be removed from the substrate prior to contacting the substrate with probes. In some embodiments, the probes can be labeled with a detectable label (e.g., any of the detectable labels described herein). In some embodiments, probes that do not specially bind (e.g., hybridize) to an extended capture probe can be washed away. In some embodiments, probes complementary to the extended capture probe can be detected on the substrate (e.g., imaging, any of the detection methods described herein).

In some embodiments, probes complementary to an extended capture probe can be about 4 nucleotides to about 100 nucleotides long. In some embodiments, probes (e.g., detectable probes) complementary to an extended capture probe can be about 10 nucleotides to about 90 nucleotides long. In some embodiments, probes (e.g., detectable probes) complementary to an extended capture probe can be about 20 nucleotides to about 80 nucleotides long. In some embodiments, probes (e.g., detectable probes) complementary to an extended capture probe can be about 30 nucleotides to about 60 nucleotides long. In some embodiments, probes (e.g., detectable probes) complementary to an extended capture probe can be about 40 nucleotides to about 50 nucleotides long. In some embodiments, probes (e.g., detectable probes) complementary to an extended capture probe can be about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 51, about 52, about 53, about 54, about 55, about 56, about 57, about 58, about 59, about 60, about 61, about 62, about 63, about 64, about 65, about 66, about 67, about 68, about 69, about 70, about 71, about 72, about 73, about 74, about 75, about 76, about 77, about 78, about 79, about 80, about 81, about 82, about 83, about 84, about 85, about 86, about 87, about 88, about 89, about 90, about 91, about 92, about 93, about 94, about 95, about 96, about 97, about 98, and about 99 nucleotides long.

In some embodiments, about 1 to about 100 probes can be contacted to the substrate and specifically bind (e.g., hybridize) to an extended capture probe. In some embodiments, about 1 to about 10 probes can be contacted to the substrate and specifically bind (e.g., hybridize) to an extended capture probe. In some embodiments, about 10 to about 100 probes can be contacted to the substrate and specifically bind (e.g., hybridize) to an extended capture probe. In some embodiments, about 20 to about 90 probes can be contacted to the substrate and specifically bind (e.g., hybridize) to an extended capture probe. In some embodiments, about 30 to about 80 probes (e.g., detectable probes) can be contacted to the substrate and specifically bind (e.g., hybridize) to an extended capture probe. In some embodiments, about 40 to about 70 probes can be contacted to the substrate and specifically bind (e.g., hybridize) to an extended capture probe. In some embodiments, about 50 to about 60 probes can be contacted to the substrate and specifically bind (e.g., hybridize) to an extended capture probe. In some embodiments, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 51, about 52, about 53, about 54, about 55, about 56, about 57, about 58, about 59, about 60, about 61, about 62, about 63, about 64, about 65, about 66, about 67, about 68, about 69, about 70, about 71, about 72, about 73, about 74, about 75, about 76, about 77, about 78, about 79, about 80, about 81, about 82, about 83, about 84, about 85, about 86, about 87, about 88, about 89, about 90, about 91, about 92, about 93, about 94, about 95, about 96, about 97, about 98, and about 99 probes can be contacted to the substrate and specifically bind (e.g., hybridize) to an extended capture probe.

In some embodiments, the probes can be complementary to a single analyte (e.g., a single gene). In some embodiments, the probes can be complementary to one or more analytes (e.g., analytes in a family of genes). In some embodiments, the probes (e.g., detectable probes) can be for a panel of genes associated with a disease (e.g., cancer, Alzheimer’s disease, Parkinson’s disease).

(iii) Cleavage Domain

Capture probes can optionally include a "cleavage domain," where one or more segments or regions of the capture probe (e.g., spatial barcodes and/or UMIs) can be releasably, cleavably, or reversibly attached to a feature, or some other substrate, so that spatial barcodes and/or UMIs can be released or be releasable through cleavage of a linkage between the capture probe and the feature, or released through degradation of the underlying substrate or chemical substrate, allowing the spatial barcode(s) and/or UMI(s) of the cleaved capture probe to be accessed or be accessible by other reagents, or both. Non-limiting aspects of cleavage domains are described herein (e.g., in Section II(b)(ii)).

In some embodiments, the capture probe is linked, (e.g., via a disulfide bond), to a feature. In some embodiments,

the capture probe is linked to a feature via a propylene group (e.g., Spacer C3). A reducing agent can be added to break the various disulfide bonds, resulting in release of the capture probe including the spatial barcode sequence. In another example, heating can also result in degradation and release of the attached capture probe. In some embodiments, the heating is done by laser (e.g., laser ablation) and features at specific locations can be degraded. In addition to thermally cleavable bonds, disulfide bonds, photosensitive bonds, and UV sensitive bonds, other non-limiting examples of labile bonds that can be coupled to a capture probe (e.g., spatial barcode) include an ester linkage (e.g., cleavable with an acid, a base, or hydroxylamine), a vicinal diol linkage (e.g., cleavable via sodium periodate), a Diels-Alder linkage (e.g., cleavable via heat), a sulfone linkage (e.g., cleavable via a base), a silyl ether linkage (e.g., cleavable via an acid), a glycosidic linkage (e.g., cleavable via an amylase), a peptide linkage (e.g., cleavable via a protease), or a phosphodiester linkage (e.g., cleavable via a nuclease (e.g., DNAase)).

In some embodiments, the cleavage domain includes a poly(U) sequence which can be cleaved by a mixture of Uracil DNA glycosylase (UDG) and the DNA glycosylase-lyase Endonuclease VIII, commercially known as the USER™ enzyme. In some embodiments, the cleavage domain can be a single U. In some embodiments, the cleavage domain can be an abasic site that can be cleaved with an abasic site-specific endonuclease (e.g., Endonuclease IV or Endonuclease VIII).

In some embodiments, the cleavage domain of the capture probe is a nucleotide sequence within the capture probe that is cleaved specifically, e.g., physically by light or heat, chemically or enzymatically. The location of the cleavage domain within the capture probe will depend on whether or not the capture probe is immobilized on the substrate such that it has a free 3' end capable of functioning as an extension primer (e.g., by its 5' or 3' end). For example, if the capture probe is immobilized by its 5' end, the cleavage domain will be located 5' to the spatial barcode and/or UMI, and cleavage of said domain results in the release of part of the capture probe including the spatial barcode and/or UMI and the sequence 3' to the spatial barcode, and optionally part of the cleavage domain, from a feature. Alternatively, if the capture probe is immobilized by its 3' end, the cleavage domain will be located 3' to the capture domain (and spatial barcode) and cleavage of said domain results in the release of part of the capture probe including the spatial barcode and the sequence 3' to the spatial barcode from a feature. In some embodiments, cleavage results in partial removal of the cleavage domain. In some embodiments, cleavage results in complete removal of the cleavage domain, particularly when the capture probes are immobilized via their 3' end as the presence of a part of the cleavage domain can interfere with the hybridization of the capture domain and the target nucleic acid and/or its subsequent extension.

## III. Multiplexing

### (a) Multiplexing Generally

In various embodiments of spatial analysis as described herein, features can include different types of capture probes for analyzing both intrinsic and extrinsic information for individual cells. For example, a feature can include one or more of the following: 1) a capture probe featuring a capture domain that binds to one or more endogenous nucleic acids in the cell; 2) a capture probe featuring a capture domain that binds to one or more exogenous nucleic acids in the cell (e.g., nucleic acids from a microorganism (e.g., a virus, a bacterium)) that infects the cell, nucleic acids introduced into the cell (e.g., such as plasmids or nucleic acid derived therefrom), nucleic acids for gene editing (e.g., CRISPR-related RNA such as crRNA, guide RNA); 3) a capture probe featuring a capture domain that binds to an analyte capture agent (e.g., an antibody coupled to a oligonucleotide that includes a capture agent barcode domain having an analyte capture sequence that binds the capture domain), and 4) a capture moiety featuring a domain that binds to a protein (e.g., an exogenous protein expressed in the cell, a protein from a microorganism (e.g., a virus, a bacterium)) that infects the cell, or a binding partner for a protein of the cell (e.g., an antigen for an immune cell receptor).

In some embodiments of any of the spatial analysis methods as described herein, spatial profiling includes concurrent analysis of two different types of analytes. A feature can be a gel bead, which is coupled (e.g., reversibly coupled) to one or more capture probes. The capture probes can include a spatial barcode sequence and a poly(T) priming sequence that can hybridize with the poly(A) tail of an mRNA transcript. The capture probe can also include a UMI sequence that can uniquely identify a given transcript. The capture probe can also include a spatial barcode sequence and a random N-mer priming sequence that is capable of randomly hybridizing with gDNA. In this configuration, capture probes can include the same spatial barcode sequence, which permits association of downstream sequencing reads with the feature.

In some embodiments of any of the spatial analysis methods as described herein, a feature can be a gel bead, which is coupled (e.g., reversibly coupled) to capture probes. The Capture probe can include a spatial barcode sequence and a poly(T) priming sequence that can hybridize with the poly(A) tail of an mRNA transcript. The capture probe can also include a UMI sequence that can uniquely identify a given transcript. The capture probe can include a spatial barcode sequence and a capture domain that is capable of specifically hybridizing with an analyte capture agent. The analyte capture agent can includes an oligonucleotide that includes an analyte capture sequence that interacts with the capture

domain coupled to the feature. The oligonucleotide of the analyte capture agent can be coupled to an antibody that is bound to the surface of a cell. The oligonucleotide includes a barcode sequence (e.g., an analyte binding moiety barcode) that uniquely identifies the antibody (and thus, the particular cell surface feature to which it is bound). In this configuration, the capture probes include the same spatial barcode sequence, which permit downstream association of barcoded nucleic acids with the location on the spatial array. In some embodiments of any of the spatial profiling methods described herein, the analyte capture agents can be can be produced by any suitable route, including via example coupling schemes described elsewhere herein.

In some embodiments of any of the spatial analysis methods described herein, other combinations of two or more biological analytes that can be concurrently measured include, without limitation: (a) genomic DNA and cell surface features (e.g., via analyte capture agents that bind to a cell surface feature), (b) mRNA and a lineage tracing construct, (c) mRNA and cell methylation status, (d) mRNA and accessible chromatin (e.g., ATAC-seq, DNase-seq, and/or MNase-seq), (e) mRNA and cell surface or intracellular proteins and/or metabolites, (f) mRNA and chromatin (spatial organization of chromatin in a cell), (g) an analyte capture agent (e.g., any of the MHC multimers described herein) and a V(D)J sequence of an immune cell receptor (e.g., T-cell receptor), (h) mRNA and a perturbation agent (e.g., a CRISPR crRNA/sgRNA, TALEN, zinc finger nuclease, and/or antisense oligonucleotide as described herein), (i) genomic DNA and a perturbation agent, (j) an analyte capture agent and a perturbation agents, (k) accessible chromatin and a perturbation agent, (l) chromatin (e.g., spatial organization of chromatin in a cell) and a perturbation agent, and (m) cell surface or intracellular proteins and/or metabolites and a perturbation agent (e.g., any of the perturbation agents described herein), or any combination thereof.

In some embodiments of any of the spatial analysis methods described herein, the first analyte can include a nucleic acid molecule with a nucleic acid sequence (e.g., mRNA, complementary DNA derived from reverse transcription of mRNA) encoding at least a portion of a V(D)J sequence of an immune cell receptor (e.g., a TCR or BCR). In some embodiments, the nucleic acid molecule with a nucleic acid sequence encoding at least a portion of a V(D)J sequence of an immune cell receptor is cDNA first generated from reverse transcription of the corresponding mRNA, using a poly(T) containing primer. The cDNA that is generated can then be barcoded using a primer, featuring a spatial barcode sequence (and optionally, a UMI sequence) that hybridizes with at least a portion of the cDNA that is generated. In some embodiments, a template switching oligonucleotide in conjunction a terminal transferase or a reverse transcriptase having terminal transferase activity can be employed to generate a priming region on the cDNA to which a barcoded primer can hybridize during cDNA generation. Terminal transferase activity can, for example, add a poly(C) tail to a 3' end of the cDNA such that the template switching oligonucleotide can bind via a poly(G) priming sequence and the 3' end of the cDNA can be further extended. The original mRNA template and template switching oligonucleotide can then be denatured from the cDNA and the barcoded primer comprising a sequence complementary to at least a portion of the generated priming region on the cDNA can then hybridize with the cDNA and a barcoded construct comprising the barcode sequence (and any optional UMI sequence) and a complement of the cDNA generated. Additional methods and compositions suitable for barcoding cDNA generated from mRNA transcripts including those encoding V(D)J regions of an immune cell receptor and/or barcoding methods and composition including a template switch oligonucleotide are described, for example, in PCT Patent Application Publication No. WO 2018/075693, and in U.S. Patent Application Publication No. 2018/0105808, the entire contents of each of which are incorporated herein by reference.

In some embodiments, V(D)J analysis can be performed using methods similar to those described herein. For example, V(D)J analysis can be completed with the use of one or more analyte capture agents that bind to particular surface features of immune cells and are associated with barcode sequences (e.g., analyte binding moiety barcodes). The one or more analyte capture agents can include an MHC or MHC multimer. A barcoded oligonucleotide coupled to a bead that can be used for V(D)J analysis. The oligonucleotide is coupled to a bead by a releasable linkage, such as a disulfide linker. The oligonucleotide can include functional sequences that are useful for subsequent processing, such as functional sequence, which can include a sequencer specific flow cell attachment sequence, e.g., a P5 sequence, as well as functional sequence, which can include sequencing primer sequences, e.g., a R1 primer binding site. In some embodiments, the sequence can include a P7 sequence and a R2 primer binding site. A barcode sequence can be included within the structure for use in barcoding the template polynucleotide. The functional sequences can be selected for compatibility with a variety of different sequencing systems, e.g., Ion Torrent Proton or PGM, Illumina sequencing instruments, etc., and the requirements thereof. In some embodiments, the barcode sequence, functional sequences (e.g., flow cell attachment sequence) and additional sequences (e.g., sequencing primer sequences) can be common to all of the oligonucleotides attached to a given bead. The barcoded oligonucleotide can also include a sequence to facilitate template switching (e.g., a poly(G) sequence). In some embodiments, the additional sequence provides a unique molecular identifier (UMI) sequence segment, as described elsewhere herein.

In an exemplary method of cellular polynucleotide analysis using a barcode oligonucleotide, a cell is co-partitioned along with a bead bearing a barcoded oligonucleotide and additional reagents such as a reverse transcriptase, primers, oligonucleotides (e.g., template switching oligonucleotides), dNTPs, and a reducing agent into a partition (e.g., a droplet in an emulsion). Within the partition, the cell can be lysed to yield a plurality of template polynucleotides (e.g., DNA

such as genomic DNA, RNA such as mRNA, etc.).

A reaction mixture featuring a template polynucleotide from a cell and (i) the primer having a sequence towards a 3' end that hybridizes to the template polynucleotide (e.g., poly(T)) and (ii) a template switching oligonucleotide that includes a first oligonucleotide towards a 5' end can be subjected to an amplification reaction to yield a first amplification product. In some embodiments, the template polynucleotide is an mRNA with a poly(A) tail and the primer that hybridizes to the template polynucleotide includes a poly(T) sequence towards a 3' end, which is complementary to the poly(A) segment. The first oligonucleotide can include at least one of an adaptor sequence, a barcode sequence, a unique molecular identifier (UMI) sequence, a primer binding site, and a sequencing primer binding site or any combination thereof. In some cases, a first oligonucleotide is a sequence that can be common to all partitions of a plurality of partitions. For example, the first oligonucleotide can include a flow cell attachment sequence, an amplification primer binding site, or a sequencing primer binding site and the first amplification reaction facilitates the attachment the oligonucleotide to the template polynucleotide from the cell. In some embodiments, the first oligonucleotide includes a primer binding site. In some embodiments, the first oligonucleotide includes a sequencing primer binding site.

The sequence towards a 3' end (e.g., poly(T)) of the primer hybridizes to the template polynucleotide. In a first amplification reaction, extension reaction reagents, e.g., reverse transcriptase, nucleoside triphosphates, co-factors (e.g., $Mg^{2+}$ or $Mn^{2+}$), that are also co-partitioned, can extend the primer sequence using the cell's nucleic acid as a template, to produce a transcript, e.g., cDNA, having a fragment complementary to the strand of the cell's nucleic acid to which the primer annealed. In some embodiments, the reverse transcriptase has terminal transferase activity and the reverse transcriptase adds additional nucleotides, e.g., poly(C), to the cDNA in a template independent manner.

The template switching oligonucleotide, for example a template switching oligonucleotide which includes a poly(G) sequence, can hybridize to the cDNA and facilitate template switching in the first amplification reaction. The transcript, therefore, can include the sequence of the primer, a sequence complementary to the template polynucleotide from the cell, and a sequence complementary to the template switching oligonucleotide.

In some embodiments of any of the spatial analysis methods described herein, subsequent to the first amplification reaction, the first amplification product or transcript can be subjected to a second amplification reaction to generate a second amplification product. In some embodiments, additional sequences (e.g., functional sequences such as flow cell attachment sequence, sequencing primer binding sequences, barcode sequences, etc.) are attached. The first and second amplification reactions can be performed in the same volume, such as for example in a droplet. In some embodiments, the first amplification product is subjected to a second amplification reaction in the presence of a barcoded oligonucleotide to generate a second amplification product having a barcode sequence. The barcode sequence can be unique to a partition, that is, each partition can have a unique barcode sequence. The barcoded oligonucleotide can include a sequence of at least a segment of the template switching oligonucleotide and at least a second oligonucleotide. The segment of the template switching oligonucleotide on the barcoded oligonucleotide can facilitate hybridization of the barcoded oligonucleotide to the transcript, e.g., cDNA, to facilitate the generation of a second amplification product. In addition to a barcode sequence, the barcoded oligonucleotide can include a second oligonucleotide such as at least one of an adaptor sequence, a unique molecular identifier (UMI) sequence, a primer binding site, and a sequencing primer binding site, or any combination thereof.

In some embodiments of any of the spatial analysis methods described herein, the second amplification reaction uses the first amplification product as a template and the barcoded oligonucleotide as a primer. In some embodiments, the segment of the template switching oligonucleotide on the barcoded oligonucleotide can hybridize to the portion of the cDNA or complementary fragment having a sequence complementary to the template switching oligonucleotide or that which was copied from the template switching oligonucleotide. In the second amplification reaction, extension reaction reagents, e.g., polymerase, nucleoside triphosphates, co-factors (e.g., $Mg^{2+}$ or $Mn^{2+}$), that are also co-partitioned, can extend the primer sequence using the first amplification product as template. The second amplification product can include a second oligonucleotide, a sequence of a segment of the template polynucleotide (e.g., mRNA), and a sequence complementary to the primer.

In some embodiments of any of the spatial analysis methods described herein, the second amplification product uses the barcoded oligonucleotide as a template and at least a portion of the first amplification product as a primer. The segment of the first amplification product (e.g., cDNA) having a sequence complementary to the template switching oligonucleotide can hybridize to the segment of the barcoded oligonucleotide comprising a sequence of at least a segment of the template switching oligonucleotide. In the second amplification reaction, extension reaction reagents, e.g., polymerase, nucleoside triphosphates, co-factors (e.g., $Mg^{2+}$ or $Mn^{2+}$), that are also co-partitioned, can extend the primer sequence (e.g., first amplification product) using the barcoded oligonucleotide as template. The second amplification product can include the sequence of the primer, a sequence which is complementary to the sequence of the template polynucleotide (e.g., mRNA), and a sequence complementary to the second oligonucleotide.

In some embodiments of any of the spatial analysis methods described herein, three or more classes of biological analytes can be concurrently measured. For example, a feature can include capture probes that can participate in an assay of at least three different types of analytes via three different capture domains. A bead can be coupled to a barcoded

oligonucleotide that includes a capture domain that includes a poly(T) priming sequence for mRNA analysis; a barcoded oligonucleotide that includes a capture domain that includes a random N-mer priming sequence for gDNA analysis; and a barcoded oligonucleotide that includes a capture domain that can specifically bind a an analyte capture agent (e.g., an antibody with a spatial barcode), via its analyte capture sequence.

In some embodiments of any of the spatial analysis methods described herein, other combinations of three or more biological analytes that can be concurrently measured include, without limitation: (a) mRNA, a lineage tracing construct, and cell surface and/or intracellular proteins and/or metabolites; (b) mRNA, accessible chromatin (e.g., ATAC-seq, DNase-seq, and/or MNase-seq), and cell surface and/or intracellular proteins and/or metabolites; (c) mRNA, genomic DNA, and a perturbation reagent (e.g., a CRISPR crRNA/sgRNA, TALEN, zinc finger nuclease, and/or antisense oligonucleotide as described herein); (d) mRNA, accessible chromatin, and a perturbation reagent; (e) mRNA, an analyte capture agent (e.g., any of the MHC multimers described herein), and a perturbation reagent; (f) mRNA, cell surface and/or intracellular proteins and/or metabolites, and a perturbation agent; (g) mRNA, a V(D)J sequence of an immune cell receptor (e.g., T-cell receptor), and a perturbation reagent; (h) mRNA, an analyte capture agent, and a V(D)J sequence of an immune cell receptor; (i) cell surface and/or intracellular proteins and/or metabolites, a an analyte capture agent (e.g., the MHC multimers described herein), and a V(D)J sequence of an immune cell receptor; (j) methylation status, mRNA, and cell surface and/or intracellular proteins and/or metabolites; (k) mRNA, chromatin (e.g., spatial organization of chromatin in a cell), and a perturbation reagent; (l) a V(D)J sequence of an immune cell receptor, chromatin (e.g., spatial organization of chromatin in a cell); and a perturbation reagent; and (m) mRNA, a V(D)J sequence of an immune cell receptor, and chromatin (e.g., spatial organization of chromatin in a cell), or any combination thereof.

In some embodiments of any of the spatial analysis methods described herein, four or more classes biological analytes can be concurrently measured. A feature can be a bead that is coupled to barcoded primers that can each participate in an assay of a different type of analyte. The feature is coupled (e.g., reversibly coupled) to a capture probe that includes a capture domain that includes a poly(T) priming sequence for mRNA analysis and is also coupled (e.g., reversibly coupled) to capture probe that includes a capture domain that includes a random N-mer priming sequence for gDNA analysis. Moreover, the feature is also coupled (e.g., reversibly coupled) to a capture probe that binds an analyte capture sequence of an analyte capture agent via its capture domain. The feature can also be coupled (e.g., reversibly coupled) to a capture probe that can specifically bind a nucleic acid molecule that can function as a perturbation agent (e.g., a CRISPR crRNA/sgRNA, TALEN, zinc finger nuclease, and/or antisense oligonucleotide as described herein), via its capture domain.

In some embodiments of any of the spatial analysis methods described herein, each of the various spatially-barcoded capture probes present at a given feature or on a given bead include the same spatial barcode sequence. In some embodiments, each barcoded capture probe can be released from the feature in a manner suitable for analysis of its respective analyte. For example, barcoded constructs A, B, C and D can be generated as described elsewhere herein and analyzed. Barcoded construct A can include a sequence corresponding to the barcode sequence from the bead (e.g., a spatial barcode) and a DNA sequence corresponding to a target mRNA. Barcoded construct B can include a sequence corresponding to the barcode sequence from the bead (e.g., a spatial barcode) and a sequence corresponding to genomic DNA. Barcoded construct C can include a sequence corresponding to the barcode sequence from the bead (e.g., a spatial barcode) and a sequence corresponding to barcode sequence associated with an analyte capture agent (e.g., an analyte binding moiety barcode). Barcoded construct D can include a sequence corresponding to the barcode sequence from the bead (e.g., a spatial barcode) and a sequence corresponding to a CRISPR nucleic acid (which, in some embodiments, also includes a barcode sequence). Each construct can be analyzed (e.g., via any of a variety of sequencing methods) and the results can be associated with the given cell from which the various analytes originated. Barcoded (or even non-barcoded) constructs can be tailored for analyses of any given analyte associated with a nucleic acid and capable of binding with such a construct.

In some embodiments of any of the spatial analysis methods described herein, other combinations of four or more biological analytes that can be concurrently measured include, without limitation: (a) mRNA, a lineage tracing construct, cell surface and/or intracellular proteins and/or metabolites, and gDNA; (b) mRNA, accessible chromatin (e.g., ATAC-seq, DNase-seq, and/or MNase-seq), cell surface and/or intracellular proteins and/or metabolites, and a perturbation agent (e.g., a CRISPR crRNA/sgRNA, TALEN, zinc finger nuclease, and/or antisense oligonucleotide as described herein); (c) mRNA, cell surface and/or intracellular proteins and/or metabolites, an analyte capture agent (e.g., the MHC multimers described herein), and a V(D)J sequence of an immune cell receptor (e.g., T-cell receptor); (d) mRNA, genomic DNA, a perturbation reagent, and accessible chromatin; (e) mRNA, cell surface and/or intracellular proteins and/or metabolites, an analyte capture agent (e.g., the MHC multimers described herein), and a perturbation reagent; (f) mRNA, cell surface and/or intracellular proteins and/or metabolites, a perturbation reagent, and a V(D)J sequence of an immune cell receptor (e.g., T-cell receptor); (g) mRNA, a perturbation reagent, an analyte capture agent (e.g., the MHC multimers described herein), and a V(D)J sequence of an immune cell receptor (e.g., T-cell receptor); (h) mRNA, chromatin (e.g., spatial organization of chromatin in a cell), and a perturbation reagent; (i) a V(D)J sequence of an immune cell receptor, chromatin (e.g., spatial organization of chromatin in a cell); and a perturbation reagent; (j) mRNA, a V(D)J sequence of an immune cell

receptor, chromatin (e.g., spatial organization of chromatin in a cell), and genomic DNA; (k) mRNA, a V(D)J sequence of an immune cell receptor, chromatin (e.g., spatial organization of chromatin in a cell), and a perturbation reagent, or any combination thereof.

(b) Construction of Spatial Arrays for Multi-Analyte Analysis

This disclosure also provides methods and materials for constructing a spatial array capable of multi-analyte analysis. In some embodiments, a spatial array includes a plurality of features on a substrate where one or more members of the plurality of features include a plurality of oligonucleotides having a first type functional sequence and oligonucleotides having a second, different type of functional sequence. In some embodiments, a feature can include oligonucleotides with two types of functional sequences. A feature can be coupled to oligonucleotides comprising a TruSeq sequence and also to oligonucleotides comprising a Nextera sequence. In some embodiments, a functional sequence can include a sequencer specific flow cell attachment sequence, e.g., a P5 sequence, as well as functional sequence, which can include sequencing primer sequences, e.g., a R1 primer binding site. In some embodiments, one or more members of the plurality of features comprises both types of functional sequences. In some embodiments, one or more members of the plurality features includes a first type of functional sequence. In some embodiments, one or more members of the plurality of features includes a second type of functional sequence. In some embodiments, an additional oligonucleotide can be added to the functional sequence to generate a full oligonucleotide where the full oligonucleotide includes a spatial barcode sequence, an optional UMI sequence, a priming sequence, and a capture domain. Attachment of these sequences can be via ligation (including via splint ligation as is described in U.S. Patent Application Publication No. 20140378345, the entire contents of which are incorporated herein by reference), or any other suitable route. As discussed herein, oligonucleotides can be hybridized with splint sequences that can be helpful in constructing complete full oligonucleotides (e.g., oligonucleotides that are capable of spatial analysis).

In some embodiments, the oligonucleotides that hybridize to sequences (e.g., TruSeq and Nextera) located on the features include capture domains capable of capturing different types of analytes (e.g., mRNA, genomic DNA, cell surface proteins, or accessible chromatin). In some examples, oligonucleotides that can bind to the TruSeq sequences can include capture domains that include poly(T) capture sequences. In addition to the poly(T) capture sequences, the oligonucleotides that can bind the TruSeq groups can also include a capture domain that includes a random N-mer sequence for capturing genomic DNA (e.g., or any other sequence or domain as described herein capable of capturing any of the biological analytes described herein). In such cases, the spatial arrays can be constructed by applying ratios of TruSeq-poly(T) and TruSeq-N-mer oligonucleotides to the features comprising the functional TruSeq sequences. This can produce spatial arrays where a portion of the oligonucleotides can capture mRNA and a different portion of oligonucleotides can capture genomic DNA. In some embodiments, one or more members of a plurality of features include both TruSeq and Nextera sequences. In such cases, a feature including both types of functional sequences is capable of binding oligonucleotides specific to each functional sequence. For example, an oligonucleotide capable of binding to a TruSeq sequence could be used to deliver an oligonucleotide including a poly(T) capture domain and an oligonucleotide capable of binding to a Nextera sequence could be used to deliver an oligonucleotide including an N-mer capture domain for capturing genomic DNA. It will be appreciated by a person of ordinary skill in the art that any combination of capture domains (e.g., capture domains having any of the variety of capture sequences described herein capable of binding to any of the different types of analytes as described herein) could be combined with oligonucleotides capable of binding to TruSeq and Nextera sequences to construct a spatial array.

In some embodiments, an oligonucleotide that includes a capture domain (e.g., an oligonucleotide capable of coupling to an analyte) or an analyte capture agent can include an oligonucleotide sequence that is capable of binding or ligating to an assay primer. The adapter can allow the capture probe or the analyte capture agent to be attached to any suitable assay primers and used in any suitable assays. The assay primer can include a priming region and a sequence that is capable of binding or ligating to the adapter. In some embodiments, the adapter can be a non-specific primer (e.g., a 5' overhang) and the assay primer can include a 3' overhang that can be ligated to the 5' overhang. The priming region on the assay primer can be any primer described herein, e.g., a poly (T) primer, a random N-mer primer, a target-specific primer, or an analyte capture agent capture sequence.

In some examples, an oligonucleotide can includes an adapter, e.g., a 5' overhang with 10 nucleotides. The adapter can be ligated to assay primers, each of which includes a 3' overhang with 10 nucleotides that complementary to the 5' overhang of the adapter. The capture probe can be used in any assay by attaching to the assay primer designed for that assay.

Adapters and assay primers can be used to allow the capture probe or the analyte capture agent to be attached to any suitable assay primers and used in any suitable assays. A capture probe that includes a spatial barcode can be attached to a bead that includes a poly(dT) sequence. A capture probe including a spatial barcode and a poly(T) sequence can be used to assay multiple biological analytes as generally described herein (e.g., the biological analyte includes a poly(A) sequence or is coupled to or otherwise is associated with an analyte capture agent comprising a poly(A) sequence

as the analyte capture sequence).

A splint oligonucleotide with a poly(A) sequence can be used to facilitate coupling to a capture probe that includes a spatial barcode and a second sequence that facilitates coupling with an assay primer. Assay primers include a sequence complementary to the splint oligo second sequence and an assay-specific sequence that determines assay primer functionality (e.g., a poly(T) primer, a random N-mer primer, a target-specific primer, or an analyte capture agent capture sequence as described herein).

In some embodiments of any of the spatial profiling methods described herein, a feature can include a capture probe that includes a spatial barcode comprising a switch oligonucleotide, e.g., with a 3' end 3rG. For example, a feature (e.g., a gel bead) with a spatial barcode functionalized with a 3rG sequence can be used that enables template switching (e.g., reverse transcriptase template switching), but is not specific for any particular assay. In some embodiments, the assay primers added to the reaction can determine which type of analytes are analyzed. For example, the assay primers can include binding domains capable of binding to target biological analytes (e.g., poly (T) for mRNA, N-mer for genomic DNA, etc.). A capture probe (e.g., an oligonucleotide capable of spatial profiling) can be generated by using a reverse transcriptase enzyme/polymerase to extend, which is followed by template switching onto the barcoded adapter oligonucleotide to incorporate the barcode and other functional sequences. In some embodiments, the assay primers include capture domains capable of binding to a poly(T) sequence for mRNA analysis, random primers for genomic DNA analysis, or a capture sequence that can bind a nucleic acid molecule coupled to an analyte binding moiety (e.g., a an analyte capture sequence of an analyte capture agent) or a nucleic acid molecule that can function in as a perturbation reagent (e.g., a CRISPR crRNA/sgRNA, TALEN, zinc finger nuclease, and/or antisense oligonucleotide as described herein).

IV. Methods, Compositions, and Kits for Generating Spatial Arrays

Arrays that capture spatial information of analytes within a biological sample can be prepared in various ways. Arrays can be designed with pluralities of capture probes. Capture probes can be designed to capture a substantial population of analytes (e.g., mRNA) or to capture specific (e.g., gene specific) analytes. The methods described herein provide for ways of preparing arrays with capture probes for capturing analytes.

Generally, arrays can be manufactured in various ways including by various printing methods as described herein. However, printing methods can be time consuming and resource intense. Arrays including capture probes generally include the same capture domain (e.g., a poly(T) capture domain), however, capture probes including different capture domains allow for multiplexing (e.g., the simultaneous capture of different kinds of analytes or different species of the same type of analyte). Thus, for example, a first capture probe can be capable of capturing of a particular analyte (e.g., mRNA) and a second capture probe can be capable of capturing a particular species of mRNA, other RNA, genomic DNA, cDNA, or an analyte (e.g., protein) indirectly through an analyte capture sequence.

The present disclosure feature methods, compositions, and kits for generating a capture probe by ligating a plurality of donor oligonucleotides to a plurality of acceptor oligonucleotides including a spatial barcode. In some embodiments, the ligation reaction is facilitated by a splint oligonucleotide, where the splint oligonucleotide includes a sequence complementary to a portion of the acceptor oligonucleotide (e.g., a first ligation handle) and a portion of the donor oligonucleotide (e.g., a second ligation handle). In some embodiments, the donor oligonucleotide includes a capture domain to detect an analyte (e.g., directly or indirectly). In some embodiments, a first plurality of donor oligonucleotides including a first capture domain are ligated to acceptor oligonucleotides. In some embodiments, a second plurality of donor oligonucleotides including a second domain are ligated to acceptor oligonucleotides. In some embodiments, the first capture domain and the second capture domain are different (e.g., capture different analytes). Thus, arrays can be generated with two or more species of capture probes (e.g., a first capture probe with a first capture domain and a second capture probe with a second capture domain) with a ligation strategy that allows for multiplex detection of analytes in a biological sample. The ligation strategies described herein allow for the generation of arrays with multiple species of capture probes (e.g., capture probes with different capture domains for different analytes of interest).

Provided herein are methods for generating arrays for spatial analysis (e.g., spatial arrays). The spatial arrays can have capture probes that can interact with analytes present in a biological sample either directly or indirectly. For example, analytes can be RNA, DNA, protein, lipids, or any other analytes described herein. In some embodiments, the capture probe is a nucleic acid or a polypeptide. In some embodiments, the capture probe can interact with a conjugate (e.g., an oligonucleotide-antibody conjugate). In some embodiments, the capture probe includes a barcode (e.g., a spatial barcode and/or a unique molecular identifier (UMI)) and a capture domain. In some embodiments, a capture probe includes a cleavage domain.

Provided herein are methods for generating arrays for spatial analysis (e.g., spatial arrays). The spatial arrays can have capture probes that can interact with analytes present in a biological sample either directly or indirectly. For example, analytes can be RNA, DNA, protein, lipids, or any other analytes described herein. In some embodiments, the capture probe is a nucleic acid or a polypeptide. In some embodiments, the capture probe interacts with a conjugate (e.g.,

an oligonucleotide-antibody conjugate). In some embodiments, the capture probe includes a barcode (e.g., a spatial barcode and/or a unique molecular identifier (UMI)) and a capture domain. In some embodiments, a capture probe includes a cleavage domain.

Thus, provided herein are methods of generating a spatial array including (a) providing a substrate including a plurality of acceptor oligonucleotides, where a first acceptor oligonucleotide of the plurality of acceptor oligonucleotides includes a spatial barcode and a first ligation handle, and where the 5' end of the first acceptor oligonucleotide is attached to the substrate; (b) providing a plurality of universal splint oligonucleotides, where a universal splint oligonucleotide of the plurality of universal splint oligonucleotides includes a sequence complementary to the first ligation handle and a sequence complementary to a second ligation handle; (c) ligating a first plurality of donor oligonucleotides, where a first donor oligonucleotide of the first plurality of donor oligonucleotides includes the second ligation handle and a first capture domain, to the 3' end of a first acceptor oligonucleotide to generate a first capture probe, where the universal splint oligonucleotide is hybridized to the first ligation handle and the second ligation handle; and (d) ligating a second plurality of donor oligonucleotides, where a second donor oligonucleotide of the plurality of second donor oligonucleotides includes the second ligation handle and a second capture domain, to the 3' end of a second acceptor oligonucleotide to generate a second capture probe, where the universal splint oligonucleotide is hybridized to the first ligation handle and the second ligation handle, thereby generating a spatial array.

Also provided herein are methods of generating a spatial array including (a) providing a substrate including a plurality of acceptor oligonucleotides, where an acceptor oligonucleotide of the plurality of acceptor oligonucleotides includes a spatial barcode and a first ligation handle, and where the 5' end of the acceptor oligonucleotide is attached to the substrate; (b) providing a plurality of universal splint oligonucleotides, where a universal splint oligonucleotide of the plurality of universal splint oligonucleotides includes a sequence complementary to the first ligation handle and a sequence complementary to a second ligation handle present in a donor oligonucleotide of a plurality of donor oligonucleotides; and (c) ligating the donor oligonucleotide including a capture domain to the 3' end of the acceptor oligonucleotide to generate a capture probe, where the universal splint oligonucleotide is hybridized to the first ligation handle and the second ligation handle, thereby generating a spatial array.

In some embodiments, capture probes are attached to an array feature and an individual array feature can include one or more capture probes. In some embodiments, an individual array feature includes hundreds or thousands of capture probes. In some embodiments, the capture probes are associated with a particular individual feature, where the individual feature contains a capture probe including a spatial barcode unique to the individual feature (e.g., a defined region or location on the array).

In some embodiments, a particular feature can contain capture probes including more than one spatial barcode (e.g., one capture probe at a particular feature can include a non-common spatial barcode that is different than the non-common spatial barcode included in another capture probe at the same particular feature, while both capture probes include a second, common spatial barcode), where the non-common spatial barcodes of the capture probes corresponds to a particular defined region or location on the array. For example, multiple non-common spatial barcode sequences associated with one particular feature on an array can provide a stronger address or attribution to a given location by providing duplicate or independent confirmation of the location. In some embodiments, the multiple non-common spatial barcodes represent increasing specificity of the location of the particular array point.

As used herein, spatial analyte capture can utilize nucleic acid probes (e.g., capture probes) including spatial barcode sequences, which are immobilized on a substrate (e.g., directly or indirectly) to generate an array. The spatial barcode sequences can correspond to the location of the capture probes on the array. In some embodiments, biological samples, such as tissue sections, are placed onto the array and an analyte from the biological sample is captured. In some embodiments, the captured analyte is a protein. In some embodiments, the captured analyte is a nucleic acid. In some embodiments, the captured analyte is RNA (e.g. messenger RNA). In some embodiments, the captured analyte is DNA (e.g., genomic DNA). In some embodiments, DNA from the biological sample is processed, e.g. fragmented and/or provided with a binding domain that hybridizes to the capture probes on the array. In some embodiments, DNA polymerase extension or ligation reaction is performed in the biological sample on the array. In some embodiments, the probes to which nucleic acids in the biological sample bind are extended using the capture probe bound nucleic acid as a template to generate complementary nucleic acid strands, which is immobilized on the surface of the array. In some embodiments, extended probes (e.g., capture probes that are subjected to template-directed nucleic acid polymerization using a captured nucleic acid analyte as a template) carry information about the position of the "captured" analyte in the biological sample, e.g. tissue section. In some embodiments, the extended probes can be released from the surface of the array, and a "library" of captured nucleic acid analytes created that are associated with spatial information, the library which can then be analyzed, for example via sequencing. In some embodiments, the sequence data can then be matched to a location in the biological sample, e.g. tissue section. For instance, the biological sample can be visualized or imaged, e.g., stained and/or photographed, before or after analyte capture to facilitate the correlation of the spatial barcode in the capture probe (e.g., an extended capture probe that is associated with a given analyte) with a position within the biological sample and the sequence data can be overlaid on an image of the biological sample, e.g., using a computer, to display the distribution

pattern of any analyte of interest across the biological sample. The visualization step is not essential as the tagged analytes can be correlated with a location in the biological sample using other means, e.g., the unique profile of the analytes captured with the same spatial barcode, e.g., at the same position on the array, can enable the analyte to be correlated to a location within the biological sample based on the characteristics of cells or areas of cells within the biological sample (e.g., a tissue section). Any of a variety of analytes can be captured and analyzed in accordance with methods provided herein, including but not limited to RNA, specific sub-types of RNA, proteins, antibodies, genomic DNA, T-cell receptors, small molecules, or other analytes.

**FIG. 1** is a schematic diagram showing an example of a capture probe, as described herein. As shown, the capture probe **102** is coupled to a feature **101** by an optional cleavage domain **103,** such as a disulfide linker. The capture probe can include functional sequences that are useful for subsequent processing, such as a functional sequence **104,** which can include a sequencer specific flow cell attachment sequence, e.g., a P5 or P7 sequence, as well as functional sequence **106,** which can include sequencing primer sequences, e.g., a R1 primer binding site, a R2 primer binding site. In some embodiments, sequence **104** is a P7 sequence and sequence **106** is a R2 primer binding site. A spatial barcode **105** can be included within the capture probe for use in barcoding the target analyte. A unique molecular identifier can also be included in combination with a spatial barcode **105.** The functional sequences can generally be selected for compatibility with any of a variety of different sequencing systems, e.g., Ion Torrent Proton or PGM, Illumina sequencing instruments, PacBio, Oxford Nanopore, etc., and the requirements thereof. In some embodiments, functional sequences can be selected for compatibility with non-commercialized sequencing systems. Examples of such sequencing systems and techniques, for which suitable functional sequences can be used, include (but are not limited to) Ion Torrent Proton or PGM sequencing, Illumina sequencing, PacBio SMRT sequencing, and Oxford Nanopore sequencing. Further, in some embodiments, functional sequences can be selected for compatibility with other sequencing systems, including non-commercialized sequencing systems.

In some embodiments, the spatial barcode **105,** functional sequences **104** (e.g., flow cell attachment sequence) and **106** (e.g., sequencing primer sequences) can be common to all of the probes attached to a given feature. The capture probe can also include a capture domain **107** to facilitate capture of a target analyte.

*Capture Probes via Splint Oligonucleotides*

Capture probes for spatial analysis can be generated by different methods. In some embodiments, the capture probe is generated from two or more oligonucleotide sequences. In some embodiments, two or more oligonucleotide sequences are ligated together to generate a capture probe. In some embodiments, capture probe generation can be facilitated by a splint oligonucleotide. In some embodiments, a plurality of oligonucleotides are attached to a feature on the substrate. In some embodiments, the oligonucleotides of the array can be about 20 to about 100 nucleotides long. In some embodiments, the oligonucleotides of the array can be about 30 to about 90 nucleotides long. In some embodiments, the oligonucleotides of the array can be about 40 to about 60 nucleotides long. In some embodiments, the oligonucleotides of the array can be about 45 to about 55 nucleotides long. In some embodiments, the capture probe includes one or more constant sequences (e.g., any of the variety of constant sequences described herein). For example, the oligonucleotide can have a functional domain (e.g. any of the variety of functional domains described herein) and/or a cleavage domain (e.g. any of the variety of cleavage domains described herein).

*Generating capture probes*

Capture probes described herein can contain various functional sequences and a capture domain. In some embodiments, the capture domain captures analytes in an unbiased manner. For example, if the capture domain is a poly(T) sequence, the capture domain can interact with (e.g., hybridize to) the poly(A) tail of any given polyadenylated RNA, such as mRNA or an analyte or analyte proxy that is modified to include a poly(A) sequence, present in the biological sample, regardless of the identity of that polyadenylated RNA. In some embodiments, the capture domain can capture analytes in an unbiased manner via a capture domain including a random or degenerate sequence. As non-limiting examples, a capture domain can include a random hexamer, octamer, nonamer, or 12-mer sequence for unbiased capture.

In some embodiments, the capture domain captures analytes in a biased manner. For example, the capture domain can be gene-specific, such that a specific analyte (e.g., a specific nucleic acid, e.g., a specific mRNA or DNA sequence) or set of analytes (e.g., set of nucleic acids, e.g., a set of mRNAs or a set of DNAs, e.g., a set of mRNAs or a set of DNAs sharing a common or similar sequence) can be captured for spatial analysis in the biological sample. In some embodiments, the capture domain can capture rRNA analytes. In some embodiments, the capture domain can capture microbial rRNA. For example, the capture domain can contain a degenerate sequence capable of interacting (e.g., hybridizing) with 16S rRNA, including microbial 16S rRNA. In some embodiments, identification of microbial rRNA in a biological sample can lead to the identification of particular microbial species present in the biological sample. In some

embodiments, identification of microbial rRNA in a biological sample can reveal whether one or more microbial species can be found in proximity to one another. In some embodiments, identification of microbial rRNA can be indicative of the presence of a pathogen.

In some embodiments, the capture domain can interact with analytes containing V(D)J sequences, e.g., a V(D)J sequence associated with an immune cell receptor (e.g., T-cell receptor, B-cell receptor). In some embodiments, identifying V(D)J sequences can identify novel sequences in the antigen-binding receptor regions of immune cell receptors (e.g., T-cell receptor, B-cell receptor).

In some embodiments, the capture domain can interact with a capture agent barcode domain associated with an analyte capture agent (e.g., an analyte capture sequence).

Capture probes for both unbiased analyte capture and biased capture can be generated from an existing array. In some embodiments, an array containing a plurality of oligonucleotides (e.g., in situ synthesized or printed oligonucleotides) can be modified to generate a variety of capture probes. The oligonucleotides (e.g., acceptor oligonucleotides) can include various domains such as, without limitation, spatial barcodes, UMIs, functional domains (e.g., sequencing domain), cleavage domains, and/or ligation handles. As used herein, the term "ligation handle" or "ligation domain" refers to a portion of an acceptor oligonucleotide that is complementary to a portion of a second oligonucleotide. In some embodiments, the second oligonucleotide is a universal splint oligonucleotide comprising a sequence complementary to (or substantially complementary to) a portion of a donor oligonucleotide, such that the universal splint oligonucleotide can adjacently position the acceptor oligonucleotide and the donor oligonucleotide for ligating the donor oligonucleotide to the acceptor oligonucleotide. In some embodiments, the donor oligonucleotide includes a capture domain. For example, **FIG. 2** shows an acceptor oligonucleotide on an immobilized substrate (e.g., an array), that contains from 5' to 3' a linker, a cleavage domain, a functional domain, a spatial barcode, a UMI, a second functional domain and a ligation domain. In some embodiments, a universal splint oligonucleotide with a sequence complementary to (or substantially complementary to) the ligation domain or ligation handle of the acceptor oligonucleotide and an additional sequence complementary to (or substantially complementary to (e.g., a second ligation handle)) an oligonucleotide (e.g., a donor oligonucleotide) containing a poly(T) capture domain facilitates the ligation of the acceptor oligonucleotide and the donor oligonucleotide containing the capture domain. In some embodiments, the capture domain is a poly(T) sequence. In some embodiments, the capture domain is a sequence specific to a nucleic acid (e.g., an mRNA or DNA sequence). In some embodiments, the capture domain is a gene-specific sequence. In some embodiments, the capture domain is a unique sequence (e.g., a designed sequence to indirectly capture an analyte). In some embodiments, the capture domain is a sequence specific to a subset of nucleic acids (e.g., a subset of mRNA or DNA sequences sharing a common or similar sequence). In embodiments where the splint oligonucleotide sequence complementary to (or substantially complementary to) the capture domain is a poly(A) sequence, the splint oligonucleotide can facilitate the ligation of a donor oligonucleotide comprising a poly(T) capture domain to the oligonucleotide. In embodiments where the splint oligonucleotide sequence complementary to (or substantially complementary to) the capture domain is a sequence specific to a nucleic acid, the splint oligonucleotide can facilitate the ligation of that donor oligonucleotide with the specific capture domain to the acceptor oligonucleotide. In embodiments where the splint oligonucleotide sequence complementary to (or substantially complementary to) the capture domain is a sequence specific to a subset of nucleic acids, the splint oligonucleotide can facilitate the ligation of that capture domain to the acceptor oligonucleotide. Penk mRNA is used herein for exemplary purposes only and the present disclosure is not limited to assaying for Penk gene expression.

In some embodiments, an array containing a plurality of oligonucleotides (e.g., in situ synthesized or printed oligonucleotides) can be modified to generate a variety of capture probes. The acceptor oligonucleotides can include various domains such as, without limitation, spatial barcodes, UMIs, functional domains (e.g., sequencing domains, amplification domains, etc.), cleavage domains, and/or ligation domains. For example, **FIG. 5** shows an exemplary strategy where an acceptor oligonucleotide having a ligation domain is immobilized on a substrate (e.g., an array). In some embodiments, the acceptor oligonucleotide shown in **FIG. 5** contains from 5' to 3' one or more of (e.g., each of) an amino modifier C6, a polythymine sequence (e.g., 5 thymines), a sequencing domain (e.g., R1), a spatial barcode (e.g., a 16 base pair spatial barcode), a UMI (e.g., a 12 base pair UMI), and a ligation domain (e.g., a 7 base pair first ligation handle comprising SEQ ID NO: 9). **FIG. 5** shows the ligation domain at the 3' end of the acceptor oligonucleotide. In some embodiments, the ligation domain can be complementary to (or substantially complementary to) a sequence in the splint oligonucleotide (e.g., a universal splint oligonucleotide). In some embodiments, the splint oligonucleotide can have a sequence complementary to (or substantially complementary to) a second ligation domain that is present in an oligonucleotide that also includes a capture domain. In some embodiments, the capture domain can be any of the capture domains described herein.

In some embodiments, a plurality of capture probes can be generated using a universal splint oligonucleotide. A universal splint oligonucleotide allows any capture probe to be generated with a capture domain of interest. For example, a universal splint oligonucleotide can be designed to be complementary to (or substantially complementary to) a first ligation handle (e.g., a constant first ligation handle) and a second ligation handle (e.g., a constant second ligation handle), wherein the first ligation handle is present in a plurality of acceptor oligonucleotides and wherein the second ligation handle

is present in a plurality of donor oligonucleotides. In some embodiments, the efficiency of ligating a first donor oligonucleotide having a first capture domain to an acceptor oligonucleotide (e.g., an acceptor oligonucleotide having a spatial barcode) using a universal splint oligonucleotide is the same or approximately the same as the efficiency of ligating a second donor oligonucleotide including having a second capture domain to an acceptor oligonucleotide using the universal splint oligonucleotide (e.g., the ligation efficiency is the same or approximately the same regardless of the capture domain present in the donor oligonucleotide). Use of a universal splint oligonucleotide can reduce cost and complexity in generating capture probes on a substrate (e.g., an array). Additionally or alternatively, a universal splint oligonucleotide can facilitate the ligation of two or more different donor oligonucleotides to two or more acceptor oligonucleotides. For example, the donor oligonucleotides can have capture domains specific for two or more different analytes (e.g., RNA, DNA, protein, any other analyte described herein). As such, the use of a universal splint oligonucleotide can be used to ligate any one, two, three or four or more capture domains as found on donor oligonucleotides to acceptor oligonucleotides on an array thereby creating an array that is capable of capturing multiple, different target analytes on one array.

In some embodiments, a capture probe generated via universal splint oligonucleotide-mediated ligation is comparable at capturing an analyte as a capture probe generated by other means (e.g., printed directly on the support). In some embodiments, a capture probe generated via universal splint oligonucleotide-mediated ligation is about 80% efficient at capturing an analyte as a capture probe generated by other means (e.g., printed directly on the support). In some embodiments, a capture probe generated via universal splint oligonucleotide-mediated ligation is are about 85% efficient at capturing an analyte as a capture probe generated by other means (e.g., printed directly on the support). In some embodiments, a capture probe generated via universal splint oligonucleotide-mediated ligation is about 90% efficient at capturing an analyte as a capture probe generated by other means (e.g., printed directly on the support). In some embodiments, a capture probe generated via universal splint oligonucleotide-mediated ligation is about 95% efficient at capturing an analyte as a capture probe generated by other means (e.g., printed directly on the support). In some embodiments, a capture probe generated via universal splint oligonucleotide-mediated ligation is about 96%, 97%, 98%, or 99% efficient at capturing an analyte as a capture probe generated by other means (e.g., printed directly on the support). In some embodiments, a capture probe generated via universal splint oligonucleotide-mediated ligation is just as efficient at capturing an analyte as a capture probe generated by other means (e.g., printed directly on the support).

In some embodiments, provided herein is a universal splint oligonucleotide that comprises SEQ ID NO: 13. In some embodiments, provided herein is a universal splint oligonucleotide that includes, SEQ ID NO: 13, wherein the 3'-most nucleotide of the universal splint oligonucleotide is an inverted nucleotide (e.g., 3' to 3' reversed linkage). In some embodiments, provided herein is a universal splint oligonucleotide that comprises SEQ ID NO: 13, wherein the universal splint oligonucleotide includes an inverted dideoxynucleotide (e.g., 5' to 5' reversed linkage).

Also provided herein are compositions and kits for capturing analytes in a biological sample. In some embodiments, a composition or a kit including a) an acceptor oligonucleotide b) a universal splint oligonucleotide is provided. In some embodiments, a composition or a kit including a) an acceptor oligonucleotide comprising a ligation handle SEQ ID NO: 12 and b) a universal splint oligonucleotide comprising SEQ ID NO: 13 is provided. In some embodiments, a composition or a kit including a) an acceptor oligonucleotide b) a universal splint oligonucleotide, and c) a donor oligonucleotide is provided. In some embodiments, a composition or a kit including a) an acceptor oligonucleotide comprising a ligation handle SEQ ID NO: 12 and b) a universal splint oligonucleotide comprising SEQ ID NO: 13, and c) a donor oligonucleotide comprising SEQ ID NO: 15 is provided. In some embodiments, a composition or a kit including a) an acceptor oligonucleotide comprising a ligation handle SEQ ID NO: 12 and b) a universal splint oligonucleotide comprising SEQ ID NO: 13, and c) a donor oligonucleotide comprising SEQ ID NO: 16 is provided. In some embodiments, a composition or a kit including a) an acceptor oligonucleotide comprising a ligation handle SEQ ID NO: 12 and b) a universal splint oligonucleotide comprising SEQ ID NO: 13, and c) a donor oligonucleotide comprising SEQ ID NO: 17 is provided. In some embodiments, a composition (e.g., a kit) including a) an acceptor oligonucleotide comprising a ligation handle SEQ ID NO: 12 and b) a universal splint oligonucleotide comprising SEQ ID NO: 13, and c) a donor oligonucleotide comprising SEQ ID NO: 18 is provided. In some embodiments, a composition or a kit including a) an acceptor oligonucleotide comprising a ligation handle SEQ ID NO: 12 and b) a universal splint oligonucleotide comprising SEQ ID NO: 13, and c) a donor oligonucleotide comprising SEQ ID NO: 19 is provided. In some embodiments, a composition or a kit including a) an acceptor oligonucleotide comprising a ligation handle SEQ ID NO: 12 and b) a universal splint oligonucleotide comprising SEQ ID NO: 13, wherein the 3'-most nucleotide of the universal splint oligonucleotide is an inverted thymine nucleotide (e.g., 3' to 3' reversed linkage) is provided. In some embodiments, a composition or a kit including a) an acceptor oligonucleotide comprising a ligation handle SEQ ID NO: 12 and b) a universal splint oligonucleotide comprising SEQ ID NO: 13, wherein the 3'-most nucleotide of the universal splint oligonucleotide is an inverted adenine nucleotide (e.g., 3' to 3' reversed linkage) is provided. In some embodiments, a composition or a kit including a) an acceptor oligonucleotide comprising a ligation handle SEQ ID NO: 12 and b) a universal splint oligonucleotide comprising SEQ ID NO: 13, wherein the 3'-most nucleotide of the universal splint oligonucleotide is an inverted cytosine nucleotide (e.g., 3' to 3' reversed linkage) is provided. In some embodiments, a composition or a kit including a) an acceptor oligonucleotide comprising a

ligation handle SEQ ID NO: 12 and b) a universal splint oligonucleotide comprising SEQ ID NO: 13, wherein the 3'-most nucleotide of the universal splint oligonucleotide is an inverted guanine nucleotide (e.g., 3' to 3' reversed linkage) is provided.

In some embodiments, a composition or a kit including a) an acceptor oligonucleotide comprising a ligation handle SEQ ID NO: 12, b) a universal splint oligonucleotide comprising SEQ ID NO: 13, wherein the 3'-most nucleotide of the universal splint oligonucleotide is an inverted thymine nucleotide (e.g., 3' to 3' reversed linkage), and c) a donor oligonucleotide comprising one of SEQ ID NOs: 15, 16, 17, 18, or 19 is provided. In some embodiments, a composition or a kit including a) an acceptor oligonucleotide comprising a ligation handle SEQ ID NO: 12 b) a universal splint oligonucleotide comprising SEQ ID NO: 13, wherein the 3'-most nucleotide of the universal splint oligonucleotide is an inverted adenine nucleotide (e.g., 3' to 3' reversed linkage), and c) a donor oligonucleotide comprising one of SEQ ID NOs: 15, 16, 17, 18, or 19 is provided. In some embodiments, a composition or a kit including a) an acceptor oligonucleotide comprising a ligation handle SEQ ID NO: 12 b) a universal splint oligonucleotide having SEQ ID NO: 13, wherein the 3'-most nucleotide of the universal splint oligonucleotide is an inverted cytosine nucleotide (e.g., 3' to 3' reversed linkage), and c) a donor oligonucleotide comprising one of SEQ ID NOs: 15, 16, 17, 18, or 19 is provided. In some embodiments, a composition or a kit including a) an acceptor oligonucleotide comprising a ligation handle SEQ ID NO: 12, b) a universal splint oligonucleotide having SEQ ID NO: 13, wherein the 3'-most nucleotide of the universal splint oligonucleotide is an inverted guanine nucleotide (e.g., 3' to 3' reversed linkage), c) a donor oligonucleotide comprising one of SEQ ID NOs: 15, 16, 17, 18, or 19 is provided.

In some embodiments, a composition or a kit including a) an acceptor oligonucleotide comprising a ligation handle SEQ ID NO: 12 and b) a universal splint oligonucleotide having SEQ ID NO: 13, and c) a donor oligonucleotide having SEQ ID NO: 19 is provided. In some embodiments, a composition or a kit including a) an acceptor oligonucleotide comprising a ligation handle SEQ ID NO: 12 and b) a universal splint oligonucleotide having SEQ ID NO: 13, where the universal splint oligonucleotide includes an inverted dideoxy-thymine nucleotide (e.g., 5' to 5' reversed linkage) is provided. In some embodiments, a composition or a kit including a) an acceptor oligonucleotide comprising a ligation handle SEQ ID NO: 12 and b) a universal splint oligonucleotide having SEQ ID NO: 13, wherein the universal splint oligonucleotide includes an inverted dideoxy-adenine nucleotide (e.g., 5' to 5' reversed linkage) is provided. In some embodiments, a composition or a kit including a) an acceptor oligonucleotide comprising a ligation handle SEQ ID NO: 12 and b) a universal splint oligonucleotide having SEQ ID NO: 13, wherein the universal splint oligonucleotide includes an inverted dideoxy-cytosine nucleotide (e.g., 5' to 5' reversed linkage) is provided. In some embodiments, a composition or a kit including a) an acceptor oligonucleotide comprising a ligation handle SEQ ID NO: 12 and b) a universal splint oligonucleotide having SEQ ID NO: 13, wherein the universal splint oligonucleotide includes an inverted dideoxy-guanine nucleotide (e.g., 5' to 5' reversed linkage) is provided.

In some embodiments, a composition or a kit including a) an acceptor oligonucleotide comprising a ligation handle SEQ ID NO: 12 and b) a universal splint oligonucleotide having SEQ ID NO: 13, and c) a donor oligonucleotide having SEQ ID NO: 19 is provided. In some embodiments, a composition or a kit including a) an acceptor oligonucleotide comprising a ligation handle SEQ ID NO: 12, b) a universal splint oligonucleotide having SEQ ID NO: 13, where the universal splint oligonucleotide includes an inverted dideoxy-thymine nucleotide (e.g., 5' to 5' reversed linkage), and c) a donor oligonucleotide comprising one of SEQ ID NOs: 15, 16, 17, 18, or 19 is provided. In some embodiments, a composition or a kit including a) an acceptor oligonucleotide comprising a ligation handle SEQ ID NO: 12, b) a universal splint oligonucleotide having SEQ ID NO: 13, wherein the universal splint oligonucleotide includes an inverted dideoxy-adenine nucleotide (e.g., 5' to 5' reversed linkage), and c) a donor oligonucleotide comprising one of SEQ ID NOs: 15, 16, 17, 18, or 19 is provided. In some embodiments, a composition or a kit including a) an acceptor oligonucleotide comprising a ligation handle SEQ ID NO: 12, b) a universal splint oligonucleotide having SEQ ID NO: 13, wherein the universal splint oligonucleotide includes an inverted dideoxy-cytosine nucleotide (e.g., 5' to 5' reversed linkage), and c) a donor oligonucleotide comprising one of SEQ ID NOs: 15, 16, 17, 18, or 19 is provided. In some embodiments, a composition or a kit including a) an acceptor oligonucleotide comprising a ligation handle SEQ ID NO: 12, b) a universal splint oligonucleotide having SEQ ID NO: 13, wherein the universal splint oligonucleotide includes an inverted dideoxy-guanine nucleotide (e.g., 5' to 5' reversed linkage), and c) a donor oligonucleotide comprising one of SEQ ID NOs: 15, 16, 17, 18, or 19 is provided.

In some embodiments, a composition or a kit including a) an acceptor oligonucleotide comprising a ligation handle SEQ ID NO: 30 b) a splint oligonucleotide SEQ ID NO: 21, and c) a donor oligonucleotide SEQ ID NO: 25 is provided. In some embodiments, a composition or a kit including a) an acceptor oligonucleotide comprising a ligation handle SEQ ID NO: 31 b) a splint oligonucleotide SEQ ID NO: 22, and c) a donor oligonucleotide SEQ ID NO: 26 is provided. In some embodiments, a composition or a kit including a) an acceptor oligonucleotide comprising a ligation handle SEQ ID NO: 32 b) a splint oligonucleotide SEQ ID NO: 23, and c) a donor oligonucleotide SEQ ID NO: 27 is provided. In some embodiments, a composition or a kit including a) an acceptor oligonucleotide comprising a ligation handle SEQ ID NO: 33 b) a splint oligonucleotide SEQ ID NO: 24, and c) a donor oligonucleotide SEQ ID NO: 28 is provided.

In any of the compositions described above, the donor oligonucleotides (e.g., a donor oligonucleotide having SEQ ID NOs: 15, 16, 17, 18, and 19) can include one or more phosphorothioate bonds at its 3' end. For example, in any of the compositions described above a phosphorothioate bond can be between the last nucleotide and the second to last nucleotide of the donor oligonucleotide (e.g., a donor oligonucleotide having SEQ ID NOs: 15, 16, 17, 18, and 19), between the second to last nucleotide and the third to last nucleotide of the donor oligonucleotide, between the third to last nucleotide and the fourth to last nucleotide, and combinations thereof.

**FIG. 5** shows an example of a ligation strategy with an acceptor oligonucleotide that includes a first ligation handle at the 3' end of the acceptor oligonucleotide. In some embodiments, the acceptor oligonucleotide shown in in **FIG. 5** includes a cleavage domain (e.g., a polythymine sequence, e.g., a pentathymine sequence.) In some embodiments, the acceptor oligonucleotide shown in **FIG. 5** includes a functional domain (e.g., a sequencing handle, e.g., R1, e.g., an amplification domain). In some embodiments, the acceptor oligonucleotide shown in **FIG. 5** includes a unique molecular identifier. SEQ ID NO: 12 is an example of a first ligation handle of an acceptor oligonucleotide. **FIG. 5** also shows a donor oligonucleotide that includes a second ligation handle and a capture domain. Ligation of the acceptor oligonucleotide and the donor oligonucleotide can be facilitated by a splint oligonucleotide (e.g., a universal splint oligonucleotide comprising SEQ ID NO: 13). In some embodiments, a universal splint oligonucleotide differs from SEQ ID NO: 13 by one, two, three, or four nucleotides. In some embodiments, a universal splint oligonucleotide includes a sequence that is at least 80%, 85%, 90%, or 95% identical to SEQ ID NO: 13. In some embodiments where the universal splint oligonucleotide differs from SEQ ID NO: 13, the first ligation handle, the second ligation handle, or both can be altered to include corresponding complementary nucleotide changes. In some embodiments where the universal splint oligonucleotide differs from SEQ ID NO: 13, the first ligation handle, the second ligation handle, or both can be unaltered such that although there are mismatches between the universal splint oligonucleotide and the first ligation handle, the second ligation handle, or both, the splint oligonucleotide is still able to hybridize to both the first and second ligation handles.

In some embodiments, the universal splint oligonucleotide can have sequence substantially complementary to a ligation handle (e.g., a first ligation handle) present in the acceptor oligonucleotide. In some embodiments, the universal splint oligonucleotide can have a sequence substantially complementary to a ligation handle (e.g., a second ligation handle) present in the donor oligonucleotide. In some embodiments, the capture domain of the donor oligonucleotide can be any capture domain described herein. In some embodiments, the capture domain of the donor oligonucleotide can interact with analytes containing a poly(A) sequence, gene specific sequence(s), a V(D)J sequence, analyte capture sequences, ribosomal sequences (e.g., 16s), or any other analyte described herein. In some embodiments, a first plurality of donor oligonucleotides including a first capture domain are ligated to a first plurality of acceptor oligonucleotides. In some embodiments, a second plurality of donor oligonucleotides inducing a second capture domain are ligated to a second plurality of acceptor oligonucleotides. In some embodiments, the first capture domain and the second capture domain are different (e.g., the first capture domain and the second capture domain capture different analytes).

In some embodiments, the donor oligonucleotide includes a second ligation handle substantially complementary or complementary to a portion of the universal splint oligonucleotide (e.g., nucleotides 1-7 of SEQ ID NO: 15) and the capture domain includes a poly(T) sequence (e.g., nucleotides 8 to 37 of SEQ ID NO: 15). In some embodiments, the donor oligonucleotide includes a second ligation handle substantially complementary or complementary to a portion of the universal splint oligonucleotide (e.g., nucleotides 1-7 of SEQ ID NO: 16) and the capture domain includes a random hexamer sequence (e.g., nucleotides 8-13 of SEQ ID NO: 16). In some embodiments, the donor oligonucleotide includes a second ligation handle substantially complementary or complementary to a portion of the universal splint oligonucleotide (e.g., nucleotides 1-7 of SEQ ID NO: 17) and the capture domain includes a random nonamer sequence (e.g., nucleotides 8-16 of SEQ ID NO: 17). In some embodiments, the donor oligonucleotide includes a second ligation handle substantially complementary or complementary to a portion of the universal splint oligonucleotide (e.g., nucleotides 1-7 of SEQ ID NO: 18) and the capture domain includes a random 12-mer sequence (e.g., nucleotides 8-19 of SEQ ID NO: 18). In some embodiments, the donor oligonucleotide includes a second ligation handle substantially complementary or complementary to a portion of the universal splint oligonucleotide (e.g., nucleotides 1-7 of SEQ ID NO: 18) and the capture domain includes capture sequence 1 (e.g., nucleotides 8-28 of SEQ ID NO: 19). In some embodiments, the capture domain includes capture sequence 2 (SEQ ID NO: 20). In some embodiments, capture sequence 2 can be complementary to an analyte capture sequence as described herein.

In some embodiments, the donor oligonucleotide includes the unique molecular identifier instead of the acceptor oligonucleotide. For example, in a 5' to 3' direction the donor oligonucleotide can include the second ligation handle, the unique molecular identifier (UMI), and the capture domain (e.g., any of the capture domains described herein). In some embodiments, the donor oligonucleotide can have a ligation handle (e.g., a second ligation handle) that is substantially complementary to a sequence in the splint oligonucleotide. In some embodiments, the second ligation handle can be about 5 to 15 nucleotides in length. In some embodiments, the second ligation handle can be about 5, 6, 7, 8, 9, 10, 11, 12, 13, 14, or 15 nucleotides in length. For example, the second ligation handle present in the donor oligonucleotide can be the first 11 nucleotides (nucleotide positions 1 through 11) in SEQ ID NOs: 25, 26, 27, 28, and 29. In some embodiments, the capture domain is a random sequence. In some embodiments, the capture domain is a gene-specific sequence. In some

embodiments, the capture domain includes a poly(T) sequence. In some embodiments, the poly(T) sequence can be about 15 nucleotides to about 50 nucleotides in length. In some embodiments, the poly(T) sequence can be about 20 nucleotides to about 45 nucleotides in length. In some embodiments, the poly(T) sequence can be about 25 nucleotides to about 35 nucleotides in length. In some embodiments, the poly(T) sequence can be about 30 nucleotides in length. In some embodiments, the poly(T) sequence (e.g., capture domain) can be 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more nucleotides in length. In some embodiments, the unique molecular identifier is a 12-mer sequence. For example, in donor oligonucleotides with SEQ ID NOs: 25, 26, 27, 28, and 29, the UMI is denoted by the 12-mer "N" sequence which follows the second ligation handle. In some embodiments, the donor oligonucleotide includes a fluorescent label. In some embodiments, the donor oligonucleotide includes a fluorescent label at its 3' end.

In some embodiments, the acceptor oligonucleotide is prepared by *in situ* synthesis on the array. Non-limiting examples of *in situ* oligonucleotide (e.g., acceptor oligonucleotide) synthesis includes light-directed synthesis or solid-phase synthesis. In some embodiments, the acceptor oligonucleotide can have a ligation handle (e.g., a first ligation handle) that is substantially complementary to a sequence in the splint oligonucleotide. In some embodiments, the first ligation handle can be about 5 to 15 nucleotides in length. In some embodiments, the first ligation handle can be about 5, 6, 7, 8, 9, 10, 11, 12, 13, 14, or 15 nucleotides in length. For example, the first ligation handle present in the acceptor oligonucleotide can be the final 11 nucleotides at the 3' end of an acceptor oligonucleotide as found in SEQ ID NOs: 30, 31, 32, and 33. In some embodiments, the acceptor oligonucleotide includes a cleavage domain, one or more functional domains, a spatial barcode, or any combination thereof.

In some embodiments, a splint oligonucleotide facilitates the ligation of the acceptor oligonucleotide and the donor oligonucleotide. In some embodiments, the splint oligonucleotide is about 10 to about 30 nucleotides in length. In some embodiments, the splint oligonucleotide is about 15 to about 25 nucleotides in length. In some embodiments, the splint oligonucleotide is about 20 nucleotides in length. In some embodiments, the splint oligonucleotide is about 10, 11, 12, 13, 14, 15, 16, 17, 18, 19, 20, 21, 22, 23, 24, 25, 26, 27, 28, 29, 30, or more nucleotides in length. In some embodiments, the splint oligonucleotide is 22 nucleotides in length. In some embodiments, the splint oligonucleotide is an oligonucleotide comprising one of SEQ ID NOs: 21, 22, 23, or 24. In some embodiments, the splint oligonucleotide includes a sequence substantially complementary to the first ligation handle of the acceptor nucleotide. In some embodiments, the splint oligonucleotide includes a sequence substantially complementary to the second ligation handle of the donor oligonucleotide. In some embodiments, the splint oligonucleotide can hybridize both the first ligation handle of the acceptor oligonucleotide and the second ligation handle of the donor oligonucleotide at the same time. In some embodiments, the splint oligonucleotide facilitates the ligation of the acceptor oligonucleotide to the donor oligonucleotide. In some embodiments, the splint oligonucleotide is released from the acceptor oligonucleotide and the donor oligonucleotide. In some embodiments, the splint oligonucleotide is released after the ligation of the acceptor oligonucleotide and the donor oligonucleotide.

In some embodiments, splint oligonucleotide V2-1 (SEQ ID NO: 21) facilitates the ligation of acceptor oligonucleotide V2-1 ligation handle (SEQ ID NO: 30) to donor oligonucleotide UMI-V2-1 (SEQ ID NO: 25). In some embodiments, splint oligonucleotide V2-1 (SEQ ID NO: 21) facilitates the ligation of acceptor oligonucleotide V2-1 ligation handle (SEQ ID NO: 30) to donor oligonucleotide UMI-Cy3 (SEQ ID NO: 29). In some embodiments, splint oligonucleotide V2-2 (SEQ ID NO: 22) facilitates the ligation of acceptor oligonucleotide V2-2 ligation handle (SEQ ID NO: 31) to donor oligonucleotide UMI-V2-2 (SEQ ID NO: 26). In some embodiments, splint oligonucleotide V2-3 (SEQ ID NO: 23) facilitates the ligation of acceptor oligonucleotide V2-3 ligation handle (SEQ ID NO: 32) to donor oligonucleotide UMI-V2-3 (SEQ ID NO: 27). In some embodiments, splint oligonucleotide V2-4 (SEQ ID NO: 24) facilitates the ligation of acceptor oligonucleotide V2-4 ligation handle (SEQ ID NO: 33) to donor oligonucleotide UMI-V2-4 (SEQ ID NO: 28).

In some embodiments, a donor oligonucleotide (e.g., a donor oligonucleotide comprising a sequence of SEQ ID NO: 25, 26, 27, 28, or 29) can include one or more phosphorothioate bonds at its 3' end. For example, in some embodiments, a phosphorothioate bond can be between the last nucleotide and the second to last nucleotide of the donor oligonucleotide (e.g., a donor oligonucleotide comprising a sequence of SEQ ID NOs: 25, 26, 27, 28, or 29), between the second to last nucleotide and the third to last nucleotide of the donor oligonucleotide, between the third to last nucleotide and the fourth to last nucleotide, or any combination thereof.

*Compositions and Kits*

Also included herein are kits including (a) an array including a plurality of acceptor oligonucleotides, where an acceptor oligonucleotide of the plurality of acceptor oligonucleotides includes a spatial barcode and a first ligation handle; (b) a plurality of universal splint oligonucleotides; and (c) a ligase.

In some kits, the acceptor oligonucleotide includes SEQ ID NO: 12. In some kits, a universal splint oligonucleotide of the plurality of universal splint oligonucleotides includes a sequence at least 90% identical to SEQ ID NO: 13. In some kits, the splint oligonucleotide includes SEQ ID NO: 13.

In some kits, the ligase is T4 ligase. In some kits, the kit includes a reverse transcriptase. In some kits, the kit includes a DNA polymerase.

In some kits, the kit includes one or more permeabilization reagents. In some kits, the kit includes one or more RNase inhibitors. In some kits, the kit includes instructions for performing any of the methods described herein.

Also provided herein are compositions including (i) an acceptor oligonucleotide including a spatial barcode and a first ligation handle, where the 5' end of the acceptor oligonucleotide is attached to a substrate; (ii) a first donor oligonucleotide including a second ligation handle and a poly(T) capture domain; and (iii) a universal splint oligonucleotide including a sequence complementary to the first ligation handle and a sequence complementary to the second ligation handle, and where the universal splint oligonucleotide is hybridized to the first ligation handle of the acceptor oligonucleotide and the second ligation handle of the donor oligonucleotide.

Also provided herein are compositions including (i) a first acceptor oligonucleotide including a spatial barcode and a first ligation handle, where the 5' end of the acceptor oligonucleotide is attached to a substrate; (ii) a first donor oligonucleotide including a second ligation handle and a poly(T) capture domain; (iii) a second donor oligonucleotide including the second ligation handle and a sequence including SEQ ID NO: 20; (iv) a first universal splint oligonucleotide including a sequence complementary to the first ligation handle and a sequence complementary to the second ligation handle, and where the universal splint oligonucleotide is hybridized to the first ligation handle of the first acceptor oligonucleotide and the second ligation handle of the first donor oligonucleotide; and (v) a second universal splint oligonucleotide including a sequence complementary to the first ligation handle and a sequence complementary to the second ligation handle, and where the universal splint oligonucleotide is hybridized to the first ligation handle of the second acceptor oligonucleotide and the second ligation handle of the second donor oligonucleotide.

Also provided herein are compositions including at least one of a plurality of first acceptor oligonucleotides including a spatial barcode ligated to at least one of a plurality of first donor oligonucleotides including a first capture domain, where the plurality of first acceptor oligonucleotides is attached to a substrate, at least one of the plurality of first acceptor oligonucleotides including a spatial barcode and a first ligation handle, and at least one of a plurality of second donor oligonucleotides including a second ligation handle and a second capture domain, and a universal splint oligonucleotide, where the universal splint oligonucleotide is hybridized to a first ligation handle of at least one of a plurality of first acceptor oligonucleotides and the second ligation handle of at least one of the plurality of second donor oligonucleotides.

Provided herein are kits including (a) an array comprising a plurality of acceptor oligonucleotides, where an acceptor oligonucleotide of the plurality of acceptor oligonucleotides includes a spatial barcode and a first ligation handle; (b) a plurality of splint oligonucleotides; and (c) a ligase. In some kits, the acceptor oligonucleotide comprises ligation handle SEQ ID NO: 12. In some kits, a splint oligonucleotide of the plurality of splint oligonucleotides comprises a sequence at least 90% identical to SEQ ID NO: 13. In some kits, the splint oligonucleotide comprises SEQ ID NO: 13. In some kits, the ligase is T4 ligase. In some kits, the kit includes a reverse transcriptase. In some kits, the kit includes a DNA polymerase. In some kits, the kit includes one or more permeabilization reagents. In some kits, the kit further includes one or more RNase inhibitors. In some kits, the kit includes instructions for performing any one of the methods of described herein.

In some embodiments, a composition or a kit comprising a) an acceptor oligonucleotide comprising ligation handle SEQ ID NO: 30, b) a splint oligonucleotide comprising SEQ ID NO: 21, where the 3'-most nucleotide of the splint oligonucleotide is an inverted thymine nucleotide (e.g., 3' to 3' reversed linkage), and c) a donor oligonucleotide comprising SEQ ID NOs: 25 and/or 29 is provided. In some embodiments, a composition or a kit comprising a) an acceptor oligonucleotide comprising ligation handle SEQ ID NO: 31, b) a splint oligonucleotide comprising SEQ ID NO: 22, where the 3'-most nucleotide of the splint oligonucleotide is an inverted thymine nucleotide (e.g., 3' to 3' reversed linkage), and c) a donor oligonucleotide comprising SEQ ID NO: 26 is provided. In some embodiments, a composition or a kit comprising a) an acceptor oligonucleotide comprising ligation handle SEQ ID NO: 32, b) a splint oligonucleotide comprising SEQ ID NO: 23, where the 3'-most nucleotide of the splint oligonucleotide is an inverted thymine nucleotide (e.g., 3' to 3' reversed linkage), and c) a donor oligonucleotide comprising SEQ ID NO: 27 is provided. In some embodiments, a composition or a kit comprising a) an acceptor oligonucleotide comprising ligation handle SEQ ID NO: 33, b) a splint oligonucleotide comprising SEQ ID NO: 24, where the 3'-most nucleotide of the splint oligonucleotide is an inverted thymine nucleotide (e.g., 3' to 3' reversed linkage), and c) a donor oligonucleotide comprising SEQ ID NO: 28 is provided.

In some embodiments, a composition or a kit comprising a) an acceptor oligonucleotide comprising ligation handle SEQ ID NO: 30 b) a splint oligonucleotide comprising SEQ ID NO: 21, wherein the 3'-most nucleotide of the splint oligonucleotide is an inverted adenine nucleotide (e.g., 3' to 3' reversed linkage), and c) a donor oligonucleotide comprising SEQ ID NOs: 25 and/or 29 is provided. In some embodiments, a composition or a kit comprising a) an acceptor oligonucleotide comprising ligation handle SEQ ID NO: 31 b) a splint oligonucleotide comprising SEQ ID NO: 22, where the 3'-most nucleotide of the splint oligonucleotide is an inverted adenine nucleotide (e.g., 3' to 3' reversed linkage), and c) a donor oligonucleotide comprising SEQ ID NO: 26 is provided. In some embodiments, a composition or a kit comprising a) an acceptor oligonucleotide comprising ligation handle SEQ ID NO: 32 b) a splint oligonucleotide comprising

SEQ ID NO: 23, wherein the 3'-most nucleotide of the splint oligonucleotide is an inverted adenine nucleotide (e.g., 3' to 3' reversed linkage), and c) a donor oligonucleotide comprising SEQ ID NO: 27 is provided. In some embodiments, a composition or a kit comprising a) an acceptor oligonucleotide comprising ligation handle SEQ ID NO: 33 b) a splint oligonucleotide comprising SEQ ID NO: 24, where the 3'-most nucleotide of the splint oligonucleotide is an inverted adenine nucleotide (e.g., 3' to 3' reversed linkage), and c) a donor oligonucleotide comprising SEQ ID NO: 28 is provided.

In some embodiments, a composition or a kit comprising a) an acceptor oligonucleotide comprising ligation handle SEQ ID NO: 30 b) a splint oligonucleotide comprising SEQ ID NO: 21, where the 3'-most nucleotide of the splint oligonucleotide is an inverted cytosine nucleotide (e.g., 3' to 3' reversed linkage), and c) a donor oligonucleotide comprising SEQ ID NOs: 25 and/or 29 is provided. In some embodiments, a composition or a kit comprising a) an acceptor oligonucleotide comprising ligation handle SEQ ID NO: 31 b) a splint oligonucleotide comprising SEQ ID NO: 22, where the 3'-most nucleotide of the splint oligonucleotide is an inverted cytosine nucleotide (e.g., 3' to 3' reversed linkage), and c) a donor oligonucleotide comprising SEQ ID NO: 26 is provided. In some embodiments, a composition or a kit comprising a) an acceptor oligonucleotide comprising ligation handle SEQ ID NO: 32 b) a splint oligonucleotide comprising SEQ ID NO: 23, where the 3'-most nucleotide of the splint oligonucleotide is an inverted cytosine nucleotide (e.g., 3' to 3' reversed linkage), and c) a donor oligonucleotide compring SEQ ID NO: 27 is provided. In some embodiments, a composition or a kit comprising a) an acceptor oligonucleotide comprising ligation handle SEQ ID NO: 33 b) a splint oligonucleotide comprising SEQ ID NO: 24, where the 3'-most nucleotide of the splint oligonucleotide is an inverted cytosine nucleotide (e.g., 3' to 3' reversed linkage), and c) a donor oligonucleotide comprising SEQ ID NO: 28 is provided.

In some embodiments, a composition or a kit comprising a) an acceptor oligonucleotide comprising ligation handle SEQ ID NO: 30, b) a splint oligonucleotide comprising SEQ ID NO: 21, where the 3'-most nucleotide of the splint oligonucleotide is an inverted guanine nucleotide (e.g., 3' to 3' reversed linkage), c) a donor oligonucleotide comprising SEQ ID NOs: 25 and/or 29 is provided. In some embodiments, a composition or a kit comprising a) an acceptor oligonucleotide comprising ligation handle SEQ ID NO: 31, b) a splint oligonucleotide comprising SEQ ID NO: 22, where the 3'-most nucleotide of the splint oligonucleotide is an inverted guanine nucleotide (e.g., 3' to 3' reversed linkage), c) a donor oligonucleotide comprising SEQ ID NO: 26 is provided. In some embodiments, a composition or a kit comprising a) an acceptor oligonucleotide comprising ligation handle SEQ ID NO: 32, b) a splint oligonucleotide comprising SEQ ID NO: 23, where the 3'-most nucleotide of the splint oligonucleotide is an inverted guanine nucleotide (e.g., 3' to 3' reversed linkage), c) a donor oligonucleotide comprising SEQ ID NO: 27 is provided. In some embodiments, a composition or a kit comprising a) an acceptor oligonucleotide comprising ligation handle SEQ ID NO: 33, b) a splint oligonucleotide comprising SEQ ID NO: 24, where the 3'-most nucleotide of the splint oligonucleotide is an inverted guanine nucleotide (e.g., 3' to 3' reversed linkage), c) a donor oligonucleotide comprising SEQ ID NO: 28 is provided.

In some embodiments, a composition or a kit comprising a) an acceptor oligonucleotide comprising ligation handle SEQ ID NO: 30, b) a splint oligonucleotide comprising SEQ ID NO: 21, where the splint oligonucleotide includes an inverted dideoxy-thymine nucleotide (e.g., 5' to 5' reversed linkage), and c) a donor oligonucleotide comprising SEQ ID NOs: 25 and/or 29 is provided. In some embodiments, a composition or a kit comprising a) an acceptor oligonucleotide comprising ligation handle SEQ ID NO: 31, b) a splint oligonucleotide comprising SEQ ID NO: 22, where the splint oligonucleotide comprises an inverted dideoxy-thymine nucleotide (e.g., 5' to 5' reversed linkage), and c) a donor oligonucleotide comprising SEQ ID NO: 26 is provided. In some embodiments, a composition or a kit comprising a) an acceptor oligonucleotide comprising ligation handle SEQ ID NO: 32, b) a splint oligonucleotide comprising SEQ ID NO: 23, where the splint oligonucleotide includes an inverted dideoxy-thymine nucleotide (e.g., 5' to 5' reversed linkage), and c) a donor oligonucleotide comprising SEQ ID NO: 27 is provided. In some embodiments, a composition or a kit comprising a) an acceptor oligonucleotide comprising ligation handle SEQ ID NO: 33, b) a splint oligonucleotide comprising SEQ ID NO: 24, where the splint oligonucleotide includes an inverted dideoxy-thymine nucleotide (e.g., 5' to 5' reversed linkage), and c) a donor oligonucleotide comprising SEQ ID NO: 28 is provided.

In some embodiments, a composition or a kit comprising a) an acceptor oligonucleotide comprising ligation handle SEQ ID NO: 30, b) a splint oligonucleotide comprising SEQ ID NO: 21, where the splint oligonucleotide includes an inverted dideoxy-adenine nucleotide (e.g., 5' to 5' reversed linkage), and c) a donor oligonucleotide comprising SEQ ID NOs: 25 and/or 29 is provided. In some embodiments, a composition or a kit comprising a) an acceptor oligonucleotide comprising ligation handle SEQ ID NO: 31, b) a splint oligonucleotide comprising SEQ ID NO: 22, where the splint oligonucleotide includes an inverted dideoxy-adenine nucleotide (e.g., 5' to 5' reversed linkage), and c) a donor oligonucleotide comprising SEQ ID NO: 26 is provided. In some embodiments, a composition or a kit comprising a) an acceptor oligonucleotide comprising ligation handle SEQ ID NO: 32, b) a splint oligonucleotide comprising SEQ ID NO: 23, where the splint oligonucleotide includes an inverted dideoxy-adenine nucleotide (e.g., 5' to 5' reversed linkage), and c) a donor oligonucleotide comprising SEQ ID NOs: 27 is provided. In some embodiments, a composition or a kit comprising a) an acceptor oligonucleotide comprising ligation handle SEQ ID NO: 33, b) a splint oligonucleotide comprising SEQ ID NO: 24, where the splint oligonucleotide includes an inverted dideoxy-adenine nucleotide (e.g., 5' to 5' reversed linkage), and c) a donor oligonucleotide comprising SEQ ID NO: 28 is provided.

In some embodiments, a composition or a kit comprising a) an acceptor oligonucleotide comprising ligation

handle SEQ ID NO: 30, b) a splint oligonucleotide comprising SEQ ID NO: 21, where the splint oligonucleotide includes an inverted dideoxy-cytosine nucleotide (e.g., 5' to 5' reversed linkage), and c) a donor oligonucleotide comprising SEQ ID NOs: 25 and/or 29 is provided. In some embodiments, a composition or a kit comprising a) an acceptor oligonucleotide comprising ligation handle SEQ ID NO: 31, b) a splint oligonucleotide comprising SEQ ID NO: 22, where the splint oligonucleotide includes an inverted dideoxy-cytosine nucleotide (e.g., 5' to 5' reversed linkage), and c) a donor oligonucleotide comprising SEQ ID NO: 26 is provided. In some embodiments, a composition or a kit comprising a) an acceptor oligonucleotide comprising ligation handle SEQ ID NO: 32, b) a splint oligonucleotide comprising SEQ ID NO: 23, where the splint oligonucleotide includes an inverted dideoxy-cytosine nucleotide (e.g., 5' to 5' reversed linkage), and c) a donor oligonucleotide comprising SEQ ID NO: 27 is provided. In some embodiments, a composition or a kit comprising a) an acceptor oligonucleotide comprising ligation handle SEQ ID NO: 33, b) a splint oligonucleotide comprising SEQ ID NO: 24, where the splint oligonucleotide includes an inverted dideoxy-cytosine nucleotide (e.g., 5' to 5' reversed linkage), and c) a donor oligonucleotide comprising SEQ ID NO: 28 is provided.

In some embodiments, a composition or a kit comprising a) an acceptor oligonucleotide comprising ligation handle SEQ ID NO: 30, b) a splint oligonucleotide comprising SEQ ID NO: 21, where the splint oligonucleotide includes an inverted dideoxy-guanine nucleotide (e.g., 5' to 5' reversed linkage), and c) a donor oligonucleotide comprising SEQ ID NOs: 25 and/or 29 is provided. In some embodiments, a composition or a kit comprising a) an acceptor oligonucleotide comprising ligation handle SEQ ID NO: 31, b) a splint oligonucleotide comprising SEQ ID NO: 22, where the splint oligonucleotide includes an inverted dideoxy-guanine nucleotide (e.g., 5' to 5' reversed linkage), and c) a donor oligonucleotide comprising SEQ ID NO: 26 is provided. In some embodiments, a composition or a kit comprising a) an acceptor oligonucleotide comprising ligation handle SEQ ID NO: 32, b) a splint oligonucleotide comprising SEQ ID NO: 23, where the splint oligonucleotide includes an inverted dideoxy-guanine nucleotide (e.g., 5' to 5' reversed linkage), and c) a donor oligonucleotide comprising SEQ ID NO: 27 is provided. In some embodiments, a composition or a kit comprising a) an acceptor oligonucleotide comprising ligation handle SEQ ID NO: 33, b) a splint oligonucleotide comprising SEQ ID NO: 24, where the splint oligonucleotide includes an inverted dideoxy-guanine nucleotide (e.g., 5' to 5' reversed linkage), and c) a donor oligonucleotide comprising SEQ ID NO: 28 is provided.

*Multiplexing*

As described herein, capture probes can be generated via universal splint oligonucleotide-mediated ligation to generate arrays with two or more capture domains (e.g., two or more capture domains specific for different analytes). In some embodiments, an array (e.g., a feature on the array) can be generated with capture probes including SEQ ID NO: 15 and capture probes including SEQ ID 16. In some embodiments, an array (e.g., a feature on the array) can be generated with capture probes including SEQ ID NO: 15 and capture probes including SEQ ID 17. In some embodiments, an array (e.g., a feature on the array) can be generated with capture probes including SEQ ID NO: 15 and capture probes including SEQ ID 18. In some embodiments, an array (e.g., a feature on the array) can be generated with capture probes including SEQ ID NO: 15 and capture probes including SEQ ID 19.

In some embodiments, the array (e.g., a feature on the array) includes about 5% to about 35% of capture probes with capture domains comprising SEQ ID NO: 16 (e.g., nucleotides 8-13 of SEQ ID NO: 16) and about 65% to about 95% of capture probes with capture domains comprising SEQ ID NO: 15 (e.g., nucleotides 8 to 37 of SEQ ID NO: 15). In some embodiments, the array includes about 5% to about 35% of capture probes with capture domains comprising SEQ ID NO: 17 (e.g., nucleotides 8-16 of SEQ ID NO: 17) and about 65% to about 95% of capture probes with capture domains comprising SEQ ID NO: 15 (e.g., nucleotides 8 to 37 of SEQ ID NO: 15). In some embodiments, the array includes about 5% to about 35% of capture probes with capture domains comprising SEQ ID NO: 18 (e.g., nucleotides 8-19 of SEQ ID NO: 18) and about 65% to about 95% of capture probes with capture domains comprising SEQ ID NO: 15 (e.g., nucleotides 8 to 37 of SEQ ID NO: 15). In some embodiments, the array includes about 5% to about 35% of capture probes with capture domains comprising SEQ ID NO: 19 (e.g., nucleotides 8-28 of SEQ ID NO: 19) and about 65% to about 95% of capture probes with capture domains comprising SEQ ID NO: 15 (e.g., nucleotides 8 to 37 of SEQ ID NO: 15). In some embodiments, the array includes about 5% to about 35% of capture probes with gene specific capture probes and about 65% to about 95% of capture probes with capture domains comprising SEQ ID NO: 15 (e.g., nucleotides 8 to 37 of SEQ ID NO: 15).

In some embodiments, the array (e.g., a feature on the array) includes a ratio of capture probes with a second capture domain to capture probes with a first capture domain from about 1:1 to about 1:15, from about 1:2 to about 1:12, from about 1:3 to about 1:10, from about 1:4 to about 1:9, from about 1:5 to about 1:8, or about 1:6 to about 1:7. In some embodiments, the first capture domain and the second capture domain are different. For example, the first capture domain can include a poly(T) sequence and the second capture domain can include a unique sequence, such a gene specific sequence. Further, the first capture domain can be a first unique sequence and the second capture probe can be a second unique sequence, both capable of capturing, for example, different gene specific sequences. However, it will be appreciated that a desired ratio of any combination of capture domains (e.g., a first capture domain, a second capture

domain) can be achieved by the methods described herein.

To reduce or eliminate unwanted ligation reactions (e.g., to ensure that ligation occurs only or primarily between an acceptor oligonucleotide and a donor oligonucleotide), the universal splint oligonucleotide, the donor oligonucleotide, or both can be modified. For example, the 3' end of the universal splint oligonucleotide can be modified with an inverted base to prevent unwanted ligation events (e.g., ligation of universal splint oligonucleotides to each other or ligation of universal splint oligonucleotides to a donor oligonucleotide). In some embodiments, the inverted base is a 3' to 3' reversed linkage (FIG. 18A). In some embodiments, the 3'-most nucleotide of the universal splint oligonucleotide is an inverted thymine nucleotide. In some embodiments, the 3'-most nucleotide of the universal splint oligonucleotide is an inverted adenine nucleotide. In some embodiments, the 3'-most nucleotide of the universal splint oligonucleotide is an inverted cytosine nucleotide. In some embodiments, the 3'-most nucleotide of the universal splint oligonucleotide is an inverted guanine nucleotide. In some embodiments, the inverted base is a 5' to 5' reversed linkage **(FIG. 18B).** In some embodiments, the inverted base in a 5' to 5' reversed linkage is a dideoxy-thymine nucleotide. In some embodiments, the inverted base in a 5' to 5' reversed linkage is a dideoxy-adenine nucleotide. In some embodiments, the inverted base in a 5' to 5' reversed linkage is a dideoxy-cytosine nucleotide. In some embodiments, the inverted base in a 5' to 5' reversed linkage is a dideoxy-guanine nucleotide. Other suitable methods can be used to prevent unwatned ligation and are known in the art. For example, a 3'ddC, a 3' inverted dT, a 3' C3 spacer, a 3' amino, and 3' phosphorylation are other methods that can be used to prevent unwanted ligation.

In some embodiments, the 3' end of the donor oligonucleotide (e.g., any of the donor oligonucleotides including a capture domain described herein) can be modified with phosphorothioate bonds between nucleotides to prevent degradation by a nuclease (e.g., an exonuclease). In some embodiments, a phosphorothioate bond can be present between the last and second to last nucleotides at the 3' end. Additionally or alternatively, a phosphorothioate bond can be present between the second to last nucleotide and the third to last nucleotide at the 3' end. In some embodiments, a phosphorothioate bond can be present between the second to last nucleotide and the third to last and the fourth to last nucleotides at the 3' end. In some embodiments, a phosphorothioate bond can be present between the last and second to last nucleotide, between the second to last nucleotide and third to last nucleotide, between the third to last nucleotide and fourth to last nucleotide at the 3' end, and combinations thereof.

In some embodiments, the 3' end of the acceptor oligonucleotide (e.g., any of the acceptor oligonucleotides including a capture domain described herein) can be modified with phosphorothioate bonds between nucleotides to prevent degradation by a nuclease (e.g., an exonuclease). In some embodiments, a phosphorothioate bond can be present between the last and second to last nucleotides at the 3' end. Additionally or alternatively, a phosphorothioate bond can be present between the second to last nucleotide and the third to last nucleotide at the 3' end. In some embodiments, a phosphorothioate bond can be present between the second to last nucleotide and the third to last and the fourth to last nucleotides at the 3' end. In some embodiments, a phosphorothioate bond can be present between the last and second to last nucleotide, between the second to last nucleotide and third to last nucleotide, between the third to last nucleotide and fourth to last nucleotide at the 3' end, and combinations thereof in the acceptor oligonucleotide.

In some embodiments, the universal splint oligonucleotide includes a sequence that is complementary to an oligonucleotide (e.g., an acceptor oligonucleotide, e.g., a first ligation handle present in the acceptor oligonucleotide), or a portion thereof, and a sequence that is complementary to an oligonucleotide (e.g., a donor oligonucleotide) containing a capture domain, or a portion thereof (e.g., complementary to the capture domain or a portion of the capture domain itself or to a second ligation handle). In some embodiments, the universal splint oligonucleotide includes a sequence that is perfectly complementary (e.g., is 100% complementary) to an oligonucleotide (e.g., an acceptor oligonucleotide, e.g., a first ligation handle present in the acceptor oligonucleotide), or a portion thereof, and/or a sequence that is perfectly complementary to an oligonucleotide containing a capture domain, or a portion thereof (e.g., perfectly complementary to the capture domain or a portion of the capture domain itself or to a second ligation handle). In some embodiments, the universal splint oligonucleotide includes a sequence that is not perfectly complementary (e.g., is not 100% complementary) to an oligonucleotide (e.g., an acceptor oligonucleotide, e.g., a first ligation handle present in the acceptor oligonucleotide), or a portion thereof, and/or a sequence that is not perfectly complementary to an oligonucleotide containing a capture domain, or a portion thereof (e.g., not perfectly complementary to the capture domain or a portion of the capture domain itself or to a second ligation handle). In some embodiments, the universal splint oligonucleotide, including the universal splint oligonucleotide, includes a sequence that is at least 70%, 75%, 80%, 85%, 90%, 95%, 96%, 97%, 98%, or 99% complementary to an oligonucleotide (e.g., an acceptor oligonucleotide, e.g., a first ligation handle present in the acceptor oligonucleotide), or a portion thereof, and/or a sequence that is complementary to an oligonucleotide containing a capture domain, or a portion thereof (e.g., at least 70%, 75%, 80%, 85%, 90%, 95%, 96%, 97%, 98%, or 99% complementary to the capture domain or a portion of the capture domain itself or to a second ligation handle). In some embodiments, the universal splint oligonucleotide includes a sequence that is perfectly complementary (e.g., is 100% complementary) to an oligonucleotide (e.g., an acceptor oligonucleotide, e.g., a first ligation handle present in the acceptor oligonucleotide), or a portion thereof, but is not perfectly complementary to a sequence that is complementary to an oligonucleotide containing a capture domain, or a portion thereof (e.g., not perfectly complementary to the capture domain

or a portion of the capture domain itself or to a second ligation handle). In some embodiments, the universal splint oligonucleotide includes a sequence that is not perfectly complementary (e.g., is not 100% complementary) to an oligonucleotide (e.g., an acceptor oligonucleotide, e.g., a first ligation handle present in the acceptor oligonucleotide), or a portion thereof, but is perfectly complementary to a sequence that is complementary to an oligonucleotide containing a capture domain, or a portion thereof (e.g., perfectly complementary to the capture domain or a portion of the capture domain itself or to a second ligation handle). So long as the universal splint oligonucleotide is capable of hybridizing to an oligonucleotide (e.g., an acceptor oligonucleotide, e.g., a first ligation handle present in the acceptor oligonucleotide), or a portion thereof, and to a sequence that is complementary to an oligonucleotide containing a capture domain, or a portion thereof (e.g., to the capture domain or a portion of the capture domain itself or to a second ligation handle), the universal splint oligonucleotide need not have a sequence that is perfectly complementary to either the acceptor oligonucleotide or to the donor oligonucleotide.

The universal splint oligonucleotide can be about 10 to about 80 nucleotides long. In some embodiments, the universal splint oligonucleotide can be about 20 to about 70 nucleotides long. In some embodiments, the universal splint oligonucleotide can be about 30 to about 60 nucleotides long. In some embodiments, the universal splint oligonucleotide can be about 40 to about 50 nucleotides long. In some embodiments, the universal splint oligonucleotide can be 14 nucleotides long.

In some embodiments, the spatial array is generated by ligating donor oligonucleotides including capture domains (e.g., poly(T) or gene specific capture domains) to the acceptor oligonucleotides, thus generating an array having a plurality of capture probes. The spatial array can be used with any of the spatial analysis methods described herein. For example, a biological sample can be provided to the generated spatial array. In some embodiments, the biological sample is permeabilized. In some embodiments, the biological sample is permeabilized under conditions sufficient to allow one or more analytes present in the biological sample to interact with the generated capture probes of the spatial array. After capture of analytes in the biological sample, the analytes can be analyzed (e.g., reverse transcribed, amplified, sequenced) using any of the variety of methods described herein.

*Ligation Efficiency*

As used herein, the term "ligation barcode" refers to a unique sequence present in the donor oligonucleotide. In some embodiments, a first plurality of donor oligonucleotides has a first ligation barcode. In some embodiments, a second plurality of donor oligonucleotides has a second ligation barcode. In some embodiments, a second plurality of donor oligonucleotides has a third ligation barcode. In some embodiments, a second plurality of donor oligonucleotides has a fourth ligation barcode. In some embodiments, the first ligation barcode, the second ligation barcode, the third ligation barcode, and the fourth ligation barcode are different. In some embodiments, detecting the presence of the first ligation barcode and the second ligation barcode indicates the efficiency of the first ligation reaction.

In some embodiments, the donor oligonucleotide of the second plurality of donor oligonucleotides is ligated to the 3' end of a second acceptor oligonucleotide of the plurality of acceptor oligonucleotides to generate a second ligation product using a second splint oligonucleotide of the plurality of splint oligonucleotides (e.g., any of the splint oligonucleotides described herein), where the second acceptor oligonucleotide of the plurality of acceptor oligonucleotides includes a spatial barcode, the first adaptor domain, and the first ligation handle and the second splint oligonucleotide of the plurality of splint oligonucleotides includes a sequence substantially complementary to at least a portion of the second acceptor oligonucleotide and a sequence substantially complementary to at least a portion of the donor oligonucleotide of the second plurality of donor oligonucleotides.

In some embodiments, a second strand is generated that is substantially complementary to the second ligation product and quantitatively measured to detect the presence of the second ligation barcode, or a complement thereof, thereby determining the presence of the second acceptor oligonucleotide on the substrate.

In some embodiments, a second strand is generated that is substantially complementary to the first ligation product, the second ligation product, or both, and includes the use of a DNA polymerase (e.g., any of the DNA polymerases described herein). **In** some embodiments, the polymerase is T4 DNA polymerase.

In some embodiments, the efficiency of ligating a plurality of first donor oligonucleotides having a first ligation barcode to an acceptor oligonucleotide (e.g., an acceptor oligonucleotide having a spatial barcode) using a splint oligonucleotide is from about 60% to about 100%, from about 65% to about 95%, from about 70% to about 90%, from about 75% to about 85%, or about 80%. In some embodiments, the efficiency of ligating a plurality of first donor oligonucleotides to a plurality of acceptor oligonucleotides using a splint oligonucleotide is about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100%.

In some embodiments, the first ligation reaction proceeds for a certain amount of time before the second ligation

reaction occurs (e.g., a second donor oligonucleotide ligated to a second acceptor oligonucleotide). In some embodiments, the second ligation reaction proceeds for a certain amount time before the third ligation reaction occurs. In some embodiments, the third ligation reaction proceeds for a certain amount of time before the fourth ligation reaction occurs. In some embodiments, the first ligation reaction, the second ligation reaction, the third ligation reaction, or the fourth ligation reaction proceeds from about 1 minute to about 24 hours, from about 5 minutes to about 12 hours, from about 10 minutes to about 10 hours, from about 20 minutes to about 8 hours, from about 30 minutes to about 6 hours, from about 40 minutes to about 5.5 hours, from about 50 minutes to about 5 hours, from about 60 minutes to about 4.5 hours, from about 1.5 hours to about 4 hours from about 2 hours to about 3.5 hours, from about 2.5 to about 3 hours.

A splint oligonucleotide can reduce cost and complexity in ligation products on a substrate. For example, a splint oligonucleotide can facilitate the ligation of two or more different donor oligonucleotides to the plurality of acceptor oligonucleotides. The ligation efficiency of the first ligation reaction (e.g., the first plurality of donor oligonucleotides ligated to the acceptor oligonucleotides) can be measured. For example, a first plurality of donor oligonucleotides can have a different ligation barcode (e.g., a first ligation barcode) than a ligation barcode (e.g., a second ligation barcode) present in a donor oligonucleotide of a second plurality of donor oligonucleotides.

In some embodiments, quantitatively measuring the presence of the first ligation barcode or a complement thereof, the second ligation barcode or a complement thereof, or both, includes the use of quantitative PCR. In some embodiments, amplifying via PCR includes amplifying with a primer substantially complementary to a first adaptor and a primer substantially complementary to a second adaptor. In some embodiments, quantitative PCR includes a "TAQMAN™" assay. In some embodiments, quantitative PCR includes the use of a dye. In some embodiments, the dye is a DNA intercalating dye such as "SYBR®" dye (e.g., SYBR Green). In some embodiments, quantitative PCR is performed on capillaries ("LightCycler® Capillaries"). In some embodiments, the quantification of genetic material is determined by optical absorbance in conjunction with real-time PCR. In some embodiments, the quantification of genetic material is determined by digital PCR.

## EXAMPLES

### EXAMPLE 1 - Generating gene specific capture probes

Capture probes containing various functional sequences and gene specific or general (e.g., poly(T)) capture domains were generated with a ligation strategy. FIG. 2 shows an immobilized oligonucleotide, acceptor oligonucleotide, on a substrate, from 3' to 5' containing an amino modifier C6 linker at its 3' end, followed by a cleavage domain (e.g., uracils), a sequencing handle functional domain (e.g., R1), a spatial barcode sequence, a UMI sequence, and a ligation handle (SEQ ID NO: 9). A splint oligonucleotide (SEQ ID NO: 10) with a sequence complementary to the ligation handle and an additional sequence (e.g., poly(A) or gene specific sequence) facilitates the ligation of oligonucleotide and the capture domain (SEQ ID NO: 11). Ligation of both poly(T) (e.g., mRNA) and a specific mRNA (e.g., Penk) capture domains to the immobilized oligonucleotide was completed using a mixture containing 30 $\mu$M Poly dT or capture sequence (e.g., Penk), 30 $\mu$M splint oligonucleotide, and 100 units of ligase. **FIG. 3** shows detection of cDNA with ligated and unligated polyT and gene specific, e.g., Penk gene, capture domains. The mixture was placed on top of the slide for 60 minutes then removed before washing and adding tissue. All tissues were permeabilized without pre-permeabilization for a duration of 3, 6, or 9 minutes **(FIGs. 4A-B).** The data show Poly dT was successfully ligated to the immobilized oligonucleotide and capable of capturing mRNA in a biased manner. Additionally, a capture domain for the Penk mRNA was successfully ligated and capable of capturing Penk mRNA. As shown in the Table 1 below, ligating a Poly dT capture domain did not affect sequencing efficiency. The reads mapped to the transcriptome for gene specific data are relatively high for a single gene capture (e.g., Penk), but the ligation strategy and subsequent gene specific mRNA capture is possible.

Table 1.

| Name | Reads Mapped Confidently to Transcriptome | Reads Mapped Confidently to Genome | Fraction Reads with primer or homopolymer sequence | Frace reads with any polyA sequence | Fraction Ribosomal protein UMI counts | Fraction mitochondrial UMI counts |
|---|---|---|---|---|---|---|
| **Ligated Poly(T) (N=6)** | **81.42%** | **90.94%** | **13.58%** | **4.30%** | **9.84%** | **25.80%** |
| **No Ligate Average (N=6)** | **83.12%** | **92.67%** | **13.37%** | **2.52%** | **10.18%** | **26.15%** |

(continued)

| Name | Reads Mapped Confidently to Transcriptome | Reads Mapped Confidently to Genome | Fraction Reads with primer or homopolymer sequence | Frace reads with any polyA sequence | Fraction Ribosomal protein UMI counts | Fraction mitochondrial UMI counts |
|---|---|---|---|---|---|---|
| **Penk Average (N=6)** | **13.33%** | **53.07%** | **10.78%** | **0.28%** | **1.18%** | **2.78%** |

**EXAMPLE 2 - Ligation of Capture Domains to Immobilized Oligonucleotides**

The following ligation protocol and reagents provide for the ligation of donor oligonucleotides including a capture domain to existing arrays. A portion of a splint oligonucleotide is complementary to the ligation handle of an acceptor oligonucleotide, (e.g., acceptor oligonucleotide 2), on the surface of the array. Another portion of the splint oligonucleotide is complementary to a second ligation handle sequence present in an oligonucleotide (e.g., a donor oligonucleotide) that also includes the capture domain. The splint oligonucleotide facilitates the ligation of the capture domain (e.g., via hybridization to the second ligation handle) to the acceptor oligonucleotide (e.g., ligation handle of the acceptor oligonucleotide).

Ligation Protocol:

Ligation reaction master mix was prepared according to Table 2 below. Equal amounts of a splint oligonucleotide and a capture domain are incubated at room temperature overnight. Alternatively, equal amounts of a splint oligonucleotide and a ligation probe are mixed and warmed up to 98°C for 2 minutes, and then cooled down to 4°C at a rate of 0.1°C/second.

Next, 100 μl probe and ligase mix are added to each well, and incubated at 22°C for 60 minutes. After 60 minutes, 100 μl of volume is removed and discarded. 100 μl of 2X SSC buffer that has been heated to 50°C is added to each well and incubated for 2 minutes. After 2 minutes, 100 μl of the 2X SSC buffer is removed from the well and discard. This process is repeated 2 times for a total of 3 washes in 2X SSC buffer at 50°C.

After the 3 washes in 2X SSC buffer, 100 μl of room temperature 0.2X SSC buffer is added to each well and incubated for 1 minute. 100 μl of 0.2X SSC buffer is then removed, followed by addition of 100 μl of 0.1X SSC buffer.

The slide can be stored at 4°C until ready for fluorescent probe hybridization and imaging.

Table 2. Ligation reaction reagent mix:

| | Stock Concentration | Desired Concentration | Volume (μl) |
|---|---|---|---|
| Desired final volume | | | 100 |
| $H_2O$ | | | 55 |
| ATP 100 mM | 100 mM | 2.5 mM | 2.5 |
| 5X Ligation Buffer | 5X | 1X | 20 |
| Ligase | 1000 | 100 | 10 |
| PolyT + splint oligonucleotide | 250 | 30 | 12 |

Reagents in the Ligation reaction reagent mix table:

| Item | 10x Part Number | Origin | Composition |
|---|---|---|---|
| 5X Ligation Buffer | 210007 | 10x Manufactured (bulk formulation) | 35% Glycerol, 250mM Tris-HCl pH 7.5, 50mM $MgCl_2$ |
| Bulk, ATP 100mM | 210010 | 10x Manufactured (bulk formulation) | 100mM (powder disodium salt dissolved in Nuclease-Free Water). pH adjusted to 6.8-7.2 using 10M NaOH |
| DTT-Free T4 Ligase | 310280 | Enzymatics (Qiagen) | Buffer: 10mM Tris-HCl, 50mM KCl, 0.1mM EDTA, 50% glycerol, pH 7.5 |

### Example 3 Ligation of Capture Domains to Immobilized Oligonucleotides

A portion of the universal splint oligonucleotide (SEQ ID NO: 13) is complementary to the first ligation handle of the acceptor oligonucleotide (SEQ ID NO: 12) on the surface of the array. Another portion of the universal splint oligonucleotide (SEQ ID NO: 13) is complementary to a second ligation handle sequence present in an oligonucleotide that also includes the capture domain. Experiments were performed for determining ligation conditions and it was determined that a ligation reaction containing 100U ligase, 30 uM of donor oligonucleotide and 30 uM of universal splint oligonucleotide was optimal in this instance for ligating the donor oligonucleotide to the acceptor oligonucleotide on an array. Briefly, an array including a plurality of acceptor oligonucleotides is contacted with 30 μM of a universal splint oligonucleotide (SEQ ID NO: 12). The universal splint oligonucleotide is annealed to the first ligation handle present in the acceptor oligonucleotide. Ligase (100 U) and a donor oligonucleotide (30 μM) including a second ligation handle substantially complementary to a portion of the universal splint oligonucleotide (SEQ ID NO: 12) are incubated on the array for 60 minutes. The universal splint oligonucleotide facilitates the ligation of the acceptor oligonucleotide and the donor oligonucleotide including a capture domain for an analyte of interest. Following incubation the array is washed for 35 minutes. The array is functionalized with capture probes including the donor oligonucleotide with a capture domain for an analyte of interest.

Poly(T) capture domains were ligated to acceptor oligonucleotides facilitated by a universal splint oligonucleotide (SEQ ID NO: 13). The array including poly(T) capture domains was contacted with a biological sample and analytes were captured and extended according to methods described herein. **FIG. 6B** shows exemplary qualitative capture of poly(A) analytes (mRNA) with ligated poly(T) capture probes and **FIG. 6A** shows the same biological sample stained with H&E staining. **FIGs. 7A-B** show exemplary quantitative detection of cDNA with the ligated poly(T) capture domains **(FIG. 7A)** and control probes **(FIG. 7B).**

Table 3 below shows comparable transcriptome mapped reads and genome mapped reads with either ligated poly(T) capture probes (30 Ts) or printed poly(T) capture probes (20Ts). **FIG. 8** also shows ligated poly(T) subarray 1 raw counts by printed poly(T) subarray raw counts with a Spearmen correlation coefficient = 0.911 and a Pearson correlation coefficient = 0.999. As such, the data demonstrate that ligating donor oligonucleotides with capture domains to acceptor oligonucleotides via a universal splint oligonucleotide can be used to build a spatial array for capturing target analytes resulting in target capture that is comparable to printed spatial arrays.

Table 3.

| | Transcriptome mapped reads | Genome mapped reads |
|---|---|---|
| Ligated poly(T) (30 Ts) | 81.42% | 90.94% |
| Printed poly(T) (20 Ts) | 83.12% | 92.67% |

### EXAMPLE 4 Generating Penk-Specific Capture Probes with a Universal Splint Oligonucleotide

Arrays including capture probes for the Penk mRNA were prepared according to the ligation protocol described in Example 2. **FIG. 9** shows detection of Penk spatial gene expression in **FIG. 9A** and Penk spatial gene expression with Penk-ligated capture probes in **FIG. 9B. FIG. 10** is a graph showing detection of labeled cDNA with ligated Penk-specific capture probes. The table below shows transcriptome mapped reads and genome mapped reads with ligated Penk-specific capture probes.

| | Transcriptome mapped reads | Genome mapped reads |
|---|---|---|
| Ligated Penk | 13.33% | 53.07% |

**FIGs. 11A-B** shows results from separate sub-arrays where printed poly(T) subarray 1 raw counts by Penk subarray 1 raw counts with a Spearmen correlation coefficient = 0.7597 and a Pearson correlation coefficienct = 0.0563 **(FIG. 11A)** and **FIG. 11B** shows results from separate sub-arrays where printed poly(T) subarray 2 raw counts by Penk subarray 2 raw counts with a Spearmen correlation coefficient = 0.7585 and a Pearson correlation coefficienct = 0.0593.

Table 4 below shows the Penk read percent (%) for different ligated poly(T) capture probes (30Ts), printed poly(T) capture probes (20 Ts), and Penk-specific ligated capture probes. The results show ligated Penk-specific probes captured a higher read percent than either ligated or printed poly(T) capture probes.

Table 4.

| Library type | Penk read % |
|---|---|
| Ligated Poly(T) probes (30 Ts) | 0.045 |
| Printed Poly(T) probes (20 Ts) | 0.043 |
| Ligated Penk-specific probes | 0.062 |

Two separate analyte-specific arrays were generated with Penk-specific capture domains. The first analyte-specific array included a capture domain near the 3' end of the Penk transcript ("3' probe") and the second analyte-specific array included a capture domain near the middle of the Penk transcript ("middle probe") **(FIG. 12).** The read distribution of Penk-specific primers ("3' probe" and "middle probe") correlated to their design. That is, the Penk middle probe read pileups were located near the center of the annotated Penk transcript and the 3' probe read pileups were more distributed along the Penk transcript. Also shown is a positive control (top) dT20 reverse transcriptase primer and a negative control plant gene probe (bottom).

**Example 5 Gene-Specific Capture Probes with a Universal Splint Oligonucleotide**

**FIG. 13** shows successful capture of Doc2g with a ligated Doc2g capture domain. Similarly, **FIG. 14** shows successful capture of Kctd12 with a ligated Kctd12 capture domain. Controls for both Doc2g and Kctd12 capture domains include: no ligation (e.g., no product expected), poly(A), and All Probe-1. In both experiments, gene-specific capture probes for other genes (e.g., Gad1-1, Gad1-2, Nrgn-1, and Nrgn-2) were controls to verify gene-specific capture.

**Example 6 Ligated Arrays Compared to Printed Arrays**

**FIGs. 15** and **16** show results from capture area 1 raw counts by capture area 2 raw counts with ligated capture domains in mouse brain tissue and human heart. The Pearson coefficiency in mouse brain tissue was 0.9992 and 0.9998 in human heart tissue, respectively. The data show that arrays generated with ligated capture domains capture analytes approximately with the same efficiency as arrays with printed capture probes. **FIG. 17** shows successful spatial clustering with both ligated capture probes and printed capture probes in mouse brain tissue.

**Example 7 Ligated Arrays with UMI in Donor Oligonucleotide.**

FIGs. 19A-B and **20A-B** show successful ligation of a labeled donor oligonucleotide (donor oligonucleotide UMI-Cy5, SEQ ID NO: 28) including a unique molecular identifier (UMI) to an acceptor oligonucleotide, (acceptor oligonucleotide V2-1, SEQ ID NO: 29), on the slide. The ligation was facilitated by a splint oligonucleotide (splint oligonucleotide V2-1, SEQ ID NO: 21). The reaction was performed with 42 $\mu$M of Cy5 labeled donor oligonucleotide UMI-Cy5 (SEQ ID NO: 28) and was incubated and ligated to acceptor oligonucleotide V2-1 (SEQ ID NO: 29) for 2 hours in the presence of splint oligonucleotide V2-1 (SEQ ID NO: 21). The slides with the ligated capture probes were washed and imaged for quality control. **FIGs. 20A and 20B** show successful ligation of an oligonucleotide pool (100 $\mu$M) ligated overnight at room temperature. Alternate spots were ligated with fluorescent donor oligonucleotide UMI-Cy3 (SEQ ID NO: 28) as a ligation quality control.

Data show the successful ligation of a donor oligonucleotide containing a UMI and a poly(T) capture domain to an acceptor oligonucleotide to synthesis a spatial array. Overall high spatial uniformity of feature intensity was observed with high signal/noise.

**Example 8 Ligation Efficiency**

**FIGs. 21A-D** show an exemplary ligation scheme where two ligation reactions are performed to generate a first ligation product and a second ligation product. A first plurality of donor oligonucleotides and a plurality of universal splint oligonucleotides are provided to a substrate including a plurality of acceptor oligonucleotides **(FIG. 21A).** The first plurality of donor oligonucleotides includes a first ligation barcode (BC1) and a second adaptor. After the ligation reaction, a population of first ligation products and unligated acceptor oligonucleotides are present on the substrate **(FIG. 21B).**

A second ligation reaction can be performed where a second plurality of donor oligonucleotides includes a second ligation barcode (e.g., BC2) and a second adaptor. The second plurality of donor oligonucleotides can be ligated to the second (e.g., unligated) acceptor oligonucleotides via a second splint oligonucleotide to generate a second ligation product **(FIG. 21C).** Following the second ligation reaction, second strand synthesis can be performed on both the first ligation product and the second ligation product, followed by denaturation, for example with KOH, NaOH, heat, and/or

formamide to release the second strand. The first ligation barcode and the second ligation barcode can be subsequently detected via a qPCR reaction. The first ligation product and the second ligation product can be quantitatively detected (e.g., TaqMan Assay) with probes specific to either the first ligation barcode (BC1) or the second ligation barcode (BC2) as shown in **FIG. 21D.** The percentage of second ligation barcodes detected informs the ligation efficiency of the first ligation reaction (e.g., ligation reaction to form the first ligation product).

Ligation efficiency was tested at both 22°C and 37°C. The results are shown in Table 5 below. The data demonstrate surprising and unexpected ligation efficiencies as measured by qPCR (e.g., TaqMan assay) at both 22°C and 37°C.

Table 5.

| Ligation Temperature | Experiment 1 | Experiment 2 |
| --- | --- | --- |
| 22°C | 94.2% | 99.4% |
| 37°C | N/A | 99.3% |

Ligation efficiency was also tested via a restriction enzyme test. **FIG. 22** shows an exemplary ligation efficiency testing scheme. For example, donor oligonucleotides including either a capture domain including a poly(T) capture domain (SEQ ID NO: 15) or a capture domain including SEQ ID NO: 19 were ligated to acceptor oligonucleotides. After ligation was performed, a blocking oligonucleotide (blocking probe (SEQ ID NO: 34)) was hybridized to a portion of the acceptor oligonucleotide. The double-stranded portion shown in **FIG. 22** includes a restriction enzyme site for SapI. The restriction enzyme generated either a 79 nucleotide product (poly(T) capture domain) or a 69 nucleotide product (capture domain of SEQ ID NO: 19). In the case of unligated acceptor oligonucleotides a 40 nucleotide product was generated.

**FIGs. 23A-B** show the products detected after ligation and incubation with the restriction enzyme **(FIG. 23A).** The blocking probe is also detected (e.g., 20 nucleotide product). **FIG. 23B** is a graph showing the ligation efficiency at 22°C and 37°C for both poly(T) and a capture domain including SEQ ID NO: 19 (e.g., X22_ligated).

**FIG. 24 is** a graph showing the median UMIs at 50k raw reads/spot with arrays generated by ligation reactions at either 22°C, 30°C, or 37°C. Collectively, the data demonstrate that ligation can be performed at a range of temperatures and the resulting arrays comparable to, if not superior to, printed arrays.

**SEQUENCE LISTING**

Synthetic PURAMATRIX polypeptide sequence
SEQ ID NO: 1 RADARADARADARADA

Synthetic EAK16 polypeptide sequence
SEQ ID NO: 2 AEAEAKAKAEAEAKAK

Synthetic KLD12 polypeptide sequence
SEQ ID NO: 3 KLDLKLDLKLDL

18s cDNA Probe 1 (P1)
SEQ ID NO: 4 GAGGAATTCCCAGTAAGT

18s cDNA Probe 2 (P2)
SEQ ID NO: 5 GAGATTGAGCAATAACAG

18s cDNA Probe 3 (P3)
SEQ ID NO: 6 GTAGTTCCGACCATAAAC

18s cDNA Probe 4 (P4)
SEQ ID NO: 7 GGTGACTCTAGATAACCT

Analyte poly(A) tail from a biological sample
SEQ ID NO: 8 AAAAAAAAAAANNNNNNNNNNNNNNNNNNNNNNNN

Acceptor Oligonucleotide Ligation Handle
SEQ ID NO: 9 GCGGCCCG

Splint Oligonucleotide
SEQ ID NO: 10 AAAAAAAAAACGGGCCGC

Capture Domain Poly(T) Sequence
SEQ ID NO: 11 TTTTTTTTTTTTTTTTTTTTTTTTTTT

Acceptor Oligonucleotide Ligation Handle 2
SEQ ID NO: 12
CGCTACG

Universal Splint Oligonucleotide
SEQ ID NO: 13
TCGACTACGTAGCG

Acceptor Oligonucleotide Ligation Handle 3
SEQ ID NO: 14
CGCTACG

T30VN
SEQ ID NO: 15
TAGTCGATTTTTTTTTTTTTTTTTTTTTTTTTTTTTT

N6 Random
SEQ ID NO: 16
TAGTCGANNNNNN

N9 Random
SEQ ID NO: 17
TAGTCGANNNNNNNNN

N12 Random
SEQ ID NO: 18
TAGTCGANNNNNNNNNNNN

Capture Sequence with Ligation handle
SEQ ID NO: 19
TAGTCGATTGCTAGGACCGGCCTTAAGC

Capture Domain of Capture Sequence
SEQ ID NO: 20
TTGCTAGGACCGGCCTTAAGC

Splint Oligonucleotide V2-1
SEQ ID NO: 21
GCTAGTAAGGTGCGCAATCCTC

Splint Oligonucleotide V2-2
SEQ ID NO: 22
GCACCTTGGACCGCTAAAGTTG

Splint Oligonucleotide V2-3
SEQ ID NO: 23
GCTAGTCGAGCCGACAATTGTC

Splint Oligonucleotide V2-4

SEQ ID NO: 24
GCCTGACGAACCGGTTTTGCAA

Donor Oligonucleotide UMI-V2-1
SEQ ID NO: 25
ACCTTACTAGCNNNNNNNNNNNNTTTTTTTTTTTTTTTTTTTTTTTTTTTTTTVN

Donor Oligonucleotide UMI-V2-2
SEQ ID NO: 26
GTCCAAGGTGCNNNNNNNNNNNNTTTTTTTTTTTTTTTTTTTTTTTTTTTTTTVN

Donor Oligonucleotide UMI-V2-3
SEQ ID NO: 27
GCTCGACTAGCNNNNNNNNNNNNTTTTTTTTTTTTTTTTTTTTTTTTTTTTTTVN

Donor Oligonucleotide UMI-V2-4
SEQ ID NO: 28
GTTCGTCAGGCNNNNNNNNNNNNTTTTTTTTTTTTTTTTTTTTTTTTTTTTTTVN

Donor Oligonucleotide UMI-Cy3
SEQ ID NO: 29

ACCTTACTAGCNNNNNNNNNNNNTTTTTTTTTTTTTTTTTTTTTTTTTTTTTTVN/3C
y3

Acceptor Oligonucleotide Ligation Handle V2-1
SEQ ID NO: 30
GAGGATTGCGC

Acceptor Oligonucleotide Ligation Handle V2-2
SEQ ID NO: 31
CAACTTTAGCG

Acceptor Oligonucleotide Ligation Handle V2-3
SEQ ID NO: 32
GACAATTGTCG

Acceptor Oligonucleotide Ligation Handle V2-4
SEQ ID NO: 33
GCAAAACCG

Blocking Probe
SEQ ID NO: 34
AGATCGGAAGAGCGTCGTGTAG

**EMBODIMENTS OF THE INVENTION:**

1. A method of generating a spatial array comprising:

(a)providing a substrate comprising a plurality of acceptor oligonucleotides, wherein a first acceptor oligonucleotide of the plurality of acceptor oligonucleotides comprises a spatial barcode and a first ligation handle, and wherein the 5' end of the first acceptor oligonucleotide is attached to the substrate;
(b)providing a plurality of universal splint oligonucleotides, wherein a universal splint oligonucleotide of the plurality of universal splint oligonucleotides comprises a sequence complementary to the first ligation handle and a sequence complementary to a second ligation handle;

(c)ligating a first plurality of donor oligonucleotides, wherein a first donor oligonucleotide of the first plurality of donor oligonucleotides comprises the second ligation handle and a first capture domain, to the 3' end of a first acceptor oligonucleotide to generate a first capture probe, wherein the universal splint oligonucleotide is hybridized to the first ligation handle and the second ligation handle; and

(d)ligating a second plurality of donor oligonucleotides, wherein a second donor oligonucleotide of the plurality of second donor oligonucleotides comprises the second ligation handle and a second capture domain, to the 3' end of a second acceptor oligonucleotide to generate a second capture probe, wherein the universal splint oligonucleotide is hybridized to the first ligation handle and the second ligation handle, thereby generating a spatial array.

2. The method of embodiment 1, wherein the plurality of acceptor oligonucleotides further comprises a cleavage domain, a unique molecular identifier, a functional domain, or any combination thereof.

3. The method of embodiment 1 or 2, wherein the first capture domain comprises a poly(T) sequence.

4. The method of any one of embodiments 1-3, wherein the second capture domain comprises a gene-specific sequence.

5. The method of embodiment 1 or 2, wherein the second capture domain comprises a sequence at least 80% identical to SEQ ID NO: 20.

6. The method of embodiment for 2, wherein the second capture domain comprises a sequence at least 85% identical to SEQ ID NO: 20.

7. The method of embodiment 1 or 2, wherein the second capture domain comprises a sequence at least 90% identical to SEQ ID NO: 20.

8. The method of embodiment 1 or 2, wherein the second capture domain comprises a sequence at least 95% identical to SEQ ID NO: 20.

9. The method of embodiment 1 or 2, wherein the second capture domain comprises SEQ ID NO: 20.

10.The method of embodiment 1 or 2, wherein the first capture domain comprises a random sequence.

11.The method of embodiment 10, wherein the random sequence is a hexamer.

12.The method of any one of embodiments 1-11, wherein the universal splint oligonucleotide comprises a sequence at least 85% identical to SEQ ID NO: 13.

13.The method of embodiment of any one of embodiments 1-11, wherein the universal splint oligonucleotide comprises a sequence at least 90% identical to SEQ ID NO: 13.

14.The method of any one embodiments 1-11, wherein the universal splint oligonucleotide comprises SEQ ID NO: 13.

15.The method of any one of embodiments 1-14, wherein the first ligation handle comprises a sequence at least 85% complementary to the first 7 nucleotides of SEQ ID NO: 13.

16. The method of any one of embodiments 1-14, wherein the first ligation handle comprises a sequence complementary to the first 7 nucleotides of SEQ ID NO: 13.

17. The method of any one of embodiments 1-14, wherein the second ligation handle comprises a sequence at least 85% complementary to nucleotides 8-14 of SEQ ID NO: 13.

18.The method of any one of embodiments 1-14, wherein the second ligation handle comprises a sequence complementary to nucleotides 8-14 of SEQ ID NO: 13.

19.The method of any one of embodiments 1-18, wherein the universal splint oligonucleotide comprises an inverted base at the 3' end.

20.The method of any one of embodiments 1-19, wherein the donor oligonucleotide comprises one or more phosphorothioate bonds at the 3' end.

21.The method of any one of embodiments 1-20, wherein the method further comprises contacting a biological sample to the spatial array after step (c).

22.The method of embodiment 21, wherein the method further comprises permeabilizing the biological sample to allow an analyte in the biological sample to interact with the first capture probe or the second capture probe.

23.The method of embodiment 21 or 22, wherein the method further comprises migrating the analyte in the biological sample to the first capture probe and the second capture probe.

24.The method of embodiment 23, wherein the migrating comprises passive migration.

25.The method of embodiment 23, wherein the migrating comprises active migration.

26. A method of generating a spatial array comprising:

(a) providing a substrate comprising a plurality of acceptor oligonucleotides, wherein an acceptor oligonucleotide of the plurality of acceptor oligonucleotides comprises a spatial barcode and a first ligation handle, and wherein the 5' end of the acceptor oligonucleotide is attached to the substrate;
(b) providing a plurality of universal splint oligonucleotides, wherein a universal splint oligonucleotide of the plurality of universal splint oligonucleotides comprises a sequence complementary to the first ligation handle and a sequence complementary to a second ligation handle present in a donor oligonucleotide of a plurality of donor oligonucleotides; and
(c) ligating the donor oligonucleotide comprising a capture domain to the 3' end of the acceptor oligonucleotide to generate a capture probe, wherein the universal splint oligonucleotide is hybridized to the first ligation handle and the second ligation handle, thereby generating a spatial array.

27.The method of embodiment 26, wherein the acceptor oligonucleotide further comprises a cleavage domain, a unique molecular identifier, a functional sequence, or any combination thereof.

28.The method of embodiment 26 or 27, wherein the universal splint oligonucleotide comprises a sequence at least 85% identical to SEQ ID NO: 13.

29.The method of embodiment 26 or 27, wherein the universal splint oligonucleotide comprises a sequence at least 90% identical to SEQ ID NO: 13.

30.The method of embodiment 26 or 27, wherein the universal splint oligonucleotide comprises SEQ ID NO: 13.

31. The method of any one of embodiments 26-30, wherein the first ligation handle comprises a sequence at least 85% complementary to the first 7 nucleotides of SEQ ID NO: 13.

32.The method of any one of embodiments 26-30, wherein the first ligation handle comprises a sequence complementary to the first 7 nucleotides of SEQ ID NO: 13.

33.The method of any one of embodiments 26-30, wherein the second ligation handle comprises a sequence at least 85% complementary to nucleotides 8-14 of SEQ ID NO: 13.

34.The method of any one of embodiments 26-30, wherein the second ligation handle comprises a sequence complementary to nucleotides 8-14 of SEQ ID NO: 13.

35. The method of any one of embodiments 26-34, wherein the method further comprises contacting a biological sample to the spatial array after step (c).

36. The method of embodiment 35, wherein the method further comprises permeabilizing the biological sample to allow an analyte in the biological sample to interact with the capture probe.

37. The method of embodiment 36, wherein the method further comprises migrating the analyte in the biological sample to the capture probe.

38. The method of embodiment 36, wherein the migrating comprises passive migration or active migration.

39. A kit comprising:

(a) an array comprising a plurality of acceptor oligonucleotides, wherein an acceptor oligonucleotide of the plurality of acceptor oligonucleotides comprises a spatial barcode and a first ligation handle;
(b)a plurality of universal splint oligonucleotides; and
(c)a ligase.

40. The kit of embodiment 39, wherein the acceptor oligonucleotide comprises SEQ ID NO: 12.

41. The kit of embodiment 39 or 40, wherein a universal splint oligonucleotide of the plurality of universal splint oligonucleotides comprises a sequence at least 90% identical to SEQ ID NO: 13.

42. The kit of embodiment 39 or 40, wherein the splint oligonucleotide comprises SEQ ID NO: 13.

43. The kit of any one of embodiments 39-42, wherein the ligase is T4 ligase.

44. The kit of any one of embodiments 39-43, wherein the kit further comprises a reverse transcriptase.

45. The kit of any one of embodiments 39-44, wherein the kit further comprises a DNA polymerase.

46. The kit of any one of embodiments 39-45, wherein the kit further comprises one or more permeabilization reagents.

47. The kit of any one of embodiments 39-46, wherein the kit further comprises one or more RNase inhibitors.

48. The kit of any one of embodiments 39-47, wherein the kit further comprises instructions for performing any one of the methods of embodiments 1-39.

49. A composition comprising:

(i) an acceptor oligonucleotide comprising a spatial barcode and a first ligation handle, wherein the 5' end of the acceptor oligonucleotide is attached to a substrate;
(ii)a first donor oligonucleotide comprising a second ligation handle and a poly(T) capture domain; and
(iii) a universal splint oligonucleotide comprising a sequence complementary to the first ligation handle and a sequence complementary to the second ligation handle, and wherein the universal splint oligonucleotide is hybridized to the first ligation handle of the acceptor oligonucleotide and the second ligation handle of the donor oligonucleotide.

50. A composition comprising:

(i) a first acceptor oligonucleotide comprising a spatial barcode and a first ligation handle, wherein the 5' end of the acceptor oligonucleotide is attached to a substrate;
(ii) a first donor oligonucleotide comprising a second ligation handle and a poly(T) capture domain;
(iii) a second donor oligonucleotide comprising the second ligation handle and a sequence comprising SEQ ID NO: 20;
(iv) a first universal splint oligonucleotide comprising a sequence complementary to the first ligation handle and a sequence complementary to the second ligation handle, and wherein the universal splint oligonucleotide is hybridized to the first ligation handle of the first acceptor oligonucleotide and the second ligation handle of the first donor oligonucleotide; and
(v) a second universal splint oligonucleotide comprising a sequence complementary to the first ligation handle and a sequence complementary to the second ligation handle, and wherein the universal splint oligonucleotide is hybridized to the first ligation handle of the second acceptor oligonucleotide and the second ligation handle of the second donor oligonucleotide.

51. A composition comprising:

i) at least one of a plurality of first acceptor oligonucleotides comprising a spatial barcode ligated to at least one of a plurality of first donor oligonucleotides comprising a first capture domain, wherein the plurality of first acceptor oligonucleotides is attached to a substrate,
ii)at least one of the plurality of first acceptor oligonucleotides comprising a spatial barcode and a first ligation handle, and

at least one of a plurality of second donor oligonucleotides comprising a second ligation handle and a second capture domain, and a universal splint oligonucleotide, wherein the universal splint oligonucleotide is hybridized to a first ligation handle of at least one of a plurality of first acceptor oligonucleotides and the second ligation handle of at least one of the plurality of second donor oligonucleotides.

## Claims

**1.** A composition comprising:
a substrate comprising:

(a) a plurality of acceptor oligonucleotides comprising a spatial barcode and a first ligation handle, wherein at least one of the plurality of acceptor oligonucleotides is ligated to at least one of a plurality of first donor oligonucleotides, wherein the plurality of first donor oligonucleotides comprise a second ligation handle and a first capture domain;
(b) at least one of the plurality of acceptor oligonucleotides is hybridized to a universal splint oligonucleotide of a plurality of splint oligonucleotides; and
(c) at least one of a plurality of second donor oligonucleotides is hybridized to the universal splint oligonucleotide, wherein the plurality of second donor oligonucleotides comprises the second ligation handle and a second capture domain.

**2.** The composition of claim 1, wherein the first capture domain comprises a poly(T) sequence.

**3.** The composition of claim 1 or 2, wherein the second capture domain comprises a gene-specific sequence.

**4.** The composition of claim 1, wherein the second capture domain comprises a sequence at least 80% identical to SEQ ID NO: 20, comprises a sequence at least 90% identical to SEQ ID NO: 20, or comprises SEQ ID NO: 20.

**5.** The composition of claim 1, wherein the first capture domain comprises a random sequence, and optionally, wherein the random sequence is a hexamer.

**6.** The composition of any one of claims 1-5, wherein the universal splint oligonucleotide comprises a sequence at least 85% identical to SEQ ID NO: 13, comprises a sequence at least 90% identical to SEQ ID NO: 13, or comprises SEQ ID NO: 13.

**7.** The composition of any one of claims 1-6, wherein the universal splint oligonucleotide comprises an inverted base at the 3' end.

**8.** The composition of any one of claims 1-7, wherein the plurality of first donor oligonucleotides comprises one or more phosphorothioate bonds at the 3' end.

**9.** The composition of any one of claims 1-8, wherein the plurality of second donor oligonucleotides comprises one or more phosphorothioate bonds at the 3' end.

**10.** The composition of any one of claims 1-9, wherein the plurality of acceptor oligonucleotides further comprises a cleavage domain, a unique molecular identifier, a functional domain, or any combination thereof.

**11.** The composition of any one of claims 1-10, wherein the plurality of first donor oligonucleotides further comprises a first ligation barcode.

**12.** The composition of any one of claims 1-11, wherein the plurality of second donor oligonucleotides further comprises a second ligation barcode.

**13.** The composition of any one of claims 1-12, further comprising a biological sample.

**14.** The composition of any one of claims 1-13, wherein the 5' end of the plurality of acceptor oligonucleotides is attached to the substrate.

**15.** The composition of any one of claims 1-14, wherein the universal splint oligonucleotide is hybridized to the first ligation handle of the at least one acceptor oligonucleotide of (b) and the second ligation handle of the at least one second donor oligonucleotide.

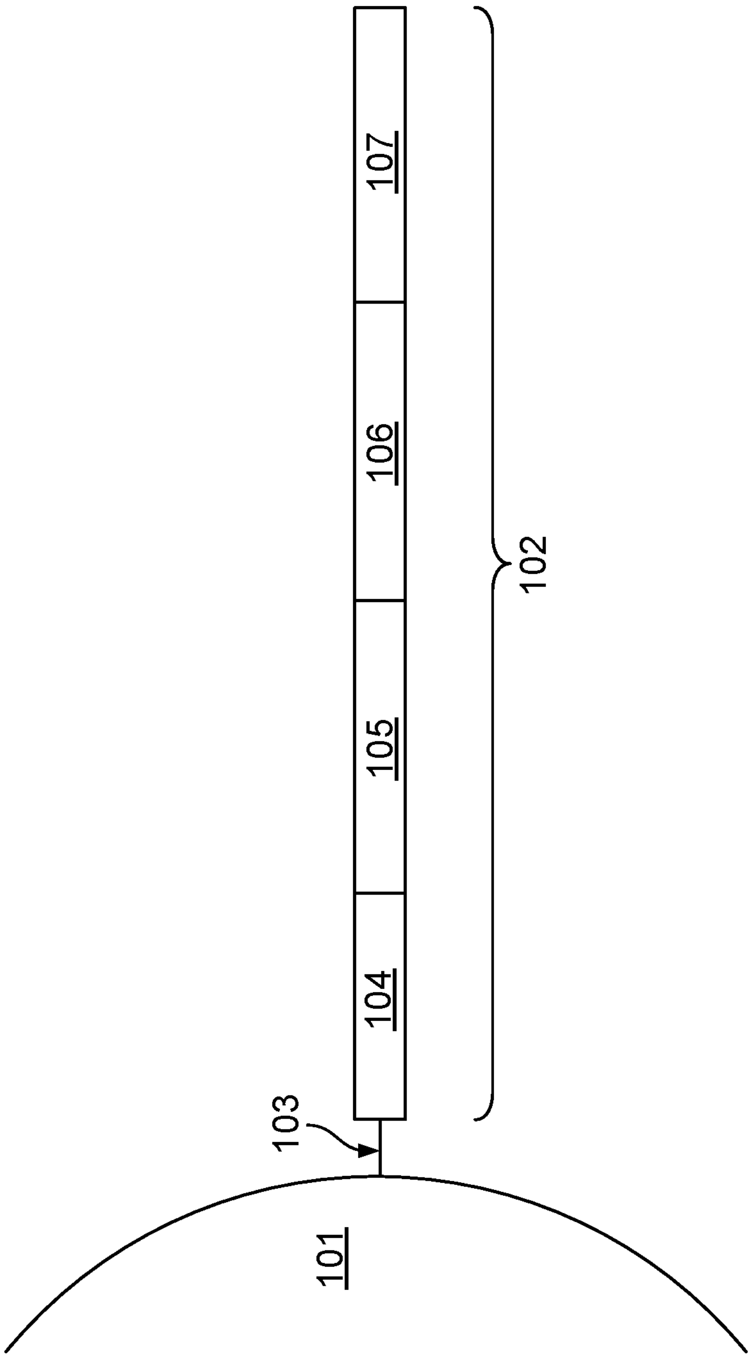


FIG. 1

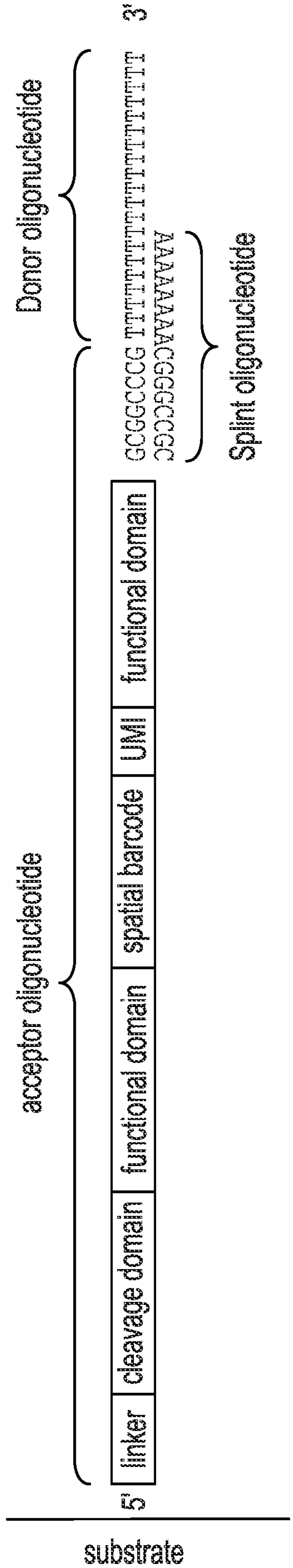
Donor oligonucleotide
acceptor oligonucleotide
3'
GCGGCCCG TTTTTTTTTTTTTTTTTTTTTTTTTTTTTT
CGCCGGGCAAAAAAAA
Splint oligonucleotide
linker
cleavage domain
functional domain
spatial barcode
UMI
functional domain
5'
substrate

FIG. 2

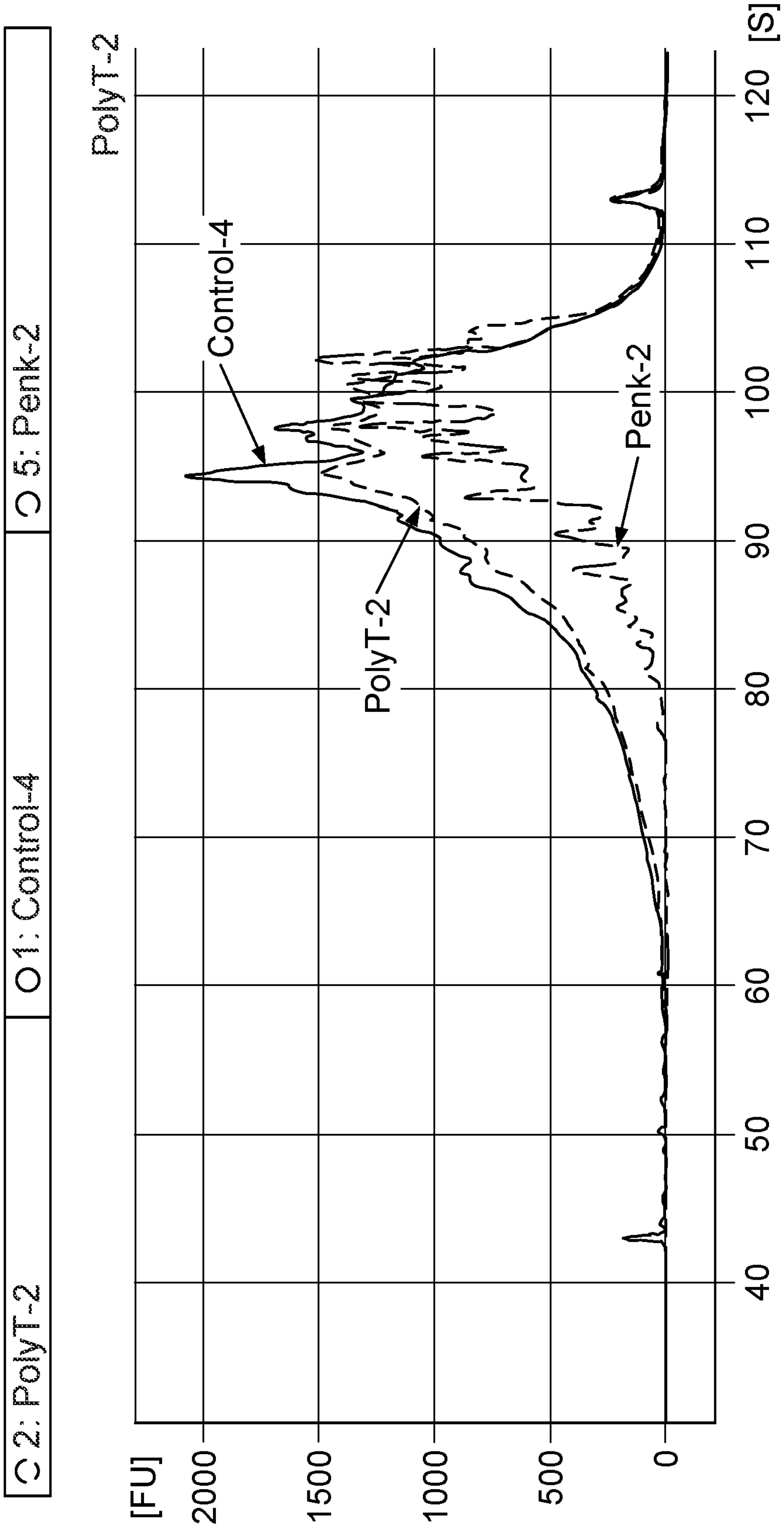
2: PolyT-2
1: Control-4
5: Penk-2
PolyT-2
[FU]
2000
1500
1000
500
0
40
50
60
70
80
90
100
110
120
[S]
Control-4
PolyT-2
Penk-2

FIG. 3

| | |
|---|---|
| 9 Minutes | 9 Minutes |
| 6 Minutes | 6 Minutes |
| 3 Minutes | 3 Minutes |

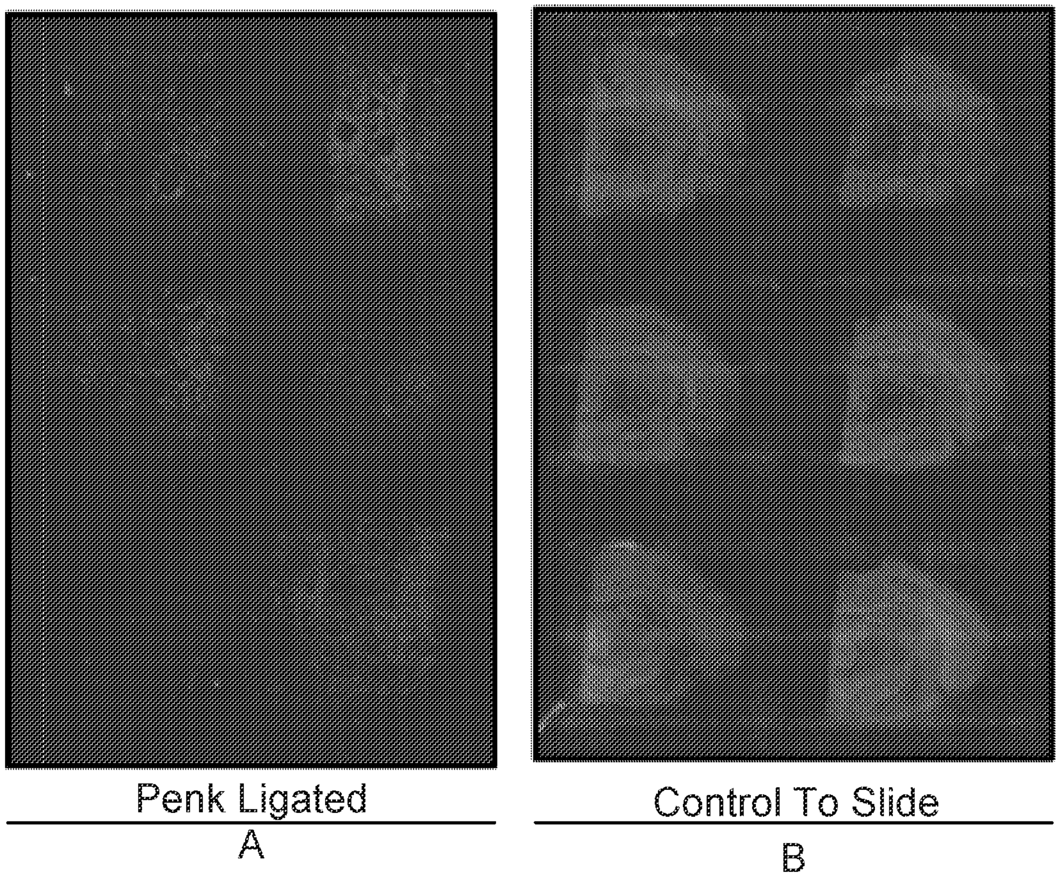


FIG. 4

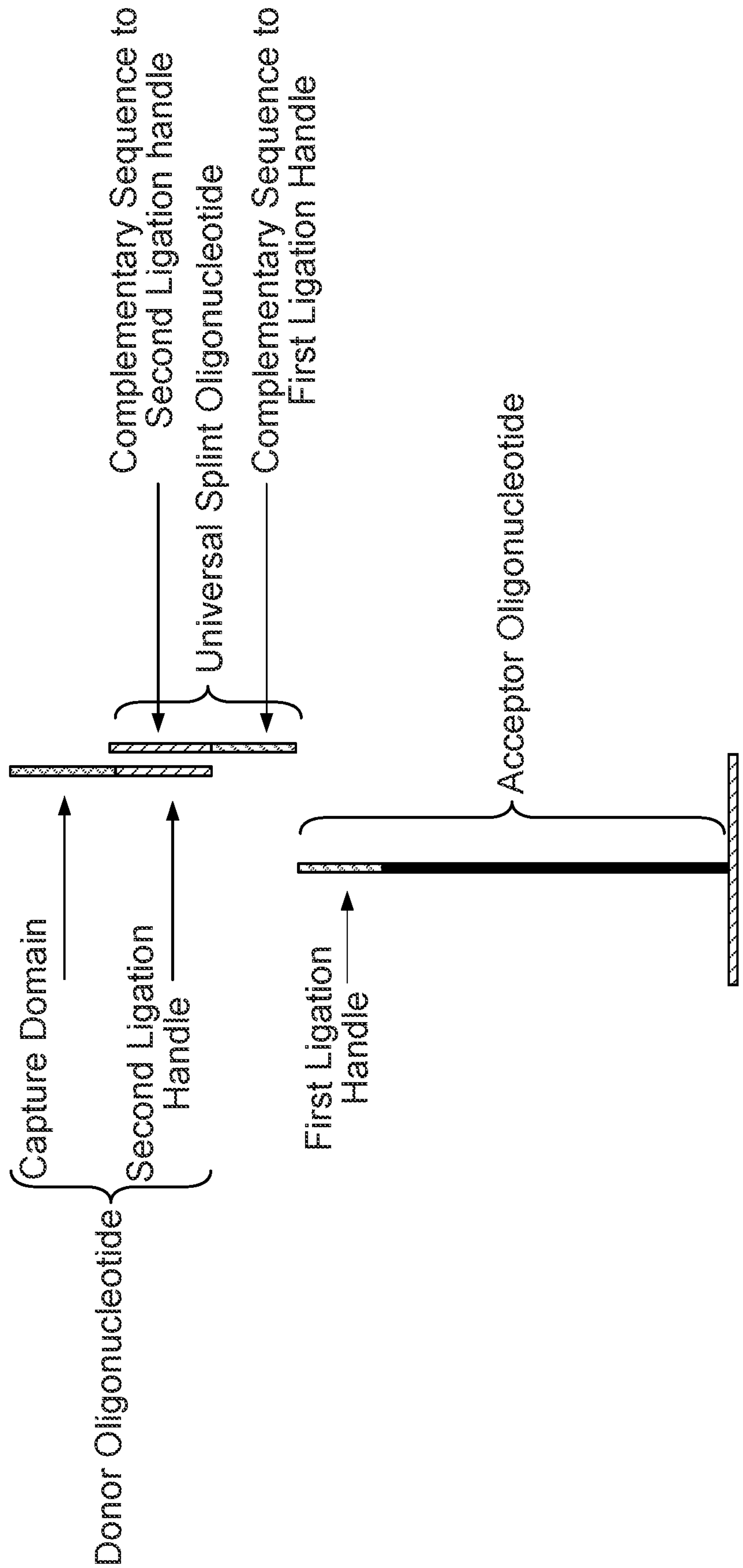
Complementary Sequence to Second Ligation handle
Universal Splint Oligonucleotide
Complementary Sequence to First Ligation Handle
Acceptor Oligonucleotide
Capture Domain
Second Ligation Handle
Donor Oligonucleotide
First Ligation Handle

FIG. 5

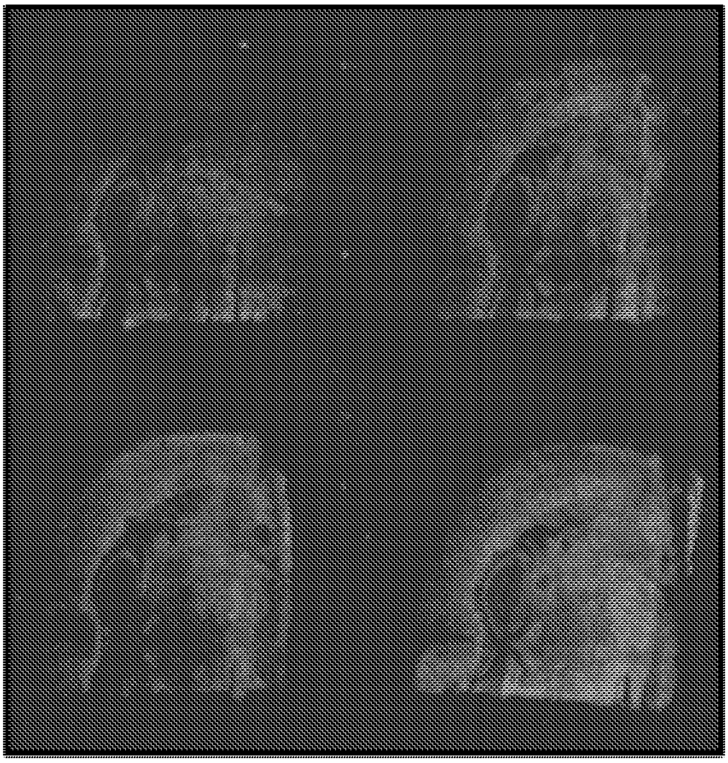

FIG. 6B

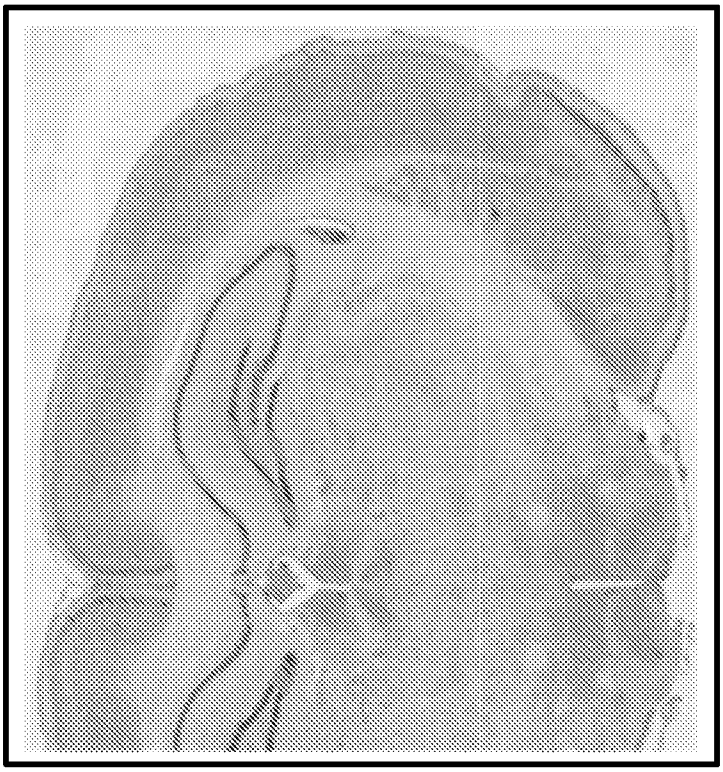

FIG. 6A

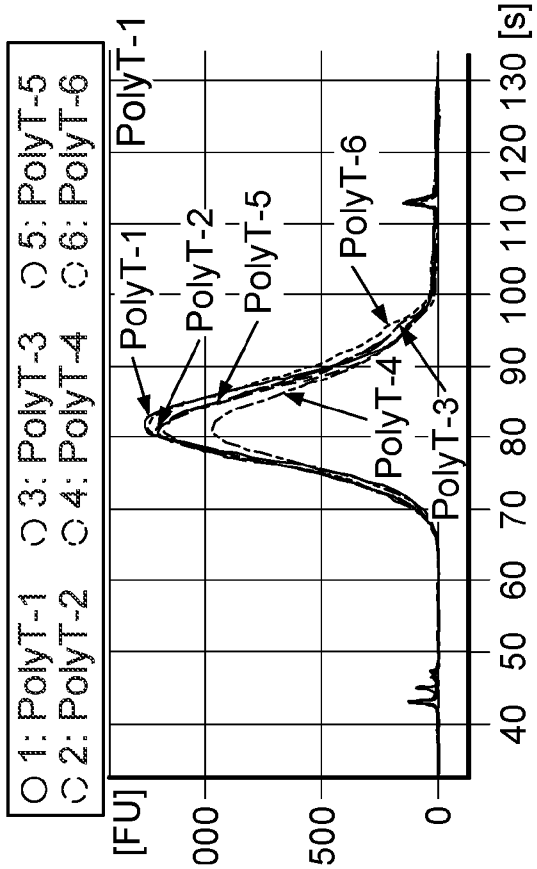
1: PolyT-1
3: PolyT-3
5: PolyT-5
2: PolyT-2
4: PolyT-4
6: PolyT-6
[FU]
1000
500
0
40
50
60
70
80
90
100
110
120
130
[s]
PolyT-1
PolyT-1
PolyT-2
PolyT-5
PolyT-6
PolyT-4
PolyT-3

FIG. 7A

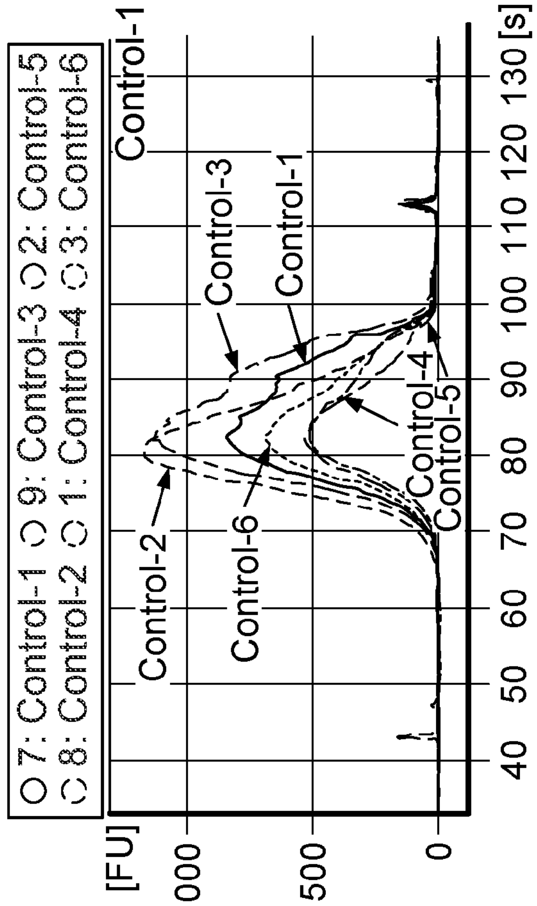
7: Control-1
9: Control-3
2: Control-5
8: Control-2
1: Control-4
3: Control-6
[FU]
1000
500
0
40
50
60
70
80
90
100
110
120
130
[s]
Control-1
Control-2
Control-6
Control-3
Control-1
Control-4
Control-5

FIG. 7B

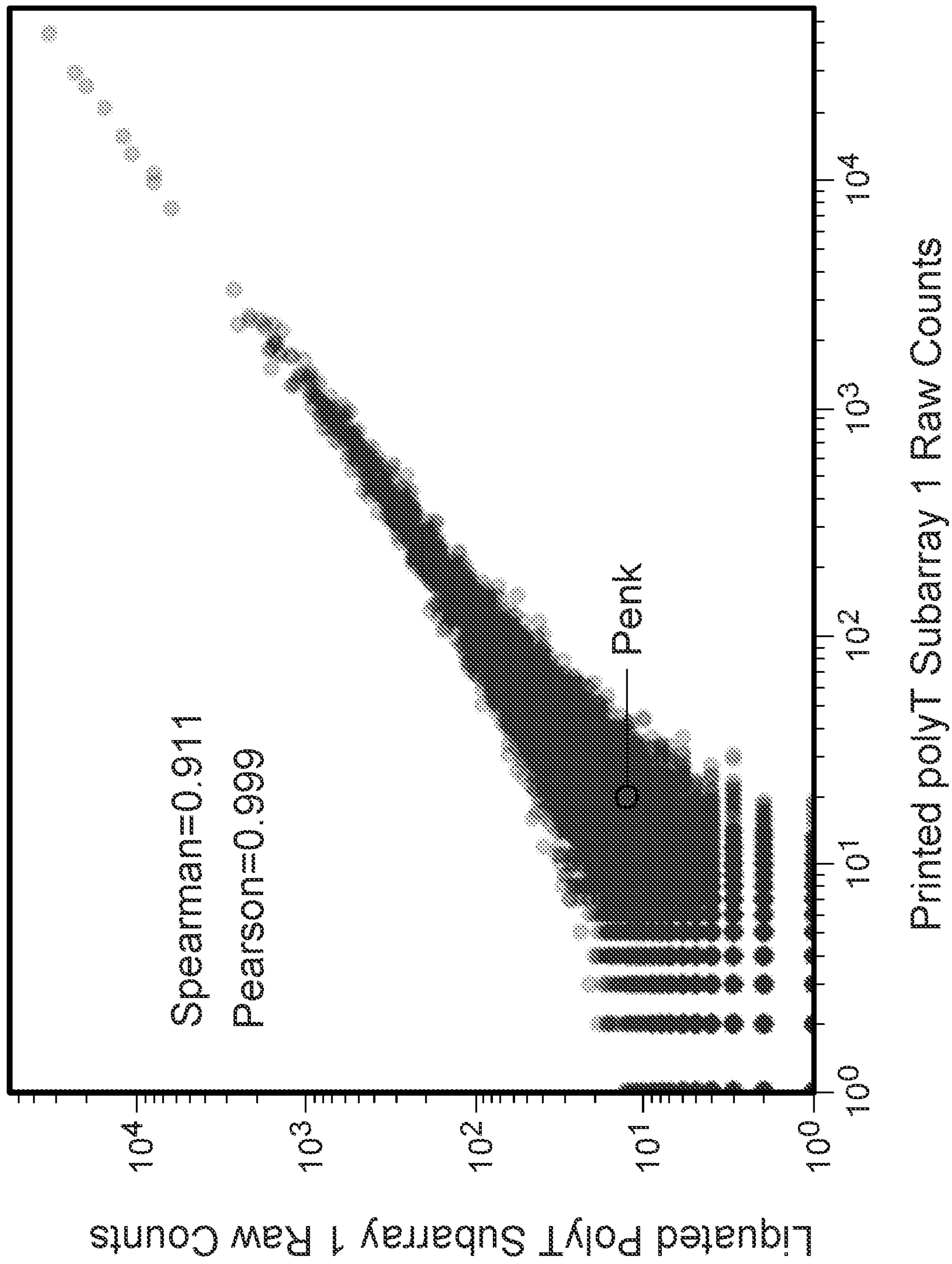
Spearman=0.911
Pearson=0.999
Penk
Printed polyT Subarray 1 Raw Counts
Liquated PolyT Subarray 1 Raw Counts
$10^0$
$10^1$
$10^2$
$10^3$
$10^4$

FIG. 8

Penk
Spatial Gene Expression

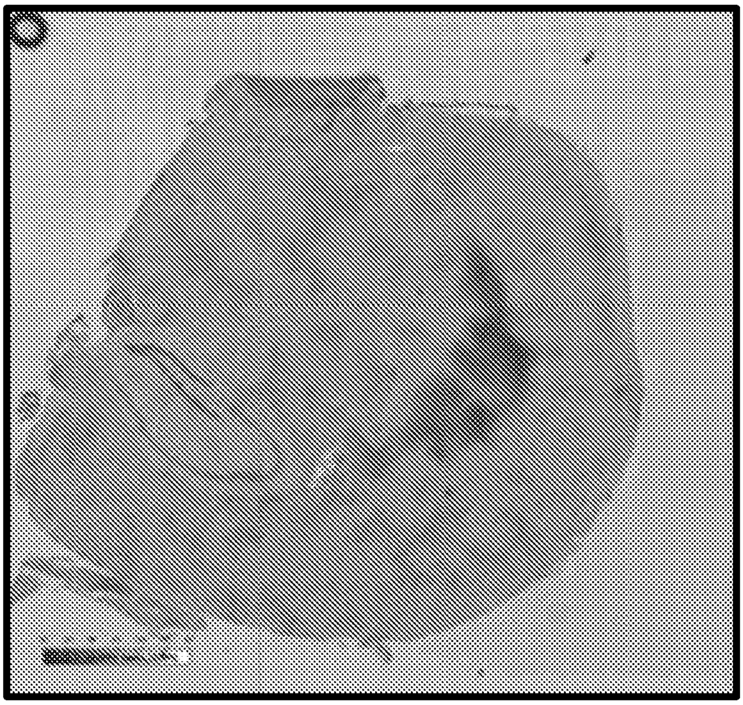

FIG. 9A

Penk- ligated
to Experiment

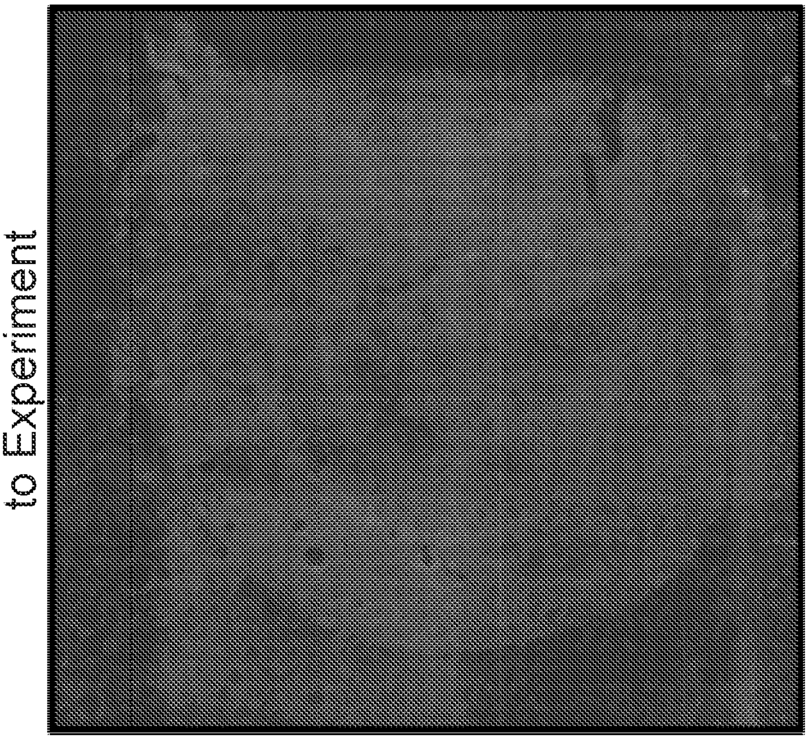

FIG. 9B

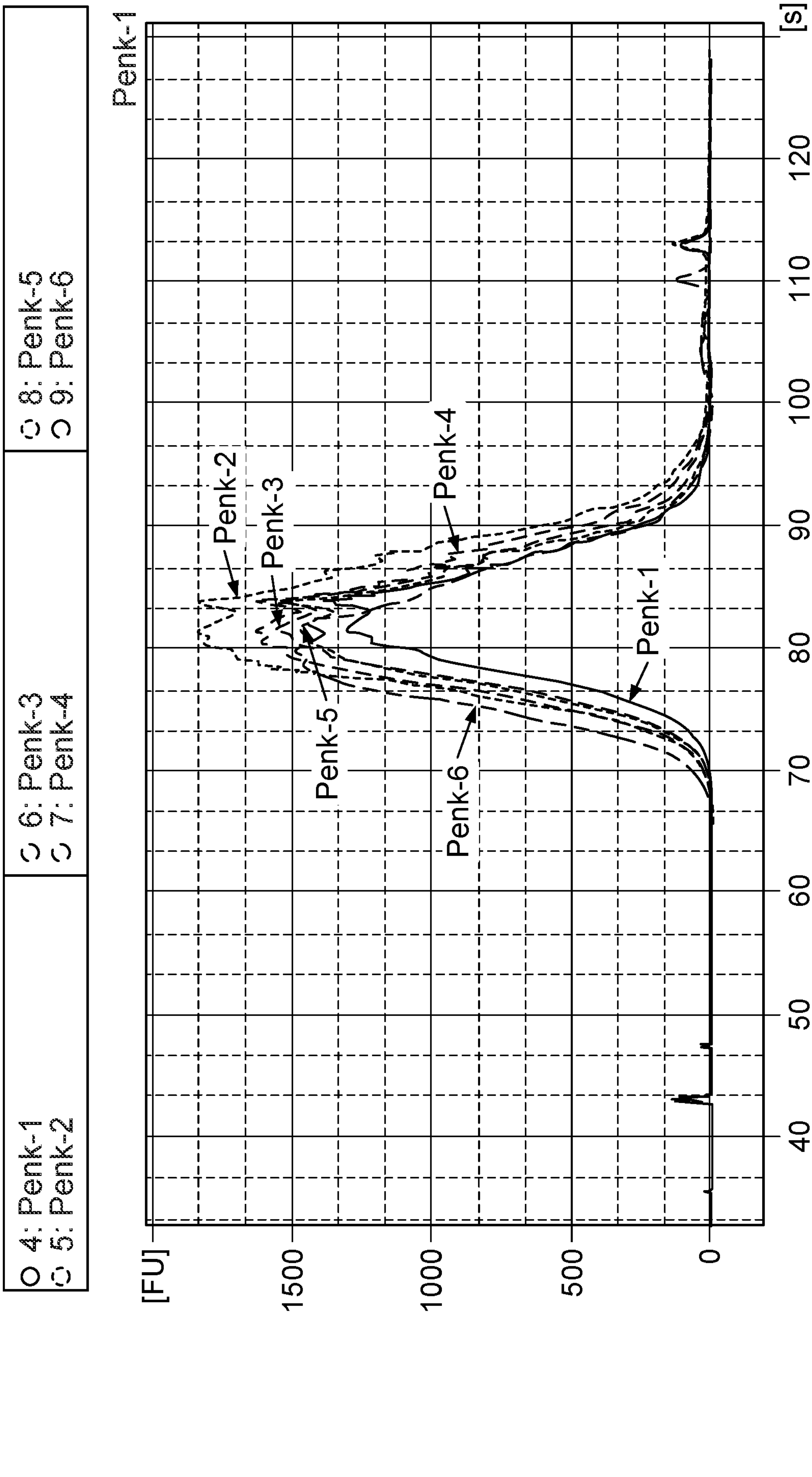
4: Penk-1
5: Penk-2
6: Penk-3
7: Penk-4
8: Penk-5
9: Penk-6
Penk-1
[FU]
1500
1000
500
0
40
50
60
70
80
90
100
110
120
[s]
Penk-2
Penk-3
Penk-4
Penk-5
Penk-6
Penk-1

FIG. 10

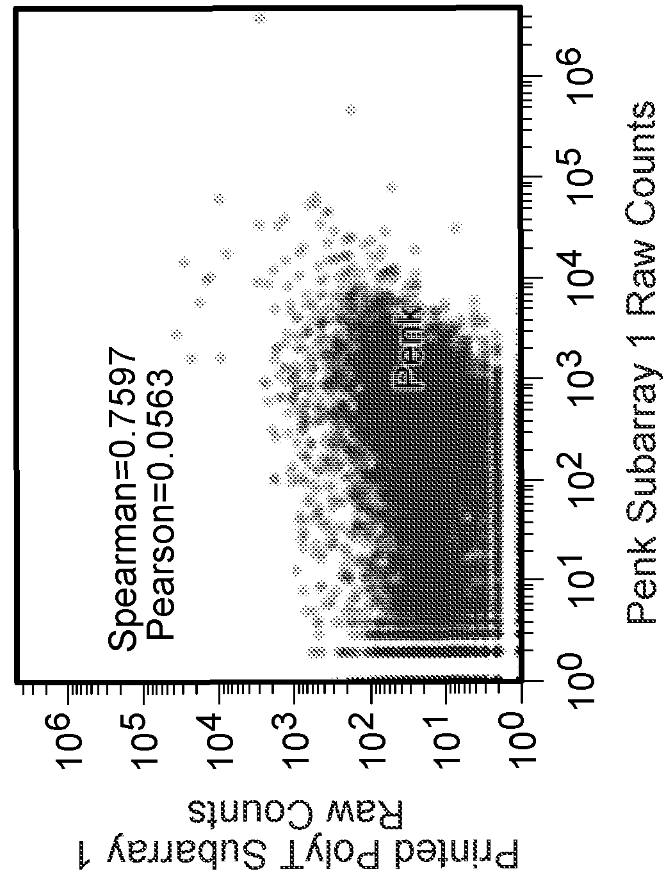


FIG. 11A

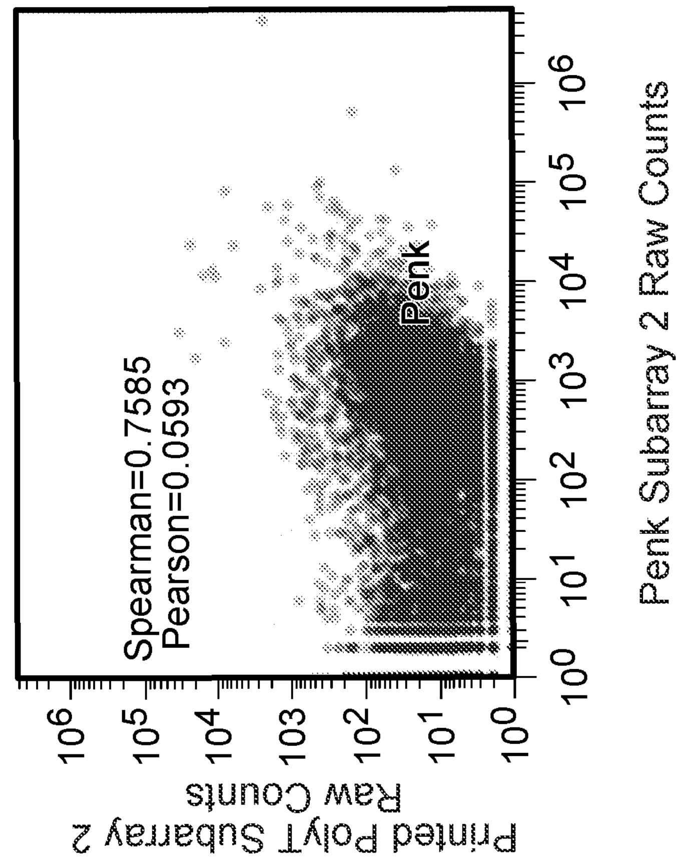


FIG. 11B

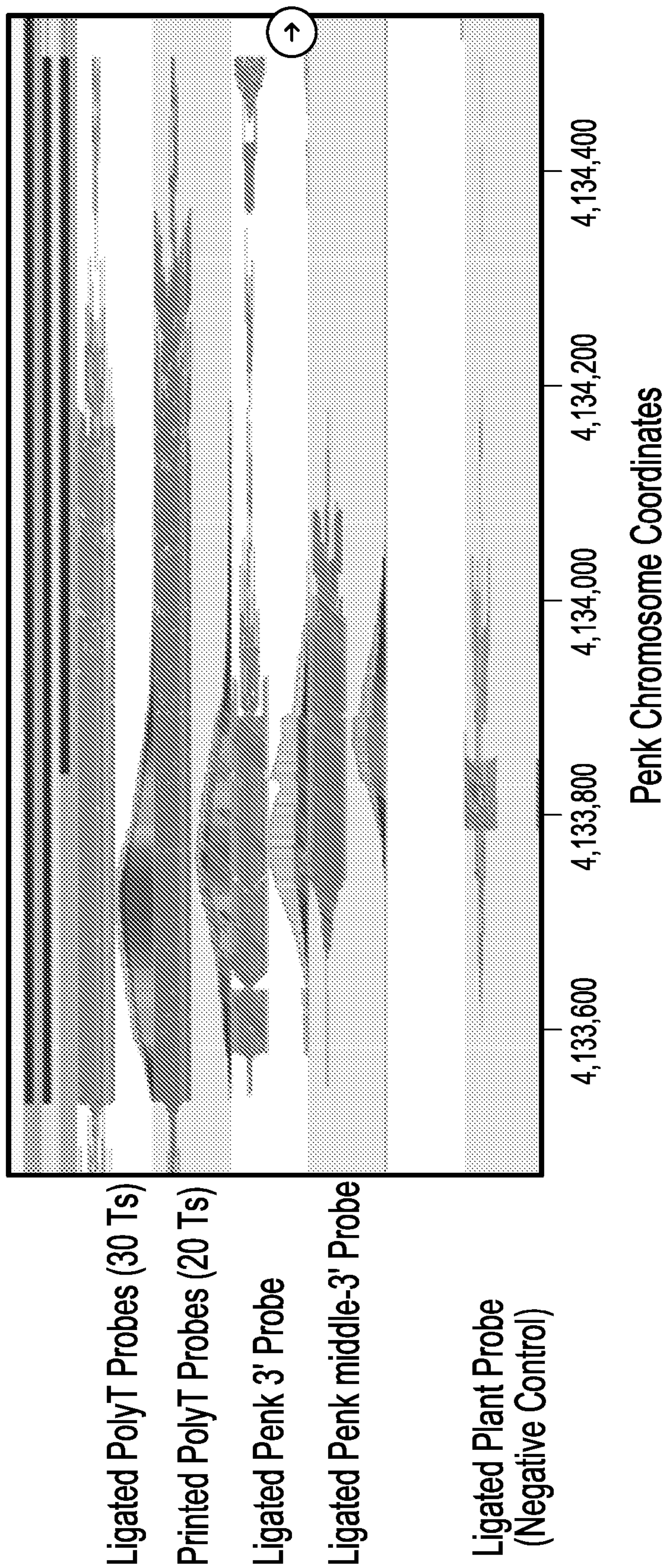
Ligated PolyT Probes (30 Ts)
Printed PolyT Probes (20 Ts)
Ligated Penk 3' Probe
Ligated Penk middle-3' Probe
Ligated Plant Probe
(Negative Control)
4,133,600
4,133,800
4,134,000
4,134,200
4,134,400
Penk Chromosome Coordinates

FIG. 12

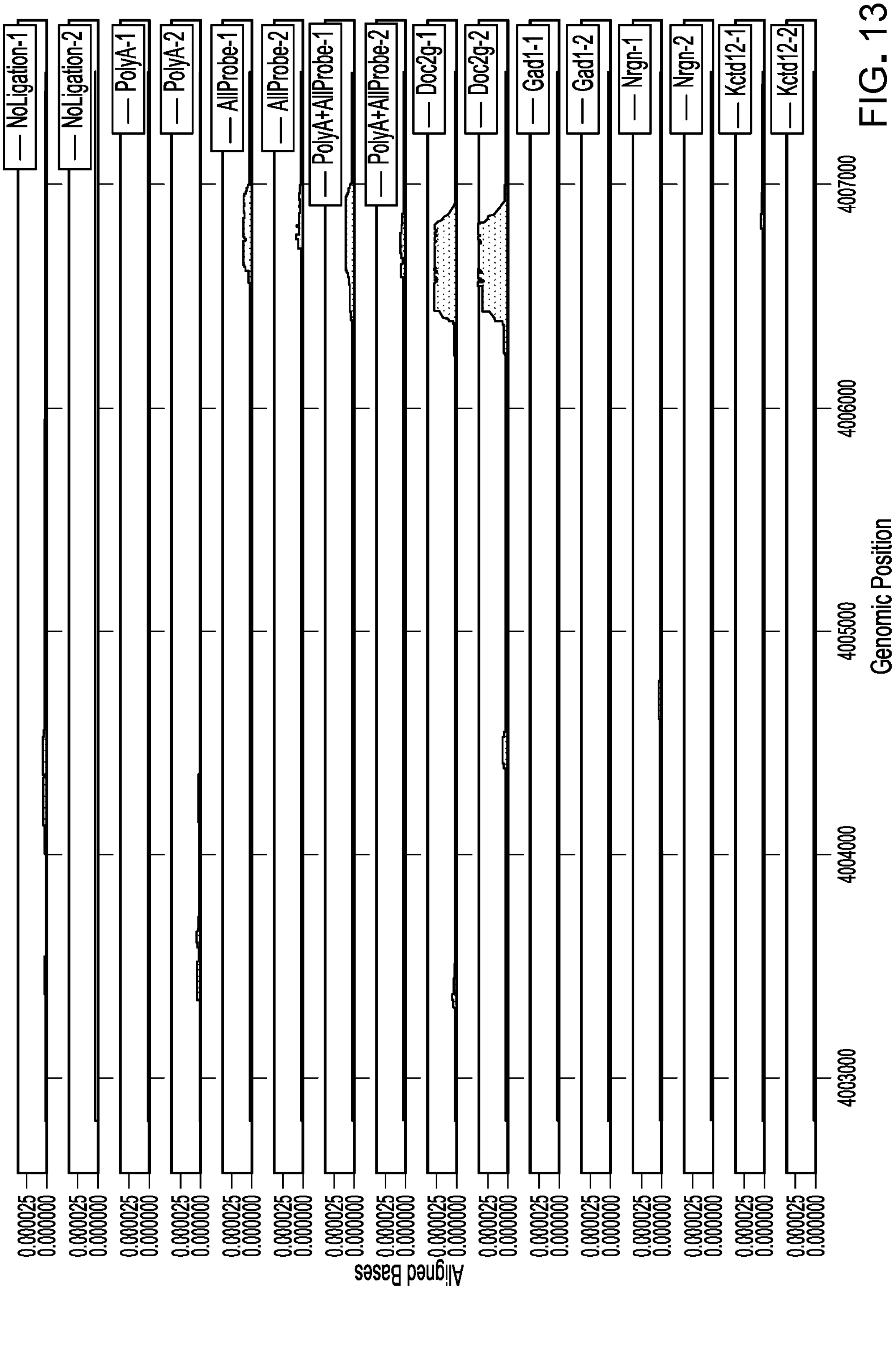
NoLigation-1
NoLigation-2
PolyA-1
PolyA-2
AllProbe-1
AllProbe-2
PolyA+AllProbe-1
PolyA+AllProbe-2
Doc2g-1
Doc2g-2
Gad1-1
Gad1-2
Nrgn-1
Nrgn-2
Kctd12-1
Kctd12-2
0.000025
0.000000
Aligned Bases
4003000
4004000
4005000
4006000
4007000
Genomic Position

FIG. 13

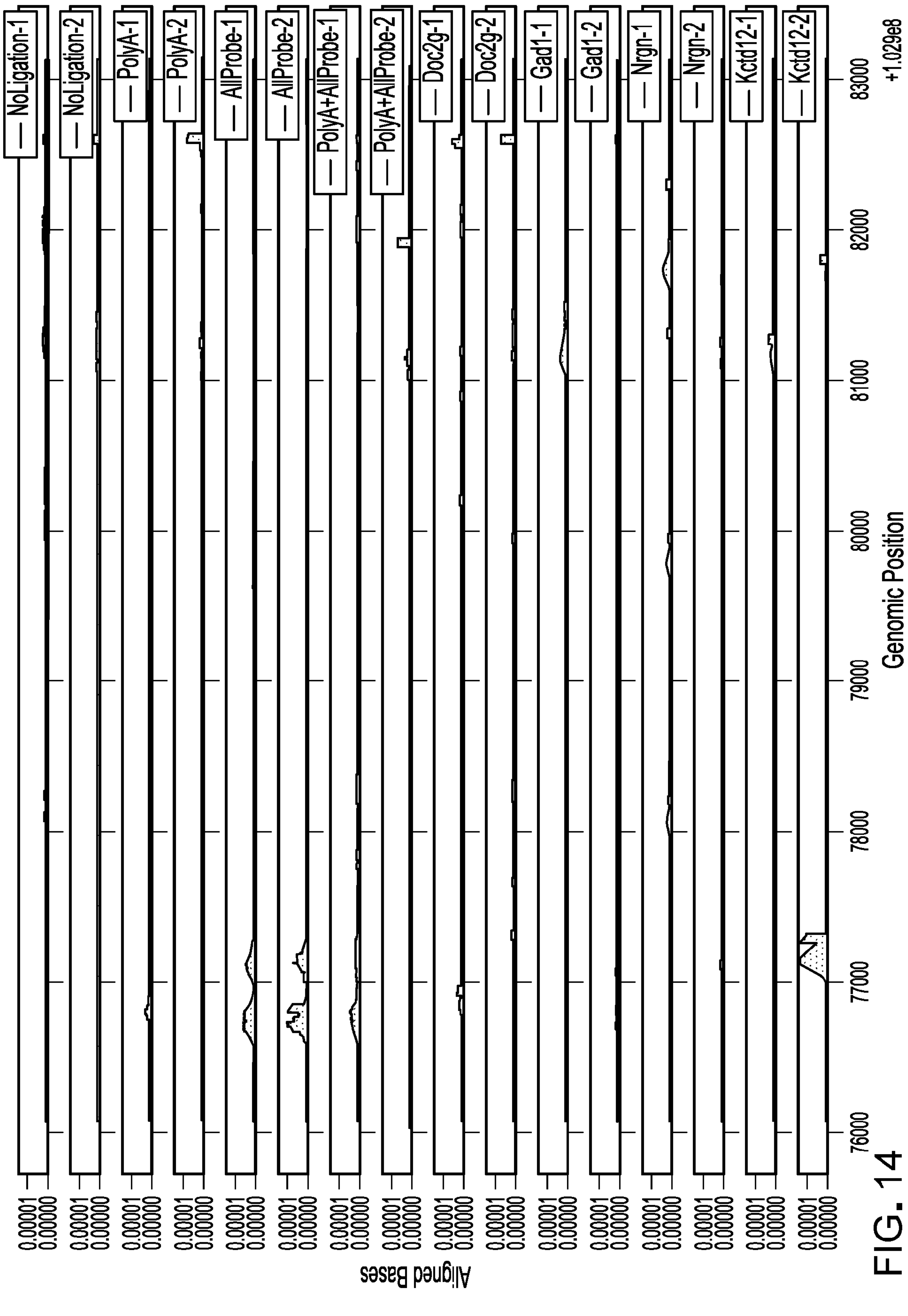
NoLigation-1
NoLigation-2
PolyA-1
PolyA-2
AllProbe-1
AllProbe-2
PolyA+AllProbe-1
PolyA+AllProbe-2
Doc2g-1
Doc2g-2
Gad1-1
Gad1-2
Nrgn-1
Nrgn-2
Kctd12-1
Kctd12-2
0.00001
0.00000
Aligned Bases
76000
77000
78000
79000
80000
81000
82000
83000
+1.029e8
Genomic Position

FIG. 14

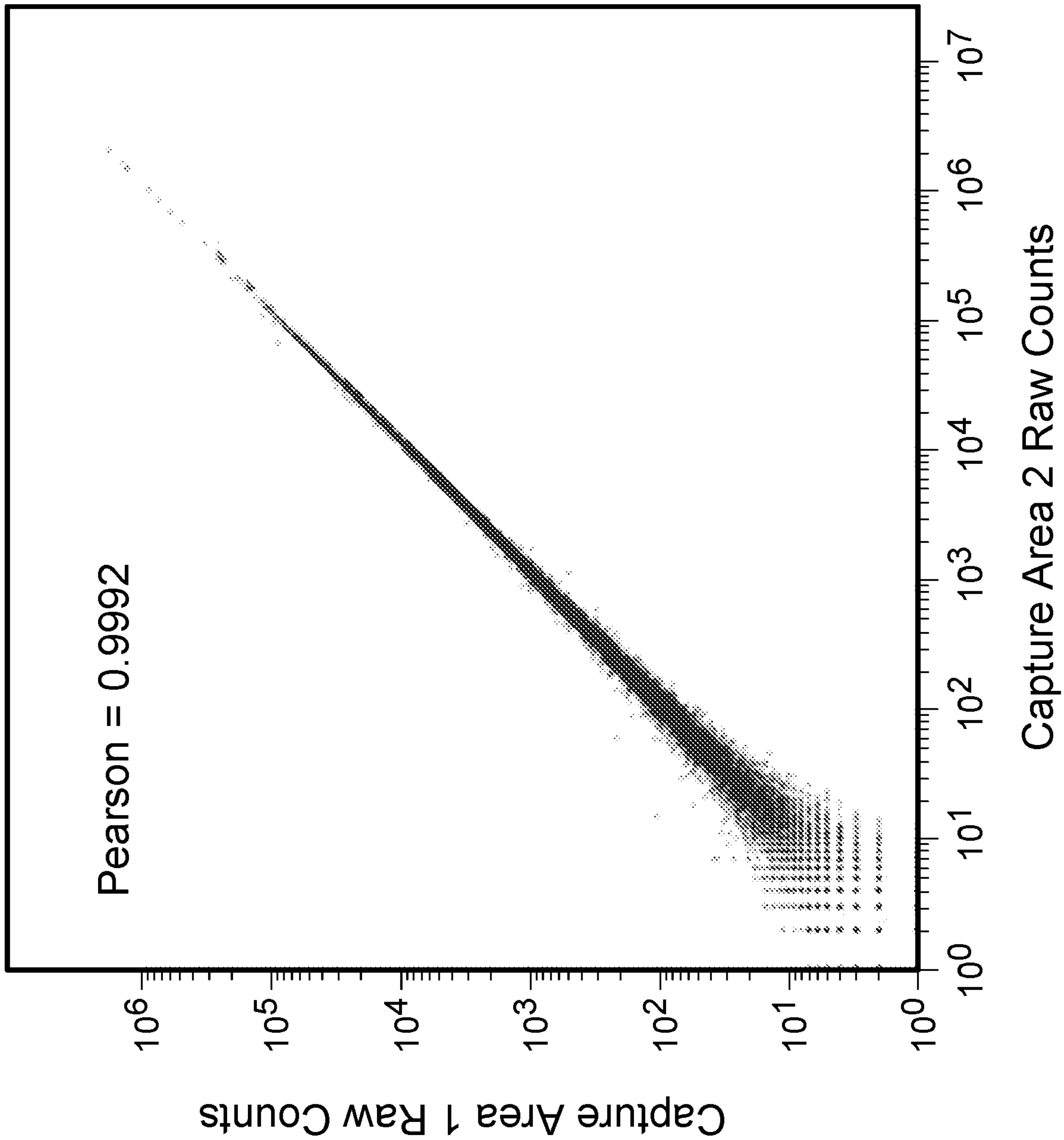
Pearson = 0.9992
Capture Area 1 Raw Counts
Capture Area 2 Raw Counts
$10^0$
$10^1$
$10^2$
$10^3$
$10^4$
$10^5$
$10^6$
$10^7$

FIG. 15

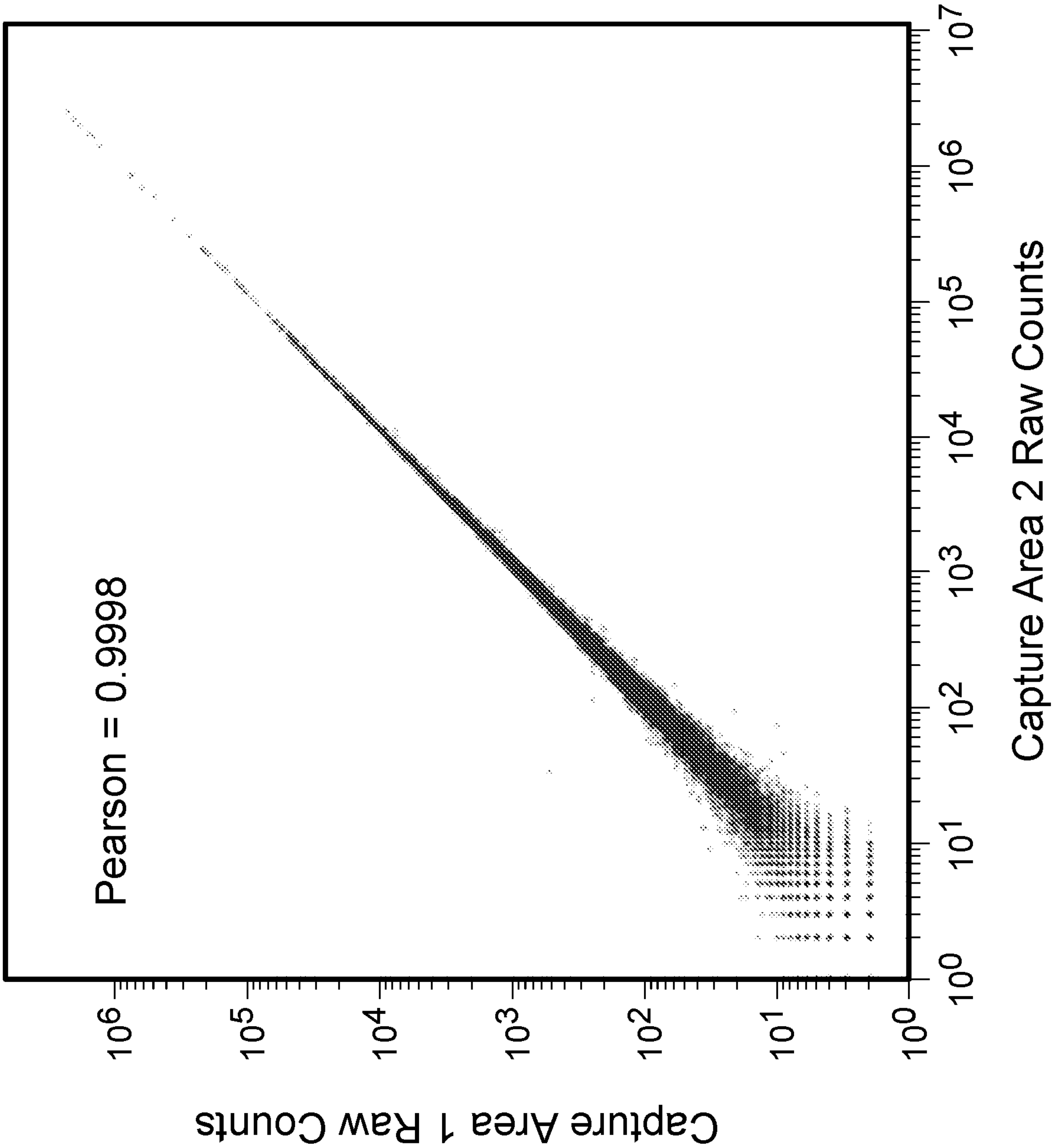
Pearson = 0.9998
Capture Area 1 Raw Counts
Capture Area 2 Raw Counts
$10^0$
$10^1$
$10^2$
$10^3$
$10^4$
$10^5$
$10^6$
$10^7$

FIG. 16

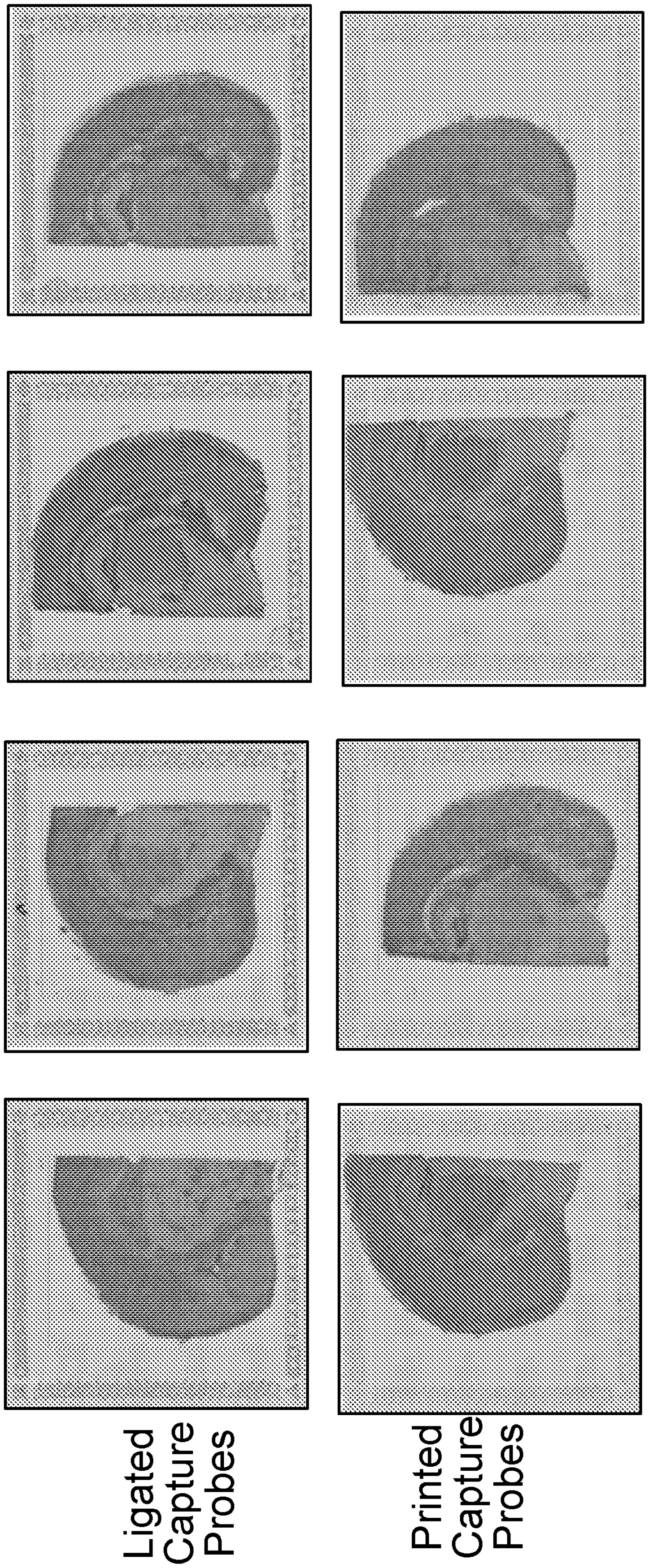


FIG. 17

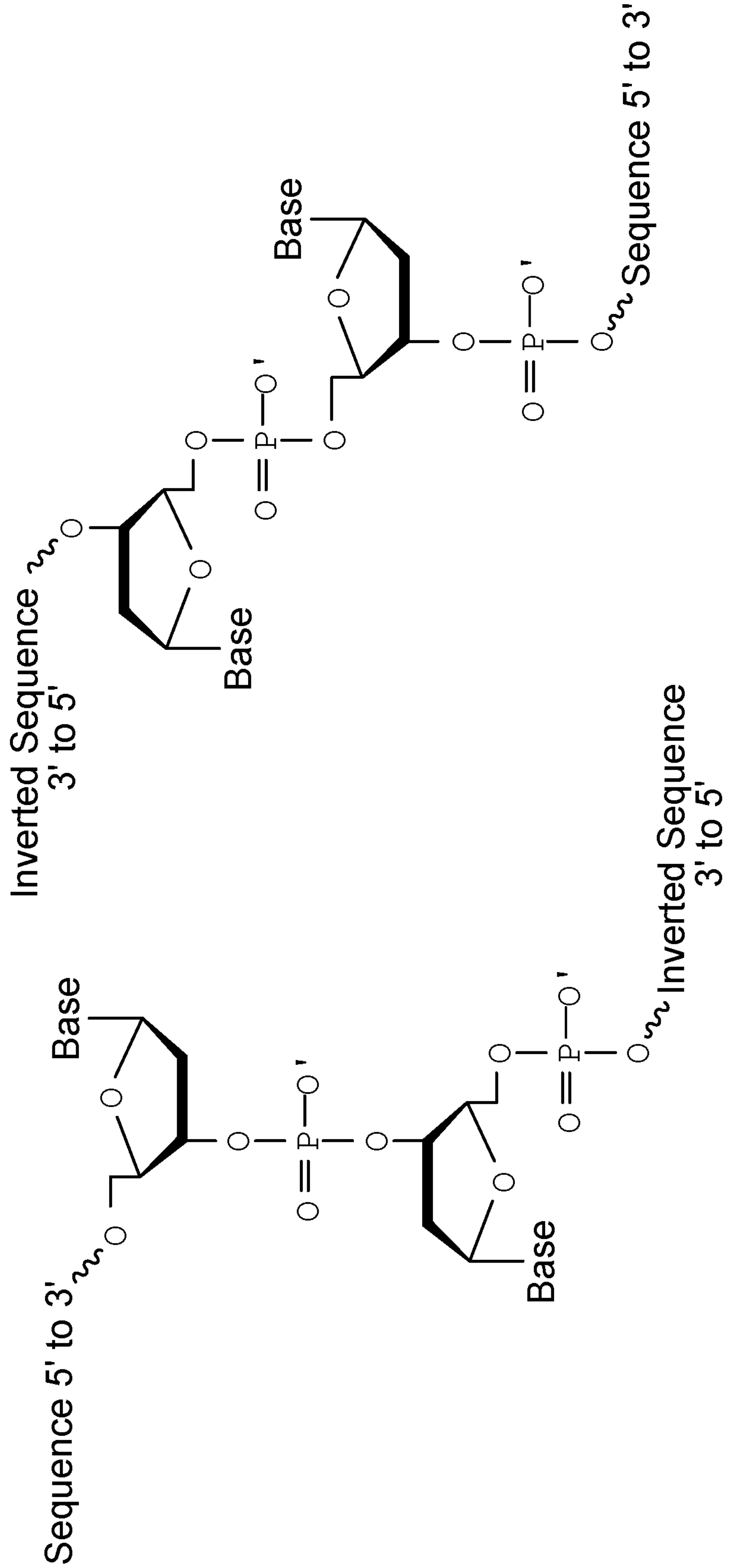

FIG. 18A

FIG. 18B

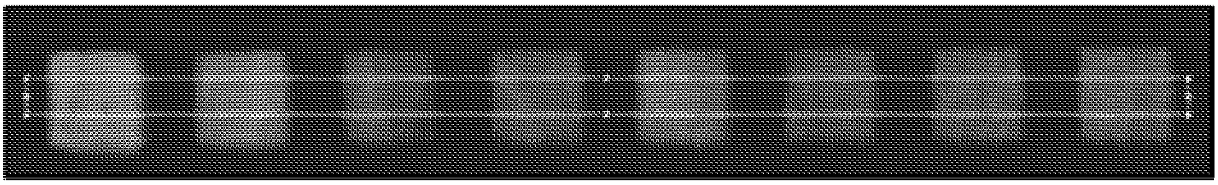

FIG. 19A

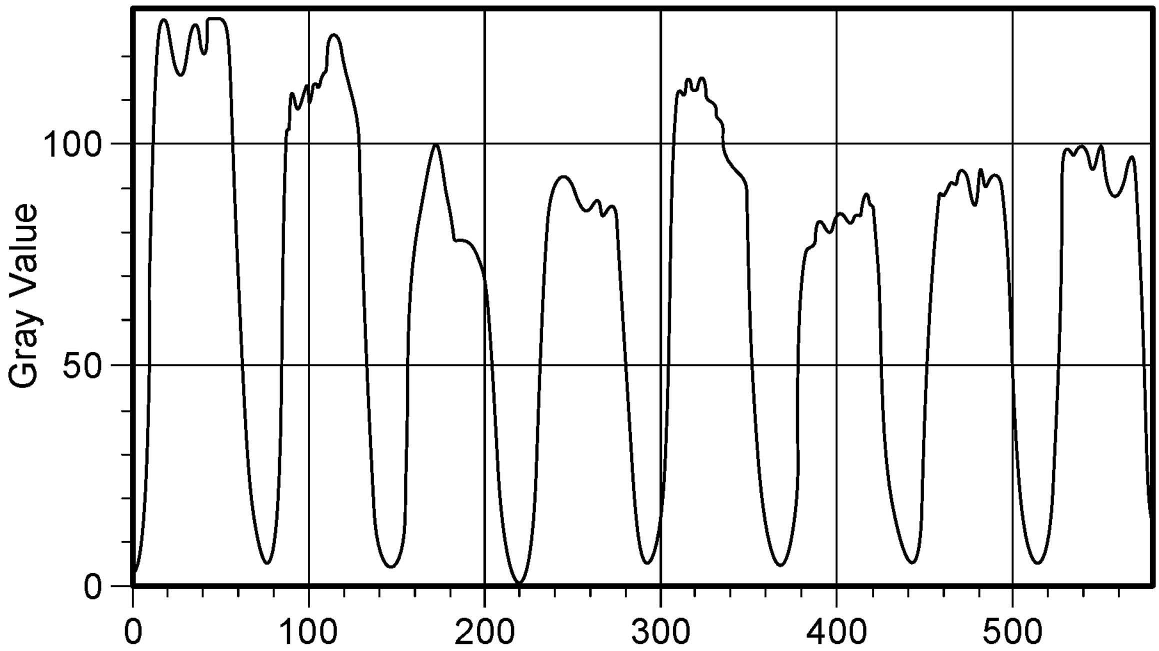


FIG. 19B

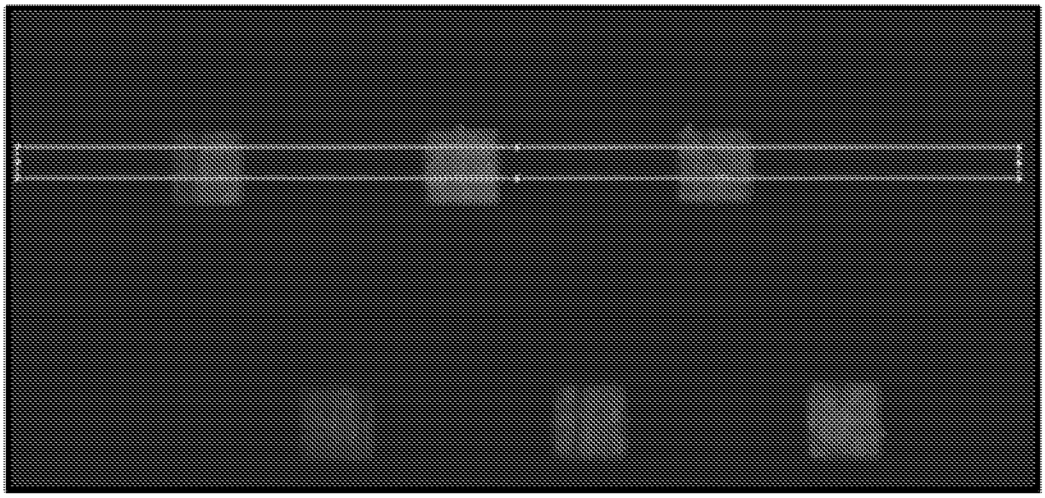

FIG. 20A

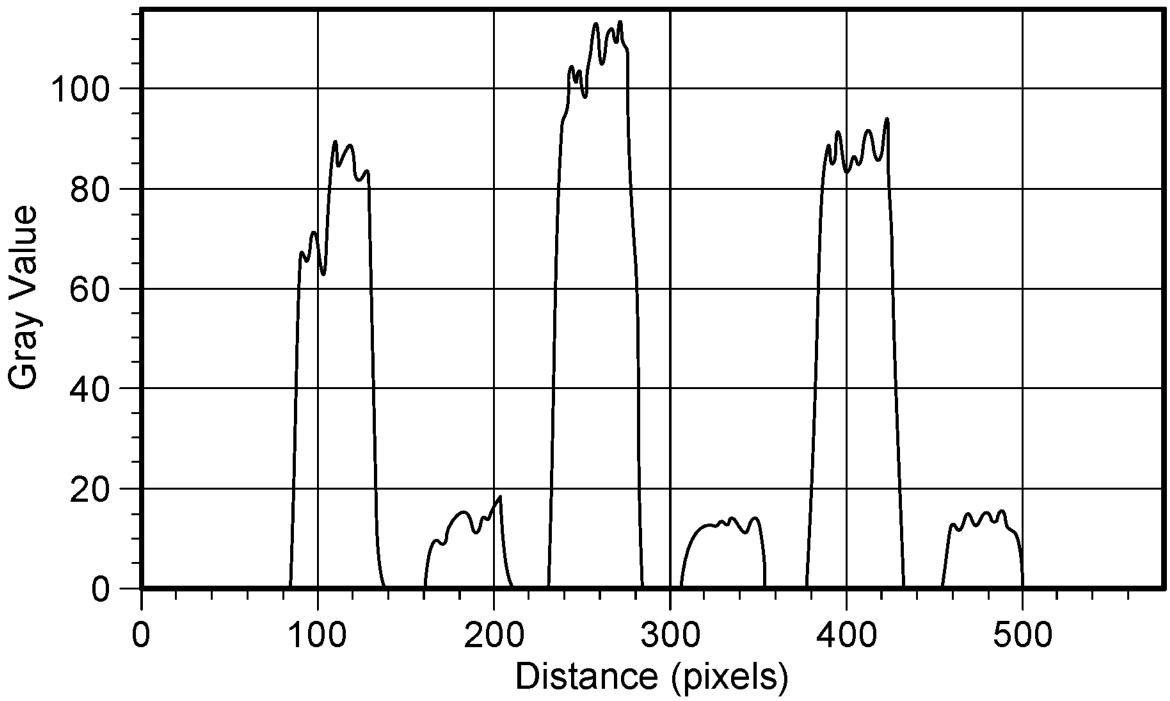


FIG. 20B

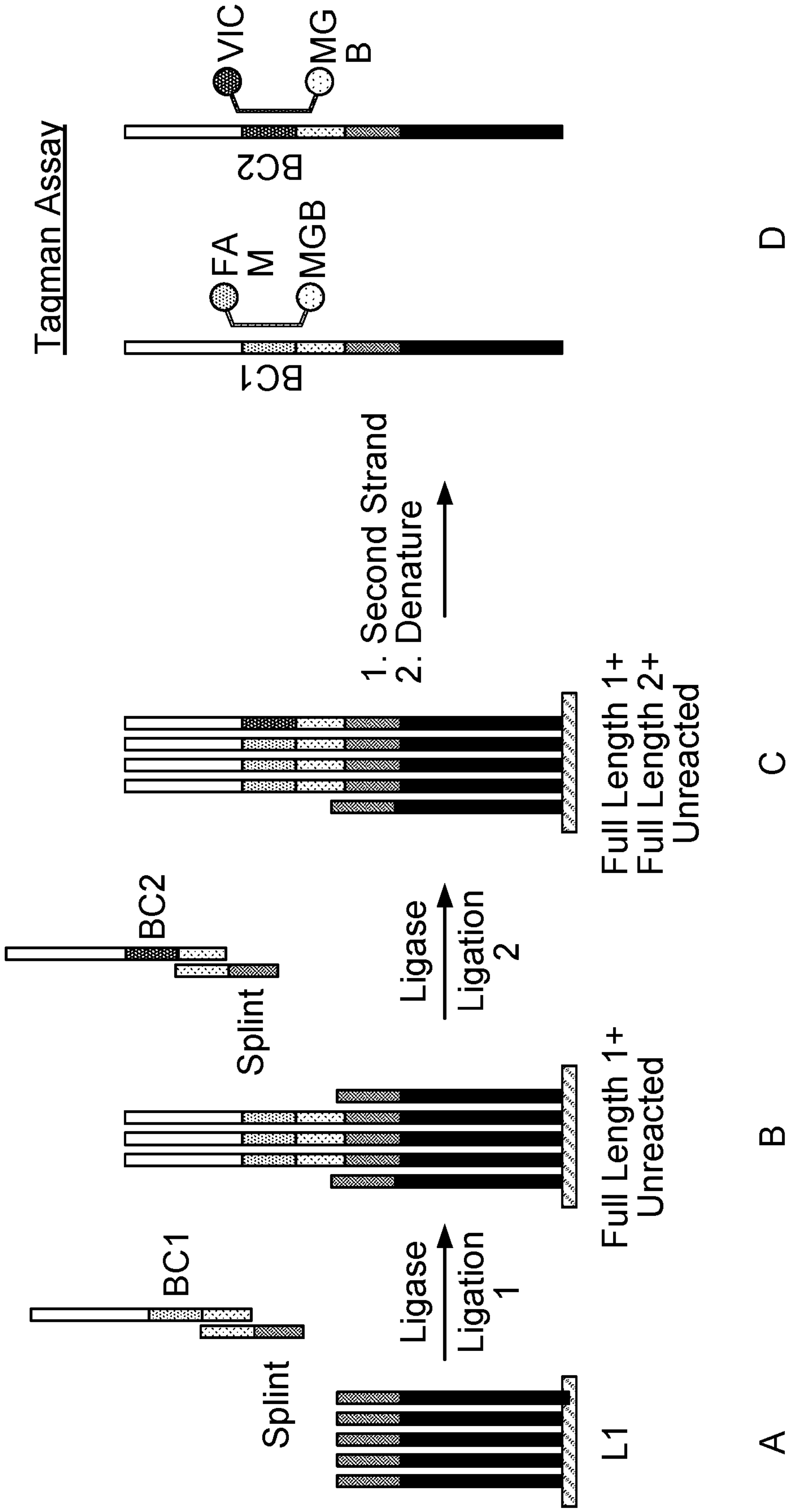
Tagman Assay
BC1
FA
M
MGB
BC2
VIC
MG
B
L1
Splint
BC1
Ligase
Ligation
1
Full Length 1+
Unreacted
Splint
BC2
Ligase
Ligation
2
Full Length 1+
Full Length 2+
Unreacted
1. Second Strand
2. Denature
A
B
C
D

FIG. 21

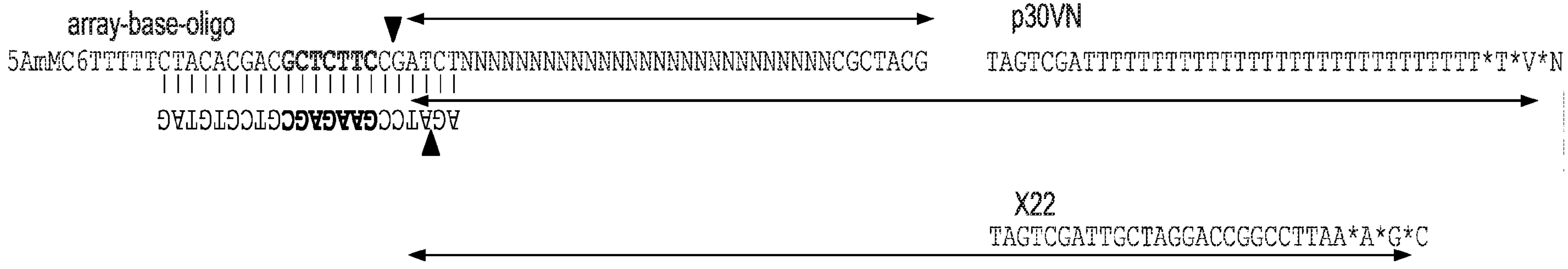
array-base-oligo
5AmMC6TTTTTCTACACGACGCTCTTCCGATCTNNNNNNNNNNNNNNNNNNNNNNNNNNNCGCTACG
AGATCCGAAGAGCGTCGTGTAG
p30VN
TAGTCGATTTTTTTTTTTTTTTTTTTTTTTTTTTTT*T*V*N
X22
TAGTCGATTGCTAGGACCGGCCTTAA*A*G*C

FIG. 22

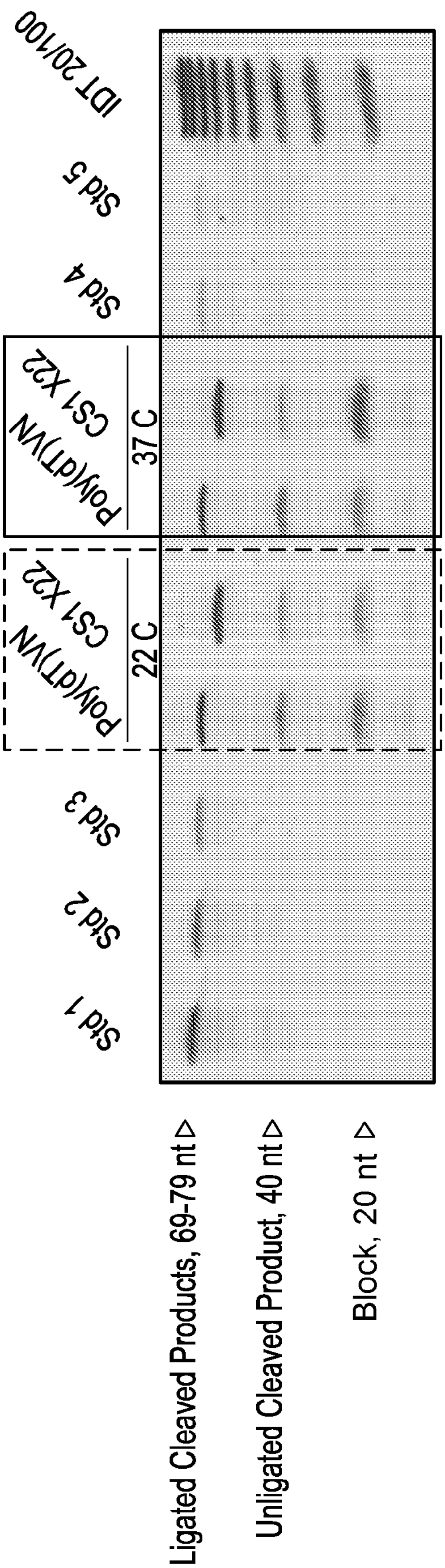


FIG. 23A

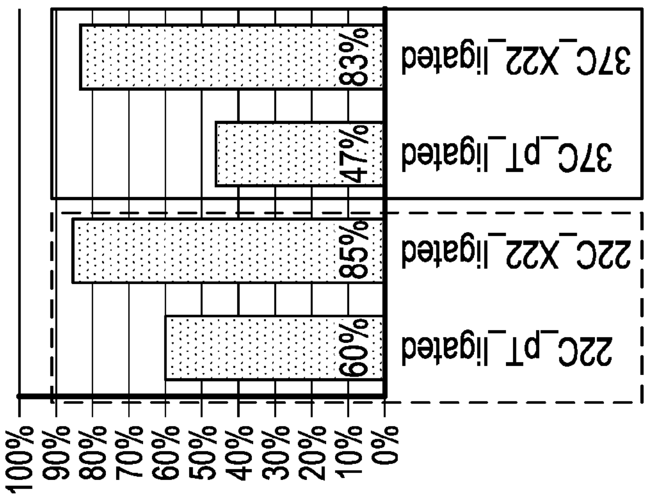


FIG. 23B

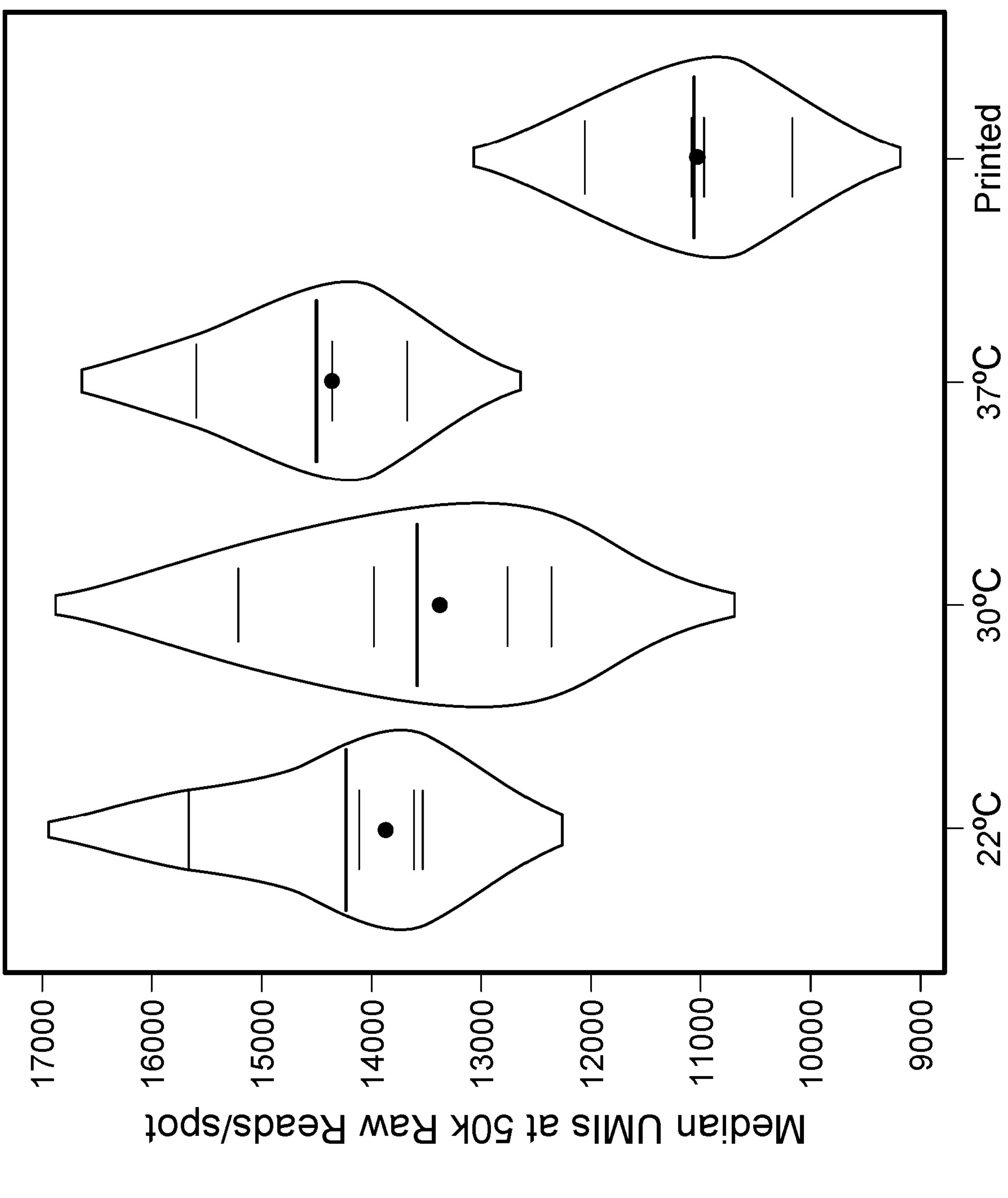
Median UMIs at 50k Raw Reads/spot
17000
16000
15000
14000
13000
12000
11000
10000
9000
22°C
30°C
37°C
Printed

FIG. 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62935043
- US 62941581
- US 63027558
- WO 2011127099 A
- WO 2014210233 A
- WO 2014210225 A
- WO 2016162309 A
- WO 2018091676 A
- WO 2012140224 A
- WO 2014060483 A
- US 10002316 B
- US 9727810 B
- US 20170016053
- WO 2018045186 A
- WO 2016007839 A
- WO 2018045181 A
- WO 2014163886 A
- US 20180245142
- WO 2017144338 A
- WO 2018107054 A
- WO 2017222453 A
- WO 2019068880 A
- WO 2011094669 A
- US 7709198 B
- US 8604182 B
- US 8951726 B
- US 9783841 B
- US 10041949 B
- WO 2016057552 A
- WO 2017147483 A
- WO 2018022809 A
- WO 2016166128 A
- WO 2017027367 A
- WO 2017027368 A
- WO 2018136856 A
- WO 2019075091 A
- US 10059990 B
- WO 2018057999 A
- WO 2015161173 A
- US 2017057269 W
- US 82574017
- US 20180156784
- WO 2012168003 A
- US 7785869 B
- US 20180312822 A
- US 62946885
- US 7282328 B
- US 20170275669 A
- US 6867028 B
- US 20140378345 A
- US 20150376609 A
- US 6265552 B
- US 20180180601
- US 20170343545 A
- US 6391937 B
- US 9512422 B
- US 9889422 B
- US 20170253918 A
- US 20180052081 A
- US 20080132429
- US 20110059865
- WO 2017019456 A
- WO 2005065814 A
- US 20080280773 A
- US 6737236 B
- US 7259258 B
- US 7375234 B
- US 7427678 B
- US 5610287 A
- US 5807522 A
- US 5837860 A
- US 5472881 A
- US 20080280773
- US 20110059865 A
- US 201314111482 A
- US 9593365 B2
- US 2019203275 A
- WO 2014085725 A
- WO 2018075693 A
- US 20180105808
- US 20140378345


### Non-patent literature cited in the description


- **RODRIQUES et al.** *Science*, 2019, vol. 363 (6434), 1463-1467
- **LEE et al.** *Nat. Protoc*, 2015, vol. 10 (3), 442-458
- **TREJO et al.** *PLoS ONE*, 2019, vol. 14 (2), e0212031
- **CHEN et al.** *Science*, 2015, vol. 348 (6233), aaa6090

- **GAO et al.** *BMC Biol*, 2017, vol. 15, 50
- **GUPTA et al.** *Nature Biotechnol.*, 2018, vol. 36, 1197-1202
- **BOLOGNESI et al.** *J. Histochem. Cytochem*, 2017, vol. 65 (8), 431-444
- **LIN et al.** *Nat Commun*, 2015, vol. 6, 8390
- **PIRICI et al.** *J. Histochem. Cytochem*, 2009, vol. 57, 567-75
- **GLASS et al.** *J. Histochem. Cytochem*, 2009, vol. 57, 899-905
- **JAMUR et al.** *Method Mol. Biol*, 2010, vol. 588, 63-66
- **LU et al.** *Lab Chip*, January 2005, vol. 5 (1), 23-9
- **NIKLAS et al.** *Anal Biochem.*, 15 September 2011, vol. 416 (2), 218-27
- **COX** ; **EMILI**. *Nat Protoc*, 2006, vol. 1 (4), 1872-8
- **CHIANG et al.** *J Biochem. Biophys. Methods*, 20 November 2000, vol. 46 (1-2), 53-68
- **YAMAUCHI** ; **HERR et al.** *Microsyst. Nanoeng*, 2017, vol. 3, 16079
- **HAN et al.** *Microsyst Nanoeng*, 17 June 2019, vol. 5, 30
- **GOULD et al.** *Oncotarget*, 20 March 2018, vol. 9 (21), 15606-15615
- **OREN** ; **SHAI**. *Biochemistry*, 18 February 1997, vol. 36 (7), 1826-35
- **ALGAYER et al.** *Molecules*, 31 May 2019, vol. 24 (11), E2079
- **HIPP et al.** *Leukemia*, October 2017, vol. 31 (10), 2278
- **GILL** ; **GHAEMI**. Nucleic acid isothermal amplification technologies: a review. *Nucleosides, Nucleotides, & Nucleic Acids*, 2008, vol. 27 (3), 224-43
- **VINCENT**. Helicase-dependent isothermal DNA amplification. *EMBO Rep.*, 2004, 795-800
- **ANDRESEN**. Helicase-dependent amplification: use in OnChip amplification and potential for point-of-care diagnostics. *Expert Rev Mol Diagn.*, 2009, vol. 9, 645-650
- **PIEPENBURG et al.** DNA Detection Using Recombinant Proteins. *PLoS Biol.*, 2006, vol. 4 (7), e204
- **LI**. Review: a comprehensive summary of a decade development of the recombinase polymerase amplification. *Analyst*, 2019, vol. 144, 31-67
- **GROKHOVSKY, S.L.** Specificity of DNA cleavage by ultrasound. *Molecular Biology*, 2006, vol. 40 (2), 276-283
- **LERICHE et al.** *Bioorg Med Chem*, 15 January 2012, vol. 20 (2), 571-82
- **DODDRIDGE et al.** *Chem. Comm.*, 1998, vol. 18, 1997-1998
- **COOK et al.** *Chemistry and Biology*, 1999, vol. 6, 451-459
- **MACOSKO et al.** *Cell*, 2015, vol. 161, 1202-1214
- **FANG et al.** *Nucleic Acids Res.*, 2003, vol. 31 (2), 708-715
- **CARTER et al.** *Applied Optics*, 2007, vol. 46, 421-427
- **WILLNER**. *Acc. Chem. Res*, 2017, vol. 50, 657-658
- **ECHEVERRIA et al.** *Gels*, 2018, vol. 4, 54
- **RUBINA, A.Y. et al.** *Biotechniques*, May 2003, vol. 34 (5)
- **VASILISKOV et al.** *Biotechniques*, September 1999, vol. 27 (3), 592-600
- **CHOI et al.** *Biotechniques*, January 2019, vol. 66 (1), 40-53
- **SHALON et al.** *Genome Research*, 1996, 639-645
- **ROGERS et al.** *Analytical Biochemistry*, 1999, vol. 266, 23-30
- **STIMPSON et al.** *Proc. Natl. Acad. Sci. USA*, 1995, vol. 92, 6379-6383
- **BEATTIE et al.** *Clin. Chem.*, 1995, vol. 45, 700-706
- **LAMTURE et al.** *Nucleic Acids Research*, 1994, vol. 22, 2121-2125
- **BEIER et al.** *Nucleic Acids Research*, 1999, vol. 27, 1970-1977
- **JOOS et al.** *Analytical Biochemistry*, 1997, vol. 247, 96-101
- **NIKIFOROV et al.** *Analytical Biochemistry*, 1995, vol. 227, 201-209
- **TIMOFEEV et al.** *Nucleic Acids Research*, 1996, vol. 24, 3142-3148
- **CHRISEY et al.** *Nucleic Acids Research*, 1996, vol. 24, 3031-3039
- **GUO et al.** *Nucleic Acids Research*, 1994, vol. 22, 5456-5465
- **RUNNING** ; **URDEA**. *BioTechniques*, 1990, vol. 8, 276-279
- **FAHY et al.** *Nucleic Acids Research*, 1993, vol. 21, 1819-1826
- **ROGERS et al.** *Gene Therapy*, 1997, vol. 4, 1387-1392
- **MILLER et al.** Basic concepts of microarrays and potential applications in clinical microbiology. *Clinical Microbiology Reviews*, 2009, vol. 22 (4), 611-633
- **GANS et al.** *Adv Mater.*, 2004, vol. 16 (3), 203-213
- **HOYER et al.** *Anal. Chem.*, 1996, vol. 68 (21), 3840-3844
- **LEE et al.** *Beilstein J. Nanotechnol.*, 2017, vol. 8, 1049-1055
- **YE et al.** *Scientific Reports*, 2016, vol. 6, 23145
- **MOSHREFZADEH et al.** *Appl. Phys. Lett.*, 1993, vol. 62, 16

- **SHARMA**. *Resonance*, 2018, vol. 23 (3), 263-275
- **NGUYEN et al.** *Materials Today*, 2017, vol. 20 (6), 314-322
- **SMEJKAL P et al.** Microfluidic isotachophoresis: A review. *Electrophoresis*, 2013, vol. 34 (11), 1493-1509
- **HERNANDEZ et al.** *New J. Chem.*, 2017, vol. 41, 462-469
- **STEVENS et al.** *Rapid Commun. Mass Specrtom.*, 2005, vol. 15, 2157-2162
- **KASHYAP et al.** *Sci Rep*, 2016, vol. 6, 29579